# EUROPEAN PATENT SPECIFICATION

(11) **EP 2 430 013 B1**
(45) Date of publication and mention of the grant of the patent: **15.10.2014**
(21) Application number: 10719514.1
(22) Date of filing: 12.05.2010
(51) Int. Cl.: C07D 401/14, C07D 403/04, C07D 403/14, C07D 471/04, C07D 487/04, C07D 519/00, A61K 31/506, A61K 31/437, A61K 31/52, A61K 31/53, A61P 35/00

(54) **HETEROARYL COMPOUNDS AS PIKK INHIBITORS**
HETERARYL VERBINDUNGEN ALS PIKK HEMMER
COMPOSÉS HETERARYLES COMME INHIBITEURS DE LA PIKK

(30) Priority: 13.05.2009 US 177825 P
(43) Date of publication of application: 21.03.2012
(73) Proprietor: Amgen Inc., Thousand Oaks, California 91320-1799 (US)
(72) Inventor: BODE, Christiane, Somerville, MA 02145 (US); BOEZIO, Alessandro, Somerville, MA 02145 (US); CHENG, Alan, C., San Francisco, CA 94131 (US); CHOQUETTE, Deborah, Medford, MA 02155 (US); COATS, James, R., Somerville MA 02143 (US); COPELAND, Katrina, W., Sudbury MA 01776 (US); HUANG, Hongbing, Lexington MA 02421 (US); LA, Daniel, Brookline MA 02446 (US); LEWIS, Richard, Framingham MA 01701 (US); LIAO, Hongyu, Thousand Oaks CA 91362 (US); POTASHMAN, Michele, Cambridge MA 02139 (US); STELLWAGEN, John, Beverly MA 01915 (US); YI, Shuyan, Cambridge MA 02139-1330 (US); NORMAN, Mark, Thousand Oaks CA 91360 (US); STEC, Markian, Moorpark CA 93021 (US); PETERSON, Emily, A., Cambridge MA 02140 (US); GRACEFFA, Russell, Hampton NH 03842 (US)
(74) Representative: HOFFMANN EITLE
(86) International application number: PCT/US2010/034596
(87) International publication number: WO 2010/132598

(56) References cited:
- WO-A1-2007/027842
- WO-A1-2007/129052
- WO-A1-2008/042639
- WO-A2-2008/138889
- WO-A2-2009/050183
- US-A1- 2007 093 490

## Description

### Field of the Invention

The present invention provides compounds that are PIKK (Phosphoinositide-3-kinase related kinase) inhibitors, more specifically, mTOR and/or PI3Kα kinase inhibitors and are therefore useful for the treatment of diseases treatable by inhibition of kinases, specifically PI3 kinases, more specifically, mTOR and/or PI3Kα, such as cancer. Also provided are pharmaceutical compositions containing such compounds and processes for preparing such compounds.

### Background

PI3 kinases are a family of lipid kinases that have been found to play a key role in the regulation of many cellular processes including proliferation, survival, carbohydrate metabolism, and motility. PI3Ks are considered to have an important role in intracellular signal transduction. In particular, the PI3Ks generate and convey signals that have important roles in cancer. PI3Ks are ubiquitously expressed, are activated by a high proportion of cell surface receptors, especially those linked to tyrosine kinases, and influence a variety of cellular functions and events. Although some PI3K activity is likely to be essential for cellular health, PI3Ks are a diverse group of enzymes for which there is increasing evidence of functional specialization. This opens up the possibility of developing isoform-selective inhibitors that can be used to treat cancer.

The primary enzymatic activity of PI3K is the phosphorylation of inositol lipids (phosphoinositides) on the 3-position of the inositol headgroup. PI3 kinases catalyze the addition of phosphate to the 3'-OH position of the inositol ring of inositol lipids generating phosphatidyl inositol monophosphate, phosphatidyl inositol diphosphate and phosphatidyl inositol triphosphate.

There are a total of eight mammalian PI3Ks, which have been divided into three main classes on the basis of sequence homology, in vitro substrate preference, and method of activation and regulation. Enzymes of a first class (Class I) have a broad substrate specificity and phosphorylate phosphatidylinositiol (Ptdlns), PtdIns(4)P and PtdIns(4,5)P₂. Class I PI3 kinases include mammalian p110α, p110β, p110δ and p110γ. Different members of the PI3-kinase family generate different lipid products. To date, four 3-phosphorylated inositol lipids have been identified in vivo. These lipids are bound by proteins that contain the appropriate lipid recognition module and which either act as effectors or transmit the PI3K signal onwards. The most familiar form of PI3K is a heterodimeric complex, consisting of a 110 kDa catalytic subunit now known as p110α and an 85 kDa regulatory/adapter subunit, p85α.

Phosphatidylinositol 3-kinase-alpha (PI3Kα), a dual specificity lipid and protein kinase, is composed of an 85 kDa regulatory subunit and a 110 kDa catalytic subunit. The protein includes a catalytic subunit, which uses ATP to phosphorylate Ptdlns, PtdIns(4)P and PtdIns(4,5)P₂. PTEN, a tumor suppressor, can dephosphorylate phosphatidylinositol (3,4,5)-trisphosphate (PIP3), the major product of PI3 kinase Class 1. PIP3, in turn, is required for translocation of protein kinase B (AKT1, PKB) to the cell membrane, where it is phosphorylated and activated by upstream kinases. The effect of PTEN on cell death is mediated through the PI3Kα/AKT1 pathway.

PI3Kα has been implicated in the control of cytoskeletal reorganization, apoptosis, vesicular trafficking and proliferation and differentiation processes. Increased copy number and expression of the p110α gene (PIK3CA) is associated with a number of cancers such as ovarian cancer, cervical cancer, breast cancer, colon cancer, rectal cancer, endometrial cancer, stomach cancer, liver cancer, lung cancer, thyroid cancer, acute myelogenous leukemia (AML), chronic myelogenous leukemia (CML), and glioblastomas.

Mammalian target of rapamycin (mTOR) is a serine/threonine kinase of approximately 289 kDa in size and a member of the evolutionary conserved eukaryotic TOR kinases. The mTOR protein is a member of the PIKK family of proteins due to its C-terminal homology (catalytic domain) with PI3-kinase and the other family members, e.g. DNA dependent protein kinase (DNA-PKcs), Ataxia-telangiectasia mutated (ATM).

It has been demonstrated that mTOR kinase is a central regulator of cell growth and survival by mediating multiple important cellular functions including translation, cell cycle regulation, cytoskeleton reorganization, apoptosis and autophagy. mTOR resides in two biochemically and functionally distinct complexes that are conserved from yeast to human. The rapamycin sensitive mTOR-Raptor complex (mTORC1) regulates translation by activation of p70S6 kinase and inhibition of eIF4E binding protein 4EBP1 through phosphorylation, which is the best-described physiological function of mTOR signaling. mTORC1 activity is regulated by extracellular signals (growth factors and hormones) through the PI3K/AKT pathway, and by nutrient availability, intracellular energy status and oxygen through the regulators like LKB 1 and AMPK. Rapamycin and its analogues inhibit mTORC1 activity by disrupting the interaction between mTOR and raptor. The rapamycin-insensitive complex, mTORC2, was discovered only recently. Unlike mTORC1 which contains raptor, the mTORC2 complex contains other proteins including Rictor and mSin1. mTORC2 phosphorylates AKT at the hydrophobic Ser473 site, and appears to be essential for AKT activity. Other substrates of mTORC2 include PKC*α* and SGK1. How mTORC2 activity is regulated is not well understood.

The mTORC1 pathway can be activated by elevated PI3K/AKT signaling or mutations in the tumor suppressor genes PTEN or TSC2, providing cells with a growth advantage by promoting protein synthesis. Cancer cells treated with the mTORC1 inhibitor rapamycin show growth inhibition and, in some cases, apoptosis. Three rapamycin analogues, CCI-779 (Wyeth), RAD001 (Novartis) and AP23573 (Ariad) are in clinical trials for the treatment of cancer. However response rates vary among cancer types from a low of less than 10% in patients with glioblastoma and breast cancer to a high of around 40% in patients with mantle cell lymphoma.

Recent studies demonstrated that rapamycin can actually induce a strong AKT phosphorylation in tumors by attenuating the feedback inhibition on receptor tyrosine kinases mediated by p70S6K, one of the downstream effectors of mTORC1. For example, in Phase I clinical trials of RAD001, an increase in pAKT (+22.2 to 63.1% of initial values) was observed after dosing. If mTORC1 inhibition-induced phospho-AKT leads to increased cancer cell survival and acquisition of additional lesions, this could counteract the effects of growth inhibition by rapamycin analogues and explain the variable response rate. Therefore, identifying and developing small molecules that target the catalytic activity of mTOR (inhibiting both mTORC1 and mTORC2) will lead to more effective therapeutics to treat cancer patients by preventing the activation of AKT that is caused by mTORC1 specific inhibitors like rapamycin and its analogues. Dysregulated mTOR activity has been shown to associate with variety of human cancers such as breast, lung, kidney, brain, ovarian, colon, cervical, endometrial, prostate, liver, thyroid, GI tract, blood and lymphoma and other diseases such as hamartoma syndromes, rheumatoid arthritis, multiple sclerosis. In view of the important role of mTOR in biological processes and disease states, catalytic inhibitors of this protein kinase are desirable.

In view of the important role of PI3Kα and mTOR in biological processes and disease states, inhibitors of these protein kinases are desirable. The present invention provides PIKK inhibitors, particularly PI3Kα and mTOR inhibitors, which are useful for treating PI3Kα and mTOR mediated diseases and conditions.

Document WO200905183 discloses phenyl- or pyridin-imidazopyridine derivatives useful for treating diseases mediated by the ALK-5 and/or ALK-4 receptor, in particular for the treatment of inflammatory or obstructive airways diseases, cancer, muscle diseases and systemic skeletal disorders such as osteoporosis. The compounds may not display more than one substitution on the imidazo part of the structure and thus differ from the present compounds.

Document US2007093490 describes aryl- or heteroaryl-imidazopyridazine derivatives inhibiting kinases, and thus suitable for treating a large number of disorders, including immunological and general inflammatory processes, oncological processes, type II diabetes,asthma, and transplants. The compounds may not display more than one substitution on the imidazo part of the structure and thus differ from the present compounds.

### SUMMARY

In one aspect, provided is a compound of Formula (1): 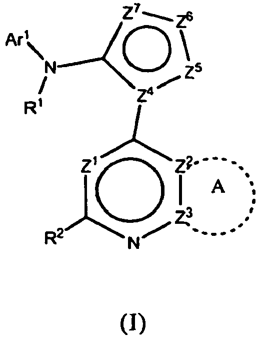 wherein:
Ar¹ is an aryl, heteroaryl, cycloalkyl or heterocyclyl ring, wherein each ring is substituted with R^{d}, R^{e}, or R^{f} where R^{d}, R^{e}, or R^{f} are independently selected from hydrogen, halo, haloalkyl, haloalkoxy, cyano, nitro, alkyl, alkenyl, alkynyl, substituted alkyl, aryl, heteroaryl, heterocycloalkyl, -C(=O)NR^{a}R^{a}, -C(=O)R^{b}, -C(=O)OR^{b}, -C(=NR^{a})NR^{a}R^{a}, -OR^{a}, -OC(=O)R^{b}, -OC(=O)NR^{a}R^{a}, -O-alkylN(R^{a})C(=O)OR^{b}, -OC(=O)N(R^{a})S(=O)₂R^{b}, -O-alky[NR^{a}R^{a}, -O-alkylOR³, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=S)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}-alkylene-NR^{a}R^{a}, or -NR^{a}-alkylene-OR^{a};
R¹ is hydrogen or alkyl;
R² is methyl or ethyl;
Z¹-N- or -CR³- where R³ isH or alkyl;
Z² is -N- or -CR⁴- where R⁴ is R^{d}, -(alkylene)heterocycloalkyl, -(alkylene)ₙO(alkylene)ₙaryl, -(alkylene)ₙN(R^{a})(alkylene)ₙaryl, -(alkylene)ₙ-N(R^{a})-(alkylene)ₙheteroaryl, or -(alkylene)ₙO(alkylene)ₙheteroaryl;
Z³ is -N- or -CR⁵- where R⁵ is R^{d}, -(alkylene)ₙaryl, -(alkylene)-heteroaryl, -(alkylene)heterocycloalkyl, -(alkylene)ₙO-(alkylene)ₙaryl, -(alkylene)ₙN(R^{a})(alkylene)ₙaryl, -(alkylene)ₙN(R^{a})(alkylene)ₙheteroaryl, or -(alkylene)ₙO(alkylene)ₙheteroaryl provided that only two of Z¹, Z² and Z³ can simultaneously be -N-;
or when Z² is -CR⁴- and Z³ is -CR⁵- then R⁴ and R⁵ together with the atoms to which they are attached can form ring A which is phenyl or a 5 or 6 membered heteroaryl ring and ring A is substituted with R^{g} or R^{h} where R^{g} or R^{h} are independently R^{d}, -(alkylene)ₙ-heterocycloalkyl, -(alkylene)ₙO(alkylene)ₙaryl, -(alkylene)ₙN(R^{a})(alkylene)ₙaryl, -(alkylene)ₙN(R^{a})(alkylene)ₙheteroaryl, or -(alkylene)ₙO(alkylene)ₙheteroaryl;
each R^{a} is independently hydrogen or R^{b}; or when two R^{a} are attached to a nitrogen atom, either alone or part of another group, the two R^{a}s together with the nitrogen atom to which they are attached can form a monocyclic heterocyclyl ring with is optionally substituted with one, two or three substitutents independently selected from oxo, halo, alkyl, alkenyl, alkynyl, cyano, nitro, alkylcarbonyl, carboxy, alkoxycarbonyl, hydroxyl, alkoxy, alkonyloxy, alkylthio, alkylsulfonyl, -alkyl-OH, aminosulfonyl, sulfonylamino, amino, alkylamino, or dialkylamino;
each R^{b} is independently alkyl, cycloalkyl, phenyl, heteroaryl, or benzyl wherein the alkyl, cycloalkyl, phenyl, or benzyl is substituted with 0, 1, 2 or 3 substituents independently selected from halo, alkyl, haloalkyl, alkoxy, amino, cyano, hydroxyl, unsubstituted heterocycloalkyl, phenyl, alkylcarbonylamino, alkylamino or dialkylamino; and
each n is independently 0 or 1; or
a pharmaceutically acceptable salt thereof;

With compound of Formula (I), in one embodiment the invention is directed to compounds of Formula (Ia) where: 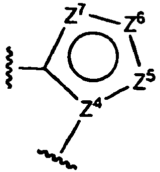 is 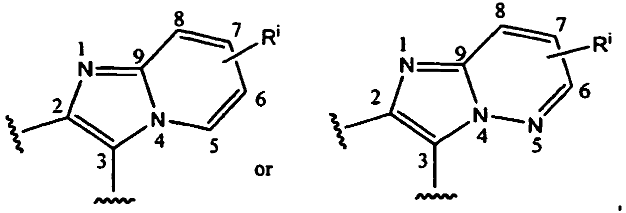
where Rⁱ is R^{d}, -(alkylene)ₙaryl, -(alkylene)heteroaryl,-(alkylene)heterocycloalkyl, -(alkylene)ₙO(alkylene)ₙaryl, - (alkylene)ₙN(R^{a})(alkylene)ₙaryl, -(alkylene₂)ₙN(R^{a})(alkylene)ₙheteroaryl, or -( alkylene)ₙO(alkylene)ₙheteroaryl;
each R^{a} is independently hydrogen or R^{b}; or when two R^{a} are attached to a nitrogen atom, either alone or part of another group, the two R^{a}s together with the nitrogen atom to which they are attached can form a monocyclic heterocyclyl ring with is optionally substituted with one, two or three substitutents independently selected from oxo, halo, alkyl, alkenyl, alkynyl, cyano, nitro, alkylcarbonyl, carboxy, alkoxycarbonyl, hydroxyl, alkoxy, alkonyloxy, alkylthio, alkylsulfonyl, aminosulfonyl, sulfonylamino, amino, alkylamino, or dialkylamino;
each R^{b} is independently alkyl, cycloalkyl, phenyl, heteroaryl, or benzyl wherein the alkyl, cycloalkyl, phenyl, or benzyl is substituted with 0, 1, 2 or 3 substituents independently selected from halo, alkyl, haloalkyl, alkoxy, amino, cyano, alkylamino or dialkylamino; and other groups are as defined in Formula (I) above.

Compounds of Formula (Ia) are a subset of compounds of Formula (I).

In a second aspect, this invention is directed to a method of treating PI3K mediated diseases and disorders in a patient in need thereof which method comprises administering to the patient a therapeutically effective amount of a compound of Formula I or a pharmaceutically acceptable salt thereof. In one embodiment, the PI3Kalpha and/or mTOR mediated disease such as cancer.

In a third aspect, this invention is directed to a pharmaceutical composition comprising a therapeutically effective amount of a compound of Formula I or a pharmaceutically acceptable salt thereof and a pharmaceutically acceptable excipient.

In a fourth aspect, the present invention is directed to the use of a compound of Formula I or a pharmaceutically acceptable salt thereof, in the manufacture of a medicament for the treatment of a PI3Kalpha and/or mTOR mediated disease such as cancer.

In a fifth aspect, the present invention is directed to compound of Formula (I) for use in therapy. Preferably the therapy is cancer.

Cancers which may be treated with compounds of the present invention include, without limitation, carcinomas such as cancer of the bladder, breast, colon, rectum, kidney, liver, lung (small cell lung cancer, and non-small-cell lung cancer), esophagus, gall-bladder, ovary, pancreas, stomach, cervix, thyroid, prostate, and skin (including squamous cell carcinoma); hematopoietic tumors of lymphoid lineage (including leukemia, acute lymphocitic leukemia, chronic lyelogenous leukemia, acute lymphoblastic leukemia, B-cell lymphoma, T-cell-lymphoma, Hodgkin's lymphoma, non-Hodgkin's lymphoma, hairy cell lymphoma and Burkett's lymphoma); hematopoietic tumors of myeloid lineage (including acute and chronic myelogenous leukemias, myelodysplastic syndrome and promyelocytic leukemia); tumors of mesenchymal origin (including fibrosarcoma and rhabdomyosarcoma, and other sarcomas, e.g., soft tissue and bone); tumors of the central and peripheral nervous system (including astrocytoma, neuroblastoma, glioma and schwannomas); and other tumors (including melanoma, seminoma, teratocarcinoma, osteosarcoma, xenoderoma pigmentosum, keratoctanthoma, thyroid follicular cancer and Kaposi's sarcoma). Other cancers that can be treated with a compound of the present invention include endometrial cancer, head and neck cancer, glioblastoma, malignant ascites, and hematopoietic cancers.

### Detailed Description

### Definitions:

Unless otherwise stated, the following terms used in the specification and claims are defined for the purposes of this Application and have the following meaning:

"Alkyl" means a linear saturated monovalent hydrocarbon radical of one to six carbon atoms or a branched saturated monovalent hydrocarbon radical of three to six carbon atoms, e.g., methyl, ethyl, propyl, 2-propyl, butyl (including all isomeric forms), pentyl (including all isomeric forms), and the like.

"Alkenyl" means a linear monovalent hydrocarbon radical of two to six carbon atoms or a branched monovalent hydrocarbon radical of three to six carbon atoms containing a double bond, e.g., ethenyl, propenyl, 2-propenyl, butenyl (including all isomeric forms), pentyl (including all isomeric forms), and the like.

"Alkynyl" means a linear monovalent hydrocarbon radical of two to six carbon atoms or a branched monovalent hydrocarbon radical of three to six carbon atoms containing a triple bond, e.g., ethynyl, propynyl, 2-propynyl, butynyl (including all isomeric forms), pentyl (including all isomeric forms), and the like.

"Alkylene" means a linear saturated divalent hydrocarbon radical of one to six carbon atoms or a branched saturated divalent hydrocarbon radical of three to six carbon atoms unless otherwise stated e.g., methylene, ethylene, propylene, 1-methylpropylene, 2-methylpropylene, butylene, pentylene, and the like.

"Amino" means a -NH₂.

"Alkylamino" means a -NHR radical where R is alkyl as defined above, e.g., methylamino, ethylamino, propylamino, or 2-propylamino, and the like.

"Alkoxy" means a -OR radical where R is alkyl as defined above, e.g., methoxy, ethoxy, propoxy, or 2-propoxy, *n-, iso-,* or *tert*-butoxy, and the like.

"Alkylthio" means a -SR radical where R is alkyl as defined above, e.g., methylthio, and the like.

"Alkylsulfonyl" means a -SO₂R radical where R is alkyl as defined above, e.g., methylsulfonyl, ethylsulfonyl, and the like.

"Alkoxycarbonyl" means a -C(O)OR radical where R is alkyl as defined above, e.g., methoxycarbonyl, ethoxycarbonyl, and the like.

"Alkylcarbonylamino" means a -NHC(O)R radical where R is alkyl as defined above, e.g., methylcarbonylamino, ethylcarbonylamino, and the like.

"Alkonyloxy" means a -OC(O) R radical where R is alkyl as defined above, e.g., acetyloxy, and the like.

"Alkoxyalkyl" means a linear monovalent hydrocarbon radical of one to six carbon atoms or a branched monovalent hydrocarbon radical of three to six carbons substituted with one or two alkoxy groups, as defined above, e.g., 2-methoxyethyl, 1-, 2-, or 3-methoxypropyl, 2-ethoxyethyl, and the like.

"Alkoxyalkyloxy" or "alkoxyalkoxy" means a -OR radical where R is alkoxyalkyl as defined above, e.g., methoxyethoxy, 2-ethoxyethoxy, and the like.

"Aminoalkyl" means a linear monovalent hydrocarbon radical of one to six carbon atoms or a branched monovalent hydrocarbon radical of three to six carbons substituted with one or two, -NRR' where R is hydrogen, alkyl, or -COR^{a} where R^{a} is alkyl, each as defined above, and R' is selected from hydrogen, alkyl, hydroxyalkyl, alkoxyalkyl, aryl, aralkyl, heteroaryl, heteroaralkyl, or haloalkyl, each as defined herein, e.g., aminomethyl, methylaminoethyl, 2-ethylamino-2-methylethyl, 1,3-diaminopropyl, dimethylaminomethyl, diethylaminoethyl, acetylaminopropyl, and the like.

"Aminoalkoxy" means a -OR radical where R is aminoalkyl as defined above, e.g., 2-aminoethoxy, 2-dimethylaminopropoxy, and the like.

"Aminosulfonyl" means a -SO₂NRR' radical where R and R' are independently hydrogen or alkyl as defined above, e.g., methylaminosulfonyl, aminosulfonyl, and the like.

"Acyl" means a -COR radical where R is alkyl, haloalkyl, cycloalkyl, cycloalkylalkyl, aryl, aralkyl, heteroaryl, heteroaralkyl, heterocyclyl, or heterocyclylalkyl, each as defined herein, e.g., acetyl, propionyl, benzoyl, pyridinylcarbonyl, and the like. When R is alkyl, the radical is also referred to herein as alkylcarbonyl.

"Aryl" means a monovalent monocyclic or bicyclic aromatic hydrocarbon radical of 6 to 10 ring atoms e.g., phenyl or naphthyl. The aryl ring can optionally be substituted with one, two or three substituents independently selected from alkyl, hydroxy, alkoxy, or halo, and/or one or two substituents independently selected from -C(=O)R^{y}, -C(=O)OR^{y}, -C(=O)NR^{x}R^{x}, -C(=NR^{x})NR^{x}R^{x}, -OR^{x}, -OC(=O)R^{x}, -OC(=O)NR^{x}R^{x}, -OC(=O)N(R^{x})S(=O)₂R^{x}, -O-alkylNR^{x}R^{x}, -O-alkylOR^{x}, -SR^{x}, -S(=O)R^{y}, -S(=O)₂R^{y}, -S(=O)₂NR^{x}R^{x}, -S(=O)₂N(R^{x})C(=O)R^{y}, -S(=O)₂N(R^{x})C(=O)OR^{y}, -S(=O)₂N(R^{x})C(=O)NR^{x}R^{x}, -NR^{x}R^{x}, -N(R^{x})C(=O)R^{y}, - (CR^{x}R^{x})ₘN(R^{x})C(=O)R^{y}, -N(R^{x})C(=O)OR^{y}, -N(R^{x})C(=O)NR^{x}R^{x}, -N(R^{x})C(=NR^{x})NR^{x}R^{x}, -N(R^{x})S(=O)₂R^{y}, -N(R^{x})S(=O)₂NR^{x}R^{x}, -alkylNR^{x}R^{x}, -NR^{x}C₂₋₆alkylNR^{x}R^{x}, -NR^{x}C₂₋₆alkylOR^{x}, -N(R^{x})(CR^{x}R^{x})ₘ-Y, -(CR^{x}R^{x})ₘ Y, or -(CR^{x}R^{x})ₘOR^{x} where m is 0-3, each R^{x} is hydrogen or R^{y} and R^{y} is independently hydrogen, alkyl, cycloalkyl, phenyl, or benzyl wherein the alkyl, cycloalkyl, phenyl, or benzyl group is substituted with one, two or three substitutents independently selected from halo, alkyl, haloalkyl, alkoxy, amino, cyano, alkylamino, or dialkylamino and Y is aryl, heteroaryl, or heterocyloalkyl substituted with 0, 1, or 2 substitutents independently selected from alkyl, halo, haloalkoxy, hydroxyl, haloalkyl -CN, nitro, acyl, carboxy, alkoxycarbonyl, hydroxyalkyl, alkoxyalkyl, hydroxyalkoxy, aminoalkyl, or aminoalkoxy. In compounds of Formula (Ia), aryl is not substituted with - (CR^{x}R^{x})ₘN(R^{x})C(=O)R^{x}, either alone or in combination with other substituents.

"Aralkyl" means a -(alkylene)-R radical where R is aryl as defined above.

"Cycloalkyl" means a cyclic saturated monovalent hydrocarbon radical of three to ten carbon atoms, e.g., cyclopropyl, cyclobutyl, cyclopentyl, or cyclohexyl, and the like.

"Cycloalkylalkyl" means a -(alkylene)-R radical where R is cycloalkyl as defined above; e.g., cyclopropylmethyl, cyclobutylmethyl, cyclopentylethyl, or cyclohexylmethyl, and the like.

"Carboxy" means -COOH.

"Dialkylamino" means a -NRR' radical where R and R' are independently alkyl as defined herein, e.g., dimethylamino, and the like.

"Halo" means fluoro, chloro, bromo, or iodo, preferably fluoro or chloro.

"Haloalkyl" means alkyl radical as defined above, which is substituted with one or more halogen atoms, preferably one to five halogen atoms, preferably fluorine or chlorine, including those substituted with different halogens, e.g., -CH₂Cl, -CF₃, -CHF₂, -CH₂CF₃, -CF₂CF₃,-CF(CH₃)₂, and the like. When the alkyl is substituted with only fluoro, it is referred to in this Application as fluoroalkyl.

"Haloalkoxy" means a -OR radical where R is haloalkyl as defined above e.g., -OCF₃,-OCHF₂, and the like. When R is haloalkyl where the alkyl is substituted with only fluoro, it is referred to in this Application as fluoroalkoxy.

"Hydroxyalkyl" means a linear monovalent hydrocarbon radical of one to six carbon atoms or a branched monovalent hydrocarbon radical of three to six carbons substituted with one or two hydroxy groups, provided that if two hydroxy groups are present they are not both

on the same carbon atom. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 1-(hydroxymethyl)-2-hydroxyethyl, 2,3-dihydroxybutyl, 3,4-dihydroxybutyl and 2-(hydroxymethyl)-3-hydroxypropyl, preferably 2-hydroxyethyl, 2,3-dihydroxypropyl, and 1-(hydroxymethyl)-2-hydroxyethyl.

"Hydroxyalkoxy" or "hydroxyalkyloxy" means a -OR radical where R is hydroxyalkyl as defined above.

"Heterocyclyl" or "heterocycloalkyl" means a saturated or unsaturated monovalent monocyclic group of 4 to 8 ring atoms in which one or two ring atoms are heteroatom selected from N, O, or S(O)ₙ, where n is an integer from 0 to 2, the remaining ring atoms being C. The heterocyclyl ring is optionally fused to a (one) aryl or heteroaryl ring as defined herein provided the aryl and heteroaryl rings are monocyclic. The heterocyclyl ring fused to monocyclic aryl or heteroaryl ring is also referred to in this Application as "bicyclic heterocyclyl" ring and is a subset of fused heterocyclyl. Additionally, one or two ring carbon atoms in the heterocyclyl ring can optionally be replaced by a -CO- group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, and the like. When the heterocyclyl ring is unsaturated it can contain one or two ring double bonds provided that the ring is not aromatic. When the heterocyclyl group contains at least one nitrogen atom, it is also referred to herein as heterocycloamino and is a subset of the heterocyclyl group. When the heterocyclyl group is a saturated ring and is not fused to aryl or heteroaryl ring as stated above, it is also referred to herein as saturated monocyclic heterocyclyl. The heterocyclyl ring can optionally be substituted with one, two or three substituents independently selected from alkyl, hydroxy, alkoxy, or halo, and/or one or two substituents independently selected from -C(=O)R^{y}, -C(=O)OR^{y}, -C(=O)NR^{x}R^{x}, -C(=NR^{x})NR^{x}R^{x}, -OR^{x}, -OC(=O)R^{x}, -OC(=O)NR^{x}R^{x}, -OC(=O)N(R^{x})S(=O)₂R^{x}, -O-alkylNR^{x}R^{x}, -O-alkylOR^{x}, -SR^{x}, -S(=O)R^{y}, -S(=O)₂R^{y}, -S(=O)₂NR^{x}R^{x}, -S(=O)₂N(R^{x})C(=O)R^{y}, -S(=O)₂N(R^{x})C(=O)OR^{y}, -S(=O)₂N(R^{x})C(=O)NR^{x}R^{x}, -NR^{x}R^{x}, -N(R^{x})C(=O)R^{y}, -N(R^{x})C(=O)OR^{y}, -N(R^{x})C(=O)NR^{x}R^{x}, -N(R^{x})C(=NR^{x})NR^{x}R^{x}, -N(R^{x})S(=O)₂R^{y}, -N(R^{x})S(=O)₂NR^{x}R^{x}, -alkylNR^{x}R^{x}, -NR^{x}C₂₋₆alkylNR^{x}R^{x}, -NR^{x}C₂₋₆alkylOR^{x}, -N(R^{x})(CR^{x}R^{x})ₘ-Y, -(CR^{x}R^{x})ₘY, or -(CR^{x}R^{x})ₘOR^{x} where m is 0-3, each R^{x} is hydrogen or R^{y} and R^{y} is independently hydrogen, alkyl, cycloalkyl, phenyl, or benzyl wherein the alkyl, cycloalkyl, phenyl, or benzyl group is substituted with one, two or three substitutents independently selected from halo, alkyl, haloalkyl, alkoxy, amino, cyano, alkylamino, or dialkylamino and Y is aryl, heteroaryl, or heterocyloalkyl substituted with 0, 1, or 2 substitutents independently selected from alkyl, halo, haloalkoxy, hydroxyl, haloalkyl -CN, nitro, acyl, carboxy, alkoxycarbonyl, hydroxyalkyl, alkoxyalkyl, hydroxyalkoxy, aminoalkyl, or aminoalkoxy.

Heterocyclylalkyl" means a -(alkylene)-R radical where R is heterocyclyl ring as defined above e.g., tetraydrofuranylmethyl, piperazinylmethyl, morpholinylethyl, and the like.

"Heteroaryl" means a monovalent monocyclic or bicyclic aromatic radical of 5 to 10 ring atoms where one or more, preferably one, two, or three, ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like. The heteroaryl ring can optionally be substituted with one, two or three substituents independently selected from alkyl, hydroxy, alkoxy, or halo, and/or one or two substituents independently selected from -C(=O)R^{y}, -C(=O)OR^{y}, -C(=O)NR^{x}R^{x}, -C(=NR^{x})NR^{x}R^{x}, -OR^{x}, -OC(=O)R^{x}, -OC(=O)NR^{x}R^{x}, -OC(=O)N(R^{x})S(=O)₂R^{x}, -O-alkylNR^{x}R^{x}, -O-alkylOR^{x}, -SR^{x}, -S(=O)R^{y}, -S(=O)₂R^{y}, -S(=O)₂NR^{x}R^{x}, -S(=O)₂N(R^{x})C(=O)R^{y}, -S(=O)₂N(R^{x})C(=O)OR^{y}, -S(=o)₂N(R^{x})C(=O)NR^{x}R^{x}, -NR^{x}R^{x}, -N(R^{x})C(=O)R^{y}, -N(R^{x})C(=O)OR^{y}, -N(R^{x})C(=O)NR^{x}R^{x}, -N(R^{x})C(=NR^{x})NR^{x}R^{x}, -N(R^{x})S(=O)_{z}R^{y}, -N(R^{x})S(=O)₂NR^{x}R^{x}, -NR^{x}C₂₋₆alkylNR^{x}R^{x}, -NR^{x}C₂₋₆alkylOR^{x}, -N(R^{x})(CR^{x}R^{x})ₙ-Y, -(CR^{x}R^{x})ₙY, or -(CR^{x}R^{x})ₙOR^{x} where n is 0-3, each R^{x} is hydrogen or R^{y} and R^{y} is independently hydrogen, alkyl, cycloalkyl, phenyl, or benzyl wherein the alkyl, cycloalkyl, phenyl, or benzyl group is substituted with one, two or three substitutents independently selected from halo, alkyl, haloalkyl, alkoxy, amino, cyano, alkylamino, or dialkylamino and Y is aryl, heteroaryl, or heterocyloalkyl substituted with 0, 1, or 2 substitutents independently selected from alkyl, halo, haloalkoxy, hydroxyl, haloalkyl - CN, nitro, acyl, carboxy, alkoxycarbonyl, hydroxyalkyl, alkoxyalkyl, hydroxyalkoxy, aminoalkyl, or aminoalkoxy.

"Heteroaralkyl" means a -(alkylene)-R radical where R is heteroaryl as defined above.

"Substituted alkyl" means a alkyl radical as defined above in which one, two, or three hydrogen atoms are independently replaced by hydroxyl, alkoxy, or halo, and/or one or two hydrogen atoms are independently replaced by -C(=O)R^{y}, -C(=O)OR^{y}, -C(=O)NR^{x}R^{x}, -C(=NR^{x})NR^{x}R^{x}, -OR^{x}, -OC(=O)R^{x}, -OC(=O)NR^{x}R^{x}, -OC(=O)N(R^{x})S(=O)₂R^{x}, -O-alkylNR^{x}R^{x}, -O-alkylOR^{x}, -SR^{x}, -S(=O)R^{y}, -S(=O)₂R^{y}, -S(=O)₂NR^{x}R^{x}, -S(=O)₂N(R^{x})C(=O)R^{y}, -S(=O)₂N(R^{x})C(=O)OR^{y}, -S(=O)₂N(R^{x})C(=O)NR^{x}R^{x}, -NR^{x}R^{x}, -N(R^{x})C(=O)R^{y}, -N(R^{x})C(=O)OR^{y}, -N(R^{x})C(=O)NR^{x}R^{x}, -N(R^{x})C(=NR^{x})NR^{x}R^{x}, -N(R^{x})S(=O)₂R^{y}, -N(R^{x})S(=O)₂NR^{x}R^{x}, -NR^{x}C₂₋₅alkylNR^{x}R^{x}, -NR^{x}C₂₋₆alkylOR^{x}, -N(R^{x})(CR^{x}R^{x})ₘ-Y, - (CR^{x}R^{x})ₘ Y, or -(CR^{x}R^{x})ₘOR^{x} where m is 0-3, each R^{x} is hydrogen or R^{y} and R^{y} is independently hydrogen, alkyl, cycloalkyl, phenyl, or benzyl wherein the alkyl, cycloalkyl, phenyl, or benzyl group is substituted with one, two or three substitutents independently selected from halo, alkyl, haloalkyl, alkoxy, amino, cyano, alkylamino, or dialkylamino and Y is aryl, heteroaryl, or heterocyloalkyl R^{x} is hydrogen or R^{y} and R^{y} is independently hydrogen, alkyl, cycloalkyl, phenyl, or benzyl wherein the alkyl, cycloalkyl, phenyl, or benzyl group is substituted with one, two or three substitutents independently selected from halo, alkyl, haloalkyl, alkoxy, amino, cyano, alkylamino, or dialkylamino and Y is aryl, heteroaryl, or heterocyloalkyl substituted with 0, 1, or 2 substitutents independently selected from alkyl, halo, haloalkoxy, hydroxyl, haloalkyl -CN, nitro, acyl, carboxy, alkoxycarbonyl, hydroxyalkyl, alkoxyalkyl, hydroxyalkoxy, aminoalkyl, or aminoalkoxy.

The term "oxo", when used as a substitutent, means the =O group, which is typically attached to a carbon atom.

"Optional" or "optionally" means that the subsequently described event or circumstance may but need not occur, and that the description includes instances where the event or circumstance occurs and instances in which it does not. For example, "heterocyclyl group optionally substituted with an alkyl group" means that the alkyl may but need not be present, and the description includes situations where the heterocyclyl group is substituted with an alkyl group and situations where the heterocyclyl group is not substituted with alkyl.

A "pharmaceutically acceptable salt" of a compound means a salt that is pharmaceutically acceptable and that possesses the desired pharmacological activity of the parent compound. Such salts include: acid addition salts, formed with inorganic acids such as hydrochloric acid, hydrobromic acid, sulfuric acid, nitric acid, phosphoric acid, and the like; or formed with organic acids such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stcaric acid, muconic acid, and the like; or salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, *N*-methylglucamine, and the like. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, which is incorporated herein by reference.

The term "pharmaceutically acceptable" means that the referenced substance, such as a compound of Formula 1 or a salt of a compound of Formula I or a formulation containing a compound of Formula I or a particular excipent, are suitable for administration to a patient.

The term "excipient" means any pharmaceutically acceptable additive, carrier, diluent, adjuvant, or other ingredient, other than the active pharmaceutical ingredient (API), which is typically included for formulation and/or administration to a patient.

The phrase in the definition of groups Ar¹ in the claims and in the specification of this Application "....each ring substituted with R^{d}, R^{e}, or R^{f} where R^{d}, R^{e}, or R^{f} are independently selected from hydrogen, halo, ......." and similar phrases used for others groups in the claims and in the specification with respect to the compound of Formula (I) means that the rings can be unsubstituted, mono-, di-, or trisubstituted unless indicated otherwise.

"Sulfonylamino" means -NHSO₂R' where R' is alkyl.

"Treating" or "treatment" of a disease includes:
preventing the disease, i.e. causing the clinical symptoms of the disease not to develop in a mammal that may be exposed to or predisposed to the disease but does not yet experience or display symptoms of the disease;
inhibiting the disease, i.e., arresting or reducing the development of the disease or its clinical symptoms; or
relieving the disease, i.e., causing regression of the disease or its clinical symptoms.

A "therapeutically effective amount" means the amount of a compound of Formula (I) that, when administered to a mammal for treating a disease, is sufficient to effect such treatment for the disease. The "therapeutically effective amount" will vary depending on the compound, the disease and its severity and the age, weight, etc., of the mammal to be treated.

The term "prodrug" means compounds that are transformed in vivo to yield a compound of the present invention. The transformation may occur by various mechanisms, such as through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

To illustrate, if the compound of the invention contains a carboxylic add functional group, a prodrug can comprise an ester formed by the replacement of the hydrogen atom of the add group with a group such as (C₁-C₈ alkyl, (C₂-C₁₂)alkanoyloxymethyl, 1-(alkanoyloxy)ethyl having from 4 to 9 carbon atoms, 1-methyl-1-(alkanoyloxy)ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)-ethyl having from 5 to 8 carbon atoms, N-(alkoxycarbonyl)aminomethyl having from 3 to 9 carbon atoms, 1-(N-(alkoxycarbonyl)aminomethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-₃)alkyl.

Similarly, if a compound of the present invention comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl, (C₁-C₆)alkoxycarbonyloxymethyl, N-(C₁-C₆)alkoxycarbonyl-aminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, -P(O)(OH)₂, -P(O)(O(C₁-C₆)alkyl)₂ or glycosyl (the radical resulting from the removal of a hydroxyl group of the hemiacetal form of a carbohydrate).

The compounds of the present invention may contain asymmetric or chiral centers, and therefore, exist in different stereoisomeric forms. It is contemplated that all stereoisomeric forms of the compounds as well as mixtures thereof, including racemic mixtures, form part of the present invention. In addition, the present invention contemplates all geometric and positional isomers. For example, if the compound contains a double bond, both the cis and trans forms (designated as Z and E, respectively), as well as mixtures, are contemplated.

Mixture of stereoisomers, such as diastereomeric mixtures, can be separated into their individual stereochemical components on the basis of their physical chemical differences by known methods such as chromatography and/or fractional crystallization. Enantiomers can can also be separated by converting the enantiomeric mixture into a diasteromeric mixture by reaction with an appropriate optically active compound (e.g., an alcohol), separating the diastereomers and converting (e.g., hydrolyzing) the individual diastereomers to the corresponding pure enantiomers. Also, some compounds may be atropisomers (e.g., substituted biaryls).

The compounds of the present invention may exist in unsolvated as well as solvated forms with pharmaceutically acceptable solvents such as water (hydrate), ethanol, and the like. The present invention contemplates and encompasses both the solvated and unsolvated forms.

It is also possible that compounds of the present invention may exist in different tautomeric forms. All tautomers of compounds of the present invention are contemplated. For example, all of the tautomeric forms of the imidazole moiety are included in this invention. Also, for example, all keto-enol or imine-enamine forms of the compounds are included in this invention.

Those skilled in the art will recognize that the compound names and structures contained herein may be based on a particular tautomer of a compound. While the name or structure for only a particular tautomer may be used, it is intended that all tautomers are encompassed by the present invention, unless stated otherwise.

The present invention also includes isotopically-labelled compounds, which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine and chlorine, such as ²H, ³H, ¹³C, ¹⁴C, ¹⁵N, ¹⁶O, ¹⁷O, ³¹P, ³²P, ³⁵S, ¹⁸F, and ³⁶Cl.

Compounds of the present invention that contain the aforementioned isotopes and/or other isotopes of other atoms are within the scope of this invention. Certain isotopically-labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detection. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically labelled compounds of this invention can generally be prepared by substituting a readily available isotopically labelled reagent for a non-isotopically labelled reagent.

In synthesizing compounds of the present invention, it may be desirable to use certain leaving groups. The term "leaving groups" ("LG") generally refer to groups that are displaceable by a nucleophile. Such leaving groups arc known in the art. Examples of leaving groups include, but are not limited to, halides (e.g., I, Br, F, CI), sulfonates (e.g., mesylate, tosylate), sulfides (e.g., SCH₃), N-hydroxsuccinimide, N-hydroxybenzotriazole, and the like. Examples of nucleophiles include, but are not limited to, amines, thiols, alcohols, Grignard reagents, anionic species (e.g., alkoxides, amides, carbanions) and the like.

### Embodiments:

A. In one embodiment, the compounds of Formula (Ia) are those wherein:
   R¹ is hydrogen and 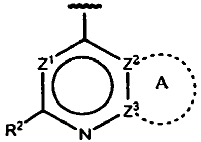 is a ring of formula 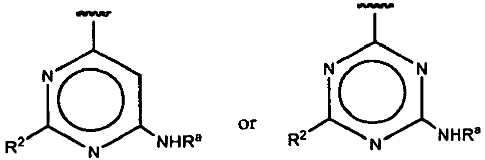 where R² is methyl and R^{a} is hydrogen, alkyl, or cyanomethyl, preferably hydrogen or methyl.
B. In another embodiment, the compounds of Formula (Ia) are those wherein: R¹ is hydrogen and 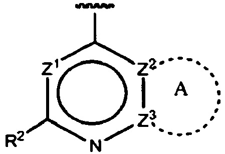 is a ring of formula: 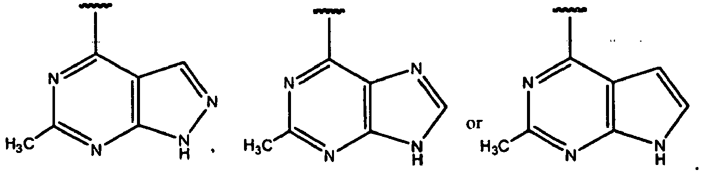
C. In another embodiment, the compounds of Formula (Ia), and in embodiments A, B, C above and groups contained therein, the compounds of Formula (Ia) are those wherein R¹ is hydrogen and Ar¹ is aryl substituted as defined in the Summary. Within this group, in one group of compounds Ar¹ is phenyl substituted at the 3-position of the pheny ring with hydroxyl or 4-position with -N(R^{a})C(=O)NR^{a}R^{a}, or -NHCOR^{b}.
D. In another embodiment the compounds of Formula (Ia) and in embodiments A, B, C above and groups contained therein, the compounds of Formula (Ia) are those wherein R¹ is hydrogen and Ar¹ is heteroaryl substituted as defined in the Summary. Within this group of compounds in one group, Ar¹ is pyrazolyl, preferably pyrazol-3-yl or pyrazol-5-yl, substituted as defined in the Summary. Within this group of compounds in another group, Ar¹ is pyridyl, benzoxazolyl, pyrimidinyl, indazolyl, indolyl, benzthiazolyl, pyrazinyl, pyridazinyl, isobenzoxazolyl, or isobenzthiazolyl, each ring substituted as defined in the Summary. Preferably, Ar¹ is pyridyl, benzoxazolyl, pyrimidinyl, indazolyl, indolyl, benzthiazolyl, pyrazinyl, pyridazinyl, isobenzoxazolyl, isobenzthiazolyl, each ring substituted with R^{d} selected from hydrogen, -N(R^{a})C(=O)NR^{a}R^{a}, -NHCOR^{b}, amino, -OR^{a} (where R^{a} is hydrogen or alkyl), haloalkyl, or halo.
F. In another embodiment, the compounds of Formula (I) are those wherein:
   Rⁱ is R^{d} selected from hydrogen, halo, haloalkyl, substituted alkyl, aryl, heteroaryl, heterocycloalkyl, -C(=O)NR^{a}R^{a}, -C(=O)OR^{b},-COR^{b}, -OR^{a}, -O-alkylNR^{a}R^{a}, -O-alkylOR^{a}, -NR^{a}R^{a}, or -NR^{a}-alkylene-OR^{a}; each R^{a} is independently hydrogen or R^{b} or when two R^{a} are attached to a nitrogen atom, either alone or part of another group, the two R^{a}s together with the nitrogen atom to which they are attached can form a monocyclic heterocyclyl ring,
   each R^{b} is independently alkyl, phenyl, or heteroaryl wherein the alkyl is substituted with 0, 1, 2 or 3 substituents independently selected from alkyl or alkoxy.

Preferably, Rⁱ is R^{d} where R^{d} is as defined above and wherein the aryl ring can optionally be substituted with one, two or three substituents independently selected from alkyl, alkoxy, or halo, and/or one or two substituents independently selected from, -C(=O)OR^{y}, -C(=O)NR^{x}R^{x}, -OR^{x}, **-**SR^{x}**,** -S(=O)₂R^{y}, -S(=O)₂NR^{x}R^{x}, -NR^{x}R^{x}, -N(R^{x})C(=O)R^{y}, -(CR^{x}R^{x})ₘN(R^{x})C(=O)R^{y} or -(CR^{x}R^{x})ₘOR^{x} where m is 1-3, each R^{x} is hydrogen or R^{y} and R^{y} is independently hydrogen, alkyl, or cycloalkyl;
the heterocycloalkyl ring is optionally be substituted with one, two or three substituents independently selected from alkyl, hydroxy, -C(=O)R^{y}, -C(=O)OR^{y}, -C(=O)NR^{x}R^{x}, -S(=O)₂R^{y}, or -(CR^{x}R^{x})ₘOR^{x} where m is 0-3, each R^{x} is hydrogen or R^{y} and R^{y} is independently hydrogen or alkyl;
the heteroaryl ring is optionally be substituted with one, two or three substituents independently selected from alkyl, -OR^{x}, -C(=O)NR^{x}R^{x}, or -(CR^{x}R^{x})ₘOR^{x} where m is 1-3, each R^{x} is hydrogen or R^{y} and R^{y} is independently hydrogen or alkyl; and
substituted alkyl is an alkyl radical in which one, two, or three hydrogen atoms arc independently replaced by hydroxyl or -NCOR^{y} where R^{y} is alkyl.

Preferably Rⁱ is hydrogen, 1-piperazinyl, 4-methyl-1-piperazinyl, 4-(1-methylethyl)-1-piperazinyl, 4-(2-methoxyethyl)-1-piperazinyl, fluoro, 3-(methylsulfonyl)phenyl, 4-pyridinyl, 2-methoxyethoxy, 4-morpholinyl, 2-(1-pyrrolidinyl)ethoxy, 1-tert-butoxycarbonyl-3,6-dihydro-1(2H)-pyridin-4-yl, 3-methoxyphenyl, 3-fluoro-4-methylaminocarbonylphenyl, 4-acetylaminomethylphenyl, 1-(2-methoxyethyl)-1H-pyrazol-4-yl, 2-fluorophenyl, 2-methylphenyl, 2-methoxy-3-pyridinyl, 3-furanyl, 3,5-dimethyl-4-isoxazolyl, 4-fluoro-2-methylphenyl, 2-(1-methylethoxy)phenyl, 3-(methylsulfanyl)phenyl, 1,2,3,6-tetrahydro-4-pyridinyl, 3-methyl-5-isoxazolyl, 1-(2-methoxyethyl)-1H-pyrazol-4-yl, 3-acetylaminophenyl, 3-cyclopropylaminophenyl, 3-isopropanoylaminophenyl, 3-pyridazinyl, 1-methyl-1,2,3,6-tetrahydro-4-pyridinyl, 4-acetylaminophenyl, 2-methyl-4-pyridinyl, 2-methyl-3-pyridinyl, 5-pyrimidinyl, 3-pyridinyl, phenylamino, 4-methoxyphenylamino, methoxy, 3-fluorophenyl, 4-methylaminocarbonylphenyl, pyridine-3-ylamino, 4-acetylaminophenyl, 3-methoxy-phenylamino, pyridine-4-ylamino, pyrimidin-5-ylamino, 4-acetylaminophenylamino, 2-methyl-4-pyridinylamino, 4-methyl-3-pyridinyl, 2,6-dimethyl-4-pyridinyl, 4-isopropylamino-carbonylphenyl, 3,4-dihydro-1(2H)-isoquinolinone-6-yl, 4-ethylaminocarbonylphenyl, 4-carboxy-2-methylphenyl, 4-methyl-5-pyrimidinyl, 2-methyl-4-methylaminocarbonylphenyl, hydroxyl, 6-methylaminocarbonylpyridin-3-yl, 2-(1-pyrrolidinyl)ethoxy, 4-N,N-dimethylaminocarbonylpiperazin-1-yl, 4-methylsulfonylpiperazin-1-yl, 4-N-methylaminocarbonylpiperazin-1-yl, 4-acetylpiperazin-1-yl, 3-hydroxymethyl-4-methoxyphenyl, pyridine-4-yl, 3,6-dihydro-2H-pyran-4-yl, 2-methyl-4-pyridinyl, 1-methyl-1H-imidazol-5-yl, 3,4-dihydroxybutyl, 1-(1-methylethyl)-1H-pyrazol-4-yl, aminocarbonyl, quinolin-7-yl, 4-methylsulfonylphenyl, 4-methylaminosulfonyl-phenyl, 3-methyl-sulfonylphenyl, 2-methoxyethylamino, 4-hydroxypiperidin-1-yl, 1-methyl-1H-pyrazol-4-yl, 2-hydroxyprop-2-yl, acetyl, or trifluoromethyl. More preferably, Rⁱ is hydrogen, 6-piperazin-1-yl, 8-fluoro, 6-(2-methoxyethoxy)-, 6-morpholin-4-yl-, 6-(3-methoxyphenyl)-, 6-(3-fluoro-4-methylaminocarbonylphenyl)-, 6-(4-methylcarbonylaminomethylphenyl)-, 6-(1-(2-methoxyethylpyrazol-4-yl)-, 6-(2-fluorophenyl)-, 6-(2-methylphenyl)-, 6-(2-methoxypyridin-3-yl)-, 6-furan-3-yl, 6-(3,5-isoxazol-4-yl)-, 6-(4-fluoro-2-methylphenyl)-, 6-(3-methylisoxazol-5-yl)-, 6-(3-acetylaminophenyl)-, 6-(3-cyclopropylcarbonylaminophenyl)-, 6-(4-isopropylcarbonylaminophenyl)-, 6-(pyridazin-3-yl)-, 6-(1-methyl-1,2,3,4-tetrahydropyridin-4-yl)-, 6-(4-methylaminocarbonylphenyl)-, 6-pyridin-3-yl-, 6-phenylamino-, 7-methoxy, 6-(pyrimidin-5-yl)-, 6-(pyridine-3-ylamino)-, 6-(4-acetylaminophenyl)-, 6-(3-methoxyphenylamino)-, 6-(pyridine-4-ylamino)-, 6-(pyrimidin-5-ylamino)-, 6-(4-acetylaminophenylamino)-, 6-(2-methylpyridin-3-ylamino)-, 6-(2-methylpyridin-4-ylamino)-, 6-(4-methylpyridin-3-yl)-, 6-(2,6-dimethylpyridin-4-yl)-, 6-(3,4-dihydro-1(2H)-isoquinolon-6-yl)-, 6-(4-ethylaminocarbonylphenyl)-, 6-(4-methylpyrimidin-5-yl)-, 6-(4-methylaminocarbonyl-2-methylphenyl)-, 6-(6-methylaminocarbonylpyridin-3-yl)-, 6-(4-dimethylaminocarbonylpiperazin-1-yl)-, 6-(3-hydroxymethyl-4-methoxyphenyl)-, 6-pyridin-4-yl, 6-(3,6-dihydropyran-4-yl)-, 6-(2-methylpyridin-4-yl)-, 6-(1-methylimidazol-5-yl)-, 6-(1-isopropylpyrazol-4-yl)-, 6-(6-methylsulfonylphenyl)-, 6-(methylaminosulfonylphenyl)-, 6-(3-methylsulfonylphenyl)-, 6-(4-methylsulfonylpiperazin-1-yl)-, 6-(2-hydroxypropan-2-yl)-, 6-trifluoromethyl, or 7-trifluoromethyl (i.e., Rⁱ is located at the 6-position of the bicyclic rings above unless indicated otherwise).
G. In another embodiment, within compounds of Formula (1) and embodiment F and groups contained therein, in one group of compounds Ar¹ is aryl, preferably phenyl, substituted as defined above. Within this embodiment, in another group of compounds Ar¹ is heteroaryl, preferably pyrazol-3-yl or pyrazol-5-yl substituted as defined above. Within this embodiment, in one group of compounds Ar¹ is heterocycloalkyl substituted as defined above.

Within this group in yet another group of compounds Ar¹ is an aryl, heteroaryl, or heterocyclyl ring, wherein each ring is substituted with R^{d}, R^{e}, or R^{f} where R^{d}, R^{e}, or R^{f} are independently selected from hydrogen, halo, haloalkyl, alkyl, alkyl substituted with -NR^{y}R^{y} (where R^{y} is alkyl) or alkoxy, -(CR^{y}R^{y})ₙY (where Y is aryl or heteroaryl), -OR^{x} (where R^{x} is hydrogen or alkyl), heteroaryl, heterocycloalkyl, -C(=O)NR^{a}R^{a}, -C(=O)R^{b}, -C(=O)OR^{b}, -OR^{a}, -O-alkylOR^{a}, -S(=O)₂R^{b}, -NR^{a}R^{a}, -N(R^{a})C(=O)R^{b}, -N(R^{a})C(=O)NR^{a}R^{a}, -N(R^{a})S(=O)₂R^{b}, or -NR^{a}-alkylene-OR^{a};
each R^{a} is independently hydrogen or R^{b}; or when two R^{a} are attached to a nitrogen atom, either alone or part of another group, the two R^{a}s together with the nitrogen atom to which they are attached can form a monocyclic heterocyclyl ring with is optionally substituted with one, two or three substitutents independently selected from alkyl, alkylcarbonyl, - alkylOH, or hydroxyl;
each R^{b} is independently alkyl, cycloalkyl, phenyl, heteroaryl, or benzyl wherein the alkyl, cycloalkyl, phenyl, or benzyl is substituted with 0, 1, 2 or 3 substituents independently selected from halo, alkyl, haloalkyl, alkoxy, amino, cyano, alkylamino dialkylamino, hydroxyl, unsubstituted heterocycloalkyl, or phenyl. Preferably, Ar¹ is 5-fluoro-6-methoxy-3-pyridinyl, 1H-pyrazol-3-yl, 1-methyl-1H-pyrazol-3-yl, 4-(3-ethylureido)phenyl, 1H-pyrrolo[2,3-b]pyridin-6-yl, 6-(trifluoromethyl)-2-pyridinyl, 2-pyridinyl, 1H-pyrrolo[3,2-b]pyridine-5-yl, 4-hydroxypyridin-2-yl, 4-(3-phenylureido)phenyl, 5-(3-ethylureido)pyridine-2-yl, 4-(3-methylureido)phenyl, 3-hydroxyphenyl, 4-acetylaminophenyl, 4-(3-(4-pyridinyl)ureidophenyl, 3-amino-pyrimidin-5-yl, 4-pyridazinyl, 1-acetyl-1H-pyrazol-3-yl, 3-(methylsulfonyl)phenyl, 3-pyridinyl, 3-methyl-5-isothiazolyl, 4-(4-methyl-1-piperazinyl)phenyl, 1H-pyrazolo[3,4-c]pyridin-3-yl, 4-fluoro-1H-indazol-3-yl)imidazo[1,2-b]pyridazin-2-yl, 1-(2-(dimethylamino)ethyl)-1H-pyrazol-3-yl, 1H-pyrazolo[3,4-b]pyridin-3-yl, 3-(4-methyl-1-piperazinyl)-phenyl, 3-acetylaminophenyl, pyridine-2-yl, 1-ethyl-1H-pyrazol-3-yl, quinolin-3-yl, isoquinolin-3-yl, 1-methylaminocarbonyl-1H-pyrazol-3-yl, 1-methylaminocarbonyl-1H-pyrazol-5-yl, 1-(2-hydroxyethylaminocarbonyl)-1H-pyrazol-3-yl, 1-(2-hydroxyethylaminocarbonyl)-1H-pyrazol-5-yl, 1-(morpholin-4-ylcarbonyl)-1H-pyrazol-3-yl, 1-(morpholin-4-ylcarbonyl)-1H-pyrazol-5-yl, 1-(phenoxycarbonyl)-1H-pyrazol-3-yl, 1-(phenylaminocarbonyl)-1H-pyrazol-3-yl, 1-(phenylaminocarbonyl)-1H-pyrazol-5-yl, 1-(methoxycarbonyl)-1H-pyrazol-3-yl, 1-(methoxycarbonyl)-1H-pyrazol-5-yl, 1-(phenoxy-carbonyl)-1H-pyrazol-4-yl, 1-(methylaminocarbonyl)-1H-pyrazol-4-yl, 1-(pyridine-2-yl)-1H-pyrazol-3-yl, 1-(methylsulfonyl)-1H-pyrazol-3-yl, 1-(benzoxazol-2-yl)-1H-pyrazol-3-yl, 1-(benzimidazol-2-yl)-1H-pyrazol-5-yl, 1-(isopropylaminocarbonyl)-1H-pyrazol-3-yl, 1-(benzylaminocarbonyl)-1H-pyrazol-3-yl, 1-(ethylaminocarbonyl)-1H-pyrazol-3-yl, 1-(2-methoxyphenyl-aminocarbonyl)-1H-pyrazol-3-yl, 1-(2-methylphenylaminocarbonyl)-1H-pyrazol-3-yl, 1-(4-methoxyphenylaminocarbonyl)-1H-pyrazol-3-yl, 1-(2-fluorophenyl-aminocarbonyl)-1H-pyrazol-3-yl, 1-(4-cyanophenylaminocarbonyl)-1H-pyrazol-3-yl, 1-(2-trifluoromethyl-phenylaminocarbonyl)-1H-pyrazol-3-yl, 1-(3-methylphenylaminocarbonyl)-1H-pyrazol-3-yl, 1-(2,5-difluorophenylaminocarbonyl)-1H-pyrazol-3-yl, 1-(3,5-difluorophenylaminocarbonyl)-1H-pyrazol-3-yl, 1-(4-methylphenylaminocarbonyl)-1H-pyrazol-3-yl, 1-(3,5-dichlorophenyl-aminocarbonyl)-1H-pyrazol-3-yl, 1-(2-chloro-5-trifluoromethylphenylaminocarbonyl)-1H-pyrazol-3-yl, 1-(2,5-dichlorophenylamino-carbonyl)-1H-pyrazol-3-yl, 1-(2,3-dichloro-phenylaminocarbonyl)-1H-pyrazol-3-yl, 1-(2-chloro-4-trifluoromethyl-phenylaminocarbonyl)-1H-pyrazol-3-yl, 1-(benzimidazol-2-yl)-1H-pyrazol-3-yl, 1-(imidazol-2-yl)-1H-pyrazol-5-yl, 1-methylsulfonyl-1H-pyrazol-3-yl, 1-aminocarbonyl-1H-pyrazol-3-yl, 1-aminocarbonyl-1H-pyrazol-5-yl, 1-azetidin-1-ylcarbonyl-1H-pyrazol-3-yl, 1-azetidin-1-ylcarbonyl-1H-pyrazol-5-yl, 1-(2-methylaminoethylamino)-carbonyl-1H-pyrazol-3-yl, 1-(2-methylaminoethylamino)carbonyl-1H-pyrazol-5-yl, 1-dimethylaminocarbonyl-1H-pyrazol-3-yl, 1-dimethylaminocarbonyl-1H-pyrazol-5-yl, 1-piperazin-1-ylcarbonyl-1H-pyrazol-3-yl, 1-(2-methoxyethylamino)carbonyl-1H-pyrazol-3-yl, 1-(2-methoxyethylamino)carbonyl-1H-pyrazol-5-yl, 1-(2-morpholin-4-ylethylamino)-carbonyl-1H-pyrazol-3-yl, 1-(2-methylpropylaminocarbonyl)-1H-pyrazol-3-yl, 1-(1-phenethylaminocarbonyl)-1H-pyrazol-3-yl, 1-(benzyl)-1H-pyrazol-3-yl, 1-(pyridine-3-ylmethyl)-1H-pyrazol-3-yl, 1-(1,4-benzoxazin-3-(4H)-one-7-yl)-1H-pyrazol-3-yl, 5-(4-hydroxyazetidin-1-yl)pyridine-3-yl, 5-(4-(propan-2-ol)azetidin-1-yl)pyridine-3-yl, quinolin-7-yl, quinolin-6-yl, isoquinolin-7-yl, 6-methoxypyridin-3-yl, 6-ethoxypyridin-3-yl, 1H-indazol-3-yl, 5-fluoro-6-methoxypyridin-3-yl, 1-(6-(2-methoxyethoxy)pyrimidin-3-yl)-1H-pyrazol-3-yl, 3-cyclopropylureidophenyl, 3-benzylcarbonylaminophenyl, 3-benzoylaminophenyl, 3-(4-chlorophenylsulfonylamino)-phenyl, 3-methylsulfonylaminophenyl, 3-(4-fluorophenyl-ureido)phenyl, 3-(benzylureido)-phenyl, 3-ethylaminocarbonylphenyl, 6-ethylamino-carbonylpyridin-3yl, 3-ethylcarbonyl-aminophenyl, 3-methylaminocarbonylphenyl, 3-ethylaminocarbonylphenyl, 3-isopropylaminocarbonylphenyl, 3-phenylaminocarbonylphenyl, 6-methylaminocarbonyl-pyridin-3yl, 6-isopropylaminocarbonylpyridin-3yl, 6-phenylamino-carbonylpyridin-3yl, 3-aminocarbonylphenyl, 4-(2-diethylaminoethylaminocarbonyl)phenyl, 4-phenylaminocarbonylphenyl, 1-(3-methylbutanoyl)-1H-pyrazol-3-yl, 1-isopropyloxy-carbonyl-1H-pyrazol-3-yl, 1-isopropyloxycarbonyl-1H-pyrazol-5-yl, 2-methoxypyridin-4-yl, 1-isopropylcarbonyl-1H-pyrazol-3-yl, 1-isopropylcarbonyl-1H-pyrazol-5-yl, 1-(3,3-dimethylbutanoyl)-1H-pyrazol-3-yl, 1-cyclohexyloxycarbonyl-1H-pyrazol-5-yl, 1-cyclohexyloxycarbonyl-1H-pyrazol-3-yl, 2-methoxypyridin-3-yl, 2-oxopyridin-3-yl, 1-(3-methylbutyl)-1H-pyrazol-3-yl, 1-(2-methoxyethyl)-1H-pyrazol-3-yl, 1H-indazol-4-yl, 6-chloro-5-methylsulfonylaminopyridin-3-yl, 6-methoxy-5-methylsulfonylaminopyridin-3-yl, 1H-indazol-6-yl, 1H-benzimidazol-6-yl, 4-(3-cyclopropylureido)phenyl, 4-piperidin-1-ylcarbonylaminophenyl, 4-pyrrolidin-1-ylcarbonylaminophenyl. 4-azetidin-1-ylcarbonylaminophenyl, 4-(1-methylpiperazin-1-ylcarbonylaminophenyl, 4-(3-(2-methoxyethyl)ureido)phenyl, 4-morpholin-4-yl-ylcarbonylaminophenyl, 4-(1-acetylpiperazin-1-ylcarbonylaminophenyl, thiazol-3-yl, 4-(2-methylmorpholin-4-yl-ylcarbonylamino)phenyl, or 4-(3-methylmorpholin-4-yl-ylcarbonylamino)phenyl. More preferably, Ar¹ is 5-fluoro-6-methoxy-3-pyridinyl, 1H-pyrazol-3-yl, 1-methyl-1H-pyrazol-3-yl, 4-(3-ethylureido)phenyl, 4-(3-phenylureido)phenyl, 4-(3-methylureido)phenyl, 3-acetylaminophenyl, 1-(phenylaminocarbonyl)-11H-pyrazol-3-yl, 1-(2-methylphenylaminocarbonyl)-1H-pyrazol-3-yl, 1-(2-fluorophenyl-aminocarbonyl)-1H-pyrazol-3-yl, 1-(4-cyanophenylaminocarbonyl)-1H-pyrazol-3-yl, 1-(2-trifluoromethylphenylaminocarbonyl)-1H-pyrazol-3-yl, 1-(2,5-difluorophenylaminocarbonyl)-1H-pyrazol-3-yl, 1-(2,5-dichlorophenylamino-carbonyl)-1H-pyrazol-3-yl, 1-(2,3-dichlorophenylaminocarbonyl)-1H-pyrazol-3-yl, 1-(imidazol-2-yl)-1H-pyrazol-5-yl, 1-aminocarbonyl-1H-pyrazol-3-yl, 1-aminocarbonyl-1H-pyrazol-5-yl, 6-methoxypyridin-3-yl, 6-chloro-5-methylsulfonylaminopyridin-3-yl, 6-methoxy-5-methylsulfonylaminopyridin-3-yl, 1H-indazol-6-yl, 4-(3-cyclopropylureido)phenyl, or 4-morpholin-4-yl-ylcarbonylaminophenyl.
H. In another embodiment, within compounds of Formula (I) and embodiments F and/or G and groups contained therein, in one group of compounds: 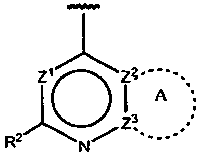
   where Z¹ is -N-, Z² is -N- or -CR⁴- where R⁴ is R^{d},-(alkylene)heterocycloalkyl, -(alkylene)ₙO(alkylene)ₙaryl, -(alkylene)ₙN(R^{a})(alkylene)ₙaryl, -( alkylene)ₙ-N(R^{a})-(alkylene)ₙheteroaryl, or -(alkylene)ₙO(alkylene)ₙheteroaryl;
   Z³ is -N- or -CR⁵- where R⁵ is R^{d}, -(alkylene)ₙaryl, -(alkylene)-heteroaryl, -(alkylene)heterocycloalkyl, -(alkylene)ₙO-(alkylene)ₙaryl, -(alkylene)ₙN(R^{a})(alkylene)ₙaryl, -(alkylene)ₙN(R^{a})(alkylene)ₙheteroaryl, or -(alkylene)ₙO(alkylene)ₙheteroaryl provided that only two of Z¹, Z² and Z³ can simultaneously be -N-;
   or when Z² is -CR⁴- and Z³ is -CR⁵- then R⁴ and R⁵ together with the atoms to which they are attached can form ring A which is phenyl or a 5 or 6 membered heteroaryl ring and ring A is substituted with R^{g} or R^{h} where R^{g} or R^{h} are independently R^{d}, -(alkylene)ₙ-heterocycloalkyl, -(alkylene)ₙO(alkylene)ₙaryl, -(alkylene)ₙN(R^{a})(alkylene)ₙaryl, -(alkylene)ₙN(R^{a})(alkylene)ₙheteroaryl, or -(alkylene)ₙO(alkylene)ₙheteroaryl. Preferably, Z¹ is -N-, Z² is -CH-, and Z³ is -CR⁵- where R⁵ is R^{d}, -(alkylene)ₙaryl, -(alkylene)-heteroaryl, -(alkylene)heterocycloalkyl, -(alkylene)ₙO-(alkylene)ₙaryl, -(alkylene)ₙN(R^{a})(alkylene)ₙaryl, -(alkylene)ₙN(R^{a})(alkylene)ₙheteroaryl, or -(alkylene)ₙO(alkylene)ₙheteroaryl.

More preferably, Z¹ is -N-, Z² is -CH-, and Z³ is -CR⁵- where R⁵ is R^{d}, or-(alkylene)ₙN(R^{a})(alkylene)ₙ₋heteroaryl; more preferably where R^{d} is -O-alkylOR^{a}, -NR^{a}R^{a}, -N(R^{a})C(=O)R^{b}, -N(R^{a})C(=O)NR^{a}R^{a}, or -N(R^{a})S(=O)₂R^{b}; each R^{a} is independently hydrogen or R^{b}; or when two R^{a} are attached to a nitrogen atom, either alone or part of another group, the two R^{a}s together with the nitrogen atom to which they are attached can form a monocyclic heterocyclyl ring with is optionally substituted with one, two or three substitutents independently selected from alkyl; and
each R^{b} is independently alkyl, cycloalkyl, phenyl, heteroaryl, or benzyl wherein the alkyl, cycloalkyl, phenyl, or benzyl is substituted with 0, 1, 2 or 3 substituents independently selected from halo, alkyl, alkoxy, amino, cyano, or dialkylamino.

Within compounds of Formula (I) and embodiments F and/or G and/or G and groups contained therein, in one group of compounds R¹ is hydrogen or alkyl; preferably hydrogen; R² is methyl.
1. In another embodiment, within compounds of Formula (I) and embodiments F and/or G and groups contained therein, in one group of compounds 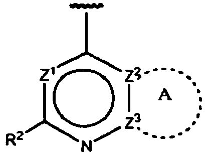 4-amino-6-methyl-1,3,5-triazin-2-yl, 2-methyl-6-(phenylamino)-4-pyrimidinyl, 6-((2-fluorophenyl)amino)-2-methyl-4-pyrimidinyl, 6-((4-fluorophenyl)amino)-2-methyl-4-pyrimidinyl, 6-((4-methoxyphenyl)amino)-2-methyl-4-pyrimidinyl, 2-methyl-6-(3-pyridazinylamino)-4-pyrimidinyl, 6-(4-methyl-1-piperazinyl)-3-pyridazinylamino-4-pyrimidinyl, 6-(5-methoxy-2-pyridinyl)amino-2-methyl-4-pyrimidinyl, 6-((3-fluorophenyl)amino)-2-methyl-4-pyrimidinyl, 6-((4-methoxy-2-pyridinyl)amino)-2-methyl-4-pyrimidinyl, 6-((6-(4-morpholinyl)-3-pyridazinyl)amino)-4-pyrimidinyl, 6-amino-2-methyl-4-pyrimidinyl, 6-((5-(2-methoxyethoxy)-3-pyridazinyl)amino)-2-methyl-4-pyrimidinyl, 2-methyl-6-((6-(4-morpholinyl)-3-pyridazinyl)amino)-4-pyrimidinyl, 6-((5-methoxy-3-pyridazinyl)amino)-2-methyl-4-pyrimidinyl, 2-methyl-6-((5-methyl-3-pyridazinyl)amino)-4-pyrimidinyl, 6-acetylamino-2-methylpyrimidin-4-yl, 2-methyl-6-((5-(2,2,2-trifluoroethoxy)-3-pyridazinyl)amino)-4-pyrimidinyl, 2-methyl-6-(methylamino)-4-pyrimidinyl, 2-methyl-6-(2-pyrazinylamino)-4-pyrimidinyl, 2-methyl-6-(1,1-dimethylureido)-4-pyrimidinyl, 2-methyl-6-(1-ethylureido)-4-pyrimidinyl, 2-methyl-6-(4-methylpiperazin-1-carbonylamino)-4-pyrimidinyl, 2-methyl-6-(4-morpholin-4-carbonylamino-)-4-pyrimidinyl, 2-methyl-6-(1H-pyrazol-3-ylamino)-4-pyrimidinyl, 6-(dimethylamino)-2-methyl-4-pyrimidinyl, 2-methyl-6-(1,3-thiazol-2-ylamino)4-pyrimidinyl, 2-methyl-6-(2-pyridinylamino)-4-pyrimidinyl, 2-methyl-6-(3-pyridazinylamino)-4-pyrimidinyl, 2-methyl-6-(4-pyrimidinylamino)-4-pyrimidinyl, 2-methyl-6-methylsulfonylamino-4-pyrimidinyl, 6-methyl-2-cyanomethylamino-4-pyrimidinyl, 2-methyl-6-dimethylaminomethyl-carbonylamino-4-pyrimidinyl, 2-methyl-6-aminomethyl-carbonylamino-4-pyrimidinyl, 2-methyl-6-cyclopropylcarbonylamino-4-pyrimidinyl, 2-methyl-6-methoxymethyl-carbonylamino-4-pyrimidinyl, 2-methyl-6-cyanomethylcarbonylamino-4-pyrimidinyl, 2-methyl-6-(3-methoxybenzyl)carbonyl-amino-4-pyrimidinyl, 2-methyl-6-(3-fluorobenzyl)carbonylamino-4-pyrimidinyl, 2-methyl-6-(2,4-difluorobenzyl)carbonyl-amino-4-pyrimidinyl, 2-methyl-6-(3-cyanophenyl)carbonylamino-4-pyrimidinyl, 2-methyl-6-(3,3,3-trifluoropropanoyl)amino-4-pyrimidinyl, 6-((6-(2-methoxyethoxy)-3-pyridazinyl)amino)-2-methyl-4-pyrimidinyl, 6-((6-(2-(2-hydroxyethyl)-morpholin-4-yl)-3-pyridazinyl)amino)-2-methyl-4-pyrimidinyl, 6-((6-(2-methylmorpholin-4-yl)-3-pyridazinyl)amino)-2-methyl-4-pyrimidinyl, 6-((6-(3-methylmorpholin-4-yl)-3-pyridazinyl)amino)-2-methyl-4-pyrimidinyl, 6-((6-(morpholin-4-yl)-3-pyridazinyl)-amino)-2-methyl-4-pyrimidinyl, 2-methyl-6-(3,3,3-trifluoropropyl)amino-4-pyrimidinyl, 2-methyl-6-(pyridine-3-yl)amino-4-pyrimidinyl, 2-methyl-6-(1,3-oxazol-5-yl)carbonylamino-4-pyrimidinyl, 2-methyl-6-(isoxazol-3-yl)amino-4-pyrimidinyl, 2-methyl-6-(1-(2-methoxy-ethyl)pyrazol-4-yl)amino-4-pyrimidinyl, 2-methyl-6-((1-((3RS)-tetrahydro-3-furanyl)-1H-pyrazol-4-yl)amino)-4-pyrimidinyl, 6-(imidazo[1,2-a]pyridin-2-ylamino)-2-methyl-4-pyrimidinyl, 6-(furo[3,2-b]pyridin-6-ylamino)-2-methyl-4-pyrimidinyl, or 6-((6-(2-methoxy-ethoxy)-4-pyrimidinyl. Preferably, 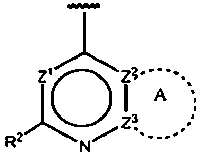 is 4-amino-6-methyl-1,3,5-triazin-2-yl, 2-methyl-6-(3-pyridazinylamino)-4-pyrimidinyl, 6-(4-methyl-1-piperazinyl)-3-pyridazinylamino-4-pyrimidinyl, 6-((5-(2-methoxyethoxy)-3-pyridazinyl)amino)-2-methyl-4-pyrimidinyl, 2-methyl-6-((6-(4-morpholinyl)-3-pyridazinyl)amino)-4-pyrimidinyl, 6-((5-methoxy-3-pyridazinyl)amino)-2-methyl-4-pyrimidinyl, 2-methyl-6-((5-methyl-3-pyridazinyl)amino)-4-pyrimidinyl, 6-acetylamino-2-methylpyrimidin-4-yl, 2-methyl-6-(methylamino)-4-pyrimidinyl, 2-methyl-6-(2-pyrazinylamino)-4-pyrimidinyl, 2-methyl-6-(3-pyridazinylamino)-4-pyrimidinyl, 2-methyl-6-(4-pyrimidinylamino)-4-pyrimidinyl, 6-methyl-2-cyanomethylamino-4-pyrimidinyl, 2-methyl-6-cyanomethylcarbonylamino-4-pyrimidinyl, 2-methyl-6-(isoxazol-3-yl)amino-4-pyrimidinyl, 2-methyl-6-(1-(2-methoxy-ethyl)pyrazol-4-yl)amino-4-pyrimidinyl, 2-methyl-6-((1-((3RS)-tetrahydro-3-furanyl)-1H-pyrazol-4-yl)amino)-4-pyrimidinyl, or 6-((6-(2-methoxy-ethoxy)-4-pyrimidinyl.

Representative compounds of Formula (I) are:

| CPD # | Name | Mass Cal'd | MS observed |
|---|---|---|---|
| 1 | 3-(4-amino-6-methyl-1,3,5-triazin-2-yl)-N-(1H-pyrazol-3-yl)imidazo[1,2-a]pyridin-2-amine | 307.3 | 308 |
| 2 | 3-(3-(2-methyl-9H-purin-4-yl)imidazo[1,2-a]pyridin-2-ylamino)phenol | 357.4 | 358.0 |
| 3 | 3-(4-methyl-6-(methylamino)-1,3,5-triazin-2-yl)-N-(1H-pyrazol-3-yl)imidazo[1,2-a]pyridin-2-amine | 321.3 | 322.2 |
| 4 | 3-(4-amino-6-methyl-1,3,5-triazin-2-yl)-N-(6-methoxypyridin-3-yl)imidazo[1,2-a]pyridin-2-amine | 348.4 | 348.8 |
| 5 | N-(3-(2-methyl-9H-purin-4-yl)imidazo[1,2-a]pyridin-2-yl)-1H-indazol-4-amine | 381.4 | 382 |
| 6 | 7-chloro-3-(4-amino-6-methyl)-1,3,5-triazin-2-yl)-N-(1H-pyrazol-3-yl)imidazo[1,2-a]pyridin-2-amine | | |
| 7 | 3-(6-amino-2-methylpyrimidin-4-yl)-N-(1H-pyrazol-3-yl)imidazo[1,2-b]pyridazin-2-amine | 341.8 | 342 |
| 8 | 3-(4-amino-6-methyl-1,3,5-triazin-2-yl)-N-(5-fluoro-6-methoxypyridin-3-yl)imidazo[1,2-a]pyridin-2-amine | 307.3 | 308.1 |
| 9 | 3-(4-amino-6-methyl-1,3,5-triazin-2-yl)-N-(6-methoxypyridin-3-yl)-6-fluoroimidazo[1,2-a]pyridin-2-amine | 296.3 | 298.2 |
| 26 | 3-(4-amino-6-methyl-1,3,5-triazin-2-yl)-N-(5-fluoro-6-methoxy-3-pyridinyl)-6-(1-piperazinyl)imidazo[1,2-a]pyridin-2-amine | 450.2 | 451.1 |
| 27 | 3-(4-amino-6-methyl-1,3,5-triazin-2-yl)-N-(5-fluoro-6-methoxy-3-pyridinyl)-6-(4-methyl-1-piperazinyl)imidazo[1,2-a]pyridin-2-amine | 464.2 | 465.2 |
| 28 | 3-(4-amino-6-methyl-1,3,5-triazin-2-yl)-N-(5-fluoro-6-methoxy-3-pyridinyl)-6-(4-(1-methylethyl)-1-piperazinyl)-imidazo[1,2-a]pyridin-2-amine | 492.2 | 493.2 |
| 29 | 3-(4-amino-6-methyl-1,3,5-triazin-2-yl)-N-(5-fluoro-6-methoxy-3-pyridinyl)-6-(4-(2-methoxyethyl)-1-piperazinyl)imidazo[1,2-a]pyridin-2-amine | 508.2 | 509.1 |
| 30 | 3-(2-methyl-6-(phenylamino)-4-pyrimidinyl)-N-1H-pyrazol-3-ylimidazo[1,2-b]pyridazin-2-amine | 383.16 | 384.0 |
| 31 | 3-(6-((2-fluorophenyl)amino)-2-methyl-4-pyrimidinyl)-N-1H-pyrazol-3-ylimidazo[1,2-b]pyridazin-2-amine | 401.15 | 402.0 |
| 32 | 3-(6-((4-fluorophenyl)amino)-2-methyl-4-pyrimidinyl)-N-1H-pyrazol-3-ylimidazo[1,2-b]pyridazin-2-amine | 401.15 | 402.0 |
| 33 | 3-(6-((4-methoxyphenyl)amino)-2-methyl-4-pyrimidinyl)-N-1H-pyrazol-3-ylimidazo[1,2-b]pyridazin-2-amine | 413.17 | 414.0 |
| 34 | N-(1-methyl-1H-pyrazol-3-yl)-3-(2-methyl-6-(3-pyridazinylamino)-4-pyrimidinyl)imidazo[1,2-b]pyridazin-2-amine | 399.17 | 400.2 |
| 35 | 3-(2-methyl-6-((6-(4-methyl-1-piperazinyl)-3-pyridazinyl)amino)-4-pyrimidinyl)-N-1H-pyrazol-3-ylimidazo[1,2-b]pyridazin-2-amine | 483.24 | 484.2 |
| 36 | 3-(6-((5-methoxy-2-pyridinyl)amino)-2-methyl-4-pyrimidinyl)-N-1H-pyrazol-3-ylimidazo[1,2-b]pyridazin-2-amine | 414.17 | 415.2 |
| 37 | 3-(6-((3-fluorophenyl)amino)-2-methyl-4-pyrimidinyl)-N-1H-pyrazol-3-ylimidazo[1,2-b]pyridazin-2-amine | 401.15 | 402.0 |
| 38 | 3-(6-((4-methoxy-2-pyridinyl)amino)-2-methyl-4-pyrimidinyl)-N-1H-pyrazol-3-ylimidazo[1,2-b]pyridazin-2-amine | 414.17 | 415.0 |
| 39 | 3-(2-methyl-6-((6-(4-morpholinyl)-3-pyridazinyl)amino)-4-pyrimidinyl)-N-1H-pyrazol-3-ylimidazo[1,2-b]pyridazin-2-amine | 470.20 | 471.3 |
| 40 | 1-(4-((3-(6-amino-2-methyl-4-pyrimidinyl)imidazo[1,2-a]pyridin-2-yl)amino)phenyl)-3-ethylurea | 402.19 | 403.2 |
| 41 | 3-(6-amino-2-methyl-4-pyrimidinyl)-N-1H-pyrrolo[2,3-b]pyridin-6-ylimidazo[1,2-a]pyridin-2-amine | 356.15 | 357.3 |
| 42 | 3-(6-amino-2-methyl-4-pyrimidinyl)-N-(6-(trifluoromethyl)-2-pyridinyl)imidazo[1,2-a]pyridin-2-amine | 385.13 | 386.1 |
| 43 | 3-(6-amino-2-methyl-4-pyrimidinyl)-N-2-pyridinylimidazo[1,2-a]pyridin-2-amine | 317.14 | 318.0 |
| 44 | 3-(6-((5-(2-methoxyethoxy)-3-pyridazinyl)amino)-2-methyl-4-pyrimidinyl)-N-1H-pyrazol-3-ylimidazo[1,2-b]pyridazin-2-amine | 459.19 | 460.0 |
| 45 | N-(3-(6-amino-2-methyl-4-pyrimidinyl)imidazo[1,2-a]pyridin-2-yl)-1H-pyrrolo[3,2-b]pyridin-5-amine | 356.15 | 357.0 |
| 46 | 2-((3-(6-amino-2-methyl-4-pyrimidinyl)imidazo[1,2-a]pyridin-2-yl)amino)-4-pyridinol | 333.13 | 334.2 |
| 47 | 1-(4-((3-(6-amino-2-methyl-4-pyrimidinyl)imidazo[1,2-a]pyridin-2-yl)amino)phenyl)-3-phenylurea | 450.19 | 451.2 |
| 48 | 1-(6-((3-(6-amino-2-methyl-4-pyrimidinyl)imidazo[1,2-a]pyridin-2-yl)amino)-3-pyridinyl)-3-ethylurea | 403.19 | 404.1 |
| 49 | 3-(2-methyl-6-((6-(4-morpholinyl)-3-pyridazinyl)amino)-4-pyrimidinyl)-N-1H-pyrazol-3-ylimidazo[1,2-a]pyridin-2-amine | 469.21 | 470.1 |
| 50 | 1-(4-((3-(6-amino-2-methyl-4-pyrimidinyl)imidazo[1,2-a]pyridin-2-yl)amino)phenyl)-3-methylurea | 388.18 | 389.4 |
| 52 | 3-((3-(6-((5-(2-methoxyethoxy)-3-pyridazinyl)amino)-2-methyl-4-pyrimidinyl)imidazo[1,2-b]pyridazin-2-yl)amino)phenol | 485.19 | 486.0 |
| 53 | N-(4-((3-(6-((5-(2-methoxyethoxy)-3-pyridazinyl)amino)-2-methyl-4-pyrimidinyl)imidazo[1,2-b]pyridazin-2-yl)amino)phenyl)acetamide | 526.22 | 527.0 |
| 54 | 3-(6-((5-methoxy-3-pyridazinyl)amino)-2-methyl-4-pyrimidinyl)-N-1H-pyrazol-3-ylimidazo[1,2-b]pyridazin-2-amine | 415.16 | 416.0 |
| 55 | 1-ethyl-3-(4-((3-(6-((5-(2-methoxyethoxy)-3-pyridazinyl)amino)-2-methyl-4-pyrimidinyl)imidazo[1,2-b]pyridazin-2-yl)amino)phenyl)urea | 555.25 | 556.0 |
| 56 | 3-(2-methyl-6-((5-methyl-3-pyridazinyl)amino)-4-pyrimidinyl)-N-1H-pyrazol-3-ylimidazo[1,2-b]pyridazin-2-amine | 399.17 | 400.0 |
| 57 | 1-(4-((3-(6-amino-2-methyl-4-pyrimidinyl)imidazo[1,2-a]pyridin-2-yl)amino)phenyl)-3-(4-pyridinyl)urea | 451.19 | 452.0 |
| 58 | 3-(6-((5-(2-methoxyethoxy)-3-pyridazinyl)amino)-2-methyl-4-pyrimidinyl)-N-1H-pyrazol-3-ylimidazo[1,2-a]pyridin-2-amine | 458.19 | 459.0 |
| 59 | N-(6-(2-((4-((ethylcarbamoyl)amino)phenyl)amino)imidazo[1,2-a]pyridin-3-yl)-2-methyl-4-pyrimidinyl)acetamide | 444.20 | 445.2 |
| 60 | 3-(2-methyl-6-((5-(2,2,2-trifluoroethoxy)-3-pyridazinyl)amino)-4-pyrimidinyl)-N-1H-pyrazol-3-ylimidazo[1,2-b]pyridazin-2-amine | 483.15 | 484.0 |
| 61 | N∼5∼-(3-(6-((5-(2-methoxyethoxy)-3-pyridazinyl)amino)-2-methyl-4-pyrimidinyl)imidazo[1,2-b]pyridazin-2-yl)-2,5-pyrimidinediamine | 486.20 | 487.0 |
| 62 | 8-fluoro-3-(6-((5-(2-methoxyethoxy)-3-pyridazinyl)amino)-2-methyl-4-pyrimidinyl)-N-1H-pyrazol-3-ylimidazo[1,2-a]pyridin-2-amine | 476.18 | 477.0 |
| 63 | N-(6-(8-fluoro-2-((1-methyl-1H-pyrazol-3-yl)amino)imidazo[1,2-a]pyridin-3-yl)-2-methyl-4-pyrimidinyl)acetamide | 380.15 | 381.0 |
| 64 | 3-(6-amino-2-methyl-4-pyrimidinyl)-N-4-pyridazinylimidazo[1,2-b]pyridazin-2-amine | 319.13 | 320.1 |
| 65 | 3-(6-amino-2-methyl-4-pyrimidinyl)-N-(1-methyl-1H-pyrazol-3-yl)-6-(3-(methylsulfonyl)phenyl)imidazo[1,2-a]pyridin-2-amine | 474.16 | 475.0 |
| 66 | 3-(2-methyl-6-(methylamino)-4-pyrimidinyl)-N-1H-pyrazol-3-ylimidazo[1,2-b]pyridazin-2-amine | 321.15 | 322 |
| 67 | N-(1-acetyl-1H-pyrazol-3-yl)-3-(6-amino-2-methyl-4-pyrimidinyl)imidazo[1,2-b]pyridazin-2-amine | 349.14 | 350 |
| 68 | 3-(6-amino-2-methyl-4-pyrimidinyl)-N-1H-pyrazol-3-yl-6-(4-pyridinyl)imidazo[1,2-b]pyridazin-2-amine | 384.16 | 385 |
| 69 | 3-(6-amino-2-methyl-4-pyrimidinyl)-6-(2-methoxyethoxy)-N-1H-pyrazol-3-ylimidazo[1,2-b]pyridazin-2-amine | 381.17 | 382.2 |
| 70 | N-(2-methyl-6-(2-(1H-pyrazol-3-ylamino)imidazo[1,2-b]pyridazin-3-yl)-4-pyrimidinyl)acetamide | 349.14 | 350 |
| 71 | 3-(6-amino-2-methyl-4-pyrimidinyl)-6-(4-morpholinyl)-N-1H-pyrazol-3-ylimidazo[1,2-b]pyridazin-2-amine | 392.18 | 393.2 |
| 72 | 3-(2-methyl-6-(methylamino)-4-pyrimidinyl)-6-(4-morpholinyl)-N-1H-pyrazol-3-ylimidazo[1,2-b]pyridazin-2-amine | 406.2 | 407.2 |
| 73 | 3-(6-amino-2-methyl-4-pyrimidinyl)-N-1H-pyrazol-3-yl-6-(2-(1-pyrrolidinyl)ethoxy)imidazo[1,2-b]pyridazin-2-amine | 420.21 | 421 |
| 74 | 3-(2-methyl-6-(2-pyrazinylamino)4-pyrimidinyl)-N-1H-pyrazol-3-ylimidazo[1,2-b]pyridazin-2-amine | 385.15 | 386.1 |
| 75 | 1,1-dimethyl-3-(2-methyl-6-(2-(1H-pyrazol-3-ylamino)imidazo[1,2-b]pyridazin-3-yl)-4-pyrimidinyl)urea | 378.17 | 379.2 |
| 76 | 1-ethyl-3-(2-methyl-6-(2-(1H-pyrazol-3-ylamino)imidazo[1,2-b]pyridazin-3-yl)-4-pyrimidinyl)urea | 378.17 | 379 |
| 77 | 4-methyl-N-(2-methyl-6-(2-1H-pyrazol-3-ylamino)imidazo[1,2-b]pyridazin-3-yl)-4-pyrimidinyl)-1-piperazinecarboxamide | 433.21 | 434 |
| 78 | N-(2-methyl-6-(2-(1H-pyrazol-3-ylamino)imidazo[1,2-b]pyridazin-3-yl)-4-pyrimidinyl)-4-morpholinecarboxamide | 420.18 | 421 |
| 79 | 3-(2-methyl-6-(1H-pyrazol-3-ylamino)-4-pyrimidinyl)-N-1H-pyrazol-3-ylimidazo[1,2-b]pyridazin-2-amine | 373.15 | 374 |
| 80 | 3-(6-(dimethylamino)-2-methyl-4-pyrimidinyl)-N-(1-methyl-1H-pyrazol-3-yl)imidazo[1,2-b]pyridazin-2-amine | 349.18 | 350 |
| 81 | 3-(2-methyl-6-(1,3-thiazol-2-ylamino)-4-pyrimidinyl)-N-1H-pyrazol-3-ylimidazo[1,2-b]pyridazin-2-amine | 390.11 | 391 |
| 82 | 3-(2-methyl-6-(2-pyridinylamino)-4-pyrimidinyl)-N-1H-pyrazol-3-ylimidazo[1,2-b]pyridazin-2-amine | 384.16 | 385 |
| 83 | 3-(2-methyl-6-(3-pyridazinylamino)-4-pyrimidinyl)-N-1H-pyrazol-3-ylimidazo[1,2-b]pyridazin-2-amine | 385.15 | 386 |
| 84 | 3-(2-methyl-6-(4-pyrimidinylamino)-4-pyrimidinyl)-N-1H-pyrazol-3-ylimidazo[1,2-b]pyridazin-2-amine | 385.15 | 386 |
| 85 | 3-(2-methyl-6-(2-pyrimidinylamino)-4-pyrimidinyl)-N-1H-pyrazol-3-ylimidazo[1,2-b]pyridazin-2-amine | 385.15 | 386 |
| 86 | N-(2-methyl-6-(2-((1-methyl-1H-pyrazol-3-yl)amino)imidazo[1,2-b]pyridazin-3-yl)-4-pyrimidinyl)acetamide | 363.16 | 364 |
| 87 | 3-(6-amino-2-methyl-4-pyrimidinyl)-7-(4-morpholinyl)-N-1H-pyrazol-3-ylimidazo[1,2-b]pyridazin-2-amine | 392.18 | 393.2 |
| 88 | 3-(2-methyl-6-(methylamino)-4-pyrimidinyl)-7-(4-morpholinyl)-N-1H-pyrazol-3-ylimidazo[1,2-b]pyridazin-2-amine | 406.2 | 407.2 |
| 89 | N-(2-methyl-6-(2-(1H-pyrazol-3-ylamino)imidazo[1,2-b]pyridazin-3-yl)-4-pyrimidinyl)methanesulfonamide | 385.11 | 386 |
| 90 | ((2-methyl-6-(2-(1H-pyrazol-3-ylamino)imidazo[1,2-b]pyridazin-3-yl)-4-pyrimidinyl)amino)acetonitrile | 346.14 | 347 |
| 91 | 3-(6-amino-2-methyl-4-pyrimidinyl)-8-fluoro-N-1H-pyrazol-3-ylimidazo[1,2-a]pyridin-2-amine | 324.12 | 325 |
| 92 | N~2~,N~2∼-dimethyl-N-(2-methyl-6-(2-((1-methyl-1H-pyrazol-3-yl)amino)imidazo[1,2-b]pyridazin-3-yl)-4-pyrimidinyl)glycinamide | 406.2 | 407 |
| 93 | N-(2-methyl-6-(2-((1-methyl-1H-pyrazol-3-yl)amino)imidazo[1,2-b]pyridazin-3-yl)-4-pyrimidinyl)glycinamide | 378.17 | 379 |
| 94 | 3-(6-amino-2-methyl-4-pyrimidinyl)-N-(3-(methylsulfonyl)phenyl)imidazo[1,2-b]pyridazin-2-amine | 395.44 | 396.0 |
| 95 | 3-(6-amino-2-methyl-4-pyrimidinyl)-N-3-pyridinylimidazo[1,2-b]pyridazin-2-amine | 318.34 | 319.2 |
| 96 | 3-(6-amino-2-methyl-4-pyrimidinyl)-N-(3-methyl-5-isothiazolyl)imidazo[1,2-b]pyridazin-2-amine | 338.39 | 339.3 |
| 97 | tert-butyl 4-(3-(4-amino-6-methyl-1,3,5-thazin-2-yl)-2-(1H-pyrazol-3-ylamino)imidazo[1,2-a]pyridin-6-yl)-3,6-dihydro-1(2H)-pyridinecarboxylate | 488.2 | 489.3 |
| 98 | 3-(4-amino-6-methyl-1,3,5-triazin-2-yl)-6-(3-methoxyphenyl)-N-1H-pyrazol-3-ylimidazo[1,2-a]pyridin-2-amine | 413.2 | 414.0 |
| 99 | 4-(3-(4-amino-6-methyl-1,3,5-triazin-2-yl)-2-(1H-pyrazol-3-ylamino)imidazo[1,2-a]pyridin-6-yl)-2-fluoro-N-methylbenzamide | 458.2 | 459.0 |
| 100 | N-(4-(3-(4-amino-6-methyl-1,3,5-triazin-2-yl)-2-(1H-pyrazol-3-ylamino)imidazo[1,2-a]pyridin-6-yl)benzyl)acetamide | 454.2 | 455 |
| 101 | 3-(4-amino-6-methyl-1,3,5-triazin-2-yl)-6-(1-(2-methoxyethyl)-1H-pyrazol-4-yl)-N-1H-pyrazol-3-ylimidazo[1,2-a]pyridin-2-amine | 431.2 | 432.0 |
| 102 | 3-(4-amino-6-methyl-1,3,5-triazin-2-yl)-6-(2-fluorophenyl)-N-1H-pyrazol-3-ylimidazo[1,2-a]pyridin-2-amine | 401.2 | 402.0 |
| 103 | 3-(4-amino-6-methyl-1,3,5-triazin-2-yl)-6-(2-methylphenyl)-N-1-pyrazol-3-ylimidazo[1,2-a]pyridin-2-amine | 397.2 | 398.0 |
| 104 | 3-(4-amino-6-methyl-1,3,5-triazin-2-yl)-6-(2-methoxy-3-pyridinyl)-N-1H-pyrazol-3-ylimidazo[1,2-a]pyridin-2-amine | 414.2 | 415.0 |
| 105 | 3-(4-amino-6-methyl-1,3,5-triazin-2-yl)-6-(3-furanyl)-N-1H-pyrazol-3-ylimidazo[1,2-a]pyridin-2-amine | 373.1 | 374.0 |
| 106 | 3-(4-amino-6-methyl-1,3,5-triazin-2-yl)-6-(3,5-dimethyl-4-isoxazolyl)-N-1H-pyrazol-3-ylimidazo[1,2-a]pyridin-2-amine | 402.2 | 403.0 |
| 107 | 3-(4-amino-6-methyl-1,3,5-triazin-2-yl)-6-(4-fluoro-2-methylphenyl)-N-1H-pyrazol-3-ylimidazo[1,2-a]pyridin-2-amine | 415.2 | 416.1 |
| 108 | 3-(4-amino-6-methyl-1,3,5-triazin-2-yl)-6-(2-(1-methylethoxy)phenyl)-N-1H-pyrazol-3-ylimidazo[1,2-a]pyridin-2-amine | 441.2 | 442.0 |
| 109 | 3-(4-amino-6-methyl-1,3,5-triazin-2-yl)-6-(3-(methylsulfanyl)phenyl)-N-1H-pyrazol-3-ylimidazo[1,2-a]pyridin-2-amine | 429.2 | 430.0 |
| 110 | N-(4-(3-(4-methyl-6-(methylamino)-1,3,5-triazin-2-yl)-2-(1H-pyrazol-3-ylamino)imidazo[1,2-a]pyridin-6-yl)benzyl)acetamide | | |
| 111 | 3-(4-amino-6-methyl-1,3,5-triazin-2-yl)-N-1H-pyrazol-3-yl-6-(1,2,3,6-tetrahydro-4-pyridinyl)imidazo[1,2-a]pyridin-2-amine | 388.2 | 389.2 |
| 112 | 3-(4-amino-6-methyl-1,3,5-triazin-2-yl)-6-(3-methyl-5-isoxazolyl)-N-1H-pyrazol-3-ylimidazo[1,2-a]pyridin-2-amine | 388.2 | 389.2 |
| 113 | 6-(1-(2-methoxyethyl)-1H-pyrazol-4-yl)-3-(4-methyl-6-(methylamino)-1,3,5-triazin-2-yl)-N-1H-pyrazol-3-ylimidazo[1,2-a]pyridin-2-amine | 445.2 | 446.2 |
| 114 | N-(3-(3-(4-amino-6-methyl-1,3,5-triazin-2-yl)-2-(1H-pyrazol-3-ylamino)imidazo[1,2-a]pyridin-6-yl)phenyl)acetamide | 440.2 | 441.2 |
| 115 | N-(3-(3-(4-amino-6-methyl-1,3,5-triazin-2-yl)-2-(1H-pyrazol-3-ylamino)imidazo[1,2-a]pyridin-6-yl)phenyl)cyclopropanecarboxamide | 466.2 | 467.2 |
| 116 | N-(4-(3-(4-amino-6-methyl-1,3,5-triazin-2-yl)-2-(1H-pyrazol-3-ylamino)imidazo[1,2-a]pyridin-6-yl)phenyl)-2-methylpropanamide | 468.2 | 469.2 |
| 117 | 3-(4-amino-6-methyl-1,3,5-triazin-2-yl)-N-1H-pyrazol-3-yl-6-(3-pyridazinyl)imidazo[1,2-a]pyridin-2-amine | 385.2 | 386.2 |
| 118 | 3-(4-amino-6-methyl-1,3,5-triazin-2-yl)-6-(1-methyl-1,2,3,6-tetrahydro-4-pyridinyl)-N-1H-pyrazol-3-ylimidazo[1,2-a]pyridin-2-amine | 402.2 | 403.2 |
| 119 | N-(4-(3-(6-amino-2-methyl-4-pyrimidinyl)-2-(1H-pyrazol-3-ylamino)imidazo[1,2-b]pyridazin-6-yl)benzyl)acetamide | | |
| 120 | N-(2-methyl-6-(2-(1H-pyrazol-3-ylamino)imidazo[1,2-b]pyridazin-3-yl)-4-pyrimidinyl)cyclopropanecarboxamide | 375.2 | 376.0 |
| 121 | N-(4-(3-(6-amino-2-methyl-4-pyrimidinyl)-2-(1H-pyrazol-3-ylamino)imidazo[1,2-b]pyridazin-6-yl)phenyl)acetamide | 440.2 | 441.3 |
| 122 | 4-(3-(6-amino-2-methyl-4-pyrimidinyl)-2-(1H-pyrazol-3-ylamino)imidazo[1,2-b]pyridazin-6-yl)-N-methylbenzamide | 440.2 | 441.2 |
| 123 | 2-methoxy-N-(2-methyl-6-(2-((1-methyl-1H-pyrazol-3-yl)amino)imidazo[1,2-b]pyridazin-3-yl)-4-pyrimidinyl)acetamide | 393.2 | 394.2 |
| 124 | 3-(6-amino-2-methyl-4-pyrimidinyl)-N-1H-pyrazol-3-ylimidazo[1,2-a]pyridin-2-amine | 306.1 | 307.2 |
| 125 | 3-(6-amino-2-methyl-4-pyrimidinyl)-N-(1-methyl-1H-pyrazol-3-yl)imidazo[1,2-a]pyridin-2-amine | 320.2 | 321.3 |
| 126 | 2-cyano-N-(2-methyl-6-(2-((1-methyl-1H-pyrazol-3-yl)amino)imidazo[1,2-a]pyridin-3-yl)-4-pyrimidinyl)acetamide | 387.2 | 388.2 |
| 127 | 2-(3-methoxyphenyl)-N-(2-methyl-6-(2-((1-methyl-1H-pyrazol-3-yl)amino)imidazo[1,2-a]pyridin-3-yl)-4-pyrimidinyl)acetamide | 468.2 | 469.2 |
| 128 | 2-(3-fluorophenyl)-N-(2-methyl-6-(2-((1-methyl-1H-pyrazol-3-yl)amino)imidazo[1,2-a]pyridin-3-yl)-4-pyrimidinyl)acetamide | 456.2 | 457.2 |
| 129 | 2-(2,4-difluorophenyl)-N-(2-methyl-6-(2-((1-methyl-1H-pyrazol-3-yl)amino)imidazo[1,2-b]pyridazin-3-yl)-4-pyrimidinyl)acetamide | 475.2 | 476.2 |
| 130 | 3-cyano-N-(2-methyl-6-(2-((1-methyl-1H-pyrazol-3-yl)amino)imidazo[1,2-b]pyridazin-3-yl)-4-pyrimidinyl)benzamide | 450.2 | 451.2 |
| 131 | 3,3,3-trifluoro-N-(1-methyl-1H-pyrazol-3-yl)-N-(3-(2-methyl-6-((3,3,3-trifluoropropanoyl)amino)-4-pyrimidinyl)imidazo[1,2-a]pyridin-2-yl)propanamide | 540.2 | 541.2 |
| 132 | 3-(6-amino-2-methyl-4-pyrimidinyl)-N-(1-methyl-1H-pyrazol-3-yl)-6-(2-methyl-4-pyridinyl)imidazo[1,2-b]pyridazin-2-amine | 412.2 | 413.2 |
| 133 | 3-(6-amino-2-methyl-4-pyrimidinyl)-N-(l-methyl-1H-pyrazol-3-yl)-6-(2-methyl-3-pyridinyl)imidazo[1,2-b]pyridazin-2-amine | 412.2 | 413.2 |
| 134 | 3,3,3-trifluoro-N-(2-methyl-6-(2-((1-methyl-1H-pyrazol-3-yl)amino)imidazo[1,2-b]pyridazin-3-yl)-4-pyrimidinyl)propanamide | 431.1 | 432.2 |
| 135 | 3,3,3-trifluoro-N-(2-methyl-6-(2-((1-methyl-1H-pyrazol-3-yl)amino)imidazo[1,2-a]pyridin-3-yl)-4-pyrimidinyl)propanamide | 430.2 | 431.2 |
| 136 | 3-(6-amino-2-methyl-4-pyrimidinyl)-N-(1-methyl-1H-pyrazol-3-yl)-6-(5-pyrimidinyl)imidazo[1,2-b]pyridazin-2-amine | 399.2 | 400.2 |
| 137 | 3-(6-((6-(2-methoxyethoxy)-3-pyridazinyl)amino)-2-methyl-4-pyrimidinyl)-N-1H-pyrazol-3-ylimidazo[1,2-b]pyridazin-2-amine | 459.2 | 460.2 |
| 138 | 2-(4-(6-((2-methyl-6-(2-(1H-pyrazol-3-ylamino)imidazo[1,2-b]pyridazin-3-yl)-4-pyrimidinyl)amino)-3-pyridazinyl)-2-morpholinyl)ethanol | 514.2 | 515.2 |
| 139 | 3-(6-amino-2-methyl-4-pyrimidinyl)-N-(4-(4-methyl-1-piperazinyl)phenyl)imidazo[1,2-b]pyridazin-2-amine | 415.2 | 416.4 |
| 140 | 3-(2-methyl-6-((6-(2-methyl-4-morpholinyl)-3-pyridazinyl)amino)-4-pyrimidinyl)-N-1H-pyrazol-3-ylimidazo[1,2-b]pyridazin-2-amine | 484.2 | 485.4 |
| 141 | 3-(2-methyl-6-((6-(3-methyl-4-morpholinyl)-3-pyridazinyl)amino)-4-pyrimidinyl)-N-1H-pyrazol-3-ylimidazo[1,2-b]pyridazin-2-amine | 484.2 | 485.2 |
| 142 | N-(3-(6-amino-2-methyl-4-pyrimidinyl)imidazo[1,2-b]pyridazin-2-yl)-1H-pyrazolo[3,4-c]pyridin-3-amine | 358.1 | 359.2 |
| 143 | 3-(6-amino-2-methyl-4-pyrimidinyl)-N-(4-fluoro-1H-indazol-3-yl)imidazo[1,2-b]pyridazin-2-amine | 375.1 | 376.2 |
| 144 | N-(3-(6-amino-2-methyl-4-pyrimidinyl)imidazo[1,2-b]pyridazin-2-yl)-1H-pyrazolo[4,3-c]pyridin-3-amine | 358.1 | 359.2 |
| 145 | N-(2-methyl-6-(2-(1H-pyrazol-3-ylamino)imidazo[1,2-a]pyridin-3-yl)-4-pyrimidinyl)acetamide | 348.1 | 349.2 |
| 146 | 3-(6-amino-2-methyl-4-pyrimidinyl)-N-(1-(2-(dimethylamino)ethyl)-1H-pyrazol-3-yl)imidazo[1,2-b]pyridazin-2-amine | 378.2 | 379.2 |
| 147 | N-(6-(2-((1-(2-(dimethylamino)ethyl)-1H-pyrazol-3-yl)amino)imidazo-[1,2-b]pyridazin-3-yl)-2-methyl-4-pyrimidinyl)acetamide | 420.2 | 421.2 |
| 148 | N-(2-methyl-6-(2-((1-methyl-1H-pyrazol-3-yl)amino)imidazo[1,2-a]pyridin-3-yl)-4-pyrimidinyl)-acetamide | 362.2 | 363.2 |
| 149 | 3-(2-methyl-6-((6-(4-morpholinyl)-3-pyridazinyl)amino)-4-pyrimidinyl)-N-(1-methyl-1H-pyrazol-3-yl)imidazo[1,2-b]pyridazin-2-amine | 484.2 | 485.2 |
| 150 | N-(3-(6-amino-2-methyl-4-pyrimidinyl)imidazo[1,2-b]pyridazin-2-yl)-1H-pyrazolo[3,4-b]pyridin-3-amine | 358.1 | 359.2 |
| 151 | 3-(6-amino-2-methyl-4-pyrimidinyl)-N-(3-(4-methyl-1-piperazinyl)-phenyl)imidazo[1,2-b]pyridazin-2-amine | 415.2 | 416.2 |
| 152 | N-(3-((3-(6-(acetylamino)-2-methyl-4-pyrimidinyl)imidazo[1,2-b]pyridazin-2-yl)amino)phenyl)acetamide | 416.2 | 417.3 |
| 153 | 3-(6-amino-2-methyl4-pyrimidinyl)-6-(1-(2-methoxyethyl)-1H-pyrazol-4-yl)-N-(1-methyl-1H-pyrazol-3-yl)imidazo[1,*2-*a]pyridin-2-amine | 444.2 | 445.2 |
| 154 | 1-(4-((3-(4-amino-6-methyl-1,3,5-triazin-2-yl)imidazo[1,2-a]pyridin-2-yl)amino)phenyl)-3-methylurea | 389.2 | 390.0 |
| 155 | 3-(4-amino-6-methyl-1,3,5-triazin-2-yl)-N-1H-pyrazol-3-yl-6-(3-pyridinyl)imidazo[1,2-a]pyridin-2-amine | 384.2 | 385.0 |
| 156 | 3-(4-amino-6-methyl-1,3,5-triazin-2-yl)-N∼6∼-phenyl-N~2∼-1H-pyrazol-3-ylimidazo[1,2-a]pyridine-2,6-diamine | 398.2 | 399.3 |
| 157 | 3-(4-amino-6-methyl-1,3,5-triazin-2-yl)-N∼6∼-(4-methoxyphenyl)-N∼2∼-1H-pyrazol-3-ylimidazo[1,2-a]pyridine-2,6-diamine | 428.2 | 429.0 |
| 158 | 3-(4-amino-6-methyl-1,3,5-triazin-2-yl)-7-methoxy-N-1H-pyrazol-3-ylimidazo[1,2-a]pyridin-2-amine | 337.1 | 338.0 |
| 159 | 3-(4-amino-6-methyl-1,3,5-triazin-2-yl)-N-1H-pyrazol-3-yl-6-(5-pyrimidinyl)imidazo[1,2-a]pyridin-2-amine | 385.2 | 386.0 |
| 160 | 3-(4-methyl-6-(methylamino)-1,3,5-triazin-2-yl)-N-1H-pyrazol-3-yl-6-(5-pyrimidinyl)imidazo[1,2-a]pyridin-2-amine | 399.2 | 400.0 |
| 161 | 3-(4-amino-6-methyl-1,3,5-triazin-2-yl)-6-(3-fluorophenyl)-N-1H-pyrazol-3-ylimidazo[1,2-a]pyridin-2-amine | 401.2 | 402.0 |
| 162 | 4-(3-(4-amino-6-methyl-1,3,5-triazin-2-yl)-2-(1H-pyrazol-3-ylamino)imidazo[1,2-a]pyridin-6-yl)-N-methylbenzamide | 440.2 | 441.0 |
| 163 | 3-(4-amino-6-methyl-1,3,5-triazin-2-yl)-N∼2∼-1H-pyrazol-3-yl-N∼6∼-3-pyridinylimidazo[1,2-a]pyridine-2,6-diamine | 399.2 | 400.0 |
| 164 | N-(4-(3-(4-amino-6-methyl-1,3,5-triazin-2-yl)-2-(1H-pyrazol-3-ylamino)imidazo[1,2-a]pyridin-6-yl)phenyl)acetamide | 440.2 | 441.0 |
| 165 | 3-(4-amino-6-methyl-1,3,5-triazin-2-yl)-N~6∼-(3-methoxyphenyl)-N∼2∼-1H-pyrazol-3-ylimidazo[1,2-a]pyridine-2,6-diamine | 428.2 | 429.0 |
| 166 | 3-(4-amino-6-methyl-1,3,5-triazin-2-yl)-N∼2∼-1H-pyrazol-3-yl-N∼6∼-4-pyridinylimidazo[1,2-a]pyridine-2,6-diamine | 399.2 | 400.0 |
| 167 | 3-(4-amino-6-methyl-1,3,5-triazin-2-yl)-N∼2∼-1H-pyrazol-3-yl-N∼6∼-5-pyrimidinylimidazo[1,2-a]pyridine-2,6-diamine | 400.2 | 401.0 |
| 168 | N-(4-((3-(4-amino-6-methyl-1,3,5-triazin-2-yl)-2-(1H-pyrazol-3-ylamino)imidazo[1,2-a]pyridin-6-yl)amino)phenyl)acetamide | 455.2 | 456.0 |
| 169 | 3-(4-amino-6-methyl-1,3,5-triazin-2-yl)-6-(2-methyl-3-pyridinyl)-N-1H-pyrazol-3-ylimidazo[1,2-a]pyridin-2-amine | 398.2 | 399.2 |
| 170 | 3-(4-amino-6-methyl-1,3,5-triazin-2-yl)-N∼6∼-(2-methyl-4-pyridinyl)-N∼2∼-1H-pyrazol-3-ylimidazo[1,2-a]pyridine-2,6-diamine | 413.2 | 414.2 |
| 171 | N-methyl-4-(3-(4-methyl-6-(methylamino)-1,3,5-triazin-2-yl)-2-(1H-pyrazol-3-ylamino)imidazo[1,2-a]pyridin-6-yl)benzamide | 454.2 | 455.2 |
| 172 | 3-(4-amino-6-methyl-1,3,5-triazin-2-yl)-6-(4-methyl-3-pyridinyl)-N-1H-pyrazol-3-ylimidazo[1,2-a]pyridin-2-amine | 398.2 | 399.2 |
| 173 | 3-(4-amino-6-methyl-1,3,5-triazin-2-yl)-6-(2,6-dimethyl-4-pyridinyl)-N-1H-pyrazol-3-ylimidazo[1,2-a]pyridin-2-amine | 412.2 | 413.2 |
| 174 | 4-(3-(4-amino-6-methyl-1,3,5-triazin-2-yl)-2-(1H-pyrazol-3-ylamino)imidazo[1,2-a]pyridin-6-yl)-N-(1-methylethyl)benzamide | 468.2 | 469.2 |
| 175 | 6-(3-(4-amino-6-methyl-1,3,5-triazin-2-yl)-2-(1H-pyrazol-3-ylamino)imidazo[1,2-a]pyridin-6-yl)-3,4-dihydro-1(2H)-isoquinolinone | 452.2 | 453.2 |
| 176 | 4-(3-(4-amino-6-methyl-1,3,5-triazin-2-yl)-2-(1H-pyrazol-3-ylamino)imidazo[1,2-a]pyridin-6-yl)-N-ethylbenzamide | 454.2 | 455.2 |
| 177 | 4-(3-(4-amino-6-methyl-1,3,5-triazin-2-yl)-2-(1H-pyrazol-3-ylamino)imidazo[1,2-a]pyridin-6-yl)-3-methylbenzoic acid | 441.2 | 442.2 |
| 178 | 3-(2-methyl-6-(methylamino)-4-pyrimidinyl)-N-1H-pyrazol-3-yl-6-(5-pyrimidinyl)imidazo[1,2-b]pyridazin-2-amine | 399.2 | 400.2 |
| 179 | 3-(4-amino-6-methyl-1,3,5-triazin-2-yl)-6-(4-methyl-5-pyrimidinyl)-N-1H-pyrazol-3-ylimidazo[1,2-a]pyridin-2-amine | 399.2 | 400.2 |
| 180 | 4-(3-(4-amino-6-methyl-1,3,5-triazin-2-yl)-2-(1H-pyrazol-3-ylamino)imidazo[1,2-a]pyridin-6-yl)-N,3-dimethylbenzamide | 454.2 | 455.2 |
| 181 | 5-(3-(4-amino-6-methyl-1,3,5-triazin-2-yl)-2-(1H-pyrazol-3-ylamino)imidazo[1,2-a]pyridin-6-yl)-N-methyl-2-pyridinecarboxamide | 441.2 | 442.2 |
| 182 | 3-(4-amino-6-methyl-1,3,5-triazin-2-yl)-2-(1H-pyrazol-3-ylamino)imidazo[1,2-a]pyridin-7-ol | 323.1 | 324.2 |
| 183 | tert-butyl 4-(3-(4-amino-6-methyl-1,3,5-triazin-2-yl)-2-(1H-pyrazol-3-ylamino)imidazo[1,2-a]pyridin-7-yl)-3,6-dihydro-1 (2H)-pyridinecarboxylate | 488.2 | 489.2 |
| 184 | 3-(4-amino-6-methyl-1,3,5-triazin-2-yl)-N-1H-pyrazol-3-yl-7-(1,2,3,6-tetrahydro-4-pyridinyl)imidazo[1,2-a]pyridin-2-amine | 388.2 | 389.2 |
| 185 | 3-(4-amino-6-methyl-1,3,5-triazin-2-yl)-7-(2-methoxyethoxy)-N-1H-pyrazol-3-ylimidazo[1,2-a]pyridin-2-amine | 381.2 | 382.2 |
| 186 | 3-(4-amino-6-methyl-1,3,5-triazin-2-yl)-N-1H-pyrazol-3-yl-6-(1H-pyrrolo[2,3-b]pyridin-5-yl)imidazo[1,2-a]pyridin-2-amine | 423.2 | 424.2 |
| 187 | 4-(3-(4-amino-6-methyl-1,3,5-triazin-2-yl)-2-(1H-pyrazol-3-ylamino)-imidazo[1,2-a]pyridin-7-yl)-N-methylbenzamide | 440.2 | 441.2 |
| 188 | 3-(4-amino-6-methyl-1,3,5-triazin-2-yl)-N-1H-pyrazol-3-yl-7-(2-(1-pyrrolidinyl)ethoxy)imidazo[1,2-a]pyridin-2-amine | 420.2 | 421.2 |
| 189 | 4-(3-(6-amino-2-methyl-4-pyrimidinyl)-2-(1H-pyrazol-3-ylamino)-imidazo[1,2-b]pyridazin-6-yl)-N,N-dimethyl-1-piperazinecarboxamide | 462.2 | 463.2 |
| 190 | 3-(6-amino-2-methyl-4-pyrimidinyl)-N-2-pyridinylimidazo[1,2-b]pyridazin-2-amine | 318.1 | 319.2 |
| 191 | 3-(6-amino-2-methyl-4-pyrimidinyl)-N-(1-ethyl-1H-pyrazol-3-yl)imidazo[1,2-b]pyridazin-2-amine | 335.2 | 336.2 |
| 192 | N-(3-(6-amino-2-methyl-4-pyrimidinyl)imidazo[1,2-b]pyridazin-2-yl)-3-quinolinamine | 368.2 | 369.2 |
| 193 | N-(3-(6-amino-2-methyl-4-pyrimidinyl)imidazo[1,2-b]pyridazin-2-yl)-3-isoquinolinamine | 368.2 | 369.2 |
| 194 | N-(3-(6-amino-2-methyl-4-pyrimidinyl)imidazo[1,2-b]pyridazin-2-yl)-2-quinolinamine | 368.2 | 369.2 |
| 195 | 3-(6-amino-2-methyl-4-pyrimidinyl)-N-(1-methyl-1H-pyrazol-3-yl)imidazo[1,2-b]pyridazin-2-amine | 321.2 | 322.2 |
| 196 | 3-((3-(4-amino-6-methyl-1,3,5-triazin-2-yl)imidazo[1,2-a]pyridin-2-yl)amino)-N-methyl-1H-pyrazole-1-carboxamide | 364.2 | 365.2 |
| 197 | 5-((3-(4-amino-6-methyl-1,3,5-triazin-2-yl)imidazo[1,2-a]pyridin-2-yl)amino)-N-methyl-1H-pyrazole-1-carboxamide | 364.2 | 365.4 |
| 198 | 3-((3-(4-amino-6-methyl-1,3,5-triazin-2-yl)imidazo[1,2-a]pyridin-2-yl)amino)-N-(2-hydroxyethyl)-1H-pyrazole-1-carboxamide | 394.2 | 395.4 |
| 199 | 5-((3-(4-amino-6-methyl-1,3,5-triazin-2-yl)imidazo[1,2-a]pyridin-2-yl)amino)-N-(2-hydroxyethyl)-1H-pyrazole-1-carboxamide | 394.2 | 395.4 |
| 200 | 3-(4-amino-6-methyl-1,3,5-triazin-2-yl)-N-(1-(4-morpholinylcarbonyl)-1H-pyrazol-3-yl)imidazo[1,2-a]pyridin-2-amine | 420.2 | 421.2 |
| 201 | 3-(4-amino-6-methyl-1,3,5-triazin-2-yl)-N-(1-(4-morpholinylcarbonyl)-1H-pyrazol-5-yl)imidazo[1,2-a]pyridin-2-amine | 420.2 | 421.2 |
| 202 | 5-((3-(4-amino-6-methyl-1,3,5-triazin-2-yl)imidazo[1,2-a]pyridin-2-yl)amino)-N-3-pyridinyl-1H-pyrazole-1-carboxamide | 427.2 | 428.1 |
| 203 | 4-((3-(4-amino-6-methyl-1,3,5-triazin-2-yl)imidazo[1,2-a]pyridin-2-yl)amino)-N-methyl-1H-pyrazole-1-carboxamide | 364.2 | 365.2 |
| 204 | 3-((3-(4-amino-6-methyl-1,3,5-triazin-2-yl)imidazo[1,2-a]pyridin-2-yl)amino)-N-phenyl-1H-pyrazole-1-carboxamide | 426.2 | 427.5 |
| 205 | 5-((3-(4-amino-6-methyl-1,3,5-triazin-2-yl)imidazo[1,2-a]pyridin-2-yl)amino)-N-phenyl-1H-pyrazole-1-carboxamide | 426.2 | 427.5 |
| 206 | 3-(4-amino-6-methyl-1,3,5-triazin-2-yl)-N-1H-pyrazol-4-ylimidazo[1,2-a]pyridin-2-amine | 307.1 | 308.2 |
| 207 | methyl 3-((3-(4-amino-6-methyl-1,3,5-triazin-2-yl)imidazo[1,2-a]pyridin-2-yl)amino)-1H-pyrazole-1-carboxylate | 365.1 | 366.3 |
| 208 | methyl 5-((3-(4-amino-6-methyl-1,3,5-triazin-2-yl)imidazo[1,2-a]pyridin-2-yl)amino)-1H-pyrazole-1-carboxylate | 365.1 | 366.3 |
| 209 | phenyl 4-((3-(4-amino-6-methyl-1,3,5-triazin-2-yl)imidazo[1,2-a]pyridin-2-yl)amino)-1H-pyrazole-1-carboxylate | 427.2 | 428.2 |
| 210 | 3-((3-(6-amino-2-methyl-4-pyrimidinyl)imidazo[1,2-a]pyridin-2-yl)amino)-N-phenyl-1H-pyrazole-1-carboxamide | 425.2 | 426.3 |
| 211 | 5-((3-(6-amino-2-methyl-4-pyrimidinyl)imidazo[1,2-a]pyridin-2-yl)amino)-N-phenyl-1H-pyrazole-1-carboxamide | 425.2 | 426.3 |
| 212 | 3-((3-(6-amino-2-methyl-4-pyrimidinyl)imidazo[1,2-a]pyridin-2-yl)amino)-N-methyl-1H-pyrazole-1-carboxamide | 363.2 | 364.5 |
| 213 | 5-((3-(6-amino-2-methyl-4-pyrimidinyl)imidazo[1,2-a]pyridin-2-yl)amino)-N-methyl-1H-pyrazole-1-carboxamide | 363.2 | 364.5 |
| 214 | 4-((3-(6-amino-2-methyl-4-pyrimidinyl)imidazo[1,2-a]pyridin-2-yl)amino)-N-phenyl-1H-pyrazole-1-carboxamide | 425.2 | 426.3 |
| 215 | 4-((3-(6-amino-2-methyl-4-pyrimidinyl)imidazo[1,2-a]pyridin-2-yl)amino)-N-methyl-1H-pyrazole-1-carboxamide | 363.2 | 364.5 |
| 216 | 3-(6-amino-2-methyl-4-pyrimidinyl)-N-(1-(2-pyridinyl)-1H-pyrazol-3-yl)imidazo[1,2-a]pyridin-2-amine | 383.2 | 384.2 |
| 217 | 3-(6-amino-2-methyl-4-pyrimidinyl)-N-(1-(methylsulfonyl)-1H-pyrazol-3-yl)imidazo[1,2-a]pyridin-2-amine | 384.1 | 385.2 |
| 218 | 3-(6-amino-2-methyl-4-pyrimidinyl)-N-(1-(1,3-benzoxazol-2-yl)-1H-pyrazol-5-yl)imidazo[1,2-a]pyridin-2-amine | 423.2 | 424.5 |
| 219 | phenyl 3-((3-(6-amino-2-methyl-4-pyrimidinyl)imidazo[1,2-a]pyridin-2-yl)amino)-1H-pyrazole-1-carboxylate | 426.2 | 427.2 |
| 220 | 3-(6-amino-2-methyl-4-pyrimidinyl)-N-(1-(1H-benzimidazol-2-yl)-1H-pyrazol-3-yl)imidazo[1,2-a]pyridin-2-amine | 422.2 | 423.2 |
| 221 | 3-((3-(6-amino-2-methyl-4-pyrimidinyl)imidazo[1,2-a]pyridin-2-yl)amino)-N-(1-methylethyl)-1H-pyrazole-1-carboxamide | 391.2 | 392.4 |
| 222 | 3-((3-(6-amino-2-methyl-4-pyrimidinyl)imidazo[1,2-a]pyridin-2-yl)amino)-N-benzyl-1H-pyrazole-1-carboxamide | 439.2 | 440.2 |
| 223 | methyl 4-(((3-((3-(6-amino-2-methyl-4-pyrimidinyl)imidazo[1,2-a]pyridin-2-yl)amino)-1H-pyrazol-1-yl)carbonyl)amino)benzoate | 482.2 | 483.5 |
| 224 | 3-((3-(6-amino-2-methyl-4-pyrimidinyl)imidazo[1,2-a]pyridin-2-yl)amino)-N-ethyl-1H-pyrazole-1-carboxamide | 377.2 | 378.2 |
| 225 | 3-((3-(6-amino-2-methyl-4-pyrimidinyl)imidazo[1,2-a]pyridin-2-yl)amino)-N-(2-methoxyphenyl)-1H-pyrazole-1-carboxamide | 455.2 | 456.2 |
| 226 | 3-(6-amino-2-methyl-4-pyrimidinyl)-N-1H-pyrazol-4-ylimidazo[1,2-a]pyridin-2-amine | 306.1 | 307.2 |
| 227 | 3-(3-(6-amino-2-methyl-4-pyrimidinyl)imidazo[1,2-a]pyridin-2-yl)amino)-N-(2-methylphenyl)-1H-pyrazole-1-carboxamide | 439.2 | 440.2 |
| 228 | 3-((3-(6-amino-2-methyl-pyrimidinyl)imidazo[1,2-a]pyridin-2-yl)amino)-N-(4-methoxyphenyl)-1H-pyrazole-1-carboxamide | 455.2 | 456.2 |
| 229 | 3-((3-(6-amino-2-methyl-4-pyrimidinyl)imidazo[1,2-b]pyridazin-2-yl)amino)-N-phenyl-1H-pyrazole-1-carboxamide, trifluoroacetic acid | 426.2 | 427.5 |
| 230 | 3-((3-(6-amino-2-methyl-4-pyrimidinyl)imidazo[1,2-b]pyridazin-2-yl)amino)-N-methyl-1H-pyrazole-1-carboxamide | 364.2 | 365.4 |
| 231 | 5-((3-(6-amino-2-methyl-4-pyrimidinyl)imidazo[1,2-b]pyridazin-2-yl)amino)-N-methyl-1H-pyrazole-1-carboxamide | 364.2 | 365.4 |
| 232 | methyl 4-((3-(6-amino-2-methyl-4-pyrimidinyl)imidazo[1,2-a]pyridin-2-yl)amino)-1H-pyrazole-1-carboxylate | 364.1 | 365.4 |
| 233 | 3-(6-amino-2-methyl-4-pyrimidinyl)-N-(1-(1,3-benzoxazol-2-yl)-1H-pyrazol-3-yl)imidazo[1,2-b]pyridazin-2-amine | 424.2 | 425.4 |
| 234 | methyl 5-((3-(6-amino-2-methyl-4-pyrimidinyl)imidazo[1,2-b]pyridazin-2-yl)amino)-1H-pyrazole-1-carboxylate | 365.1 | 366.2 |
| 235 | 3-((3-(6-amino-2-methyl-4-pyrimidinyl)imidazo[1,2-b]pyridazin-2-yl)amino)-N-(2-methylphenyl)-1H-pyrazole-1-carboxamide | 440.2 | 441.2 |
| 236 | 3-((3-(6-amino-2-methyl-4-pyrimidinyl)imidazo[1,2-b]pyridazin-2-yl)amino)-N-(2-fluorophenyl)-1H-pyrazole-1-carboxamide | 444.2 | 445.2 |
| 237 | 3-(6-amino-2-methyl-4-pyrimidinyl)-N-(1-(1,3-benzoxazol-2-yl)-1H-pyrazol-5-yl)imidazo[1,2-b]pyridazin-2-amine | 424.2 | 425.4 |
| 238 | 3-((3-(6-amino-2-methyl-4-pyrimidinyl)imidazo[1,2-b]pyridazin-2-yl)amino)-N-(4-cyanophenyl)-1H-pyrazole-1-carboxamide | 451.2 | 452.4 |
| 239 | 3-((3-(6-amino-2-methyl-4-pyrimidinyl)imidazo[1,2-b]pyridazin-2-yl)amino)-N-(2-(trifluoromethyl)phenyl)-1H-pyrazole-1-carboxamide | 494.2 | 495.2 |
| 240 | 3-((3-(6-amino-2-methyl-4-pyrimidinyl)imidazo[1,2-b]pyridazin-2-yl)amino)-N-(3-methylphenyl)-1H-pyrazole-1-carboxamide | 440.2 | 441.2 |
| 241 | 3-((3-(6-amino-2-methyl-4-pyrimidinyl)imidazo[1,2-b]pyridazin-2-yl)amino)-N-(2,5-difluorophenyl)-1H-pyrazole-1-carboxamide | 462.2 | 463.2 |
| 242 | 3-((3-(6-amino-2-methyl-4-pyrimidinyl)imidazo[1,2-b]pyridazin-2-yl)amino)-N-(3,5-difluorophenyl)-1H-pyrazole-1-carboxamide | 462.2 | 463.2 |
| 243 | 3-((3-(6-amino-2-methyl-4-pyrimidinyl)imidazo[1,2-b]pyridazin-2-yl)amino)-N-(1-methylethyl)-1H-pyrazole-1-carboxamide | 392.2 | 393.2 |
| 244 | 3-((3-(6-amino-2-methyl-4-pyrimidinyl)imidazo[1,2-b]pyridazin-2-yl)amino)-N-(4-methylphenyl)-1H-pyrazole-1-carboxamide | 440.2 | 441.2 |
| 245 | 3-((3-(6-amino-2-methyl-4-pyrimidinyl)imidazo[1,2-b]pyridazin-2-yl)amino)-N-(3,5-dichlorophenyl)-1H-pyrazole-1-carboxamide | 494.1 | 495.3 |
| 246 | 3-((3-(6-amino-2-methyl-4-pyrimidinyl)imidazo[1,2-b]pyridazin-2-yl)amino)-N-(2-chloro-5-(trifluoromethyl)phenyl)-1H-pyrazole-1-carboxamide | 528.1 | 529.2 |
| 247 | 3-((3-(6-amino-2-methyl-4-pyrimidinyl)imidazo[1,2-b]pyridazin-2-yl)amino)-N-(2,5-dichlorophenyl)-1H-pyrazole-1-carboxamide | 494.1 | 495.0 |
| 248 | 3-((3-(6-amino-2-methyl-4-pyrimidinyl)imidazo[1,2-b]pyridazin-2-yl)amino)-N-(2,3-dichlorophenyl)-1H-pyrazole-1-carboxamide | 494.1 | 495.0 |
| 249 | 3-((3-(6-amino-2-methyl-4-pyrimidinyl)imidazo[1,2-b]pyridazin-2-yl)amino)-N-(2-chloro-4-(trifluoromethyl)phenyl)-1H-pyrazole-1-carboxamide | 528.1 | 529.0 |
| 250 | 3-((3-(6-(acetylamino)-2-methyl-4-pyrimidinyl)imidazo[1,2-b]pyridazin-2-yl)amino)-N-(2-methylphenyl)-1H-pyrazole-1-carboxamide | 482.2 | 483.3 |
| 251 | 3-(6-amino-2-methyl-4-pyrimidinyl)-N-(1-(1H-benzimidazol-2-yl)-1H-pyrazol-3-yl)imidazo[1,2-b]pyridazin-2-amine | 423.2 | 424.5 |
| 252 | 3-(6-amino-2-methyl-4-pyrimidinyl)-N-(1-(1H-imidazol-2-yl)-1H-pyrazol-3-yl)imidazo[1,2-b]pyridazin-2-amine | 423.2 | 424.5 |
| 253 | 3-(6-amino-2-methyl-4-pyrimidinyl)-N-(1-(methylsulfonyl)-1H-pyrazol-3-yl)imidazo[1,2-b]pyridazin-2-amine | 385.1 | 386.4 |
| 254 | 3-((3-(6-amino-2-methyl-4-pyrimidinyl)imidazo[1,2-b]pyridazin-2-yl)amino)-1H-pyrazole-1-carboxamide | 350.1 | 351.3 |
| 255 | 5-((3-(6-amino-2-methyl-4-pyrimidinyl)imidazo[1,2-b]pyridazin-2-yl)amino)-1H-pyrazole-1-carboxamide | 350.1 | 351.3 |
| 256 | 3-(6-amino-2-methyl-4-pyrimidinyl)-N-(1-(1-azetidinylcarbonyl)-1H-pyrazol-3-yl)imidazo[1,2-b]pyridazin-2-amine | 390.1 | 391.5 |
| 257 | 3-(6-amino-2-methyl-4-pyrimidinyl)-N-(1-(1-azetidinylcarbonyl)-1H-pyrazol-5-yl)imidazo[1,2-b]pyridazin-2-amine | 390.1 | 391.5 |
| 258 | 3-((3-(6-amino-2-methyl-4-pyrimidinyl)imidazo[1,2-b]pyridazin-2-yl)amino)-N-(2-(methylamino)ethyl)-1H-pyrazole-1-carboxamide | 407.2 | 408.3 |
| 259 | 5-((3-(6-amino-2-methyl-4-pyrimidinyl)imidazo[1,2-b]pyridazin-2-yl)amino)-N-(2-(methylamino)ethyl)-1H-pyrazole-1-carboxamide | 407.2 | 408.2 |
| 260 | 3-((3-(6-amino-2-methyl-4-pyrimidinyl)imidazo[1,2-b]pyridazin-2-yl)amino)-N,N-dimethyl-1H-pyrazole-1-carboxamide | 378.2 | 379.5 |
| 261 | 5-((3-(6-amino-2-methyl-4-pyrimidinyl)imidazo[1,2-b]pyridazin-2-yl)amino)-N,N-dimethyl-1H-pyrazole-1-carboxamide | 378.2 | 379.5 |
| 262 | 3-(6-amino-2-methyl-4-pyrimidinyl)-N-(1-(1-piperazinylcarbonyl)-1H-pyrazol-3-yl)imidazo[1,2-b]pyridazin-2-amine | 419.2 | 420.3 |
| 263 | 5-((3-(6-amino-2-methyl-4-pyrimidinyl)imidazo[1,2-b]pyridazin-2-yl)amino)-N-(2-hydroxyethyl)-1H-pyrazole-1-carboxamide | 394.2 | 395.5 |
| 264 | 3-((3-(6-amino-2-methyl-4-pyrimidinyl)imidazo[1,2-b]pyridazin-2-yl)amino)-N-(2-methoxyethyl)-1H-pyrazole-1-carboxamide | 408.2 | 409.5 |
| 265 | 5-((3-(6-amino-2-methyl-4-pyrimidinyl)imidazo[1,2-b]pyridazin-2-yl)amino)-N-(2-methoxyethyl)-1H-pyrazole-1-carboxamide | 408.2 | 409.5 |
| 266 | 5-((3-(6-amino-2-methyl-4-pyrimidinyl)imidazo[1,2-b]pyridazin-2-yl)amino)-N-(2-methylpropyl)-1H-pyrazole-1-carboxamide | 406.2 | 407.4 |
| 267 | 3-(6-amino-2-methyl-4-pyrimidinyl)-N-1H-pyrazol-3-yl-6-(5-pyrimidinyl)imidazo[1,2-b]pyridazin-2-amine | 385.2 | 386.2 |
| 268 | 3-(6-amino-2-methyl-4-pyrimidinyl)-N-(1-(1-phenylethyl)-1H-pyrazol-3-yl)imidazo[1,2-b]pyridazin-2-amine | 411.4 | 412.2 |
| 269 | 3-(6-amino-2-methyl-4-pyrimidinyl)-N-(1-benzyl-1H-pyrazol-3-yl)imidazo[1,2-b]pyridazin-2-amine | 397.1 | 398.4 |
| 270 | 3-(6-amino-2-methyl-4-pyrimidinyl)-N-(1-(3-pyridinylmethyl)-1H-pyrazol-3-yl)imidazo[1,2-b]pyridazin-2-amine | 398.1 | 399.3 |
| 271 | 7-((3-(6-amino-2-methyl-4-pyrimidinyl)imidazo[1,2-a]pyridin2-yl)amino)-2H-1,4-benzoxazin-3(4H)-one | 387.1 | 388.5 |
| 272 | 1-(5-((3-(6-amino-2-methyl-4-pyrimidinyl)imidazo[1,2-b]pyridazin-2-yl)amino)-2-pyridinyl)-3-azetidinol | 389 | 390 |
| 273 | 2-(1-(5-((3-(6-amino-2-methyl-4-pyrimidinyl)imidazo[1,2-b]pyridazin-2-yl)amino)-2-pyridinyl)-3-azetidinyl)-2-propanol | 431 | 432 |
| 274 | N-(3-(4-amino-6-methyl-1,3,5-triazin-2-yl)imidazo[1,2-a]pyridin-2-yl)-7-quinolinamine | 368 | 369 |
| 275 | N-(3-(4-amino-6-methyl-1,3,5-triazin-2-yl)imidazo[1,2-a]pyridin-2-yl)-6-quinolinamine | 368 | 369 |
| 276 | N-(3-(4-amino-6-methyl-1,3,5-triazin-2-yl)imidazo[1,2-a]pyridin-2-yl)-7-isoquinolinamine | 368 | 369 |
| 277 | 4-(3-(4-amino-6-methyl-1,3,5-triazin-2-yl)-2-(7-quinolinylamino)-imidazo[1,2-a]pyridin-6-yl)-N,N-dimethyl-1-piperazinecarboxamide | 523 | 524 |
| 278 | N-(3-(4-amino-6-methyl-1,3,5-triazin-2-yl)-6-(4-(methylsulfonyl)-1-piperazinyl)imidazo[1,2-a]pyridin-2-yl)-7-quinolinamine | 530 | 531 |
| 279 | N-(3-(4-amino-6-methyl-1,3,5-triazin-2-yl)-6-(1-piperazinyl)-imidazo[1,2-a]pyridin-2-yl)-7-quinolinamine | 452 | 454 |
| 280 | 4-(3-(4-(acetylamino)-6-methyl-1,3,5-triazin-2-yl)-2-((6-methoxy-3-pyridinyl)amino)imidazo[1,2-a]pyridin-6-yl)-N,N-dimethyl-1-piperazinecarboxamide | 545 | 546 |
| 281 | 3-(4-amino-6-methyl-1,3,5-triazin-2-yl)-N-(6-ethoxy-3-pyridinyl)-imidazo[1,2-a]pyridin-2-amine | 362 | 363 |
| 282 | N-(3-(4-amino-6-methyl-1,3,5-triazin-2-yl)imidazo[1,2-a]pyridin-2-yl)-1H-indazol-3-amine | 357 | 358 |
| 283 | 4-(3-(4-amino-6-methyl-1,3,5-triazin-2-yl)-2-((5-fluoro-6-methoxy-3-pyridinyl)amino)imidazo[1,2-a]pyridin-6-yl)-N,N-dimethyl-1-piperazinecarboxamide | 521 | 522 |
| 284 | 4-(3-(4-amino-6-methyl-1,3,5-triazin-2-yl)-2-((6-methoxy-3-pyridinyl)amino)imidazo[1,2-a]pyridin-6-yl)-N-methyl-1-piperazinecarboxamide | 489 | 490 |
| 285 | 6-(4-acetyl-1-piperazinyl)-3-(4-amino-6-methyl-1,3,5-triazin-2-yl)-N-(6-methoxy-3-pyridinyl)imidazo[1,2-a]pyridin-2-amine | 474 | 475 |
| 286 | (5-(3-(4-amino-6-methyl-1,3,5-triazin-2-yl)-2-(1H-pyrazol-5-ylamino)imidazo[1,2-a]pyridin-6-yl)-2-methoxyphenyl)methanol | 443.2 | 444.3 |
| 287 | 3-(4-amino-6-methyl-1,3,5-triazin-2-yl)-N-1H-pyrazol-5-yl-6-(4-pyridinyl)imidazo[1,2-a]pyridin-2-amine | 384.4 | 385.2 |
| 288 | 3-(4-amino-6-methyl-1,3,5-triazin-2-yl)-6-(3,6-dihydro-2H-pyran-4-yl)-N-1H-pyrazol-5-ylimidazo[1,2-a]pyridin-2-amine | 389.4 | 390.3 |
| 289 | 3-(4-amino-6-methyl-1,3,5-triazin-2-yl)-6-(2-methyl-4-pyridinyl)-N-1H-pyrazol-5-ylimidazo[1,2-a]pyridin-2-amine | 398.2 | 399.2 |
| 290 | 3-(4-amino-6-methyl-1,3,5-triazin-2-yl)-6-(1-methyl-1H-imidazol-5-yl)-N-1H-pyrazol-5-ylimidazo[1,2-a]pyridin-2-amine | 387.2 | 388.2 |
| 291 | 4-(3-(4-amino-6-methyl-1,3,5-triazin-2-yl)-2-(1H-pyrazol-5-ylamino)-midazo[1,2-a]pyridin-6-yl)-1,2-butanediol | 388.2 | 389.4 |
| 292 | 3-(4-amino-6-methyl-1,3,5-triazin-2-yl)-6-(1-(1-methylethyl)-1H-pyrazol-4-yl)-N-1H-pyrazol-5-ylimidazo[1,2-a]pyridin-2-amine | 415.2 | 416 |
| 293 | 3-(4-amino-6-methyl-1,3,5-triazin-2-yl)-2-(1H-pyrazol-5-ylamino)-imidazo[1,2-a]pyridine-6-carboxamide | 350.1 | 351.3 |
| 294 | N-((3-(4-amino-6-methyl-1,3,5-triazin-2-yl)-2-(1H-pyrazol-5-ylamino)imidazo[1,2-a]pyridin-6-yl)methyl)acetamide | 378.2 | 379.2 |
| 295 | 3-(2-methyl-6-((2,2,2-trifluoroethyl)amino)-4-pyrimidinyl)-N-1H-pyrazol-5-ylimidazo[1,2-b]pyridazin-2-amine | 389.1 | 390.3 |
| 296 | 3-(2-methyl-6-(2-pyrazinylamino)-4-pyrimidinyl)-N-(1-methyl-1H-pyrazol-3-yl)imidazo[1,2-b]pyridazin-2-amine | 399.2 | 400.5 |
| 297 | 3-(2-methyl-6-(3-pyridinylamino)-4-pyrimidinyl)-N-1H-pyrazol-3-ylimidazo[1,2-b]pyridazin-2-amine | 384.2 | 385.2 |
| 298 | 2-cyano-N-(2-methyl-6-(2-((1-methyl-1H-pyrazol-3-yl)amino)-imidazo[1,2-b]pyridazin-3-yl)-4-pyrimidinyl)acetamide | 388.2 | 389.4 |
| 299 | N-(2-methyl-6-(2-((1-methyl-1H-pyrazol-3-yl)amino)imidazo[1,2-b]pyridazin-3-yl)-4-pyrimidinyl)-1,3-oxazole-5-carboxamide | 416.2 | 417.3 |
| 300 | 3-(6-amino-2-methyl-4-pyrimidinyl)-N-(1-methyl-1H-pyrazol-3-yl)-6-(5-pyrimidinyl)imidazo[1,2-a]pyridin-2-amine | 398.2 | 399.3 |
| 301 | N-(4-(3-(6-amino-2-methyl-4-pyrimidinyl)-2-((1-methyl-1H-pyrazol-3-yl)amino)imidazo[1,2-a]pyridin-6-yl)phenyl)acetamide | 453.2 | 454.2 |
| 302 | 3-(4-amino-6-methyl-1,3,5-triazin-2-yl)-N-(1-methyl-1H-pyrazol-3-yl)-6-(7-quinolinyl)imidazo[1,2-a]pyridin-2-amine | 448.2 | 449.2 |
| 303 | N-(4-(3-(6-amino-2-methyl-4-pyrimidinyl)-2-((1-methyl-1H-pyrazol-3-yl)amino)imidazo[1,2-b]pyridazin-6-yl)benzyl)acetamide | 468.2 | 469.5 |
| 304 | 3-(4-amino-6-methyl-1,3,5-triazin-2-yl)-N-(1-methyl-1H-pyrazol-3-yl)-6-(5-pyrimidinyl)imidazo[1,2-a]pyridin-2-amine | 399.2 | 400.5 |
| 305 | 4-(3-(6-amino-2-methyl-4-pyrimidinyl)-2-((1-methyl-1H-pyrazol-3-yl)amino)imidazo[1,2-b]pyridazin-6-yl)-N-methylbenzamide | 454.2 | 455.4 |
| 306 | 3-(6-amino-2-methyl-4-pyrimidinyl)-N-(1-(6-(2-methoxyethoxy)-4-pyrimidinyl)-1H-pyrazol-3-yl)imidazo[1,2-b]pyridazin-2-amine | 459.2 | 460.2 |
| 307 | 3-(6-(3-isoxazolylamino)2-methyl-4-pyrimidinyl)-N-1H-pyrazol-3-ylimidazo[1,2-b]pyridazin-2-amine | 374.1 | 375.3 |
| 308 | 2-cyano-N-(2-methyl-6-(2-((1-methyl-1H-pyrazol-3-yl)amino)-6-(5-pyrimidinyl)imidazo[1,2-a]pyridin-3-yl)-4-pyrimidinyl)acetamide | 465.2 | 466.2 |
| 309 | 3-(6-((1-(2-methoxyethyl)-1H-pyrazol-4-yl)amino)-2-methyl-4-pyrimidinyl)-N-1H-pyrazol-3-ylimidazo[1,2-b]pyridazin-2-amine | 431.2 | 432.2 |
| 310 | 3-(2-methyl-6-((1-((3R)-tetrahydro-3-furanyl)-1H-pyrazol-4-yl)amino)-4-pyrimidinyl)-N-1H-pyrazol-3-ylimidazo[1,2-b]pyridazin-2-amine, 3-(2-methyl-6-((1-((3S)-tetrahydro-3-furanyl)-1H-pyrazol-4-yl)amino)-4-pyrimidinyl)-N-1H-pyrazol-3-ylimidazo[1,2-b]pyridazin-2-amine | 443.2 | 444.3 |
| 311 | 3-(6-(imidazo[1,2-a]pyridin-2-ylamino)-2-methyl-4-pyrimidinyl)-N-1H-pyrazol-3-ylimidazo[1,2-b]pyridazin-2-amine | 423.2 | 424.5 |
| 312 | 3-(6-(furo[3,2-b]pyridin-6-ylamino)-2-methyl-4-pyrimidinyl)-N-1H-pyrazol-3-ylimidazo[1,2-b]pyridazin-2-amine | 424.2 | 425.2 |
| 313 | 3-(6-amino-2-methyl-4-pyrimidinyl)-N-1H-pyrazol-3-yl-6-(5-pyrimidinyl)imidazo[1,2-a]pyridin-2-amine | 384.2 | 385.5 |
| 314 | 3-(2-methyl-6-((1-((3R)-tetrahydro-3-furanyl)-1H-pyrazol-4-yl)amino)-4-pyrimidinyl)-N-1H-pyrazol-3-ylimidazo[1,2-a]pyridin-2-amine, 3-(2-methyl-6-((1-((3S)-tetrahydro-3-furanyl)-1H-pyrazol-4-yl)amino)-4-pyrimidinyl)-N-1H-pyrazol-3-ylimidazo[1,2-a]pyridin-2-amine | 442.2 | 443.4 |
| 315 | 3-(6-((6-(2-methoxyethoxy)-4-pyrimidinyl)amino)-2-methyl-4-pyrimidinyl)-N-1H-pyrazol-3-ylimidazo[1,2-b]pyridazin-2-amine | 459.2 | 460.2 |
| 316 | 1-(3-((3-(6-amino-2-methyl-4-pyrimidinyl)imidazo[1,2-b]pyridazin-2-yl)amino)phenyl)-3-cyclopropylurea | 415.2 | 416.2 |
| 317 | N-(3-((3-(6-amino-2-methyl-4-pyrimidinyl)imidazo[1,2-a]pyridin-2-yl)amino)phenyl)acetamide | 373.17 | 374.2 |
| 318 | N-(3-((3-(6-amino-2-methyl-4-pyrimidinyl)imidazo[1,2-a]pyridin-2-yl)amino)phenyl)-2-phenylacetamide | 449.2 | 450.2 |
| 319 | N-(3-((3-(6-amino-2-methyl-4-pyrimidinyl)imidazo[1,2-a]pyridin-2-yl)amino)phenyl)benzamide | 435.18 | 436.2 |
| 320 | N-(3-((3-(6-amino-2-methyl-4-pyrimidinyl)imidazo[1,2-a]pyridin-2-yl)amino)phenyl)-4-chlorobenzenesulfonamide | 505.11 | 506.2 |
| 321 | N-(3-((3-(6-amino-2-methyl-4-pyrimidinyl)imidazo[1,2-a]pyridin-2-yl)amino)phenyl)methanesulfonamide | 539.07 | 410.2 |
| 322 | 1-(3-((3-(6-amino-2-methyl-4-pyrimidinyl)imidazo[1,2-a]pyridin-2-yl)amino)phenyl)-3-phenylurea | 450.19 | 451.2 |
| 323 | 1-(3-((3-(6-amino-2-methyl-4-pyrimidinyl)imidazo[1,2-a]pyridin-2-yl)amino)phenyl)-3-(4-fluorophenyl)urea | 468.18 | 469.2 |
| 324 | 1-(3-((3-(6-amino-2-methyl-4-pyrimidinyl)imidazo[1,2-a]pyridin-2-yl)amino)phenyl)-3-benzylurea | 464.21 | 465.2 |
| 325 | 1-(3-((3-(6-amino-2-methyl-4-pyrimidinyl)imidazo[1,2-a]pyridin-2-yl)amino)phenyl)-3-methylurea | 388.18 | 389.2 |
| 326 | 1-(3-((3-(6-amino-2-methyl-4-pyrimidinyl)imidazo[1,2-a]pyridin-2-yl)amino)phenyl)-3-ethylurea | 402.19 | 403.2 |
| 327 | 4-((3-(6-amino-2-methyl-4-pyrimidinyl)imidazo[1,2-a]pyridin-2-yl)amino)-N-ethylbenzamide | 387.18 | 388.2 |
| 328 | 5-((3-(6-amino-2-methyl-4-pyrimidinyl)imidazo[1,2-a]pyridin-2-yl)amino)-N-ethyl-2-pyridinecarboxamide | 388.18 | 389.2 |
| 329 | N-(3-((3-(6-amino-2-methyl-4-pyrimidinyl)imidazo[1,2-b]pyridazin-2-yl)amino)phenyl)acetamide | 374.16 | 375.2 |
| 330 | N-(3-((3-(6-amino-2-methyl-4-pyrimidinyl)imidazo[1,2-b]pyridazin-2-yl)amino)phenyl)propanamide | 388.18 | 389.2 |
| 331 | 3-((3-(6-amino-2-methyl-4-pyrimidinyl)imidazo[1,2-a]pyridin-2-yl)amino)-N-methylbenzamide | 373.17 | 374.2 |
| 332 | 3-((3-(6-amino-2-methyl-4-pyrimidinyl)imidazo[1,2-a]pyridin-2-yl)amino)-N-ethylbenzamide | 387.18 | 388.2 |
| 333 | 3-((3-(6-amino-2-methyl-4-pyrimidinyl)imidazo[1,2-a]pyridin-2-yl)amino)-N-(1-methylethyl)benzamide | 401.2 | 402.2 |
| 334 | 3-((3-(6-amino-2-methyl-4-pyrimidinyl)imidazo[1,2-b]pyridazin-2-yl)amino)-N-methylbenzamide | 374.16 | 375.2 |
| 335 | 3-((3-(6-amino-2-methyl-4-pyrimidinyl)imidazo[1,2-b]pyridazin-2-yl)amino)-N-ethylbenzamide | 388.18 | 389.2 |
| 336 | 3-((3-(6-amino-2-methyl-4-pyrimidinyl)imidazo[1,2-b]pyridazin-2-yl)amino)-N-phenylbenzamide | 436.18 | 437.2 |
| 337 | 3-((3-(6-amino-2-methyl-4-pyrimidinyl)imidazo[1,2-b]pyridazin-2-yl)amino)-N-(1-methylethyl)benzamide | 402.19 | 403.2 |
| 338 | 5-((3-(6-amino-2-methyl-4-pyrimidinyl)imidazo[1,2-a]pyridin-2-yl)amino)-N-methyl-2-pyridinecarboxamide | 374.16 | 375.2 |
| 339 | 5-((3-(6-amino-2-methyl-4-pyrimidinyl)imidazo[1,2-a]pyridin-2-yl)amino)-N-(1-methylethyl)-2-pyridinecarboxamide | 402.19 | 403 |
| 340 | 5-((3-(6-amino-2-methyl-4-pyrimidinyl)imidazo[1,2-a]pyridin-2-yl)amino)-N-phenyl-2-pyridinecarboxamide | 436.18 | 437.2 |
| 341 | 4-((3-(6-amino-2-methyl-4-pyrimidinyl)imidazo[1,2-a]pyridin-2-yl)amino)benzamide | 359.15 | 360.2 |
| 342 | 4-((3-(6-amino-2-methyl-4-pyrimidinyl)imidazo[1,2-a]pyridin-2-yl)amino)-N-(2-(diethylamino)ethyl)benzamide | 458.25 | 459.2 |
| 343 | 4-((3-(6-amino-2-methyl-4-pyrimidinyl)imidazo[1,2-a]pyridin-2-yl)amino)-N-phenylbenzamide | 435.18 | 436.2 |
| 344 | 3-(4-amino-6-methyl-1,3,5-triazin-2-yl)-6-(4-(methylsulfonyl)phenyl)-N-1H-pyrazol-3-ylimidazo[1,2-a]pyridin-2-amine | 461.14 | 462.2 |
| 345 | 3-(6-amino-2-methyl-4-pyrimidinyl)-N-(1-(3-methylbutanoyl)-1H-pyrazol-3-yl)imidazo[1,2-b]pyridazin-2-amine | 391.19 | 392.2 |
| 346 | 1-methylethyl 5-((3-(6-amino-2-methyl-4-pyrimidinyl)imidazo[1,2-b]pyridazin-2-yl)amino)-1H-pyrazole-1-carboxylate | 393.17 | 394.2 |
| 347 | 1-methylethyl 3-((3-(6-amino-2-methyl-4-pyrimidinyl)imidazo[1,2-b]pyridazin-2-yl)amino)-1H-pyrazole-1-carboxylate | 393.17 | 394.2 |
| 348 | 3-(6-amino-2-methyl-4-pyrimidinyl)-N-(2-methoxy-4-pyridinyl)imidazo[1,2-b]pyridazin-2-amine | 348.14 | 349.2 |
| 349 | 3-(6-amino-2-methyl-4-pyrimidinyl)-N-(1-(2-methylpropanoyl)-1H-pyrazol-3-yl)imidazo[1,2-b]pyridazin-2-amine | 377.17 | 378.2 |
| 350 | 3-(6-amino-2-methyl-4-pyrimidinyl)-N-(1-(2-methylpropanoyl)-1H-pyrazol-5-yl)imidazo[1,2-b]pyridazin-2-amine | 377.17 | 378.2 |
| 351 | 4-(3-(4-amino-6-methyl-1,3,5-triazin-2-yl)-2-(1H-pyrazol-3-ylamino)imidazo[1,2-a]pyridin-6-yl)-N-methylbenzenesulfonamide | 476.15 | 477.2 |
| 352 | 3-(4-amino-6-methyl-1,3,5-triazin-2-yl)-6-(3-(methylsulfonyl)phenyl)-N-1H-pyrazol-3-ylimidazo[1,2-a]pyridin-2-amine | 461.14 | 462.2 |
| 353 | 3-(6-amino-2-methyl-4-pyrimidinyl)-N-(1-(3,3-dimethylbutanoyl)-1H-pyrazol-3-yl)imidazo[1,2-b]pyridazin-2-amine | 405.2 | 406.2 |
| 354 | 3-(4-amino-6-methyl-1,3,5-triazin-2-yl)-N-(1-methyl-1H-pyrazol-3-yl)-6-(4-(methylsulfonyl)phenyl)imidazo[1,2-a]pyridin-2-amine | 475.15 | 476.2 |
| 355 | cyclohexyl 5-((3-(6-amino-2-methyl-4-pyrimidinyl)imidazo[1,2-b]pyridazin-2-yl)amino)-1H-pyrazole-1-carboxylate | 433.2 | 434.2 |
| 356 | cyclohexyl 3-((3-(6-amino-2-methyl-4-pyrimidinyl)imidazo[1,2-b]pyridazin-2-yl)amino)-1H-pyrazole-1-carboxylate | 433.2 | 434.2 |
| 357 | 3-(6-amino-2-methyl-4-pyrimidinyl)-N-(2-methoxy-3-pyridinyl)imidazo[1,2-b]pyridazin-2-amine | 348.14 | 349.2 |
| 358 | 3-((3-(6-amino-2-methyl-4-pyrimidinyl)imidazo[1,2-b]pyridazin-2-yl)amino)-2(1H)-pyridinone | 334.13 | 335.2 |
| 359 | 3-(6-amino-2-methyl-4-pyrimidinyl)-N-(1-(3-methylbutyl)-1H-pyrazol-3-yl)imidazo[1,2-b]pyridazin-2-amine | 377.21 | 378.2 |
| 360 | 3-(6-amino-2-methyl-4-pyrimidinyl)-N-(1-(2-methoxyethyl)-1H-pyrazol-3-yl)imidazo[1,2-b]pyridazin-2-amine | 365.17 | 366.2 |
| 361 | 3-(6-amino-2-methyl-4-pyrimidinyl)-6-(3-(methylsulfonyl)phenyl)-N-1H-pyrazol-3-ylimidazo[1,2-b]pyridazin-2-amine | 461.14 | 462.2 |
| 362 | N-(3-(4-amino-6-methyl-1,3,5-triazin-2-yl)imidazo[1,2-a]pyridin-2-yl)-1H-indazol-4-amine | | |
| 363 | 3-(6-amino-2-methyl-4-pyrimidinyl)-N-(6-methoxy-3-pyridinyl)-6-(4-morpholinyl)imidazo[1,2-b]pyridazin-2-amine | | |
| 364 | 3-(4-amino-6-methyl-1,3,5-triazin-2-yl)-N-(6-methoxy-3-pyridinyl)-6-(4-morpholinyl)imidazo[1,2-b]pyridazin-2-amine | | |
| 365 | 3-(6-amino-2-methyl-4-pyrimidinyl)-N~6∼-(2-methoxyethyl)-N∼2∼-(6-methoxy-3-pyridinyl)imidazo[1,2-b]pyridazine-2,6-diamine | | |
| 366 | 3-(6-amino-2-methyl-4-pyrimidinyl)-N∼2∼-1H-indazol-4-yl-N∼6∼-(2-methoxyethyl)imidazo[1,2-b]pyridazine-2,6-diamine | | |
| 367 | 1-(3-(6-amino-2-methyl-4-pyrimidinyl)-2-((6-methoxy-3-pyridinyl)amino)imidazo[1,2-b]pyridazin-6-yl)-4-piperidinol | | |
| 368 | 4-(3-(4-amino-6-methyl-1,3,5-triazin-2-yl)-2-((6-methoxy-3-pyridinyl)amino)imidazo[1,2-a]pyridin-7-yl)-N,N-dimethyl-1-piperazinecarboxamide | | |
| 369 | 4-(3-(4-amino-6-methyl-1,3,5-triazin-2-yl)-2-((5-fluoro-6-methoxy-3-pyridinyl)amino)imidazo[1,2-a]pyridin-7-yl)-N,N-dimethyl-1-piperazinecarboxamide | | |
| 370 | 3-(4-amino-6-methyl-1,3,5-triazin-2-yl)-N-(5-fluoro-6-methoxy-3-pyridinyl)-7-(4-(methylsulfonyl)-1-piperazinyl)imidazo[1,2-a]pyridin-2-amine | | |
| 371 | N-(5-((3-(4-amino-6-methyl-1,3,5-triazin-2-yl)imidazo[1,2-a]pyridin-2-yl)amino)-2-chloro-3-pyridinyl)methanesulfonamide | | |
| 372 | N-(5-((3-(4-amino-6-methyl-1,3,5-triazin-2-yl)imidazo[1,2-a]pyridin-2-yl)amino)-2-methoxy-3-pyridinyl)methanesulfonamide | | |
| 373 | 3-(6-amino-2-methyl-4-pyrimidinyl)-N-1H-indazol-6-ylimidazo[1,2-b]pyridazin-2-amine | 357.15 | 358.20 |
| 374 | 3-(6-amino-2-methyl-4-pyrimidinyl)-N-1H-benzimidazol-6-ylimidazo[1,2-b]pyridazin-2-amine | 357.15 | 358.20 |
| 375 | N-(3-(6-amino-2-methyl-4-pyrimidinyl)imidazo[1,2-a]pyridin-2-yl)-1H-benzimidazol-6-amine | 356.15 | 357.20 |
| 376 | 3-(4-amino-6-methyl-1,3,5-triazin-2-yl)-N-(6-methoxy-3-pyridinyl)-7-(1-methyl-1H-pyrazol-4-yl)imidazo[1,2-a]pyridin-2-amine | 428.2 | 428.9 |
| 377 | 3-(4-amino-6-methyl-1,3,5-triazin-2-yl)-N-(6-methoxy-3-pyridinyl)-6-(1-methyl-1H-pyrazol-4-yl)imidazo[1,2-a]pyridin-2-amine | 428.2 | 428.9 |
| 378 | 3-(4-amino-6-methyl-1,3,5-triazin-2-yl)-N-(6-methoxy-3-pyridinyl)-6-(4-morpholinyl)imidazo[1,2-a]pyridin-2-amine | 433.2 | 433.9 |
| 379 | 3-(6-amino-2-methyl-4-pyrimidinyl)-N-(6-methoxy-3-pyridinyl)-6-(4-morpholinyl)imidazo[1,2-a]pyridin-2-amine | 432.2 | 433 |
| 380 | 3-(4-amino-6-methyl-1,3,5-triazin-2-yl)-N-(6-methoxy-3-pyridinyl)-6-(3-pyridinyl)imidazo[1,2-a]pyridin-2-amine | 425.2 | 425.9 |
| 381 | 4-(3-(4-amino-6-methyl-1,3,5-triazin-2-yl)-2-((6-methoxy-3-pyridinyl)amino)imidazo[1,2-a]pyridin-6-yl)-N,N-dimethyl-1-piperazinecarboxamide | 503.2 | 503.9 |
| 382 | 3-(4-amino-6-methyl-1,3,5-triazin-2-yl)-N-(6-methoxy-3-pyridinyl)-6-(4-(methylsulfonyl)-1-piperazinyl)imidazo[1,2-a]pyridin-2-amine | 510.2 | 511.1 |
| 383 | 2-(3-(4-amino-6-methyl-1,3,5-triazin-2-yl)-2-((6-methoxy-3-pyridinyl)-amino)imidazo[1,2-a]pyridin-6-yl)-2-propanol | 406.2 | 407 |
| 384 | 3-(4-amino-6-methyl-1,3,5-triazin-2-yl)-N-(6-methoxy-3-pyridinyl)-6-(1-piperazinyl)imidazo[1,2-a]pyridin-2-amine | 432 | 433 |
| 385 | 1-(3-(4-amino-6-methyl-1,3,5-triazin-2-yl)-2-(7-quinolinylamino)-imidazo[1,2-a]pyridin-6-yl)ethanone | 410.2 | 411 |
| 386 | 2-(3-(4-amino-6-methyl-1,3,5-triazin-2-yl)-2-(7-quinolinylamino)-imidazo[1,2-a]pyridin-6-yl)-2-propanol | 426.2 | 427 |
| 387 | 1-(3-(4-amino-6-methyl-1,3,5-triazin-2-yl)-2-((5-fluoro-6-methoxy-3-pyridinyl)amino)imidazo[1,2-a]pyridin-6-yl)ethanone | 408.1 | 409 |
| 388 | 2-(3-(4-amino-6-methyl-1,3,5-triazin-2-yl)-2-((5-fluoro-6-methoxy-3-pyridinyl)amino)imidazo[1,2-a]pyridin-6-yl)-2-propanol | 424.2 | 425 |
| 389 | 4-(3-(6-amino-2-methyl-4-pyrimidinyl)-2-((5-fluoro-6-methoxy-3-pyridinyl)amino)imidazo[1,2-b]pyridazin-6-yl)-N,N-dimethyl-1-piperazinecarboxamide | 521.2 | 521.9 |
| 390 | 1-(3-(6-amino-2-methyl-4-pyrimidinyl)-2-((5-fluoro-6-methoxy-3-pyridinyl)amino)imidazo[1,2-a]pyridin-6-yl)ethanone | 407.1 | 407.9 |
| 391 | 2-(3-(6-amino-2-methyl-4-pyrimidinyl)-2-((5-fluoro-6-methoxy-3-pyridinyl)amino)imidazo[1,2-a]pyridin-6-yl)-2-propanol | 423.2 | 424 |
| 392 | 4-(3-(6-amino-2-methyl-4-pyrimidinyl)-2-((5-fluoro-6-methoxy-3-pyridinyl)amino)imidazo[1,2-a]pyridin-6-yl)-N,N-dimethyl-1-piperazinecarboxamide | 520.2 | 521 |
| 393 | 3-(4-amino-6-methyl-1,3,5-triazin-2-yl)-N-1H-pyrazol-3-yl-6-(trifluoromethyl)imidazo[1,2-a]pyridin-2-amine | 375.12 | 376.2 |
| 394 | 3-(4-amino-6-methyl-1,3,5-triazin-2-yl)-N-1H-pyrazol-3-yl-7-(trifluoromethyl)imidazo[1,2-a]pyridin-2-amine | 375.12 | 376.2 |
| 395 | 3-(6-amino-2-methyl-4-pyrimidinyl)-N-1H-pyrazol-3-yl-7-(trifluoromethyl)imidazo[1,2-a]pyridin-2-amine | 374.12 | 375.2 |
| 396 | 3-(6-amino-2-methyl-4-pyrimidinyl)-N-1H-pyrazol-3-yl-6-(trifluoromethyl)imidazo[1,2-a]pyridin-2-amine | 374.12 | 375.2 |
| 397 | 1-(4-((3-(6-amino-2-methyl-4-pyrimidinyl)imidazo[1,2-b]pyridazin-2-yl)amino)phenyl)-3-cyclopropylurea | 415.19 | 416 |
| 398 | 1-(4-((3-(6-amino-2-methyl-4-pyrimidinyl)imidazo[1,2-b]pyridazin-2-yl)amino)phenyl)-3-ethylurea | 403.19 | 404 |
| 399 | N-(4-((3-(6-amino-2-methyl-4-pyrimidinyl)imidazo[1,2-b]pyridazin-2-yl)amino)phenyl)-1-piperidinecarboxamide | 443.22 | 444.2 |
| 400 | N-(4-((3-(6-amino-2-methyl-4-pyrimidinyl)imidazo[1,2-b]pyridazin-2-yl)amino)phenyl)-1-pyrrolidinecarboxamide | 429.2 | 430.2 |
| 401 | N-(4-((3-(6-amino-2-methyl-4-pyrimidinyl)imidazo[1,2-b]pyridazin-2-yl)amino)phenyl)-1-azetidinecarboxamide | 415.19 | 416.2 |
| 402 | N-(4-((3-(6-amino-2-methyl-4-pyrimidinyl)imidazo[1,2-b]pyridazin-2-yl)amino)phenyl)-4-methyl-1-piperazinecarboxamide | 458.23 | 459.2 |
| 403 | 1-(4-((3-(6-amino-2-methyl-4-pyrimidinyl)imidazo[1,2-b]pyridazin-2-yl)amino)phenyl)-3-(2-methoxyethyl)urea | 433.2 | 434.2 |
| 404 | N-(4-((3-(6-amino-2-methyl-4-pyrimidinyl)imidazo[1,2-b]pyridazin-2-yl)amino)phenyl)-4-morpholinecarboxamide | 445.2 | 446.2 |
| 405 | 4-acetyl-N-(4-((3-(6-amino-2-methyl-4-pyrimidinyl)imidazo[1,2-b]pyridazin-2-yl)amino)phenyl)-1-piperazinecarboxamide | 486.22 | 487.2 |
| 406 | 3-(6-amino-2-methyl-4-pyrimidinyl)-N-1,3-thiazol-4-ylimidazo[1,2-b]pyridazin-2-amine | 324.09 | 325 |
| 407 | N-(4-((3-(6-amino-2-methyl-4-pyrimidinyl)imidazo[1,2-a]pyridin-2-yl)amino)phenyl)-4-morpholinecarboxamide | 444.2 | 445.2 |
| 408 | 4-acetyl-N-(4-((3-(6-amino-2-methyl-4-pyrimidinyl)imidazo[1,2-a]pyridin-2-yl)amino)phenyl)-1-piperazinecarboxamide | 485.23 | 486.2 |
| 409 | N-(4-((3-(6-amino-2-methyl-4-pyrimidinyl)imidazo[1,2-a]pyridin-2-yl)amino)phenyl)-2-methyl-4-morpholinecarboxamide | 458.22 | 459.2 |
| 410 | 3-(4-((3-(6-amino-2-methyl-4-pyrimidinyl)imidazo[1,2-a]pyridin-2-yl)amino)phenyl)-1-methyl-1-(1-methylethyl)urea | 430.51 | 431.2 |
| 411 | N-(4-((3-(6-amino-2-methyl-4-pyrimidinyl)imidazo[1,2-a]pyridin-2-yl)amino)phenyl)-3-methyl-4-morpholinecarboxamide | 458.22 | 459.2 |
| 412 | 1-(4-((3-(6-amino-2-methyl-4-pyrimidinyl)imidazo[1,2-a]pyridin-2-yl)amino)phenyl)-3-cyclopropylurea | 414.19 | 415.2 |
| 413 | 1-(4-((3-(6-amino-2-methyl-4-pyrimidinyl)imidazo[1,2-a]pyridin-2-yl)amino)phenyl)-3-(1-methylethyl)urea | 416.21 | 417.0 |
| 414 | 3-(6-amino-2-methyl-4-pyrimidinyl)-N-(1-methyl-1H-pyrazol-3-yl)-6-(2-methyl-3-pyridinyl)imidazo[1,2-a]pyridin-2-amine | | |
| 415 | 3-((3-(6-amino-2-methyl-4-pyrimidinyl)imidazo[1,2-b]pyridazin-2-yl)amino)-N-(2-chloroethyl)-1H-pyrazole-1-carboxamide | 412.13 | 413.4 |
| 416 | 3-(6-amino-2-methyl-4-pyrimidinyl)-N-(1-(1,3-oxazol-2-yl)-1H-pyrazol-3-yl)imidazo[1,2-b]pyridazin-2-amine | 374.14 | 375.3 |
| 417 | 5-((3-(6-amino-2-methyl-4-pyrimidinyl)imidazo[1,2-b]pyridazin-2-yl)amino)-N-(1-methylethyl)-1H-pyrazole-1-carboxamide | 392.18 | 393.3 |
| 418 | 3-((3-(2-methyl-4-pyrimidinyl)imidazo[1,2-b]pyridazin-2-yl)amino)-1H-pyrazole-1-carboxamide | 335.12 | 336.3 |
| 419 | 5-((3-(2-methyl-4-pyrimidinyl)imidazo[1,2-b]pyridazin-2-yl)amino)-1H-pyrazole-1-carboxamide | 335.12 | 336.3 |
| 420 | ethyl 2-((3-(6-amino-2-methyl-4-pyrimidinyl)imidazo[1,2-b]-pyridazin-2-yl)amino)-1H-imidazole-4-carboxylate | | |
| 421 | 3-(2-methyl-4-pyrimidinyl)-6-(3-(methylsulfonyl)phenyl)-N-1H-pyrazol-3-ylimidazo[1,2-b]pyridazin-2-amine | | |
| 422 | N-(3-(6-amino-2-methyl-4-pyrimidinyl)imidazo[1,2-b]-pyridazin-2-yl)-3H-imidazo[4,5-b]pyridin-5-amine | 358.14 | 359.0 |
| 423 | N-(2-methyl-6-(2-((1-methyl-1H-pyrazol-3-yl)amino)-6-(3-(methylsulfonyl)phenyl)imidazo[1,2-a]pyridin-3-yl)-4-pyrimidinyl)acetamide | 516.17 | 517.0 |
| 424 | 3-(4-amino-6-methyl-1,3,5-triazin-2-yl)-6-(3-(ethylsulfonyl)-phenyl)-N-1H-pyrazol-3-ylimidazo[1,2-a]pyridin-2-amine | | |
| 425 | 3-(6-amino-2-methyl-4-pyrimidinyl)-6-(3,6-dihydro-2H-pyran-4-yl)-N-1H-pyrazol-3-ylimidazo[1,2-b]pyridazin-2-amine | 389.17 | 390.0 |
| 426 | N-(5-((3-(6-amino-2-methyl-4-pyrimidinyl)imidazo[1,2-b]-pyridazin-2-yl)amino)-3-pyridinyl)acetamide | 375.16 | 376.0 |
| 427 | 3-(6-amino-2-methyl-4-pyrimidinyl)-N-(2-methyl-1,3-thiazol-4-yl)imidazo[1,2-b]pyridazin-2-amine | 338.11 | 339.0 |
| 428 | 3-(6-amino-2-methyl-4-pyrimidinyl)-N-1,3-thiazol-5-ylimidazo-[1,2-b]pyridazin-2-amine | 324.09 | 325.0 |
| 429 | 1-(4-((3-(6-amino-2-methyl-4-pyrimidinyl)imidazo[1,2-a]-pyridin-2-yl)amino)phenyl)-3-(2-methylpropyl)urea | 430.22 | 431.2 |
| 430 | N-(4-((3-(6-amino-2-methyl-4-pyrimidinyl)imidazo[1,2-a]-pyridin-2-yl)amino)phenyl)-4,4-difluoro-1-piperidinecarboxamide | 478.2 | 479.2 |
| 431 | (2S)-N-(4-((3-(6-amino-2-methyl-4-pyrimidinyl)imidazo[1,2-a]pyridin-2-yl)amino)phenyl)-2-methyl-1-pyrrolidine-carboxamide | 442.22 | 443.2 |
| 432 | N-(2-methyl-6-(2-((1-methyl-1H-pyrazol-3-yl)amino)-6-(2-methyl-3-pyridinyl)imidazo[1,2-a]pyridin-3-yl)-4-pyrimidinyl)acetamide | | |
| 433 | N-(6-(6-(1-(2-methoxyethyl)-1H-pyrazol-4-yl)-2-((1-methyl-1H-pyrazol-3-yl)amino)imidazo[1,2-a]pyridin-3-yl)-2-methyl-4-pyrimidinyl)acetamide | | |
| 434 | N-(5-((3-(6-(acetylamino)-2-methyl-4-pyrimidinyl)imidazo-[1,2-b]pyridazin-2-yl)amino)-3-pyridinyl)acetamide | 417.14 | 418.0 |
| 435 | N-(2-methyl-6-(2-(1H-pyrazol-3-ylamino)-6-(5-pyrimidinyl)-imidazo[1,2-a]pyridin-3-yl)-4-pyrimidinyl)acetamide | 426.2 | 427.5 |
| 436 | 8-fluoro-3-(2-methyl-4-pyrimidinyl)-N-1H-pyrazol-3-ylimidazo-[1,2-a]pyridin-2-amine | 309.11 | 310 |
| 437 | 3-(4-amino-6-methyl-1,3,5-triazin-2-yl)-6-(4-(ethylsulfonyl)-phenyl)-N-1H-pyrazol-3-ylimidazo[1,2-a]pyridin-2-amine | | |
| 438 | 3-(2,6-dimethyl-4-pyrimidinyl)-6-(3-(methylsulfonyl)phenyl)-N-1H-pyrazol-3-ylimidazo[1,2-b]pyridazin-2-amine | | |
| 439 | N-(6-(8-fluoro-2-(1H-pyrazol-3-ylamino)imidazo[1,2-a]-pyridin-3-yl)-2-methyl-4-pyrimidinyl)acetamide | 366.14 | 367 |
| 440 | N-(3-(6-amino-2-methyl-4-pyrimidinyl)-6-(3-(methylsulfonyl)-phenyl)imidazo[1,2-b]pyridazin-2-yl)-1,3-benzenediamine | | |
| 441 | 3-((8-fluoro-3-(2-methyl-4-pyrimidinyl)imidazo[1,2-a]pyridin-2-yl)amino)-N-(1-methylethyl)-1H-pyrazole-1-carboxamide | 394.17 | 395 |
| 442 | 5-((3-(6-(acetylamino)-2-methyl-4-pyrimidinyl)-8-fluoro-imidazo[1,2-a]pyridin-2-yl)amino)-1H-pyrazole-1-carboxamide | 409.14 | 410 |
| 443 | 3-((3-(6-(acetylamino)-2-methyl-4-pyrimidinyl)-8-fluoroimidazo[1,2-a]pyridin-2-yl)amino)-1H-pyrazole-1-carboxamide | 409.14 | 410 |
| 444 | N-(2-methyl-6-(2-(1 H-pyrazol-3-ylamino)-6-(5-pyrimidinyl)-imidazo[1,2-b]pyridazin-3-yl)-4-pyrimidinyl)acetamide | 427.2 | 428.4 |
| 445 | 5-((8-fluoro-3-(2-methyl-4-pyrimidinyl)imidazo[1,2-a]pyridin-2-yl)amino)-N-(1-methylethyl)-1H-pyrazole-1-carboxamide | 394.17 | 395 |
| 446 | (2R)-N-(4-((3-(6-amino-2-methyl-4-pyrimidinyl)imidazo[1,2-b]pyridazin-2-yl)amino)phenyl)-2-methyl-4-morpholine-carboxamide | 459.21 | 460.2 |
| 447 | 3-(4-((3-(6-amino-2-methyl-4-pyrimidinyl)imidazo[1,2-b]pyridazin-2-yl)amino)phenyl)-1-methyl-1-(1-methylethyl)urea | 431.22 | 432.2 |
| 448 | (3R)-N-(4-((3-(6-amino-2-methyl-4-pyrimidinyl)imidazo[1,2-b]pyridazin-2-yl)amino)phenyl)-3-methyl-4-morpholinecarboxamide | 459.21 | 460.2 |
| 449 | (2S)-N-(4-((3-(6-amino-2-methyl-4-pyrimidinyl)imidazo[1,2-b]pyridazin-2-yl)amino)phenyl)-2-methyl-1-pyrrolidine-carboxamide | 443.22 | 444.2 |
| 450 | (3S)-N-(4-((3-(6-amino-2-methyl-4-pyrimidinyl)imidazo[1,2-b]pyridazin-2-yl)amino)phenyl)-3-methyl-1-pyrrolidine-carboxamide | 443.22 | 444.2 |
| 451 | N-(3-((3-(6-amino-2-methyl-4-pyrimidinyl)-6-(3-(methyl-sulfonyl)-phenyl)imidazo[1,2-b]pyridazin-2-yl)amino)phenyl)acetamide | | |
| 452 | N-(6-(2-((4-((cyclopropylcarbamoyl)amino)phenyl)amino)-imidazo[1,2-b]pyridazin-3-yl)-2-methyl-4-pyrimidinyl)-acetamide | 457.2 | 458.1 |
| 453 | 3-(6-amino-2-methyl-4-pyrimidinyl)-6-(5,6-dihydro-2H-pyran-3-yl)-N-1H-pyrazol-3-ylimidazo[1,2-b]pyridazin-2-amine | 389.17 | 390.3 |
| 454 | 2-(4-((3-(6-amino-2-methyl-4-pyrimidinyl)imidazo[1,2-b]-pyridazin-2-yl)amino)phenyl)-N-cyclopropylacetamide | 414.19 | 415.2 |
| 455 | 3-((3-(6-amino-2-methyl-4-pyrimidinyl)imidazo[1,2-b]-pyridazin-2-yl)amino)-N-(5-fluoro-2-(trifluoromethyl)phenyl)-1H-pyrazole-1-carboxamide | 512.14 | 513.3 |
| 456 | 6-fluoro-8-methoxy-3-(2-methyl-4-pyrimidinyl)-N-1H-pyrazol-3-ylimidazo[1,2-a]pyridin-2-amine | 339.12 | 340.2 |
| 457 | N-(3-(6-amino-2-methyl-4-pyrimidinyl)-6-(3-(methylsulfonyl)-phenyl)imidazo[1,2-b]pyridazin-2-yl)-1,4-benzenediamine | | |
| 458 | N-(4-((3-(6-amino-2-methyl-4-pyrimidinyl)-6-(3-(methylsulfonyl)-phenyl)imidazo[1,2-b]pyridazin-2-yl)amino)phenyl)acetamide | | |
| 459 | 3-(6-amino-2-methyl-4-pyrimidinyl)-N-(1-methyl-1H-pyrazol-3-yl)-6-(3-(methylsulfonyl)phenyl)imidazo[1,2-b]pyridazin-2-amine | | |
| 460 | 3-(6-amino-2-methyl-4-pyrimidinyl)-N-(1-(4,5-dihydro-1,3-oxazol-2-yl)-1H-pyrazol-3-yl)imidazo[1,2-b]pyridazin-2-amine | 376.38 | 377.2 |
| 461 | 6-fluoro-3-(2-methyl-4-pyrimidinyl)-N-1H-pyrazol-3-yl-8-(3-pyridinyloxy)imidazo[1,2-a]pyridin-2-amine | 402.14 | 403.2 |

### General Synthetic Scheme

Compounds of this invention can be made by the methods depicted in the reaction schemes shown below.

The starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds of this invention can be synthesized, and various modifications to these schemes can be made and will be suggested to one skilled in the art having referred to this disclosure. The starting materials and the intermediates, and the final products of the reaction may be isolated and purified if desired using conventional techniques, including but not limited to filtration, distillation, crystallization, chromatography and the like. Such materials may be characterized using conventional means, including physical constants and spectral data.

Unless specified to the contrary, the reactions described herein take place at atmospheric pressure over a temperature range from about -78 °C to about 150 °C, more preferably from about 0 °C to about 125 °C and most preferably at about room (or ambient) temperature, e.g., about 20 °C.

Compounds of Formula (I) can be prepared as illustrated and described in Scheme 1 below.

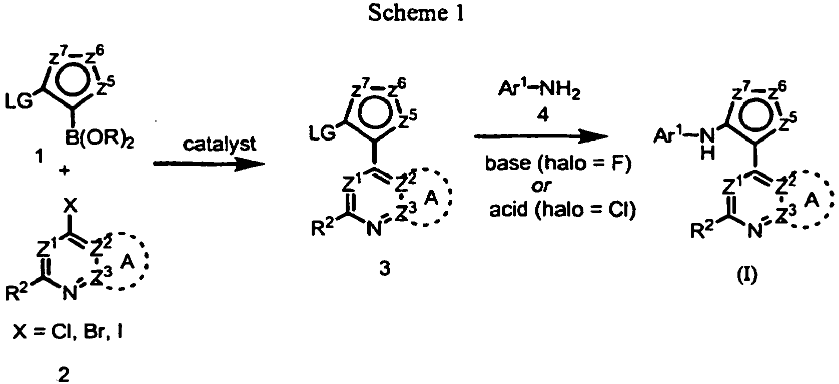

Treatment of a compound of formula 1 where LG is a leaving group such as halo, with compound of formula 2 under Suzuki coupling reaction conditions provides a compound of formula 3. The reaction is typically carried out in the presence of a transition metal catalyst (e.g., tetrakis(triphenylphosphine) palladium (0), and the like) at elevated temperature in organic solvents (e.g., ethanol, methanol, toluene, tetrahydrofuran, dioxane, and the like) and typically also requires a base (e.g., sodium carbonate, sodium acetate, and the like), and water as a co-solvent. Compounds of formula 1 and 2 are either commercially available or can be made by methods well known in the art. (please provide a list of commercially available compound within the scope and/or lit. references). Alternatively, the trialkylstannae derivative of 1 can be used instead of boronic acid derivative.

Treatment of a compound of formula 3 with an amine of formula 4 where Ar¹ is as defined in the Summary, provides a compound of Formula (I) where R¹ is hydrogen. Compounds of formula 4 are either commercially available or can be made by methods well known in the art.

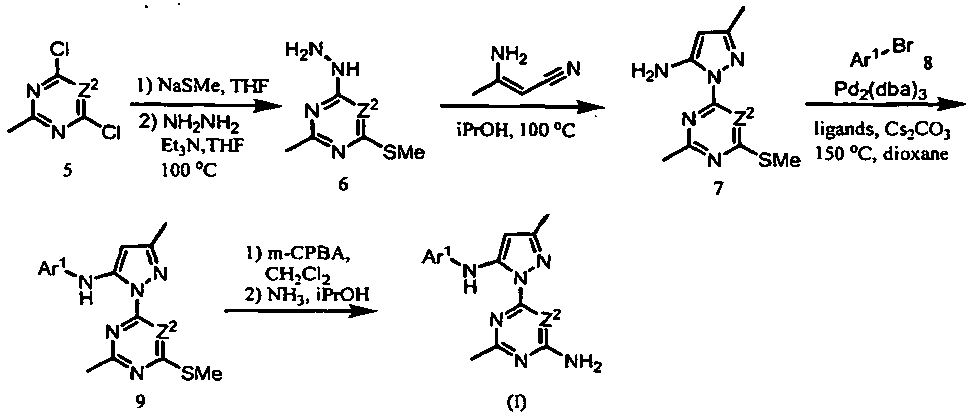

### Utility

The compounds of the present invention are useful for the treatment of P13K and/or mTOR mediated diseases and disorders such as cancer. Cancers which may be treated with compounds of the present invention include, without limitation, carcinomas such as cancer of the bladder, breast, colon, rectum, kidney, liver, lung (small cell lung cancer, and non-small- cell lung cancer), esophagus, gall-bladder, ovary, pancreas, stomach, cervix, thyroid, prostate, and skin (including squamous cell carcinoma); hematopoietic tumors of lymphoid lineage (including leukemia, acute lymphocitic leukemia, chronic lyelogenous leukemia, acute lymphoblastic leukemia, B-cell lymphoma, T-cell-lymphoma, Hodgkin's lymphoma, non-Hodgkin's lymphoma, hairy cell lymphoma and Burkett's lymphoma); hematopoietic tumors of myeloid lineage (including acute and chronic myelogenous leukemias, myelodysplastic syndrome and promyelocytic leukemia); tumors of mesenchymal origin (including fibrosarcoma and rhabdomyosarcoma, and other sarcomas, e.g., soft tissue and bone); tumors of the central and peripheral nervous system (including astrocytoma, neuroblastoma, glioma and schwannomas); and other tumors (including melanoma, seminoma, teratocarcinoma, osteosarcoma, xenoderoma pigmentosum, keratoctanthoma, thyroid follicular cancer and Kaposi's sarcoma). Other cancers that can be treated with a compound of the present invention include endometrial cancer, head and neck cancer, glioblastoma, malignant ascites, and hematopoietic cancers.

The compounds of the present invention can also be used to treat hyperproliferative disorders such as thyroid hyperplasia (especially Grave's disease), and cysts (such as hypervascularity of ovarian stroma, characteristic of polycystic ovarian syndrome (Stein-Leventhal syndrome)).

The compounds of the present invention can also be used to treat the following diseases or conditions: asthma, chronic obstructive pulmonary disease (COPD), emphysema, psoriasis, contact dermatitis, conjunctivitis, allergic rhinitis, systemic lupus erythematosus (SLE), ulcerative colitis, Crohn's disease, multiple sclerosis, rheumatoid arthritis, inflammatory bowel disease, Alzheimer's disease, atheroscleosis and Huntington's diesese.

### Administration and Pharmaceutical Composition

In addition, the compounds of the present invention can be administered alone, in combination with other compounds of the present invention, or with other pharmaceutically active compounds. The other pharmaceutically active compounds can be intended to treat the same disease or condition as the compounds of the present invention or a different disease or condition. If the patient is to receive or is receiving multiple pharmaceutically active compounds, the compounds can be administered simultaneously, or sequentially. For example, in the case of tablets, the active compounds may be found in one tablet or in separate tablets, which can be administered at once or sequentially in any order. In addition, it should be recognized that the compositions may be different forms. For example, one or more compound may be delivered via a tablet, while another is administered via injection or orally as a syrup. All combinations, delivery methods and administration sequences are contemplated.

Since one aspect of the present invention contemplates the treatment of the disease/conditions with a combination of pharmaceutically active agents that may be administered separately, the invention further relates to combining separate pharmaceutical compositions in kit form. The kit comprises two separate pharmaceutical compositions: a compound of the present invention, and a second pharmaceutical compound. The kit comprises a container for containing the separate compositions such as a divided bottle or a divided foil packet. Additional examples of containers include syringes, boxes and bags. Typically, the kit comprises directions for the use of the separate components. The kit form is particularly advantageous when the separate components are preferably administered in different dosage forms (e.g., oral and parenteral), are administered at different dosage intervals, or when titration of the individual components of the combination is desired by the prescribing physician or veterinarian.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the tablets or capsules so specified should be ingested. Another example of such a memory aid is a calendar printed on the card, e.g., as follows "First Week, Monday, Tuesday, .. etc ... Second Week, Monday, Tuesday, ..." etc. Other variations of memory aids will be readily apparent. A "daily dose" can be a single tablet or capsule or several pills or capsules to be taken on a given day. Also, a daily dose of a compound of the present invention can consist of one tablet or capsule, while a daily dose of the second compound can consist of several tablets or capsules and vice versa. The memory aid should reflect this and aid in correct administration of the active agents.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered micro-chip memory coupled with a liquid crystal readout, or audible reminder signal which, for example, reads out the date that the last daily dose has been taken and/or reminds one when the next dose is to be taken.

The compounds of the present invention and other pharmaceutically active agents, if desired, can be administered to a patient either orally, rectally, parenterally, (for example, intravenously, intramuscularly, or subcutaneously) intracisternally, intravaginally, intraperitoneally, intravesically, locally (for example, powders, ointments or drops), or as a buccal or nasal spray. All methods that are used by those skilled in the art to administer a pharmaceutically active agent are contemplated.

Compositions suitable for parenteral injection may comprise physiologically acceptable sterile aqueous or nonaqueous solutions, dispersions, suspensions, or emulsions, and sterile powders for reconstitution into sterile injectable solutions or dispersions. Examples of suitable aqueous and nonaqueous carriers, diluents, solvents, or vehicles include water, ethanol, polyols (propylene glycol, polyethylene glycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

These compositions may also contain adjuvants such as preserving, wetting, emulsifying, and dispersing agents. Microorganism contamination can be prevented by adding various antibacterial and antifungal agents, for example, parabens, chlorobutanol, phenol, sorbic acid, and the like. It may also be desirable to include isotonic agents, for example, sugars, sodium chloride, and the like. Prolonged absorption of injectable pharmaceutical compositions can be brought about by the use of agents delaying absorption, for example, aluminum monostearate and gelatin.

Solid dosage forms for oral administration include capsules, tablets, powders, and granules. In such solid dosage forms, the active compound is admixed with at least one inert customary excipient (or carrier) such as sodium citrate or dicalcium phosphate or (a) fillers or extenders, as for example, starches, lactose, sucrose, mannitol, and silicic acid; (b) binders, as for example, carboxymethylcellulose, alginates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (a) solution retarders, as for example, paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof. In the case of capsules, and tablets, the dosage forms may also comprise buffering agents.

Solid compositions of a similar type may also be used as fillers in soft and hard filled gelatin capsules using such excipients as lactose or milk sugar, as well as high molecular weight polyethylene glycols, and the like.

Solid dosage forms such as tablets, dragees, capsules, pills, and granules can be prepared with coatings and shells, such as enteric coatings and others well known in the art. They may also contain opacifying agents, and can also be of such composition that they release the active compound or compounds in a certain part of the intestinal tract in a delayed manner. Examples of embedding compositions that can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage form may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propylene glycol, 1,3-butylene glycol, dimethylformamide, oils, in particular, cottonseed oil, groundnut oil, corn germ oil, olive oil, castor oil, and sesame seed oil, glycerol, tetrahydrofurfuryl alcohol, polyethylene glycols and fatty acid esters of sorbitan, or mixtures of these substances, and the like.

Besides such inert diluents, the composition can also include adjuvants, such as wetting agents, emulsifying and suspending agents, sweetening, flavoring, and perfuming agents. Suspensions, in addition to the active compound, may contain suspending agents, as for example, ethoxylated isostearyl alcohols, polyoxyethylene sorbitol and sorbitan esters, microcrystalline cellulose, aluminum metahydroxide, bentonite, agar-agar, and tragacanth, or mixtures of these substances, and the like.

Compositions for rectal administration are preferable suppositories, which can be prepared by mixing the compounds of the present invention with suitable non-irritating excipients or carriers such as cocoa butter, polyethylene glycol or a suppository wax, which are solid at ordinary room temperature, but liquid at body temperature, and therefore, melt in the rectum or vaginal cavity and release the active component.

Dosage forms for topical administration of a compound of the present invention include ointments, powders, sprays and inhalants. The active compound or fit compounds are admixed under sterile condition with a physiologically acceptable carrier, and any preservatives, buffers, or propellants that may be required. Opthalmic formulations, eye ointments, powders, and solutions are also contemplated as being within the scope of this invention.

The compounds of the present invention can be administered to a patient at dosage levels in the range of about 0.1 to about 3,000 mg per day. For a normal adult human having a body weight of about 70 kg, a dosage in the range of about 0.01 to about 100 mg per kilogram body weight is typically sufficient. The specific dosage and dosage range that can be used depends on a number of factors, including the requirements of the patient, the severity of the condition or disease being treated, and the pharmacological activity of the compound being administered. The determination of dosage ranges and optimal dosages for a particular patient is within the ordinary skill in the art.

The compounds of Formula (I) or a pharmaceutically acceptable salt thereof, may also be administered in combination with one or more additional pharmaceutically active compounds/agents. In a particular embodiment, the additional pharmaceutically active agent is an agent that can be used to treat a cancer. For example, an additional pharmaceutically active agent can be selected from antineoplastic agents, anti-angiogenic agents, chemotherapeutic agents and peptidal cancer therapy agents. In yet another embodiment, the antineoplastic agents are selected from antibiotic-type agents, alkylating agents, antimetabolite agents, hormonal agents, immunological agents, interferon-type agents, kinase inhibitors, miscellaneous agents and combinations thereof. It is noted that the additional pharmaceutically active compounds/agents may be a traditional small organic chemical molecules or can be macromolecules such as a proteins, antibodies, peptibodies, DNA, RNA or fragments of such macromolecules.

Examples of specific pharmaceutically active agents that can be used in the treatment of cancers and that can be used in combination with one or more compound of the present invention include: methotrexate; tamoxifen; fluorouracil; 5-fluorouracil; hydroxyurea; mercaptopurine; cisplatin; carboplatin; daunorubicin; doxorubicin; etoposide; vinblastine; vincristine; paclitaxel; thioguanine; idarubicin; dactinomycin; imatinib; gemcitabine; altretamine; asparaginase; bleomycin; capecitabine; carmustine; cladibrine; cyclophosphamine; cytarabine; decarazine; docetaxel; idarubicin; ifosfamide; irinotecan; fludarabine; mitomycin; mitoxane; mitoxantrone; topotecan; vinorelbine; adriamycin; mithram; imiquimod; alemtuzmab; exemestane; bevacizumab; cetuximab; azacitidine; clofarabine; decitabine; desatinib; dexrazoxane; docetaxel; epirubicin; oxaliplatin; erlotinib; raloxifene; fulvestrant; letrozole; gefitinib; gemtuzumab; trastuzumab; gefitinib; ixabepilone; lapatinib; lenalidomide; aminolevulinic acid; temozolomide; nelarabine; sorafenib; nilotinib; pegaspargase; pemetrexed; rituximab; dasatinib; thalidomide; bexarotene; temsirolimus; bortezomib; vorinostat; capecitabine; zoledronic acid; anastrozole; sunitinib; aprepitant and nelarabine, or a pharmaceutically acceptable salt thereof.

Additional pharmaceutically active agents that can be used in the treatment of cancers and that can be used in combination with one or more compound of the present invention include: epoetin alfa; darbepoetin alfa; panitumumab; pegfilgrastim; palifermin; filgrastim; denosumab; ancestim; AMG 102; AMG 386; AMG 479; AMG 655; AMG 745; AMG 951; and AMG 706, or a pharmaceutically acceptable salt thereof.

The compounds of the present invention can also be used in combination with pharmaceutically active agents that treat nausea. Examples of agents that can be used to treat nausea include: dronabinol; granisetron; metoclopramide; ondansetron; and prochlorperazine; or a pharmaceutically acceptable salt thereof.

In addition, the compounds of the present invention can be used in combination with other agents that can be used to treat cancer such as acemannan; aclarubicin; aldesleukin; alitretinoin; amifostine; amrubicin; amsacrine; anagrelide; arglabin; arsenic trioxide; BAM 002 (Novelos); bicalutamide; broxuridine; celmoleukin; cetrorelix; cladribine; clotrimazole; DA 3030 (Dong-A); daclizumab; denileukin diftitox; deslorelin; dilazep; docosanol; doxercalciferol; doxifluridine; bromocriptine; cytarabine; HIT diclofenac; interferon alfa; tretinoin; edelfosine; edrecolomab; eflornithine; emitefur, epirubicin; epoetin beta; etoposide phosphate; exisulind; fadrozole; finasteride; fludarabine phosphate; formestane; fotemustine; gallium nitrate; gemtuzumab zogamicin; gimeracil/oteracil/tegafur combination; glycopine; goserelin; heptaplatin; human chorionic gonadotropin; human fetal alpha fetoprotein; ibandronic acid; interferon alfa; interferon alfa natural; interferon alfa-2; interferon alfa-2a; interferon alfa-2b; interferon alfa-N1; interferon alfa-n3; interferon alfacon-1; interferon alpha natural; interferon beta; interferon beta-1a; interferon beta-1b; interferon gamma natural; interferon gamma-1a; interferon gamma-1b; interleukin-1 beta; iobenguane; irsogladine; lanreotide; LC 9018 (Yakult); leflunomide; lenograstim; lentinan sulfate; letrozole; leukocyte alpha interferon; leuprorelin; levamisole + fluorouracil; liarozole; lobaplatin; lonidamine; lovastatin; masoprocol; melarsoprol; metoclopramide; mifepristone; miltefosine; mirimostim; mismatched double stranded RNA; mitoguazone; mitolactol; mitoxantrone; molgramostim; nafarelin; naloxone + pentazocine; nartograstim; nedaplatin; nilutamide; noscapine; novel erythropoiesis stimulating protein; NSC 631570 octreotide; oprelvekin; osaterone; paclitaxel; pamidronic acid; peginterferon alfa-2b; pentosan polysulfate sodium; pentostatin; picibanil; pirarubicin; rabbit antithymocyte polyclonal antibody; polyethylene glycol interferon alfa-2a; porfimer sodium; raltitrexed; rasburicase; rhenium Re 186 etidronate; RII retinamide; romurtide; samarium (153 Sm) lexidronam; sargramostim; sizofiran; sobuzoxane; sonermin; strontium-89 chloride; suramin; tasonermin; tazarotene; tegafur; temoporfin; teniposide; tetrachlorodecaoxide; thymalfasin; thyrotropin alfa; toremifene; tositumomab-iodine 131; treosulfan; tretinoin; trilostane; trimetrexate; triptorelin; tumor necrosis factor alpha natural; ubenimex; bladder cancer vaccine; Maruyama vaccine; melanoma lysate vaccine; valrubicin; verteporfin; virulizin; zinostatin stimalamer, abarelix; AE 941 (Aetema); ambamustine; antisense oligonucleotide; bcl-2 (Genta); APC 8015 (Dendreon); dexaminoglutethimide; diaziquone; EL 532 (Elan); EM 800 (Endorecherche); eniluracil; etanidazole; fenretinide; filgrastim SD01 (Amgen); galocitabine; gastrin 17 immunogen; HLA-B7 gene therapy (Vical); granulocyte macrophage colony stimulating factor, histamine dihydrochloride; ibritumomab tiuxetan; ilomastat; IM 862 (Cytran); interleukin-2; iproxifene; LDI 200 (Milkhaus); leridistim; lintuzumab; CA 125 monoclonal antibody(MAb) (Biomira); cancer MAb (Japan Pharmaceutical Development); HER-2 and Fc MAb (Medarex); idiotypic 105AD7 MAb (CRC Technology); idiotypic CEA MAb (Trilex); LYM-1-iodine 131 MAb (Techniclone); polymorphic epithelial mucin-yttrium 90 MAb (Antisoma); marimastat; menogaril; mitumomab; motexafin gadolinium; MX 6 (Galderma); nolatrexed; P 30 protein; pegvisomant; porfiromycin; prinomastat; RL 0903 (Shire); rubitecan; satraplatin; sodium phenylacetate; sparfosic acid; SRL 172 (SR Pharma); SU 5416 (SUGEN); TA 077 (Tanabe); tetrathiomolybdate; thaliblastine; thrombopoietin; tin ethyl etiopurpurin; tirapazamine; cancer vaccine (Biomira); melanoma vaccine (New York University); melanoma vaccine (Sloan Kettering Institute); melanoma oncolysate vaccine (New York Medical College); viral melanoma cell lysates vaccine (Royal Newcastle Hospital); or valspodar. It is noted that the agents recited above may also be administered as pharmaceutically acceptable salts when appropriate.

The compounds of the present invention may also be used in combination with radiation therapy, hormone therapy, surgery and immunotherapy, which therapies are well know to those skilled in the art.

### EXAMPLES

The examples presented below illustrate specific embodiments of the present invention. These examples are meant to be representative and are not intended to limit the scope of the claims in any manner. The starting materials for the specific examples below are generally available from commercial sources, unless otherwise specified. When helpful, commercial sources may be specifically indicated.

### Analytical Methods:

Unless otherwise indicated, HPLC analyses were run on an Agilent Model 1100 system with an Agilent Technologies Zorbax SB-Cg(5 µ) reverse phase column (4.6 x 150 mm) run at 30 °C with a flow rate of about 1.50 mL/min (Agilent Technologies, Santa Clara, CA). The mobile phase used solvent A (H₂O/0.1% TFA) and solvent B (ACN/0.1% TFA) with a 11 min gradient from 5% to 100% ACN. The gradient was followed by a 2 min. return to 5% ACN and about a 2.5 min. re-equilibration (flush).

### LC-MS Methods:

Unless otherwise indicated, samples were run on an Agilent model-1100 LC-MSD system with an Agilent Technologies XDB-C₈ (3.5µ) reverse phase column (4.6 x 75 mm) at 30 °C. The flow rate was constant and ranged from about 0.75 mL/min to about 1.0 mL/min. The mobile phase used a mixture of solvent A (H₂O/0.1% HCO₂H or TFA) and solvent B (ACN/0.1% HCO₂H or TFA) with a 5 to 9 min time period for a gradient from 10% to 90% solvent B. The gradient was followed by a 0.5 min period to return to 10% solvent B and a 2.5 min 10% solvent B re-equilibration (flush) of the column.

### Preparative HPLC Methods:

Where indicated, compounds of the present invention were purified via reverse phase HPLC using a Gilson (Gilson, Middleton, WI) or Shimadzu (Columbia, MD) workstation utilizing one of the following two protocols: (A) Using a 50 x 100 mm column (Waters, Exterra, C18, 5µ) (Waters, Milford, MA) at 50 mL/min. The mobile phase used was a mixture of solvent A (H₂O/10 mM ammonium carbonate at pH about 10, adjusted with conc. NH₄OH) and solvent B (85:15 ACN/water, 10 mM ammonium carbonate at pH of about 10 adjusted with conc. NH₄OH). Each purification run utilized a ≥10 min gradient from 40% to 100% solvent B followed by a 5 min flow of 100% solvent B. The gradient was followed by a 2 min return to 40% solvent B; or (B) Using a Waters 20 x 50 mm column at 20 mL/min or Phenomenex Gemni 5µ C18 100 x 30mm (Phenomenex, Torrance, CA). The mobile phase used was a mixture of solvent A (H₂O/0.1% TFA) and solvent B (ACN/0.1% TFA) with a ≥10 min gradient from 5% to 100% solvent B. The gradient is followed by a 2 min return to 5% ACN.

### Proton NMR Spectra:

Unless otherwise indicated, all ¹H NMR spectra were run on a Varian (Varian, Palo Alto, CA) series Mercury 300 MHz instrument or a Bruker (Bruker, Bilerica, MA) series 400MHz instrument. Where so characterized, all observed protons are reported as parts-per-million (ppm) downfield from tetramethylsilane (TMS) or other internal reference in the appropriate solvent indicated.

### Mass Spectra (MS)

Unless otherwise indicated, all mass spectral data for starting materials, intermediates and/or exemplary compounds are reported as mass/charge (m/z), having an (M+H⁺) or (M-H⁻) molecular ion, depending on the inonization mode (positive or negative). The molecular ion reported was obtained by electrospray detection method. Compounds having an isotopic atom, such as bromine and the like, are reported according to the detected isotopic pattern, as appreciated by those skilled in the art.

The following abbreviations may be used herein:
- Ac₂O: acetic anhydride
- ACN: acetonitrile
- A-phos: Bis[(di-tert-butylphenyl phosphine)]palladium dichloride
- aq: aqueous
- ATP: adenosine 5'-triphosphate
- Calcd or Calc'd: calculated
- Conc.: concentrated
- DCM: dichloromethane
- DMAP: dimethyl aminopyridine
- DME: dimethoxyl ethyl ether
- DMF: N,N-dimethylformamide
- DMSO: dimethyl sulfoxide
- DTT: dithiothreitol
- ESI: electrospray ionization
- Et₂O: diethyl ether
- Et₃N: triethylamine
- EtOAc: ethyl acetate
- EtOH: ethyl alcohol
- FBS: fetal bovine serum
- g: grams
- h: hour
- HCO₂H: formic acid
- Hex: hexanes
- HOAc: acetic acid
- HPLC: high pressure liquid chromatography
- IPA or iPrOH or iPr: isopropyl alcohol
- iPr₂NEt: N-ethyl diisopropylamine
- KOAc: potaisum hydroxyacetate
- LCMS, LC-MS or LC/MS: liquid chromatography mass spectroscopy
- m/z: mass divided by charge
- MeCN: acetonitrile
- MeI: iodomethane
- MeOH: methyl alcohol
- mg: milligrams
- min: minutes
- mL: milliliters
- MS: mass spectra
- MsCl: mesylchloride
- NMP: 1-methyl-2-pyrrolidinone
- NMR: nuclear magnetic resonance
- PG: protecting group
- PIP2: phosphatidylinositol bisphosphate
- Pos. ion: positive ion
- py or pyr: pyridine
- rt or RT: room temperature
- Sat.: saturated
- TFA: trifuoroacetic acid
- TFAA: trifluoroacetic anhydride
- THF: tetrahydrofuran
- TMS: tetramethylsilane
- Ts or tosyl: para-toluene sulfonyl
- TsCl: para-toluene sulfonyl chloride
- wt: Weight

Percents of solid reagents specified are percent by weight with respect to the total weight, and percents of solvents are specified by percent by volume with respect to the total volume, unless indicated otherwise.

### Example 1

### Synthesis of 6-chloro-2-methyl-9-(tetrahydro-2H-pyran-2-yl)-9H-purine

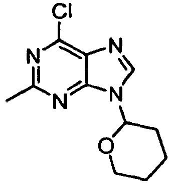

### Step 1

2-Methyl-4,6-dichloro-5-aminopyrimidine (Aldrich, 1.05 g) and ammonium hydroxide (3.0 mL, J.T. Baker, Phillipsburg, NJ, 28.0% - 30.0 %) were placed in a microwave vial. The vial was sealed and heated in a CEM microwave reactor (CEM Corporation, Matthews, NC) at 120 °C and 40 Watts for 25 minutes. The reaction mixture was cooled to room temperature. This procedure was repeated a total of nine times using the following amounts of 2-methyl-4,6-dichloro-5-aminopyrimidine under the same reaction conditions:
Run 2: 1.027 g. 2.5 mL ammonium hydroxide.
Run 3: 1.034 g, 2.5 mL ammonium hydroxide.
Run 4: 1.118 g, 2.6 mL ammonium hydroxide.
Run 5: 1.117 g, 2.5 mL ammonium hydroxide.
Run 6: 1.149 g, 2.7 mL ammonium hydroxide.
Run 7: 1.264 g, 2.6 mL ammonium hydroxide.
Run 8: 1.106 g, 2.6 mL ammonium hydroxide.
Run 9: 1.075 g, 2.7 mL ammonium hydroxide.

All the runs were combined and concentrated to give 6-chloro-2-methylpyrimidine-4,5-diamin . MS (ESI pos. ion) m/z: 159. Calcd. MS: 158.

### Step 2

6-Chloro-2-methylpyrimidine-4,5-diamine (8.89 g, 56.1 mmol, the material from Step 1) was suspended in ethyl orthoformate (100 mL, 601 mmol) in a flask fitted with a reflux condenser and placed in a preheated oil bath (100 °C) and stirred for 75 minutes. The reaction mixture was cooled to room temperature, concentrated, treated with hexanes and filtered. The solid washed with hexanes and dried to give 6-chloro-2-methyl-9H-purine. MS (ESI pos. ion) m/z: 169. Calcd: 168.

### Step 3

6-Chloro-2-methyl-9H-purine (9.45 g, 56 mmol, the material from Step 2) was suspended in DCM (100 mL) and p-toluenesulfonic acid (Acros Organics, Geel, Belgium, 12% in acetic acid, 0.90 mL, 5.6 mmol) and 2,3-dihydropyran (6.6 mL, 73 mmol) were added. The reaction flask was fitted with a reflux condenser and placed in a preheated oil bath (50 °C) and stirred under nitrogen for 30 minutes. The reaction mixture was cooled to room temperature and stirred overnight. After stirring overnight, the reaction mixture was diluted with DCM and treated with saturated sodium bicarbonate (75 mL). The layers were separated, and the aqueous phase was extracted with DCM. The organic extracts were combined, dried over sodium sulfate, filtered, concentrated, and dried under high vacuum at 45 °C (in a water bath) and then at room temperature and then at 60 °C and finally at room temperature again to afford 6-chloro-2-methyl-9-(tetrahydro-2H-pyran-2-yl)-9H-purine (13.73 g, 97% over 3 steps). MS (ESI pos. ion) m/z: 253. Calcd. MS: 252. ¹H NMR (CDCl₃, 400 MHz) δ 8.26 (s, 1H), 5.78 (d, J = 10.56 Hz, 1H), 4.19 (d, J = 11.93 Hz, 1H), 3.84 - 3.76 (m, 1H), 2.80 (s, 3H), 2.20 - 1.96 (m, 3H), 1.89 - 1.64 (m, 3H).

### Example 2

### Synthesis of 4-chloro-6-methyl-1-(tetrahydro-2H-pyran-2-yl)-1H-pyrazolo[3,4-d]pyrimidine

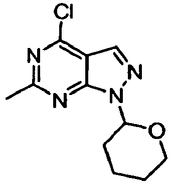

### Step 1

LDA was prepared by dropwise addition of n-butyllithium, 2.5 M solution in hexanes (Aldrich, St. Louis, MO) (14.7 mL, 36.8 mmol) to N,N-diisopropylamine (5.42 mL, 38.4 mmol) in THF (40 mL) cooled in an ice bath. The LDA solution was cooled to -78 °C and a solution of 4,6-dichloro-2-methylpyrimidine (Aldrich, St. Louis, MO) (5.448 g, 33.4 mmol) in THF (50 mL) was added dropwise over 1 h. A dark solution was obtained. A solution of N-methyl-N-(2-pyridyl)formamide (TCI Tokyo Kasei Kogyo Co., Ltd.) (4.80 mL, 40.1 mmol) in THF (20 mL) was added dropwise to the solution at -78 °C over 20 min. The resulting solution was stirred for 30 min and then quenched with a solution of acetic acid (2.10 mL, 36.8 mmol) in THF (20 mL) added dropwise at -78 °C over 10 min. The solution was stirred at -78 °C for 30 min.

The resulting solution of 4,6-dichloro-2-methylpyrimidine-5-carbaldehyde was treated dropwise with a solution of anhydrous hydrazine (1.10 mL, 35.07 mmol) at -78 °C. The reaction mixture was stirred for 15 min, and then the cooling bath was removed and the reaction mixture stirred at RT for 1 h. The reaction mixture was concentrated and partitioned between water (110 mL) and EtOAc (110 mL). The organic layer was washed with saturated aqueous NaHCO₃ (100 mL), separated, dried (MgSO₄), treated with activated charcoal and filtered through a plug of silica, washing with EtOAc. The filtrate was concentrated and purified by flash chromatography on silica eluting with 5% acetone / DCM to 25% EtOAc / hexane. The residue was suspended in DCM (3 mL), cooled in a freezer, and filtered to give 4-chloro-6-methyl-1H-pyrazolo[3,4-d]pyrimidine (330 mg, 5.86% yield) as a tan solid. ¹H NMR (400 MHz, d6-DMSO) δ 14.25 (bs, 1 H); 8.35 (s, 1 H); 2.68 (s, 3 H).

### Step 2

A suspension of 4-chloro-6-methyl-1H-pyrazolo[3,4-d]pyrimidine (325 mg, 1.93 mmol) in EtOAc (3 mL) was treated with 3,4-dihydro-2H-pyran (0.53 mL, 5.78 mmol)and MP-TsOH resin (Biotage) (72 mg, 4.3 mmol/g, 0.15 eq) and the resulting suspension was heated at 90 °C for 3 h, after which time LCMS indicated essentially complete conversion. The solution was filtered, washed with EtOAc, and concentrated to give a pale yellow oil. Purification by flash chromatography (EtOAc / hexane; 5% to 20%) gave 4-chloro-6-methyl-1-(tetrahydro-2H-pyran-2-yl)-1H-pyrazolo[3,4-d]pyrimidine (482 mg, 98.9% yield) as a colorless oil. ¹H NMR (400 MHz, d6-DMSO) 8 8.44 (s, 1 H); 5.91 - 6.02 (m, 1 H); 3.91 - 3.99 (m, 1 H); 3.67 - 3.77 (m, 1 H); 2.72 (s, 3 H); 2.36 - 2.48 (m, 1 H); 1.96 - 2.08 (m, 1 H); 1.87 - 1.95 (m, 1 H); 1.32 - 1.84 (m, 3 H).

### Example 3

### Synthesis of 2,4-cichloro-6-methyl-1,3,5-triazine

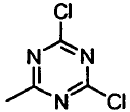

Methylmagnesium bromide, 3M in ether (Aldrich, St. Louis, MO) (10.0 mL, 30 mmol) was added slowly to a white suspension of 2,4,6-trichloro-1,3,5-triazine (Aldrich, St. Louis, MO) (3.68 g, 20 mmol) in DCM (25.0 mL, 389 mmol) at 0 °C and the resulting yellow suspension was warmed up to room temperature and stirring was continued until disappearance of starting material (TLC, KMnO₄ stain, 3 h). The reaction mixture was carefully quenched with NH₄Cl(aq) at 0 °C and then diluted with water and DCM (25.0 mL). The separated organic layer was dried, filtered and concentrated to give 2,4-dichloro-6-methyl-1,3,5-triazine as a yellow solid (2.94 g, 90%) which was used for further reaction without purification. ¹H-NMR (CDCl₃, 400 MHz) δ 2.74 (s, 3 H).

### Example 4

### Synthesis of 2-chloro-4-methyl-6-(methylthio)-1,3,5-triazine

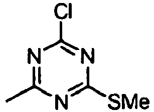

Sodium methanethiolate (0.49 g, 7.0 mmol) was added portionwise at 0 °C to a stirred cloudy solution of 2,4-dichloro-6-methyl-1,3,5-triazine (1.04 g, 6.3 mmol) in toluene (10 mL, 94 mmol)over 15 min. After addition, the pale yellow mixture was stirred at the same temperature for another 1 h, and water (10 mL) was added. The separated aqueous layer was extracted with EtOAc and the combined organic layers were washed with brine, dried over Na₂SO₄, and concentrated to give the crude residue which was purified with flash column chromatography (hexanes to 70 % DCM in hexanes) to give 2-chloro-4-methyl-6-(methylthio)-1,3,5-triazine (0.87 g, 78% yield) as a white solid. MS (API-ES) *m*/*z* 176 (M+H)⁺; ¹H NMR (d6-DMSO, 400 MHz) δ 2.55 (s, 3 H) 2.51 (br. s., 3 H).

### Example 5

### Synthesis of 4-chloro-6-methyl-1,3,5-triazin-2-amine

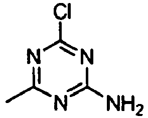

A solution of ammonia, 2.0M methyl alcohol (Aldrich, St. Louis, MO) (36.0 mL, 72 mmol) was added dropwise at room temperature (slightly exothermic) to a stirred yellow suspension of 2,4-dichloro-6-methyl-1,3,5-triazine (2.9373 g, 18 mmol) in toluene (20.0 mL, 188 mmol) over 1.5 h. The resulting mixture was stirred for an additional 2.5 h, concentrated and purified (ISCO, DCM το 10 % MeOH in DCM) to give the desired product 4-chloro-6-methyl-1,3,5-triazin-2-amine (1.88 g, 73%) as a yellow solid. MS (API-ES) *m*/*z* 145 (M+H)⁺; ¹H NMR (CD₃OD, 300 MHz) δ 2.32 (s, 3H).

### Example 6

### Synthesis of 6-chloro-2-methylpyrimidin-4-amine

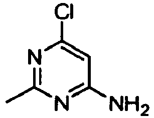

Ammonia, 2.0 M in methyl alcohol (6.0 mL, 12 mmol) was added to a stirred yellow suspension of 4,6-dichloro-2-methylpyrimidine (Aldrich) (0.487 g, 3 mmol) in 1,4-dioxane (10.0 mL) at room temperature. The resulting mixture was sealed and stirred at 70 °C overnight. After cooling, the reaction mixture was concentrated and the crude residue was dissolved in DCM/MeOH and mixed with SiO₂. The solvent was evaporated and the residue was purified by flash column chromatography (pure DCM το 10% MeOH in DCM) to give 6-chloro-2-methylpyrimidin-4-amine (0.25 g, 58%) as a white solid. MS (API-ES) *m*/*z* 144 (M+H)⁺.

### Example 7

### Synthesis of 6-chloro-5-fluoro-2-methylpyrimidin-4-amine

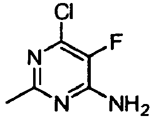

A mixture of 4,6-dichloro-5-fluoro-2-methylpyrimidine (1.55 g, 8.6 mmol) in aqueous ammonium hydroxide (10.00 mL, 90 mmol) and MeOH (1.00 mL, 25 mmol) was heated at 70 °C for 2 h (sealed tube). After cooling, 10 mL water was added and stirred for 30 min. The solid was isolated, washed with water, and dried to give the desired product 6-chloro-5-fluoro-2-methylpyrimidin-4-amine (0.9244 g, 67%) as a white solid. MS (API-ES) *m*/*z* 163 (M+H)⁺.

### Example 8

### Synthesis of 2-methyl-4-(methylthio)-6-(tributylstannyl)pyrimidine

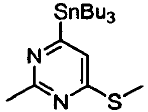

### Step 1

n-Butyllithium solution, 1.6 M in hexane (0.184 mL, 2.200 mmol, Aldrich, St. Louis, MO) was added to a solution of diisopropylamine (0.314 mL, 2.200 mmol) in THF (5 mL) at 0 °C. The reaction mixture was stirred at 0 °C for 15 min. Tributyltin hydride (0.527 mL, 2.000 mmol, Aldrich, St. Louis, MO) was added dropwisely. The solution was stirred at 0 °C for 15 min. The reaction mixture was cooled down to -78 °C, 4,6-dichloro-2-methylpyrimidine (326 mg, 2.000 mmol, Aldrich, St. Louis, MO) in THF (2 mL) was then added and the mixture was stirred at -78 °C for 8 h. The reaction mixture was quenched by saturated aqueous KF (4 mL), and extracted with EtOAc (30 mL). The organic extract was washed with saturated NaCl (5 mL) and dried over Na₂SO₄. The solution was filtered and concentrated in vacuo to give 4-chloro-2-methyl-6-(tributylstannyl)pyrimidine as a light-yellow oil. The crude material was used directly in the next step without purification.

### Step 2

Sodium thiomethoxide (140 mg, 2 mmol) was added to 4-chloro-2-methyl-6-(tributylstannyl)pyrimidine (835 mg, 2 mmol) in tetrahydrofuran (10 mL). The reaction mixture was stirred at room temperature for 48 h. The reaction mixture was diluted with water (20 mL) and extracted with EtOAc. The organic extract was washed with saturated NaCl and dried over Na₂SO₄. The solution was filtered and concentrated in vacuo to give the crude material as an orange oil. The crude product was purified by silica gel chromatography, eluting with 5% EtOAc/hexanes to give 2-methyl-4-(methylthio)-6-(tributylstannyl)-pyrimidine (256 mg, 30 % yield). ¹H NMR (300 MHz, CDCl₃) δ 7.07 (s, 1 H); 2.62 (s, 3 H); 2.51 (s, 3 H); 1.44 - 1.70 (m, 6 H); 1.21 - 1.42 (m, 6 H); 0.98 - 1.21 (m, 6 H); 0.91 (t, 9 H).

### Example 9

### Synthesis of 4-chloro-N,N-bis(4-methoxybenzyl)-6-methyl-1,3,5-triazin-2-amine

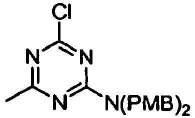

Cesium carbonate (0.860 mL, 10.74 mmol) was added to a mixture of 4-chloro-6-methyl-1,3,5-triazin-2-amine (1.10 g, 7.61 mmol) and 1-(chloromethyl)4-methoxybenzene (1.10 mL, 8.11 mmol) in DMF (8.0 mL) at rt. After 40 min, more 1-(chloromethyl)-4-methoxybenzene (1.10 mL, 8.11 mmol) was added. After another 1 h, more cesium carbonate (0.860 mL, 10.74 mmol) was added. After another 30 min, the reaction mixture was diluted with EtOAc (30 mL) and filtered through a pad of Celite® (diatomaceous earth). The filtrate was transferred to a separatory funnel, diluted with more EtOAc, and washed with water. The organic layer was dried over Na₂SO₄ and concentrated. The resulting slurry was filtered and washed with 1:1 hexane-EtOAc. The filtrate was purified by chromatography on silica using 5 to 100% DCM in hexane to give the product as a soft white solid (1.8 g). LCMS (ES, pos.): cacld for C₂₀H₂₁ClN₄O₂: 384.1; found: 385.1 (M+H)⁺. ¹H NMR (400 MHz, CDCl₃) δ 7.16 (t, J=8.12 Hz, 4 H); 6.81 - 6.95 (m, 4 H); 4.74 (s, 2 H); 4.69 (s, 2 H); 3.81 (s, 6 H); 2.45 (s, 3 H).

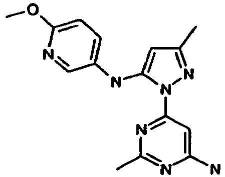

### Example 13

### Synthesis of 3-(3-(2-methyl-9H-purin-6-yl)imidazo[1,2-a]pyridin-2-ylamino)phenol

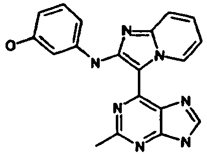

### Step 1

Triethylamine (21.1 mL, 151 mmol) was added dropwise to a mixture of chloroacetic acid (13.0 g, 138 mmol, Alfa Aesar, Ward Hill, MA) and water (35 mL). After 10 minutes, 2-aminopyridine (15.5 g, 165 mmol, Fluka, Buchs, Switzerland) was added. The reaction mixture was heated at 90 °C. After 2 h, the solution was allowed to cool to rt. EtOH (6 mL) was added and the mixture was stirred at 5 °C for 30 min. The solution was filtered and the filtercake was washed with cold EtOH and then dried under vacuum to give a white solid (19.7 g). The white solid was added to a 500-mL round-bottomed flask and suspended in toluene (140 mL). The mixture was heated to reflux and then phosphorus oxychloride (32.4 mL, 348 mmol) was added slowly over 45 min. The reaction mixture was stirred at reflux for 17 h. It was allowed to cool to rt and slowly poured into ice water (300 mL) over 15 min and then stirred for 15 min. The phases were separated and the aqueous phase was cooled to 0 °C and neutralized with 1 N NaOH and 10 N NaOH. The precipitate that formed was filtered and the filtercake was washed with water and then dried by passing air through the filter frit to afford the title compound (12.78 g, 61 %) as a pale yellow solid. *m*/*z* (ESI, +ve ion) 153 (M+H)⁺. ¹H NMR (400 MHz, d6-DMSO) δ 8.50 (d, J = 6.8 Hz, 1 H); 8.05 (s, 1 H); 7.53 (d, J = 9.2 Hz, 1 H); 7.30 - 7.35 (m, 1 H); 6.98 (t, J = 6.8 Hz, 1 H).

### Step 2

A glass microwave reaction vessel was charged with 2-chloroimidazo[1,2-a]pyridine (0.850 g, 5.57 mmol), 1,4-dioxane (8.5 mL), ethanol (4.25 mL), 6-chloro-2-methyl-9-(tetrahydro-2H-pyran-2-yl)-9H-purine (2.11 g, 8.36 mmol), potassium carbonate (1.54 g, 11.14 mmol), triphenylphosphine (0.073 g, 0.279 mmol) and palladium(II) acetate (0.125 g, 0.557 mmol, Strem). The reaction mixture was stirred and heated in an Emrys Optmizer microwave reactor (Personal Chemistry, Biotage AB, Inc., Upssala, Sweden) at 130 °C for 60 min. The mixture was poured into water (200 mL) and extracted with EtOAc (3 x 100 mL). Purification by silica gel chromatography (50 to 100% EtOAc/hexane) followed by elution with 2.0 to 5.0% MeOH (2 M in NH₃)/CH₂Cl₂ gave 6-(2-chloroimidazo[1,2-a]pyridin-3-yl)-2-methyl-9H-purine with the THP protecting group intact. This material was dissolved in 10% MeOH/CH₂Cl₂ and loaded onto Si-TsOH (4 grams, Silicycle, SiliCycle Inc., Quebec City, Canda). The Si-TsOH plug was eluted with 10% MeOH/CH₂Cl₂ and then the cartridge was eluted with 10% MeOH(2 M NH₃)/CH₂Cl₂ to elute off the product. The 10% MeOH(2 M NH₃)/CH₂Cl₂ eluent was concentrated to give 6-(2-chloroimidazo[1,2-a]pyridin-3-yl)-2-methyl-9H-purine (0.24 g, 15 %) as an off-white solid. *m*/*z* (ESI, +ve ion) 285 (M+H)⁺. ¹H NMR (400 MHz, d6-DMSO) δ 13.47 (br s, 1 H); 8.90 (d, J = 6.8 Hz, 1 H); 8.56 (br s, 1 H); 7.72 (d, J = 9.0 Hz, 1 H); 7.49 - 7.58 (m, 1 H); 7.12 (t, J = 6.7 Hz, 1 H); 2.76 (s, 3 H).

### Step 3

A mixture of 6-(2-chloroimidazo[1,2-a]pyridin-3-yl)-2-methyl-9H-purine (0.0700 g, 0.246 mmol), 3-aminophenol (0.161 g, 1.48 mmol), ethanol (1.3 mL) and one drop of conc. HCl was heated for 4 h at 155 °C in a microwave reactor. The solution was diluted with MeOH and DMSO to make it homogeneous and was purified by Prep-HPLC (Phenomenex Gemini 5 micron, C18, 100 x 30 mm, 5 to 50% CH₃CN(0.1% TFA)/H₂O(0.1% TFA) over 20 min then 100% CH₃CN(0.1% TFA) for 3 minutes at 20 mL/min) with the fractions containing product concentrated to give the title compound (0.039 g, 44 %) as an orange solid. *m*/*z* (ESI, +ve ion) 358 (M+H)⁺. ¹H NMR (400 MHz, d6-DMSO) δ 13.53 (br s, 1 H); 12.78 (s, 1 H); 10.52 (d, J=7.0 Hz, 1 H); 9.28 (s, 1 H); 8.62 (s, 1 H); 7.49 - 7.61 (m, 3 H); 7.21 (d, J=8.8 Hz, 1 H); 7.07 - 7.15 (m, 2 H); 6.37 (dd, J=7.9, 1.9 Hz, 1 H); 2.74 (s, 3 H).

### Example 14

### Synthesis of N-(3-(2-methyl-9H-purin-6-yl)imidazo[1,2-a]pyridin-2-yl)-1H-indazol-4-amine

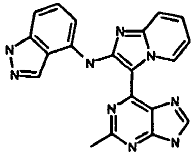

A mixture of 6-(2-chloroimidazo[1,2-a]pyridin-3-yl)-2-methyl-9H-purine (0.0730 g, 0.256 mmol), 1H-indazol-4-amine (0.205 g, 1.538 mmol), ethanol (1.25 mL) and conc HCl (1drop) was heated at 155 °C for 9 h in a microwave reactor. The reaction mixture was poured into aq. NaHCO₃ and was extracted with 25% iPrOH/CHCl₃. The combined extracts were washed with brine, dried (Na₂SO₄) and concentrated onto silica. Purification by silica gel chromatography (2.5 to 95% MeOH (2 M in NH₃)/CH₂Cl₂) gave a dark solid that was mainly product. Further purification by Prep-HPLC (Phenomenex Gemini 5 micron, C18, 100 x 30 mm, 5 to 55% CH₃CN(0.1% TFA)/H₂O(0.1% TFA) over 20 min then 100% CH₃CN(0.1% TFA) for 3 minutes at 20 mL/min) gave N-(3-(2-methyl-9H-purin-6-yl)imidazo[1,2-a]pyridin-2-yl)-1H-indazol-4-amine 2,2,2-trifluoroacetate (0.0186 g, 0.049 mmol, 19.02 % yield) as an orange solid. *m*/*z* (ESI, +ve ion) 382.0 (M+H)⁺.

### Example 15

### Synthesis of 3-(4-amino-6-methyl-1,3,5-triazin-2-yl)-N-(1H-pyrazol-3-yl)imidazo[1,2-a]pyridin-2-amine

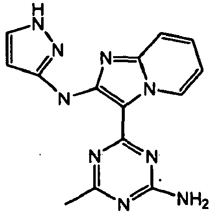

### Step 1

In a 20 mL microwave sealed tube under N₂ were dissolved 2,4-dichloro-6-methyl-1,3,5-triazine (1612 mg, 9.83 mmol), 2-chloroimidazo[1,2-a]pyridine (750 mg, 4.92 mmol), palladium(II) acetate (110 mg, 0.492 mmol), triphenylphosphine (258 mg, 0.983 mmol) and potassium carbonate (1359 mg, 9.83 mmol) in 10 mL of *p*-dioxane then stirred and heated at 150 °C with microwave for 5h. After 5h, the reaction mixture based on LC-MS showed the desired compound. The reaction mixture was diluted with DCM then neutralized with H₂O. The aqueous phase was extracted with DCM:MeOH (90:10) then the organic layer was dried over Na₂SO₄, filtered and concentrated under reduced pressure. The crude mixture was purified by MPLC (ISCO) with Hexanes:AcOEt 100:0 to 0:100 to afforded 2-chloro-3-(4-chloro-6-methyl-1,3,5-triazin-2-yl)imidazo[1,2-a]pyridine (750 mg, 2.68 mmol, 54.5 % yield) as a solid. *m*/*z* (ESI, +ve ion) 280.2 (M+H)⁺.

### Step 2

In a 25-mL round bottom flask under N₂ were dissolved 2-chloro-3-(4-chloro-6-methyl-1,3,5-triazin-2-yl)imidazo[1,2-a]pyridine (750 mg, 2.68 mmol) in ammonia in MeOH (9M) (8.92 mL, 80 mmol) and stirred at rt. After 30 min the reaction mixture based on LC-MS showed conversion to the desired compound. The crude mixture was concentrated under reduced pressure to afforded 4-(2-chloroimidazo[1,2-a]pyridin-3-yl)-6-methyl-1,3,5-triazin-2-amine (750 mg, 2.88 mmol, 107 % yield) as a solid. *m*/*z* (ESI, +ve ion) 261.0 (M+H)⁺.

### Step 3

In a 10-mL sealed tube under N₂ were dissolved 4-(2-chloroimidazo[1,2-a]pyridin-3-yl)-6-methyl-1,3,5-triazin-2-amine (750 mg, 2.88 mmol), 1H-pyrazol-3-amine (478 mg, 5.75 mmol) and TsOHH₂O (821 mg, 4.32 mmol) in 5 mL of 2-BuOH then stirred and heated at 150 °C. After 5h the reaction mixture based on LC-MS showed conversion to the desired compound. The reaction mixture was diluted with MeOH and freebased with K₂CO₃. Silica was added to the mixture and concentrated under reduced pressure. The crude mixture was purified by MPLC (ISCO) with DCM:MeOH+NH₄OH 100:0 to 90:10 to afforded 3-(4-amino-6-methyl-1,3,5-triazin-2-yl)-N-(1H-pyrazol-3-yl)imidazo[1,2-a]pyridin-2-amine (75 mg, 0.244 mmol, 8.48 % yield) as a off-white solid *m*/*z* (ESI, +ve ion) 308.0(M+H)⁺.

An alternative procedure for step 3 is found in step 5 of Example 27.

### Example 16

### Synthesis of 3-(4-methyl-6-(methylamino)-1,3,5-triazin-2-yl)-N-(1H-pyrazol-3-yl)imidazo[1,2-a]pyridin-2-amine

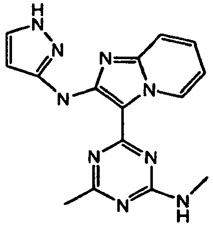

Prepared according to preparation of 3-(4-amino-6-methyl-1,3,5-triazin-2-yl)-N-(I H-pyrazol-3-yl)imidazo[1,2-a]pyridin-2-amine using MeNH₂ (2N in MeOH) in the Step 2.
*m*/*z* (ESI, +ve ion) 322.2 (M+H)⁺

### Example 17

### Synthesis of 3-(4-amino-6-methyl-1,3,5-triazin-2-yl)-7-chloro-N-(1H-pyrazol-3-yl)imidazo[1,2-a]pyridin-2-amine

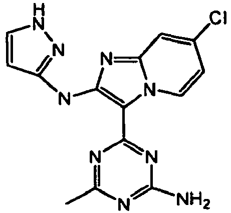

### Step 1

2,7-Cichloroimidazo[1,2-a]pyridine was prepared according to preparation of 2-chloroimidazo[1,2-a]pyridine in Example 15. 3-(4-amino-6-methyl-1,3,5-triazin-2-yl)-N-(1H-pyrazol-3-yl)imidazo[1,2-a]pyridin-2-amine using 4-chloropyridin-2-amine.
*m*/*z* (ESI, +ve ion) 187.0 (M+H)⁺

### Step 2

2,7-Dichloro-3-(4-chloro-6-methyl-1,3,5-triazin-2-yl)imidazo[1,2-a]pyridine (prepared according Org. Lett. 2009, 11, 1837-1840), was dissolved 2,7-dichloroimidazo[1,2-a]pyridine (0.6 g, 3.21 mmol) in THF (2.67 mL) and added a solution of (2,2,6,6-tetramethylpiperidin-1-yl)zinc(II) lithium chloride (11.46 mL, 4.81 mmol) dropwise and let stir for 30 min at rt under nitrogen. In a separated flask, dissolved Pd(PPh₃)₄ (0.371 g, 0.321 mmol) and 2,4-dichloro-6-methyl-1,3,5-triazine (0.789 g, 4.81 mmol) in THF (2.67 mL) under nitrogen and then transferred with cannula dropwise into the zinc reagent and let stir 16 hrs at rt until reaction was completed. The reaction mixture was neutralized with NH₄Cl and extracted with EtOAc, dried over Na₂SO₄ and filtered. The reaction mixture was concentrated under reduced and the crude mixture was purified by MPLC (ISCO) with Hexanes:AcOEt 100:0 to 0:100 to afforded 2,7-dichloro-3-(4-chloro-6-methyl-1,3,5-triazin-2-yl)imidazo[1,2-a]pyridine (120 mg, 0.381 mmol, 11.89 % yield) as a brown solid. *m*/*z* (ESI, +ve ion) 314.0 (M+H)⁺.

### Step 3

4-(2,7-Dichloroimidazo[1,2-a]pyridin-3-y)-6-methyl-1,3,5-triazin-2-amine was prepared according to preparation of 3-(4-amino-6-methyl-1,3,5-triazin-2-yl)-N-(1H-pyrazol-3-yl)imidazo[1,2-a]pyridin-2-amine Example 15 in step 3. *m*/*z* (ESI, +ve ion) 295.0 (M+H)⁺

### Step 4

In a 2 mL microwave sealed tube under N₂ were dissolved cesium carbonate (397 mg, 1.220 mmol), 1H-pyrazol-3-amine (101 mg, 1.220 mmol), and 4-(2,7-dichloroimidazo[1,2-a]pyridin-3-yl)-6-methyl-1,3,5-triazin-2-amine (120 mg, 0.407 mmol) in DMSO (407 µL) and heated to 100 °C. After 1h the reaction mixture based on LC-MS showed conversion to the desired compound. The reaction was filtered and directly purified via Gilson reverse phase (0.1% TFA in CH₃CN/H₂O, gradient 10% to 70%) and purified by MPLC (ISCO) with DCM:MeOH+NH₄OH 100:0 to 90:10 to afforded after freebasing with SCX column 3-(4-amino-6-methyl-1,3,5-triazin-2-yl)-7-chloro-N-(1H-pyrazol-3-yl)imidazo[1,2-a]pyridin-2-amine (2.0 mg, 5.85 µmol, 1.5 % yield). *m*/*z* (ESI, +ve ion) 342.0 (M+H)⁺

### Example 18

### Synthesis of 3-(6-amino-2-methylpyrimidin-4-yl)-N-(1H-pyrazol-3-yl)imidazo[1,2-b]pyridazin-2-amine

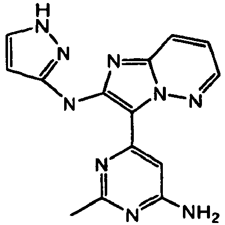

### Step 1

In a 250-mL round bottom flask under N₂ were dissolved 2-chloroacetic acid (2.94 g, 31.1 mmol), pyridazin-3-amine hydrochloride (4.09 g, 31.1 mmol) and triethylamine (12.58 mL, 93 mmol) in 50 mL of MeCN then stirred and heated at 80 °C. After 1h the reaction mixture was concentrated under reduced pressure and the crude 2-(6-iminopyridazin-1(6H)-yl)acetic acid was used without further purification in the next step.

### Step 2

In a 250-mL round bottom flask under N₂ was dissolved the crude 2-(6-iminopyridazin-1(6H)-yl)acetic acid and phosphorus oxychloride (14.49 mL, 155 mmol) in 50 mL of 1,2-dichlorobenze then stirred and heated at 100°C. After 2 h, the reaction mixture based on LC-MS showed conversion to the desired compound. The reaction mixture was diluted with DCM then neutralized with ice/H₂O. The aqueous phase was extracted with DCM then the organic layer was dried over Na₂SO₄, filtered and concentrated under reduced pressure. The crude mixture was purified by MPLC (ISCO) with Hexanes first to remove the 1,2-dichlorobenzene and then Hexanes:AcOEt 100:0 to 0:100 to afforded 2-chloroimidazo[1,2-b]pyridazine (740 mg, 4.82 mmol, 15.50 % yield) as a white solid. *m*/*z* (ESI, +ve ion) 150.2 (M+H)⁺.

### Step 3

In a 20 mL microwave sealed tube under N₂ were dissolved 4,6-dichloro-2-methylpyrimidine (1.974 g, 12.11 mmol), 2-chloroimidazo[1,2-b]pyridazine (620 mg, 4.04 mmol), triphenylphosphine (212 mg, 0.807 mmol), palladium(II) acetate (91 mg, 0.404 mmol) and potassium carbonate (1.674 g, 12.11 mmol) in 8 mL of *p*-dioxane then stirred and heated at 130 °C. After 5 h, the reaction mixture was concentrated under reduced pressure with silica and the crude mixture was purified by MPLC (ISCO) with Hexanes:AcOEt 100:0 to 0:100 to afforded 2-chloro-3-(6-chloro-2-methylpyrimidin-4-yl)imidazo[1,2-b]pyridazine (1.1 g, 3.93 mmol, 97 % yield) contaminated with PPh₃ as a yellow solid. *m*/*z* (ESI, +ve ion) 280.0 (M+H)⁺.

### Step 4

In a 20 mL microwave sealed tube under N₂ was dissolved 2-chloro-3-(6-chloro-2-methylpyrimidin-4-yl)imidazo[1,2-b]pyridazine (1.1 g, 3.93 mmol) in ammonia (9 M in MeOH) (10.0 mL, 70.0 mmol) then stirred and heated at 130 °C with microwave. After 1 h, the reaction mixture was cooled at 0°C and then the solid filtered to afforded 6-(2-chloroimidazo[1,2-b]pyridazin-3-yl)-2-methylpyrimidin-4-amine (380 mg, 1.458 mmol, 37.1 % yield) as a white solid. *m*/*z* (ESI, +ve ion) 261.2(M+H)⁺.

### Step 5

In a 10 mL microwave sealed tube under N₂ were dissolved 1 H-pyrazol-3-amine (319 mg, 3.84 mmol), 6-(2-chloroimidazo[1,2-b]pyridazin-3-yl)-2-methylpyrimidin-4-amine (200 mg, 0.767 mmol), Xantphos (89 mg, 0.153 mmol), Pd(dba)₂(88 mg, 0.153 mmol) and cesium carbonate (1500 mg, 4.60 mmol) in 3 mL of *p*-dioxane then stirred and heated at 130 °C with microwave. After 10 h, the reaction mixture was concentrated under reduced pressure with silica and purified by MPLC (ISCO) with DCM:MeOH+NH₄OH 100:0 to 90:10 and then reverse phase purified with Gilson to afforded after freebasing with SCX column 3-(6-amino-2-methylpyrimidin-4-yl)-N-(1H-pyrazol-3-yl)imidazo[1,2-b]pyridazin-2-amine as a yellow solid. *mlz* (ESI, +ve ion) 308.1 (M+H)⁺.

An alternative procedure is described in step 5 of Example 27.

### Example 23

### Synthesis of 3-(2-methyl-9H-purin-6-yl)-N-(1H-pyrazol-5-yl)imidazo[1,2-a]pyridin-2-amine

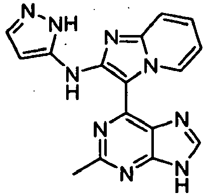

A glass microwave reaction vessel was charged with 6-(2-chloroimidazo[1,2-a]pyridin-3-yl)-2-methyl-9-(tetrahydro-2H-pyran-2-yl)-9H-purine (0.222 g, 0.602 mmol, from Example 13, Step 2) and 2-butanol (6 mL). To this mixture was added 3-aminopyrazole (250 mg, 3.01 mmol) followed by p-toluenesulfonic acid (282 mg, 1.806 mmol). The resulting slurry was stirred and heated with microwave irradiation (Biotage initiator) at 130 °C for 10 h. The mixture was poured into saturated aqueous NaHCO₃ (20 mL) and extracted with EtOAc (1x10 mL). The layers were separated and the aqueous layer was further extracted with CH₂Cl₂ (2x20 mL). The combined organic layers were dried (Na₂SO₄) and concentrated *in vacuo.* The residue was taken up in minimal MeOH/DMSO and purified by preparative HPLC (Gilson: 10-80% (0.1% TFA in CH₃CN) in H₂O over 15 min) to yield 3-(2-methyl-9H-purin-6-yl)-N-(1H-pyrazol-5-yl)imidazo[1,2-a]pyridin-2-amine as the 2,2,2-trifluoroacetate salt (8.0 mg, 0.018 mmol, 2.98 % yield). *m*/*z* (ESI, +ve ion) 332.0 (M+H)⁺. ¹H NMR (400 MHz, d6-DMSO) δ ppm 2.80 (s, 3 H); 6.75 (d, *J*=2.15 Hz, 1 H); 7.26 (td, *J*=6.92, 1.42 Hz, 1 H); 7.57 - 7.83 (m, 4 H); 8.65 (s, 1 H); 10.55 - 10.74 (m, 1 H); 13.33 (br. s., 1 H); 13.66 (br.s., 1 H).

### Example 24

### Synthesis of 3-(4-amino-6-methyl-1,3,5-triazin-2-yl)-N-(6-methoxypyridin-3-yl)imidazo[1,2-a]pyridin-2-amine

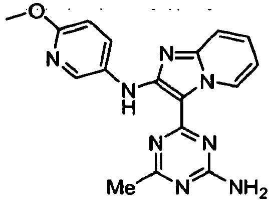

### Step 1

To a sealable vial was added 2-chloroimidazo[1,2-a]pyridine (0.30 g, 1.97 mmol), chloro[2-(dicyclohexylphosphino)-3,6-dimethoxy-2'-4'-6'-tri-i-propyl-1,1'-biphenyl][2-(2-aminoethyl)phenyl]palladium(II) (0.016 g, 0.020 mmol, Strem), and sodium tert-butoxide (0.472 g, 4.92 mmol). The mixture was carefully evacuated and backfilled with N₂. This was repeated twice. To the mixture was added 1,4-dioxane (4.0 mL) and 5-amino-2-methoxypyridine (0.25 mL, 2.36 mmol, Aldrich). The mixture was carefully evacuated and backfilled with N₂. This was repeated twice. The mixture was heated and stirred at 90 °C for 1 h and then was poured into aq. NaHCO₃ and extracted with EtOAc (3 x 100 mL). The combined extracts were washed with brine, dried (Na₂SO₄) and concentrated onto silica. Purification by silica gel chromatography (1.0 to 6.0% MeOH (2 M in NH₃)/CH₂Cl₂) afforded N-(6-methoxypyridin-3-yl)imidazo[1,2-a]pyridin-2-amine as a green solid (0.363 g, 1.51 mmol, 77% yield). MS (ESI positive ion) m/z: 241 (M+1).

### Step 2

To a 0.5-2 mL Personal Chemistry microwave vial was added was added N-(6-methoxypyridin-3-yl)imidazo[1,2-a]pyridin-2-amine (0.075 g, 0.31 mmol), 4-chloro-N,N-bis(4-methoxybenzyl)-6-methyl-1,3,5-triazin-2-amine (0.18 g, 0.47 mmol), potassium carbonate (0.086 g, 0.62 mmol), triphenylphosphine (0.016 g, 0.062 mmol), palladium (II) acetate (7.0 mg, 0.031 mmol) and 1,4-dioxane (1.0 mL). The vial was sealed and carefully evacuated and backfilled with N₂. This was repeated twice. The reaction mixture was heated at 140 °C for 3 h in the microwave. The reaction mixture was poured into aq. NaHCO₃ and extracted with EtOAc. The combined extracts were washed with brine and then dried (Na₂SO₄) and concentrated onto silica. Purification by silica gel chromatography (0.0 to 4.5% MeOH (2 M in NH₃)/CH₂Cl₂) afforded 3-(4-(bis(4-methoxybenzyl)amino)-6-methyl-1,3,5-triazin-2-yl)-N-(6-methoxypyridin-3-yl)imidazo[1,2-a]pyridin-2-amine (0.156 g, 0.265 mmol, 85% yield). MS (ESI positive ion) m/z: 589 (M+1).

### Step 3

To a sealable vial was added 3-(4-(bis(4-methoxybenzyl)amino)-6-methyl-1,3,5-triazin-2-yl)-N-(6-methoxypyridin-3-yl)imidazo[1,2-a]pyridin-2-amine (0.075 g, 0.1-3 mmol), and a solution of trifluoromethanesulfonic acid (0.040 mL, 0.450 mmol) in trifluoroacetic acid (2.00 mL, 26.0 mmol) (∼2% solution by volume). The solution was stirred at room temperature for 3 h. The reaction mixture was concentrated to about half the volume by flushing with a stream of nitrogen. The solution was then carefully pippetted into sat NaHCO₃ that had been cooled in an ice-water bath. The pH of the solution was confirmed to be near neutral. A precipitate had formed and the solution was centrifuged and the liquid was pipetted off. The precipitate was washed with water and then dried under vacuum. It was then washed with EtOAc and then dried under vacuum to afford 3-(4-amino-6-methyl-1,3,5-triazin-2-yl)-N-(6-methoxypyridin-3-yl)imidazo[1,2-a]pyridin-2-amine (0.042 g, 0.121 mmol, 95 % yield) as a light yellow solid. MS (ESI positive ion) m/z: 349 (M+1). ¹H NMR (400 MHz, DMSO-*d*₆) δ ppm 2.41 (s, 3 H), 3.85 (s, 3 H), 6.85 (d, J = 9.0 Hz, 1 H), 7.11 (t, *J* = 6.6 Hz, I H), 7.40 (br s, I H), 7.50 (t, J = 7.8 Hz, 1 H), 7.55-7.65 (m, 2 H), 8.36 (d, *J* = 7.8 Hz, 1 H), 8.71 (s, I H), 9.98 (d, J = 6.7 Hz, 1 H), 10.32 (br s, 1 H).

### Example 25

### Synthesis of 3-(4-amino-6-methyl-1,3,5-triazin-2-yl)-N-(5-fluoro-6-methoxypyridin-3-yl)imidazo[1,2-a]pyridin-2-amine.

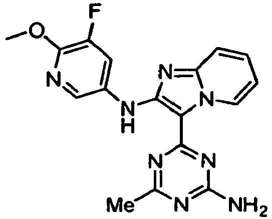

### Step 1

To a 10-20 mL Personal Chemistry microwave vial was added was added 2-chloroimidazo[1,2-a]pyridine (0.50 g, 3.3 mmol), 4-chloro-N,N-bis(4-methoxybenzyl)-6-methyl-1,3,5-triazin-2-amine (1.89 g, 4.92 mmol), potassium carbonate (0.906 g, 6.55 mmol), triphenylphosphine (0.172 g, 0.655 mmol), palladium (II) acetate (0.074 g, 0.33 mmol) and 1,4-dioxane (10 mL). The vial was sealed and carefully evacuated and backfilled with N₂. This was repeated twice. The reaction mixture was heated at 140 °C for 3 h in the microwave. The reaction mixture was poured into aq. NaHCO₃ (100 mL) and was extracted with EtOAc. The combined extracts were washed with brine and then dried (Na₂SO₄) and concentrated onto silica. Purification by silica gel chromatography (5.0 to 50% EtOAc/hexane) afforded 4-(2-chloroimidazo[1,2-a]pyridin-3-yl)-N,N-bis(4-methoxybenzyl)-6-methyl-1,3,5-triazin-2-amine ( (0.67 g, 1.33 mmol, 41 % yield). MS (ESI positive ion) m/z: 501 (M+1).

### Step 2

To a sealable vial was added 4-(2-chloroimidazo[1,2-alpyridin-3-yl)-N,N-bis(4-methoxybenzyl)-6-methyl-1,3,5-triazin-2-amine (0.125 g, 0.250 mmol), chloro[2-(dicyclohexylphosphino)-3,6-dimethoxy-2'-4'-6'-tri-i-propyl-1,1'-biphenyl][2-(2-aminoethyl)phenyl]palladium(II) (1.99 mg, 2.50 µmol, Strem), and sodium *tert*-butoxide (0.060 g, 0.624 mmol). The mixture was carefully evacuated and backfilled with N₂. This was repeated twice. To the mixture was added 1,4-dioxane (1.0 mL) and 5-fluoro-6-methoxypyridin-3-amine (0.043 g, 0.30 mmol, Anichem, LLC, North Brunswick, NJ). The mixture was carefully evacuated and backfilled with N₂. This was repeated twice. The reaction mixture was heated and stirred at 90 °C for 6 h and then was poured into aq. NaHCO₃ and extracted with EtOAc. The combined extracts were washed with brine, dried (Na₂SO₄) and concentrated onto silica. Purification by silica gel chromatography (5.0 to 30% EtOAc/hexane) afforded 3-(4-(bis(4-methoxybenzyl)amino)-6-methyl-1,3,5-triazin-2-yl)-N-(5-fluoro-6-methoxypyridin-3-yl)imidazo[1,2-a]pyridin-2-amine (0.065 g, 43 % yield) as a yellow solid. MS (ESI positive ion) m/z: 607 (M+1).

### Step 3

To a sealable vial was added 3-(4-(bis(4-methoxybenzyl)amino)-6-methyl-1,3,5-triazin-2-yl)-N-(5-fluoro-6-methoxypyridin-3-yl)imidazo[1,2-a]pyridin-2-amine (0.060 g, 0.099 mmol) and a solution of trifluoromethanesulfonic acid (0.040 mL, 0.450 mmol) in trifluoroacetic acid (2.00 mL, 26.0 mmol) (∼2% TfOH in TFA by volume). The solution was stirred at rt for 3 h. The reaction mixture was carefully pippetted into sat. aq NaHC03 (30 mL) and the pH of the solution was confirmed to be neutral. The solution was centrifuged and the supernatant liquid was pippetted off. The solid was washed with water and then dried under vacuum. The solid was washed with 1:1 EtOAc/hexane (2 x) and then dried under vacuum to afford 3-(4-amino-6-methyl-1,3,5-triazin-2-yl)-N-(5-fluoro-6-methoxypyridin-3-yl)imidazo[1,2-a]pyridin-2-amine as a white solid (0.031 g, 86% yield). MS (ESI positive ion) m/z: 367 (M+1). ¹H NMR (400 MHz, DMSO-*d*₆) δ ppm 2.42 (s, 3 H), 3.94 (s, 3 H), 7.13 (t,J = 6.7 Hz, 1 H), 7.44 (br s, 1 H), 7.52 (t, *J=* 7.6 Hz, 1 H), 7.60-7.69 (m, 2 H), 8.50 (dd, *J* = 12.8, 1.9 Hz, 1 H), 8.56 (br s, 1 *H), 9.97 (d, J= 6.8 Hz,* 1 H), 10.46 (br s, 1 H).

### Example 26

### Synthesis of 3-(4-amino-6-methyl-1,3,5-triazin-2-yl)-6-fluoro-N-(6-methoxypyridin-3-yl)imidazo[1,2-a]pyridin-2-amine

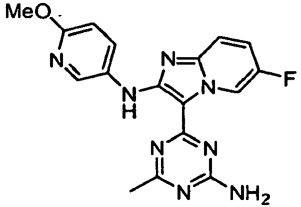

### Step 1

To a 100-mL round-bottomed flask was added 2-amino-5-fluoropyridine (1.08 g, 9.62 mmol) (Sigma-Aldrich, St. Louis, MO) and ethyl bromoacetate (3.73 mL, 33.7 mmol) (Sigma-Aldrich, St. Louis, MO). The reaction mixture was stirred at room temperature for 16 h. To the thick suspension was added ether (20 mL) and the resulting white precipitate was filtered off, washed with ether, and dried in vacuum. Ethyl 2-(5-fluoro-2-iminopyridin-1(2H)-yl)acetate hydrobromide (1.9 g, 6.81 mmol) was obtained as a white solid. MS (ESI positive ion) m/z: 199.1 (M+1).

### Stop 2

To a 25-mL, round-bottomed flask was added ethyl 2-(5-fluoro-2-iminopyridin-1(2H)-yl)acetate hydrobromide (1.92 g, 6.88 mmol) and phosphorus oxychloride (6.30 mL, 68.8 mmol). The reaction mixture was heated at 110 °C for 3 h, allowed to cool to room temperature, and the solvent was eliminated under vacuum. To the greenish syrup obtained was added ice water and NH₄OH carefully until the pH of the solution was made basic. The solution was extracted with DCM and the combined organic extracts were dried over Na₂SO₄. The solution was filtered and concentrated in vacuum to give the crude material that was absorbed onto a plug of silica gel and purified by chromatography through a Redi-Sep pre-packed silica gel column (40 g), eluting with a gradient of 30 to 40 % EtOAc in hexane. 2-Chloro-6-fluoroimidazo[1,2-a]pyridine was obtained as a white solid (0.85 g, 4.99 mmol).

### Step 3

A glass microwave reaction vessel was charged with 2-chloro-6-fluoroimidazo[1,2-a]pyridine (0.85 g, 4.99 mmol), triphenylphosphine (0.26 g" 1.0 mmol), palladium diacetate (112 mg, 0.50 mmol), potassium carbonate (0.14 g, 9.99 mmol) and 4-chloro-N,N-bis(4-methoxybenzyl)-6-methyl-1,3,5-triazin-2-amine (2691 mg, 6.99 mmol) in dioxane (15 mL). The vial was sealed and purged with argon for several minutes. The reaction mixture was stirred and heated in an Emrys Optmizer microwave reactor (Personal Chemistry, Biotage AB, Inc., Upssala, Sweden) at 140 °C for 3 h. The reaction mixture was diluted with satd. NaHCO₃ (10 mL) and extracted with DCM. The combined organic extracts were dried over Na₂SO₄. The solution was filtered and concentrated in vacuum to give the crude material. The crude material was absorbed onto a plug of silica gel and purified by chromatography through a Redi-Sep pre-packed silica gel column (40 g), eluting with a gradient of 20 % to 50 % EtOAc in hexane, to provide 4-(2-chloro-6-fluoroimidazo[1,2-a]pyridin-3-yl)-N,N-bis(4-methoxybenzyl)-6-methyl-1,3,5-triazin-2-amine (0.66g, 1.28 mmol) as white solid. MS (ESI positive ion) m/z: 518.8 (M+).

### Step 4

To a 10-mL vial was added 4-(2-chloro-6-fluoroimidazo[1,2-a]pyridin-3-yl)-N,N-bis(4-methoxybenzyl)-6-methyl-1,3,5-triazin-2-amine (139 mg, 0.27 mmol), 6-methoxypyridin-3-amine (40 mg, 0.32 mmol) (Aldrich, St Louis, MO), sodium 2-methylpropan-2-olate (64 mg, 0.67 mmol), chloro[2-(dicyclohexylphosphino)-3,6-dimethoxy-2'-4'-6'-tri-i-propyl-1,1'-biphenyl][2-(2-aminoethyl)phenyl]palladium(II) (Strem, Newburyport, MA) (2 mg, 2.7 µmol), and dioxanc (1.5 mL). The vial was sealed and purged with argon for several minutes and stirred at 90 °C for 6 h. The reaction mixture was diluted with satd NaHCO₃ (5 mL) and extracted with EtOAc. The combined organic extracts were dried over Na₂SO₄, and the solution was filtered and concentrated in vacuo to give the crude material. The crude material was absorbed onto a plug of silica gel and purified by chromatography through a Redi-Sep pre-packed silica gel column (12 g), eluting with a gradient of 20 % to 40 % EtOAc in hexane, to provide 3-(4-(bis(4-methoxybenzyl)amino)-6-methyl-1,3,5-triazin-2-yl)-6-fluoro-N-(6-methoxypyridin-3-yl)imidazo[1,2-a]pyridin-2-amine (47 mg, 0.08 mmol) as yellow solid. MS (ESI positive ion) m/z: 606.8 (M+).

### Step 5

To a 25-mL round-bottomed flask was added 3-(4-(bis(4-methoxybenzyl)amino)-6-methyl-1,3,5-triazin-2-yl)-6-fluoro-N-(6-methoxypyridin-3-yl)imidazo[1,2-a]pyridin-2-amine (47 mg, 0.08 mmol) and 2% triflic acid in TFA (1 mL). The reaction mixture was stirred at room temperature for 5 h, and the solvents were eliminated under vacuum. The residue was dissolved in DCM and washed with satd NaHCO₃ solutionand dried over Na₂SO₄. The solution was filtered and concentrated in vacuo to give the crude material, which was triturated with ether and filtered off. 3-(4-Amino-6-methyl-1,3,5-triazin-2-yl)-6-fluoro-N-(6-methoxypyridin-3-yl)imidazo[1,2-a]pyridin-2-amine was obtained as a yellow solid (12 mg, 0.03 mmol). MS (ESI positive ion) m/z: 367.0 (M+1). 1H NMR (400 MHz, DMSO-*d*₆) δ ppm 2.43 (s, 3 H) 3.84 (s, 3 H) 6.86 (d, *J*=8.41 Hz, 1 H) 7.49 (s, 1 H) 7.53 - 7.67 (m, 2 H) 7.74 (s, 1 H) 8.32 (d, J = 7.82 Hz, 1 H) 8.68 (br. s., 1 H) 10.04 (br. s., 1 H) 10.24 (br. s., 1 H).

### Example 27

### Synthesis of 3-(4-amino-6-methyl-1,3,5-triazin-2-yl)-7-methoxy-N-(1H-pyrazol-3-yl)imidazo[1,2-a]pyridin-2-amine

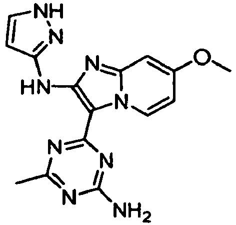

### Step 1

Dissolved 2-chloroacetic acid (1.269 g, 13.43 mmol) in acetonitrile (2.238 mL) and then added triethylamine (2.81 mL, 20.14 mmol) at rt. 4-methoxypyridin-2-amine (2.0 g, 16.11 mmol) was added and the reation mixture was and heated to 90 °C for 2 hrs until reaction was complete. After concentration, the crude product 2-(2-imino-4-methoxypyridin-1(2H)-yl)acetic acid (2.40 g, 13.2 mmol, 98% yield) was used in the next step. *m*/*z* (ESI, +ve ion) 183.2 (M+H)⁺.

### Step 2

In a 50 mL round bottom flask, dissolved 2-(2-imino-4-methoxypyridin-1(2H)-yl)acetic acid (2.44 g, 13.39 mmol) in toluene (13.39 mL) and added phosphoryl trichloride (3.68 mL, 40.2 mmol) and the reaction mixture was stirred and heated to 110 °C for 16 hrs, until the reaction was complete based on LCMS. The reaction mixture was transferred dropwise to a flask with water and ice then stirred for 30 min. The phases were seperated, organic layer discarded, and the aqueous phase was neutralized with 6 N NaOH while in an ice-water bath. The aqueous layer was extracted with DCM, and the organic layer was dried over Na₂SO₄, filtered then concentrated under rotary evaporation. Purification by MPLC (ISCO) with a gradient DCM:MeOH + NH₄OH 100:0 to 90:10 afforded 2-chloro-7-methoxyimidazo[1,2-a]pyridine (250 mg, 1.369 mmol, 10.22 % yield) as a tan solid. *m*/*z* (ESI, +ve ion) 183.0 (M+H)⁺.

### Step 3

Zinc(II) chloride (4.50 g, 33.0 mmol) was flammed dried under vacuum and then dissolved in THF (30.0 mL) to make a 1 M solution. In a separate dried 250 mL round bottom flask under nitrogen, dissolved 2,2,6,6-tetramethylpiperidine (4.24 g, 30 mmol) in dry THF (30.0 mL), and then cooled it to -40 °C. n-Bbutyllithium (12.00 mL, 30.0 mmol) was added dropwise, and allowed the reaction mixture to warm to -10 °C over 1 hr. Zinc(II) chloride (4.50 g, 33.0 mmol) solution was added dropwise, and stirring was continued for 30 min at -10 °C, then 30 min at rt. This solution in THF (0.42 M) was used for coupling reactions and was stored under nitrogen.

Dissolved 2-chloro-7-methoxyimidazo[1,2-a]pyridine (210 mg, 1.150 mmol) in THF (958 µL) and added the solution of (2,2,6,6-tetramethylpiperidin-1-yl)zinc(II) lithium chloride (4107 µL, 1.725 mmol) (0.42 M) dropwise and let stir for 30 min at rt under nitrogen. In a separate flask, dissolved palladium tetrakis (133 mg, 0.115 mmol) and 2,4-dichloro-6-methyl-1,3,5-triazine (283 mg, 1.725 mmol) in more THF (958 µL) under nitrogen and cannulated the mixture into the -chloro-7-methoxyimidazo[1,2-a]pyridine solution. The reaction mixture was stirred for 16 hrs at rt until reaction was complete by LCMS. The reaction mixture was quenched reaction with NH₄Cl and aqueous layer was extracted with EtOAc, dried organics over Na₂SO₄, and concentrated under rotary evaporation. The crude product was purified via MPLC (ISCO) with a gradient hexanes:EtOAc column 0:100 to 100:0. to give 2-chloro-3-(4-chloro-6-methyl-1,3,5-triazin-2-yl)-7-methoxyimidazo[1,2-a]pyridine (150 mg, 0.484 mmol, 42.1 % yield) as a yellow solid. *m*/*z* (ESI, +ve ion) 310.0 (M+H)⁺.

### Step 4

Dissolved 2-chloro-3-(4-chloro-6-methyl-1,3,5-triazin-2-yl)-7-methoxyimidazo[1,2-a]pyridine (150 mg, 0.484 mmol) in 0.5 M ammonia in dioxane (2902 µL, 1.451 mmol) in a sealed tube and the reaction mixture was stirred for 16 hrs at rt. Concentration of the reaction mixture gave crude 4-(2-chloro-7-methoxyimidazo-[1,2-a]pyridin-3-yl)-6-methyl-1,3,5-triazin-2-amine that was used in the next step. *m*/*z* (ESI, +ve ion) 291.0 (M+H)⁺.

### Step 5

Charged a flask with Pd₂dba₃ (132 mg, 0.144 mmol) and CyP-F*t*-Bu₂ (208 mg, 0.289 mmol) and degassed, backfilling with nitrogen three times. The solids were dissolved in t-BuOH (2094 µL) and water (130 µL, 7.22 mmol) and heated to 100 °C for 15 min. The reaction mixture was cooled to rt and 1H-pyrazol-3-amine (120 mg, 1.445 mmol), 4-(2-chloro-7-methoxyimidazo[1,2-a]pyridin-3-yl)-6-methyl-1,3,5-triazin-2-amine (210 mg, 0.722 mmol) and cesium carbonate (706 mg, 2.167 mmol) were added. The reaction mixture was degassed again with nitrogen and heated at 100 °C for 4 hrs until the reaction was complete by LCMS. The reaction mixture was then rotary evaporated onto silica, and purified with MPLC (ISCO) with a gradient DCM:MeOH + NH₄OH 100:0 to 90:10 to afford 3-(4-amino-6-methyl-1,3,5-triazin-2-yl)-7-methoxy-N-(1H-pyrazol-3-yl)imidazo[1,2-a]pyridin-2-amine (30.0 mg, 88.9 µmol, 12.3% yield). *m*/*z* (ESI, +ve ion) 338.0 (M+H)⁺. ¹H NMR (400 MHz, DMSO-*d*₆) δ ppm 2.33 (s, 3 H) 3.89 (s, 3 H) 6.72 (br. s., 1 H) 6.80 (s, 1 H) 7.02 (br. s., 1 H) 7.32 (br. s., 2 H) 7.61 (s, 1 H) 9.78 (d, *J*=7.53 Hz, 1 H) 10.41 (br. s., 1 H) 12.14 (br. s., 1 H).

### Example 28

### Synthesis of N-(1-(1H-benzo[d]imidazol-2-yl)-1H-pyrazol-3-yl)-3-(6-amino-2-methylpyrimidin-4-yl)imidazo[1,2-b]pyridazin-2-amine

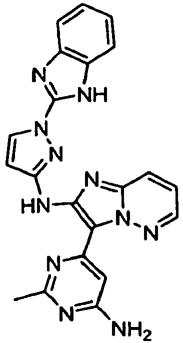

3-(6-Amino-2-methylpyrimidin-4-yl)-N-(1H-pyrazol-3-yl)imidazo[1,2-b]pyridazin-2-amine (100 mg, 0.325 mmol), p-toluenesulfonic acid (61.9 mg, 0.325 mmol), and 2-chloro-1H-benzo[d]imidazole (59.6 mg, 0.390 mmol) were dissolved in 2-butanol (651 µL) and heated in the microwave at 100 °C for 30 min until reaction was complete by LCMS. After concentration, the reaction mixture was redissolved in DMSO and purified via HPLC (Gilson) with a gradient water:acetonitrile 85:15 to 30:70 with 0.1% TFA. N-(1-(1H-benzo-[d]imidazol-2-yl)-1H-pyrazol-3-yl)-3-(6-amino-2-methylpyrimidin-4-yl)imidazo[1,2-b]pyridazin-2-amine 2,2,2-trifluoroacetate was collected directly as the TFA salt as a yellow solid. *m*/*z* (ESI, +ve ion) 424.5 (M+H)⁺. ¹H NMR (500 MHz, DMSO-*d*₆) δ ppm 2.66 (s, 3 H) 7.07 - 7.22 (m, 2 H) 7.32 (d, *J*=2.67 Hz, 1 H) 7.44 - 7.59 (m, 3 H) 7.93 (br. s., 1 H) 8.18 (dd, *J*=8.98, 1.50 Hz, 1 H) 8.56 (d, *J*=2.67 Hz, 1 H) 8.71 (dd, *J*=4.65, 1.44 Hz, 1 H) 10.87 (br. s., 1 H).

### Example 29

### Synthesis of 3-(3-(6-amino-2-methylpyrimidin-4-yl)imidazo[1,2-b]pyridazin-2-ylamino)-N-phenyl-1H-pyrazole-1-carboxamide

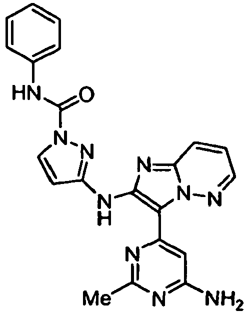

3-(6-Amino-2-methylpyrimidin-4-yl)-N-(1H-pyrazol-3-yl)imidazo[1,2-b]pyridazin-2-amine (120 mg, 0.390 mmol) was dissolved in THF (781 µL) and isocyanatobenzene (63.8 µL, 0.586 mmol) was added. The reaction mixture was stirred at rt for 2 hrs until it was complete by LCMS (two isomers on the pyrazole). The reaction mixture was concentrated, then redissolved mixture in DMSO and purified via HPLC (Gilson) with a gradient water:acetonitrile 85:15 to 10:90 with 0.1% TFA to afford 3-(3-(6-amino-2-methylpyrimidin-4-yl)imidazo[1,2-b]pyridazin-2-ylamino)-N-phenyl-1H-pyrazole-1-carboxamide (25 mg, 0.046 mmol, 12 % yield) as the TFA salt. *m*/*z* (ESI, +ve ion) 427.5 (M+H)⁺. ¹H NMR (500 MHz, DMSO-*d*₆) δ ppm 2.63 (s, 3 H) 7.16 (t, *J*=7.32 Hz, 1 H) 7.26 (d, *J*=7.91 Hz, 1 H) 7.29 (d, *J*=2.35 Hz, 1 H) 7.39 (t, *J*=7.85 Hz, 2 H) 7.44 (d, *J*=7.27 Hz, 1 H) 7.48 (dd, *J*=8.82, 4.65 Hz, 1 H) 7.74 (d, *J*=8.12 Hz, 2 H) 7.87 (br. s., 1 H) 8.09 - 8.23 (m, 1 H) 8.39 (d, *J*=2.67 Hz, 1 H) 8.63 - 8.77 (m, 1 H) 10.10 (s, 1 H) 10.99 (br. s., 1 H).

### Example 30

### Synthesis of 3-(3-(6-amino-2-methylpyrimidin-4-yl)imidazo[1,2-b]pyridazin-2-ylamino)-N-(2-methoxyethyl)-1H-pyrazole-1-carboxamide

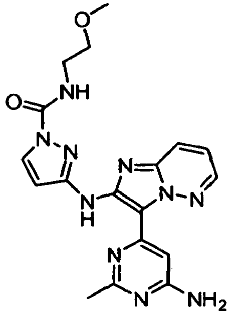

### Step 1

In a 50-mL sealed tube under N₂ were dissolved 3-(6-amino-2-methylpyrimidin-4-yl)-N-(1H-pyrazol-3-yl)imidazo[1,2-b]pyridazin-2-amine (1.20 g, 3.90 mmol), and phenyl carbonochloridate (0.539 mL, 4.30 mmol) in THF (24 mL), then added hunig'sbase (0.682 mL, 3.90 mmol). Let stir at rt for 1 hr until reaction was complete by LCMS (two isomers on the pyrazole). The crude solution of phenyl 3-(3-(6-amino-2-methylpyrimidin-4-yl)imidazo[1,2-b]pyridazin-2-ylamino)-1H-pyrazole-1-carboxylate (1.67 g, 3.9 mmol, 100% yield) was used in the next step. *m*/*z* (ESI, +ve ion) 428.4 (M+H)⁺.

### Step 2

3-(3-(6-Amino-2-methylpyrimidin-4-yl)imidazo[1,2-b]pyridazin-2-ylamino)-1H-pyrazole-1-carboxylate (133 mg, 0.311 mmol) was dissolved in THF (2 mL) (what is the second reagent??) and the reaction mixture wasstirred at rt for 1 hr until reaction was complete by LCMS (2 isomers formed). The reaction mixture was concentrated, then redissolved in DMSO and purified via HPLC (Gilson) with a gradient water:acetonitrile 90:10 to 50:50 with 0.1 % TFA to afford 3-(3-(6-amino-2-methylpyrimidin-4-yl)imidazo[1,2-b]pyridazin-2-ylamino)-N-(2-methoxyethyl)-1H-pyrazole-1-carboxamide (1.05 mg, 2.010 µmol, 1 % yield) as the TFA salt. *m*/*z* (ESI, +ve ion) 409.5 (M+H)⁺. ¹H NMR (400 MHz, METHANOL-*d*₄) δ ppm 2.75 (s, 3 H) 3.42 (s, 3 H) 3.56 - 3.64 (m, 4 H) 7.08 (d, *J*=2.84 Hz, 1 H) 7.49 (dd, *J*=9.00, 4.69 Hz, 1 H) 8.05 (dd, *J*=9.05, 1.61 Hz, 1 H) 8.18 (br. s., 1 H) 8.20 (d, *J*=2.84 Hz, 1 H) 8.63 (dd, *J*=4.60, 1.57 Hz, 1 H).

### Example 31

### Synthesis of 3-(4-amino-6-methyl-1,3,5-triazin-2-yl)-6-(2-fluorophenyl)-N-(1H-pyrazol-3-yl)imidazo[1,2-a]pyridin-2-amine 2,2,2-trifluoroacetate

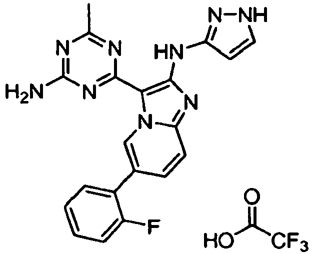

### Step 1

To a solution of 2-chloroacetic acid (1.661 mL, 24.08 mmol) and acetonitrile (4.01 mL) was added triethylamine 99% (5.02 mL, 36.1 mmol), dropwise under N₂. 2-Amino-5-bromopyridine (5.00 g, 28.9 mmol) was added as a solid in a single portion. The reaction mixture was maintained at rt for 3 min until majority of chloropyridine had dissolved and then heated at 90 °C for 60 min. The reation appeared to undergo an uncontrolled exotherm after 50 minutes at 90 °C and turned dark brown. Oil bath was removed and reaction was cooled to rt, yellow/tan solid was collected by vacuum filtration, rinsing with minimal acetonitrile. The solid was dried under reduced pressure to yield 2-(5-bromo-2-iminopyridin-1(2H)-yl)acetic acid (2.657 g, 11.50 mmol, 47.8 % yield) as a light brown solid. LCMS (trifluoroacetic acid modifier, ESI) m/z: 231.0 (M+H)⁺

### Step 2

6-Bromo-2-chloroimidazo[1,2-a]pyridine was prepared according to preparation of 2-chloroimidazo[1,2-b]pyridazine in Example 18, Step 2. LCMS (trifluoroacetic acid modifier, ESI) m/z: 231.0 (M+H)⁺

### Step 3

Added anhydrous THF (35.1 mL) to 6-bromo-2-chloroimidazo[1,2-a]pyridine (8.13 g, 35.1 mmol) under N2 atmosphere, followed by (2,2,6,6-tetramethylpiperidin-1-yl)zinc(II) lithium chloride solution in THF (122 mL, 63.2 mmol) at rt. The reaction mixture was maintained for 30 min at rt. In a separate flask combined 4,6-dichloro-2-methylpyrimidine (17.18 g, 105 mmol) and palladium tetrakis (4.06 g, 3.51 mmol) in THF (20 mL) and the slurry was to the solution containing of 6-bromo-2-chloroimidazo[1,2-a]pyridine and (2,2,6,6-tetramethylpiperidin-1-yl)zinc(II) lithium chloride at rt. After 18 h, the solution was diluted with MeOH and concentrated to generate a brown sludge. This was filtered though a buchner funnel rinsing with CH₂Cl₂ to afford pure desired product 6-bromo-2-chloro-3-(6-chloro-2-methylpyrimidin-4-yl)imidazo[1,2-a]pyridine (6.87 g, 19.19 mmol, 54.6 % yield) as an orange solid. LCMS (trifluoroacetic acid modifier, ESI) m/z: 359.1 (M+H)⁺

### Step 4

To 6-bromo-2-chloro-3-(6-chloro-2-methylpyrimidin-4-yl)imidazo[1,2-a]pyridine (2.105 g, 5.88 mmol) was added ammonia in MeOH (7N) (0.127 mL, 5.88 mmol) in a sealed vessel. Ammonia (gas) (0.127 mL, 5.88 mmol) was bubbled through solution for 1 minute and heated at 100 °C for 2 h with microwave. Solid was collected by vacuum filtration, rinsing with IPA and water, then IPA to give 6-(6-bromo-2-chloroimidazo[,2-a]pyridin-3-yl)-2-methylpyrimidin-4-amine (1.705 g, 5.04 mmol, 86 % yield) as a light yellow amorphous solid. LCMS (trifluoroacetic acid modifier, ESI) m/z: 340.0 (M+H)⁺

### Step 5

1,4-Dioxane (2.58 mL) was added to 4-(6-bromo-2-chloroimidazo[1,2-a]pyridin-3-yl)-6-methyl-1,3,5-triazin-2-amine (0.175 g, 0.515 mmol), [1,1-bis(diphenylphosphino)-ferrocene]palladium(II) dichloride dichloromethane adduct (0.042 g, 0.052 mmol), and 2-fluorobenzeneboronic acid (0.079 g, 0.567 mmol) under N₂. A solution of sodium carbonate anhydrous granular (0.086 mL, 2.061 mmol) was added as a 2M solution in water (2 mL). The reaction mixture was stirred and heated at 80 °C for 2 hr. The crude material was purified by chromatography through a Redi-Sep pre-packed silica gel column (40 g), eluting with a gradient of 0% to 10% MeOH in CH2CL2, to provide 4-(2-chloro-6-(2-fluorophenyl)imidazo[1,2-a]pyridin-3-yl)-6-methyl-1,3,5-triazin-2-amine (0.193 g, 0.544 mmol, 106 % yield) as a yellow solid. LCMS (trifluoroacetic acid modifier, ESI) m/z: 355.0 (M+H)⁺

### Step 6

3-(4-Amino-6-methyl-1,3,5-triazin-2-yl)-6-(2-fluorophenyl)-N-(1H-pyrazol-3-yl)imidazo[1,2-a]pyridin-2-amine 2,2,2-trifluoroacetate was prepared according to Example 27, step 5. The crude material was purified by reverse-phase preparative HPLC using 0.1% TFA in CH₃CN/H₂O, gradient 10% to 90% over 20 min to provide 3-(4-amino-6-methyl-1,3,5-triazin-2-yl)-6-(2-fluorophenyl)-N-(H-pyrazol-3-yl)imidazo[1,2-a]pyridin-2-amine 2,2,2-trifluoroacetate (0.028 g, 0.054 mmol, 10.71 % yield) as an orange solid. LCMS (formic acid modifier, ESI) m/z: 402.0 (M+H)^{+ 1}H NMR (400 MHz, DMSO-*d*₆) δ ppm 2.41 (s, 3 H); 6.79 (d, *J*=2.05 Hz, 1 H); 7.29 - 7.46 (m, 2 H); 7.46 - 7.62 (m, 2 H); 7.62 - 7.80 (m, 5 H); 7.83 (br. s., 1 H); 10.19 (br. s., 1 H); 10.43 (br. s., 1 H).

### Example 32

### Synthesis of 3-(6-amino-2-methylpyrimidin-4-yl)-N-(1H-pyrazol-3-yl)imidazo[1,2-a]pyridin-2-amine 2,2,2-trifluoroacetate

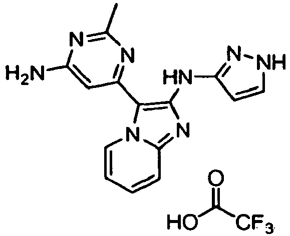

### Step 1

2-Chloro-3-(6-chloro-2-methylpyrimidin-4-yl)imidazo[1,2-a]pyridine was synthesized according to the preparation of 2-chloro-3-(6-chloro-2-methylpyrimidin-4-yl)imidazo[1,2-b]pyridazine in Example 18 step 3. LCMS (trifluoroacetic acid modifier, ESI) m/z: 279.0 (M+H)⁺

### Step 2

6-(2-Chloroimidazo[1,2-a]pyridin-3-yl)-2-methylpyrimidin-4-amine was prepared according to the preparation of 6-(6-bromo-2-chloroimidazo[1,2-a]pyridin-3-yl)-2-methylpyrimidin-4-amine in Example A, step 4. LCMS (trifluoroacetic acid modifier, ESI) m/z: 260.2 (M+H)⁺

### Step 3

3-(6-Amino-2-methylpyrimidin-4-yl)-N-(1H-pyrazol-3-yl)imidazo[1,2-a]pyridin-2-amine 2,2,2-trifluoroacetate was prepared according to procedure described in Example 27, Step 5. The crude material was purified by reverse-phase preparative HPLC using 0.1% TFA in CH₃CN/H₂O, gradient 10% to 90% over 20 min to provide 3-(6-amino-2-methyl-pyrimidin-4-yl)-N-(1H-pyrazol-3-yl)imidazo[1,2-a]pyridin-2-amine 2,2,2-trifluoroacetate (0.031 g, 0.074 mmol, 12.77 % yield) as yellow solid. LCMS (trifluoroacetic acid modifier, ESI) m/z: 307.5 (M+H)⁺. ¹H NMR (400 MHz, MeOH) δ ppm 2.68 (s, 3 H); 6.62 (br. s., 1 H); 6.89 (s, 1 H); 7.13 - 7.30 (m, 1 H); 7.66 (br. s., 3 H); 8.85 (m, 1 H).

### Example 33

### Synthesis of 2-(3-methoxyphenyl)-N-(2-methyl-6-(2-(1-methyl-1H-pyrazol-3-ylamino)imidazo[1,2-a]pyridin-3-yl)pyrimidin-4-yl)acetamide 2,2,2-trifluoroacetate

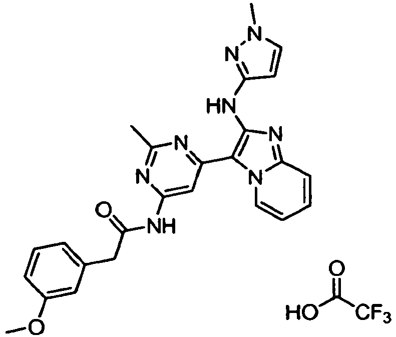

### Step 1

3-(6-Amino-2-methylpyrimidin-4-yl)-N-(1-methyl-1H-pyrazol-3-yl)imidazo[1,2-a]pyridin-2-amine was prepared according to the procedure described in Example 18, Step 5 using 1-methyl-1h-pyrazol-3-amine. LCMS (trifluoroacetic acid modifier, ESI) m/z: 321.2 (M+H)⁺

### Step 2

3-(6-Amino-2-methylpyrimidin-4-yl)-N-(1-methyl-1H-pyrazol-3-yl)imidazo[1,2-a]pyridin-2-amine (0.085 g, 0.265 mmol) and 2-(3-methoxyphenyl)acetic acid (0.057 g, 0.345 mmol) were put under N₂. Ethyl acetate (1.327 mL), followed by diisopropylethylamine (0.231 mL, 1.327 mmol) and finally 1-propanephosphonic acid cyclic anhydride, 50 wt. % solution in ethyl acetate (1.351 mL, 2.123 mmol) were added. The reaction mixture was stirred and heated to 55 °C for 2 h. The concentrated crude material was purified by chromatography through a Redi-Sep pre-packed silica gel column (40 g), eluting with a gradient of 1% to 10% MeOH in CH₂Cl₂, to provide unpure product. Purification by reverse-phase preparative HPLC, 0.1% TFA in CH₃CN/H₂O, gradient 10% to 90% over 15 min providee 2-(3-methoxyphenyl)-N-(2-methyl-6-(2-(1-methyl-1H-pyrazol-3-ylamino)-imidazo[1,2-a]pyridin-3-yl)pyrimidin-4-yl)acetamide 2,2,2-trifluoroacetate (0.047 g, 0.081 mmol, 30.4 % yield) as a yellow solid.

LCMS (trifluoroacetic acid modifier, ESI) m/z: 469.2 (M+H)⁺

¹H NMR (400 MHz, DMSO-*d*₆) δ ppm 2.62 (s, 3 H); 3.76 (m, *J*=4.60 Hz, 8 H); 6.67 (d, *J*=2.25 Hz, 1 H); 6.79 - 6.87 (m, 1 H); 6.88 - 6.97 (m, 2 H); 7.16 - 7.29 (m, 2 H); 7.49 (t, *J*=7.73 Hz, 1 H); 7.54 - 7.64 (m, 2 H); 8.30 (s, 1 H); 8.78 (d, J=6.65 Hz, 1 H); 10.40 (br. s., 1 H); 11.10 (s, 1 H).

### Example 34

### Synthesis of 3-(6-amino-2-methylpyrimidin-4-yl)-N-(1-(2-(dimethylamino)ethyl)-1H-pyrazol-3-yl)imidazo[1,2-b]pyridazin-2-amine 2,2,2-trifluoroacetate

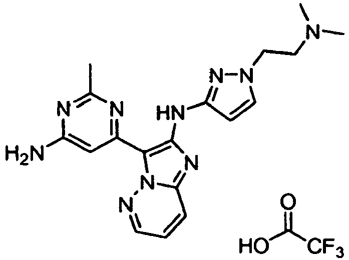

The title compound was prepared according to procedure described in of Example 27, Step 5 using 1-(2-(dimethylamino)ethyl)-1H-pyrazol-3-amine.

### Example 35

### Synthesis of 3-(6-amino-2-methylpyrimidin-4-yl)-N-(1H-pyrazol-3-yl)-6-(pyrimidin-5-yl)imidazo[1,2-b]pyridazin-2-amine

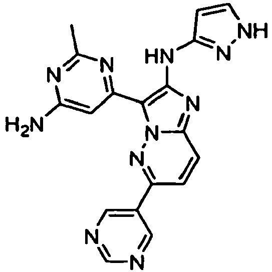

### Step 1

A sealable vial was charged with 2,6-dichloroimidazo[1,2-b]pyridazine (1.17 g, 6.22 mmol), pyrimidine-5-boronic acid (1.388 g, 11.20 mmol, Maybridge), and dichloro(1,1-bis(diphenylphosphinoferrocene))palladium(ii) dichloromethane adduct (0.356 g, 0.436 mmol, Strem). The vial was sealed with a septa cap and flushed with N₂. Dioxane (24.89 mL) was added followed by an aqueous solution of sodium carbonate (6.55 mL, 12.45 mmol). The reaction mixture was sparged with N₂ for 10 min, and then heated at 80 °C for 3 h. The reaction mixture was concentrated and purified by MPLC (Teledine Isco combiFlash Companion) as described: The crude residue was taken up in minimal CH₁Cl₂/MeOH (20 mL) and absorbed onto a minimal amount of loose silica gel, which was then packed into a 25 g custom loading cartridge and passed through a Redi-Sep® pre-packed silica gel column (80 g) using a gradient of 1% MeOH in CH₂Cl₂ to 10% MeOH in CH₂Cl₂ to afford 2-chloro-6-(pyrimidin-5-yl)imidazo[1,2-b]pyridazine (1.32 g, 5.70 mmol, 92 % yield) as a orange amorphous solid.

### Step 2

A microwave vial was charged with 2-chloro-6-(pyrimidin-5-yl)imidazo[1,2-b]pyridazine (0.366 g, 1.580 mmol), 4,6-dichloro-2-methylpyrimidine (0.567 g, 3.48 mmol, Aldrich), cesium carbonate (1.030 g, 3.16 mmol, Strem), triphenylphosphine (0.083 g, 0.316 mmol, Fluka) and palladium(ii) acetate (0.035 g, 0.158 mmol, Strem). The vial was sealed with septa cap, flushed with N₂, and dioxane was added via syringe (6.32 mL). The reaction mixture was sparged with N₂ for 10 minutes, then irradiated in the microwave (Biotage Initiator) at 130 °C for 2 h. The crude reaction mixture was absorbed directly onto a 25 g pre-packed silica loading cartridge for purification by MPLC (Teledine Isco combiFlash Companion). The residue was passed through a Redi-Sep® pre-packed silica gel column (40 g) using first a gradient of 5% EtOAc in CH₂Cl₂ to 100% EtOAc followed by a gradient of 1% MeOH in CH₂Cl₂ to 10% MeOH in CH₂Cl₂ to afford 2-chloro-3-(6-chloro-2-methylpyrimidin-4-yl)-6-(pyrimidin-5-yl)imidazo[1,2-b]pyridazine (0.380 g, 1.061 mmol, 67.1 % yield) as a tan solid.

### Step 3

A sealable microwave vial was charged with 2-chloro-3-(6-chloro-2-methylpyrimidin-4-yl)-6-(pyrimidin-5-yl)imidazo[1,2-b]pyridazine (0.380 g, 1.061 mmol) and the vial was sealed using a septa cap. 10 N ammonia in MeOH (17.10 mL, 790 mmol) was added via syringe to generate a tan slurry. Gaseous ammonia was bubbled through the slurry for 5 min, then the mixture was irradiated in the microwave (Biotage Initiator) at 100 °C for 2 h. Upon cooling to rt, a tan solid precipitated. The solid was collected by vacuum filtration to give 6-(2-chloro-6-(pyrimidin-5-yl)imidazo[1,2-b]pyridazin-3-yl)-2-methylpyrimidin-4-amine (0.2394 g, 0.707 mmol, 44.7 % yield) as a tan solid.

### Step 4

A sealable vial was charged with tris(dibenzylideneacetone)dipalladium(0) (32.4 mg, 0.035 mmol, Strem) and (r)-1-[(s)-2-(dicyclohexylphosphino)ferrocenyl]ethyl-di-tert-butylphosphine (39.3 mg, 0.071 mmol, Strem). The vial was sealed with a septa cap and flushed with N₂. *tert*-Butanol (1 mL) was added, followed by water (63.8 µL, 3.54 mmol) and the resulting mixture was sparged with N₂ for 5 min. The solution was heated at 120 °C for 10 min, and then cooled to rt.

In a separate vial was charged 6-(2-chloro-6-(pyrimidin-5-yl)imidazo[1,2-b]pyridazin-3-yl)-2-methylpyrimidin-4-amine (120.0 mg, 0.354 mmol), cesium carbonate (404 mg, 1.240 mmol, Alfa Aesar), and 3-aminopyrazole (147 µL, 1.771 mmol, Alfa Aesar). The vial was sealed with a septa cap and flushed with N₂. *tert*-Butanol (1771 µL) was added, was added and the reaction mixture was sparged with N₂ for 5 mins. The catalyst solution was transferred via syringe to the vial that contained the coupling substrates. The resulting mixture was heated at 100 °C for 18 h. The crude reaction mixture was absorbed directly onto a 25 g loading cartridge for purification by MPLC (Teledine Isco combiFlash Companion). The residue was passed through a Redi-Sep® pre-packed silica gel column (40 g) using a gradient of 0.1% NH₄OH and 1% MeOH in CH₂Cl₂ to 1% NH₄OH and 10% MeOH in CH₂Cl₂ to afford the title compound 3-(6-amino-2-methylpyrimidin-4-yl)-N-(1H-pyrazol-3-yl)-6-(pyrimidin-5-yl)imidazo[1,2-b]pyridazin-2-amine (100.0 mg, 0.259 mmol, 73.3 % yield) as a orange amorphous solid. 1 H NMR (500 MHz, DMSO-*d*₆) δ ppm 2.46 (s, 3 H) 6.82 - 6.88 (m, 1 H) 7.12 (br. s, 2 H) 7.62 - 7.65 (m, 1 H) 7.70 (s, 1 H) 7.98 - 8.06 (m, 1 H) 8.12- 8.19 (m, 1 H) 9.36 (s, 1 H) 9.59 (s, 2 H) 11.28 (s, 1 H) 12.17 (s, 1 H); LRMS *m*/*z* calcd = 385.2, observed (M +H) = 386.4.

### Example 35

### Synthesis of N-(6-(2-(1H-pyrazol-3-ylamino)-6-(pyrimidin-5-yl)imidazo[1,2-a]pyridin-3-yl)-2-methylpyrimidin-4-yl)acetamide

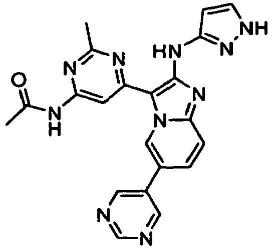

### Step 1

To a vial charged with 3-(6-amino-2-methylpyrimidin-4-yl)-N-(1H-pyrazol-3-yl)-6-(pyrimidin-5-yl)imidazo[1,2-a]pyridin-2-amine (prepared as described in Example 31) was added 1,4-dioxane (2445 µL), followed by pyridine (316 µL, 3.91 mmol, Aldrich) then acetic anhydride (231 µL, 2.445 mmol), Aldrich). The vial was sealed with a septa cap and heated at 80 °C for 2 h. Additional acetic anhydride (231 µL, 2.445 mmol) was added and the solution was heated at 80 °C for an additional 18 h. The solution was concentrated for purification by MPLC (Teledine Isco combiFlash Companion). The crude residue was taken up in minimal CH₂Cl₂ and absorbed onto a 5 g pre-packed silica loading cartridge and passed through a Redi-Sep® pre-packed silica gel column (40 g) using a gradient of 1% MeOH in CH₂Cl₂ to 10% MeOH in CH₂Cl₂ to afford N-(6-(2-(1-acetyl-1H-pyrazol-3-ylamino)-6-(pyrimidin-5-yl)imidazo[1,2-a]pyridin-3-yl)-2-methylpyrimidin-4-yl)acetamide (54.0 mg, 0.115 mmol, 23.57 % yield) as a yellow solid.

### Step 2

A sealable vial was charged with N-(6-(2-(1-acetyl-1H-pyrazol-3-ylamino)-6-(pyrimidin-5-yl)imidazo[1,2-a]pyridin-3-yl)-2-methylpyrimidin-4-yl)acetamide (54.0 mg, 0.115 mmol) followed by CH₂Cl₂ (5 mL) and MeOH (0.5 mL) and MP-Carbonate (159 mg, 3.07 mmol/gram loading, Biotage). The reaction mixture was heated at 60 °C for 1 hour. The solution was filtered to remove the MP-Carbonate and the filtrate was concentrated for purification by MPLC (Teledine Isco combiFlash Companion). The crude residue was taken up in minimal 90/10/1 CH₂Cl₂/MeOH/NH₄OH and absorbed onto a 5 g loading cartridge and passed through a Redi-Sep® pre-packed silica gel column (12 g) using a gradient of 0.1% NH₄OH and 1% MeOH in CH₂Cl₂ to 1% NH₄OH and 10% MeOH in CH₂Cl₂ to afford N-(6-(2-(1H-pyrazol-3-ylamino)-6-(pyrimidin-5-yl)imidazo[1,2-a]pyridin-3-yl)-2-methyl-pyrimidin-4-yl)acetamide (15.0 mg, 0.035 mmol, 7.19 % yield) as a yellow amorphous solid. 1H NMR (400 MHz, DMSO-*d*₆) δ ppm 2.19 (s, 3 H) 2.62 (s, 3 H) 6.82 (br. s, 1 H) 7.65 (br. s, 1 H) 7.69 - 7.77 (m, 1 H) 7.88 - 7.97 (m, 1 H) 8.50 (s,1 H) 9.13 - 9.18 (m, 1 H) 9.25 (s, 1 H) 9.28 (s, 2 H) 10.47 (s, 1 H) 10.96 (s, 1 H) 12.22 (br. s, 1 H); LRMS *m*/*z* calcd = 426.2, observed (M +H) = 427.5.

### Example 36

### Synthesis of N-(3-(3-(6-amino-2-methylpyrimidin-4-yl)imidazo[1,2-b]pyridazin-2-ylamino)phenyl)acetamide

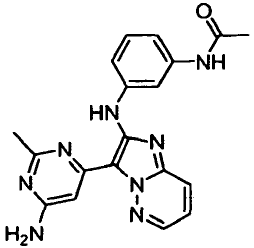

### Step 1

N1-(3-(6-amino-2-methylpyrimidin-4-yl)imidazo[1,2-b]pyridazin-2-yl)benzene-1,3-diamine was synthesized utilizing the procedure of Example 27, Step 5. m/z=333.2, cald 332.15 for C₁₇H₁₆N₈

### Step 2

To a solution of acetyl chloride (0.017 mg, 0.217 µmol) in DMF (0.453 µL) was added N1-(3-(6-amino-2-methylpyrimidin-4-yl)imidazo[1,2-b]pyridazin-2-yl)benzene-1,3-diamine (0.060 mg, 0.181 µmol) and triethylamine (0.035 µL, 0.253 µmol). The reaction mixture was shaken at rt 15 h, quenched with 100 uL MeOH, then purified directly by prepatory HPLC. ¹H NMR (500 MHz, DMSO-*d*₆) δ ppm 2.06 (s, 3 H) 6.98 (br. s., 2 H) 7.12 - 7.29 (m, 2 H) 7.35 (dd, *J*=8.93, 4.77 Hz, 1 H) 7.58 - 7.79 (m, 2 H) 7.91 (m, 1 H) 8.06 (dd, *J*=8.93, 1.22 Hz, 1 H) 8.54 - 8.72 (m, 1 H) 9.91 (s, 1 H) 11.47 (s, 1 H). m/z= 375.2, calcd 374.16 for C₁₉H₁₈N₈O.

### Example 37

### N-(5-((3-(4-amino-6-methyl-1,3,5-triazin-2-yl)imidazo[1,2-a]pyridin-2-yl)amino)-2-methoxy-3-pyridinyl)methanesulfonamide

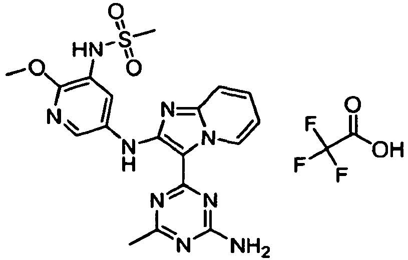

To a 2-5 mL Personal Chemistry microwave vial was added 4-(2-chloroimidazo[1,2-a]pyridin-3-yl)-6-methyl-1,3,5-triazin-2-amine (0.050 g, 0.192 mmol), N-(5-amino-2-methoxypyridin-3-yl)methanesulfonamide (0.094 g, 0.432 mmol), methanol (1.50 mL) and hydrochloric acid (conc, 5 drops). The reaction mixture was stirred and heated at 155 °C for 80 min in the microwave. The reaction mixture was diluted with DMF and purified by Prep-HPLC (Phenomenex Gemini 5 micron, C18, 100 x 30 mm, 5 to 55% CH₃CN(0.1% TFA)/H₂O(0.1% TFA) over 20 min then 100% CH₃CN(0.1% TFA) for 3 minutes at 20 ml/min) with the fractions containing suspected product concentrated to afford the title compound as a tan solid (0.011 g, 10 % yield). MS (ESI positive ion) m/z: 442 (M+1). ¹H NMR (400 MHz, DMSO-*d*₆) δ ppm 10.30 (br s, 1 H), 9.97 (d, J= 6.8 Hz, 1 H), 9.25 (s, 1 H), 8.53 (s, 1 H), 8.37 (s, 1 H), 7.77 (br s, 1 H), 7.48 - 7.60 (m, 3 H), 7.13 (t, *J =* 6.7 Hz, 1 H), 3.92 (s, 3 H), 3.09 (s, 3 H), 2.43 (s, 3 H).

### Example 38

### Synthesis of 3-(6-amino-2-methylpyrimidin-4-yl)-N-(1-(pyridin-3-ylmethyl)-1H-pyrazol-3-yl)imidazo[1,2-b]pyridazin-2-amine

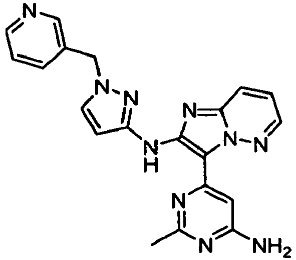

A glass reaction vessel was charged with 3-(6-(bis(4-methoxybenzyl)amino)-2-methylpyrimidin-4-yl)-N-(1H-pyrazol-3-yl)imidazo[1,2-b]pyridazin-2-amine (0.200 g, 0.365 mmol) and DMF (3 mL). The reaction vial was purged with nitrogen and sodium hydride (0.015 g, 0.621 mmol) was added. The resulting slurry was stirred at room temperature for 5 minutes, until gas evolution ceased. 3-(Bromomethyl)pyridine (0.063 g, 0.365 mmol) was added and the reaction was heated to 50 °C for 15 h. The mixture was poured into water (20 mL) and extracted with DCM. The combined organic layers were dried (MgSO₄) and concentrated *in vacuo.* The crude reaction was treated with a premixed solution of trifluoromethanesulfonic acid (6.62 mL, 74.5 mmol) in TFA (0.098 mL, 1.278 mmol). The solution was stirred at room temperature for 1 h and concentrated *in vacuo.* The residue was taken up in minimal MeOH and purified by preparative HPLC (Gilson: 10-80% (0.1% TFA in CH₃CN) in H₂O over 15 min). The combined fractions were washed with saturated NaHCO₃ and extracted with DCM (3x25 mL). The combined organic layers were dried (MgSO₄) and concentrated *in vacuo* to yield 3-(6-amino-2-methylpyrimidin-4-yl)-N-(1-(pyridin-3-ylmethyl)-1H-pyrazol-3-yl)imidazo[1,2-b]pyridazin-2-amine (37 mg, 0.093 mmol, 25.4 % yield). m/z (ESI, +ve ion) 399.3 (M+H)⁺. 1H NMR (400 MHz, DMSO-*d*₆) δ ppm 2.42 (s, 3 H) 5.32 (s, 2 H) 6.88 (d, *J*=2.25 Hz, 1 H) 6.97 (br s, 2 H) 7.32 (dd, *J*=9.00, 4.69 Hz, 2 H) 7.66 (s, 1 H) 7.80 (d, *J*=2.15 Hz, 2 H) 8.03 (dd, *J*=8.95, 1.61 Hz, 1 H) 8.49 - 8.56 (m, 2 H) 8.59 (dd, *J*=4.70, 1.56 Hz, 1 H) 11.06 (br s, 1 H)

### Example 39

### 1-(5-(3-(6-amino-2-methylpyrimidin-4-yl)imidazo[1,2-b]pyridazin-2-ylamino)pyridin-2-yl)azetidin-3-ol

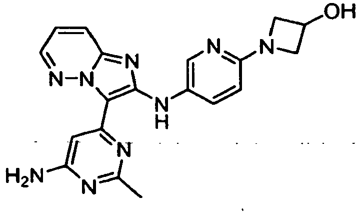

### Step I

To 2-chloro-5-nitropyridine (1.30 g, 8.20 mmol) and 3-hydroxyazetidine hydrochloride (0.923g, 8.43 mmol) was added dry DMF (5ml), and the reaction mixture was stirred in water bath at RT while triethylamine (2.349 mL, 16.85 mmol) was added dropwise. The mixture was stirred RT 3 h, then diluted EtOAc washed water, brine. Aqueous phase was further extracted with EtOAc, and the combined organics evaporated at reduced pressure. The residue was boiled with toluene then allowed to stand at RT. The resulting yellow solid 1-(5-nitropyridin-2-yl)azetidin-3-ol was collected by filtration and dried in-vacuuo

### Step 2

To 1-(5-nitropyridin-2-yl)azetidin-3-ol (0.50 g, 2.56 mmol) in ethanol (25 ml) was added ammonium formate (1.615 g, 25.6 mmol). To the reaction mixture was then added a slurry of 5% Pd/C (70 mg) in water 1ml under Ar atmosphere and the reaction mixture was stirred under Ar at rt for 45 min in a water bath. The catalyst was removed by filtration through a celite plug and washed with ethanol. Solvent was stripped from the filtrate and residue extracted from minimum of aq NaHC03 with EtOAc, and solvent stripped to afford a red oil, which was used without further purification in the next step.

### Step 3

A pressure vessel under nitrogen was charged with Pd₂(dba)₃ (0.070 g, 0.076 mmol), JosiPhos CyPF-t-Bu (0.086 g, 0.155 mmol), 1-(5-aminopyridin-2-yl)azetidin-3-ol (0.310 g, 1.877 mmol), 6-(2-chloroimidazo[1,2-b]pyridazin-3-yl)-2-methylpyrimidin-4-amine (0.200 g, 0.767 mmol), and cesium carbonate (1.25 g, 3.84 mmol) in t-BuOH (3 mL) and water (0.21 mL, 11.6 mmol). The reaction mixture was degassed with argon, the vessel sealed, then heated at 120 °C for 36 h. Solvent was then stripped at reduced pressure and the residue was chromatographed on silica gel, eluent 90:10:1 DCM MeOH: NH4OH. Fractions containing the product, eluting just above the excess aminopyridine SM were pooled and solvent stripped. The residue was crystallised from hot ethanol as an orange solid and dried in vacuuo to give 70 mg is the title compound m/z 390 MH+

### Example 40

### Synthesis of 3-(6-amino-2-methylpyrimidin-4-yl)-N-(1-isopentyl-1H-pyrazol-3-yl)imidazo[1,2-b]pyridazin-2-amine

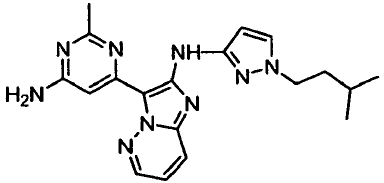

### Step 1

5-Nitro-1H-pyrazole (0.4 g, 3.54 mmol) was suspended in DMF (7.07 mL) and cooled to 0 °C under N₂. To this was added sodium hydride (60% wt in oil) (0.283 g, 7.07 mmol) portionwise and resulting mixture was stirred for 10 mins at 0 °C. 1-Bromo-3-methylbutane (1.695 mL, 14.15 mmol) was added via syringe and the mixture was warmed up to room temperature over 1 h and stirred for an additional 2 h. Water (10 mL) was added dropwise and mixture extracted with DCM. Organic layers collected, dried over sodium sulfate, concentrated and dried to completion to afford brown oil. T his was purified by MPLC (30-50% EtOAc/Hexanes) to provide 1-isopentyl-3-nitro-1H-pyrazole (400mg, 2.183 mmol, 61.7 % yield) as a tan solid. MS m/z = 184.2 [M+1]⁺. Calc'd for C₈H₁₃N₃O₂: 183.1.

### Step 2

1-Isopentyl-3-nitro-1H-pyrazole (0.37 g, 2.020 mmol) and pd/c (10% wt) (0.215 g, 0.202 mmol) under a blanket of N₂ were suspended in ethanol (26.9 mL). The reaction mixture was purged with nitrogen and a hydrogen filled-balloon was attached via an adaptor. and stirred at room temperature for 16 h. The reaction mixture was filtered through a celite cake and washed with ethanol and DCM. The filtrate was concentrated to yield 1-isopentyl-1H-pyrazol-3-amine (309mg, 2.017 mmol, 100 % yield) as a tan oil. MS m/z = 154.2 [M+1]⁺. Calc'd for C₈H₁₅N₃: 153.1.

### Step 3

3-(6-Amino-2-methylpyrimidin-4-yl)-N-(1-isopentyl-1H-pyrazol-3-yl)imidazo[1,2-b]pyridazin-2-amine was synthesized utilizing the procedure described in Example 27, Step 5.

### Example 41

### Synthesis of N-(4-((3-(6-amino-2-methyl-4-pyrimidinyl)imidazo[1,2-b]pyridazin-2-yl)amino)phenyl)-4-morpholinecarboxamide

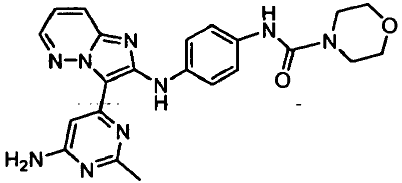

### Step 1

A glass vial was charged with benzene-1,4-diamine (1.659 g, 15.34 mmol), cesium carbonate (5.00 g, 15.34 mmol), Pd₂(dba)₃ (0.281 g, 0.307 mmol), JosiPhos (0.340 g, 0.614 mmol) and 6-(2-chloroimidazo[1,2-b]pyridazin-3-yl)-2-methylpyrimidin-4-amine (0.8 g, 3.07 mmol). The vial was sealed, evacuated and backfilled with nitrogen; this was repeated twice. *t*-BuOH (8.60 mL) and water (0.553 mL, 30.7 mmol) were added via syringe. The reaction mixture was stirred at 120 °C overnight. The crude material was purified by MPLC (Biotage), eluting with a gradient of 0% to 10% 2M NH₃·MeOH in CH₂CL₂, to provide N1-(3-(6-amino-2-methylpyrimidin-4-yl)imidazo[1,2-b]pyridazin-2-yl)benzene-1,4-diamine (0.88 g, 2.65 mmol, 86 % yield) as a brown solid. *m*/*z* (ESI, +ve ion) 333.0 (M+H)⁺.

### Step 2

To a 100 mL round-bottomed flask was added N1-(3-(6-amino-2-methylpyrimidin-4-yl)imidazo[1,2-b]pyridazin-2-yl)benzene-1,4-diamine (0.385 g, 1.158 mmol), 4-nitrophenyl carbonochloridate (0.233 g, 1.158 mmol) and N-ethyl-N-isopropylpropan-2-amine (0.202 mL, 1.158 mmol) in THF (7.72 mL). The solution was stirred at room temperature overnight. The reaction mixture was concentrated to give a brown solid. This solid and DIPEA (0.588 mL) were dissolved in DMF (7 mL) to provide a stock solution of 4-nitrophenyl 4-(3-(6-amino-2-methylpyrimidin-4-yl)imidazo[1,2-b]pyridazin-2-ylamino)phenylcarbamate, which was used immediately for the next step.

### Step 3

To a screw capped vial was added the above stock solution (1 mL) and morpholine (0.021 mL, 0.241 mmol). The reaction mixture was stirred at room temperature overnight. The crude material was purified by preparative HPLC to provide N-(4-(3-(6-amino-2-methylpyrimidin-4-yl)imidazo[1,2-b]pyridazin-2-ylamino)phenyl)morpholine-4-carboxamide as a yellow solid. *m*/*z* (ESI, +ve ion) 446.2 (M+H)⁺. ¹H NMR (500 MHz, DMSO-*d*6) δ 11.22 (s, 1H), 8.52 - 8.57 (m, 1 H), 8.37 (s, 1 H), 7.99 - 8.05 (m, 1H), 7.62 - 7.71 (m, 3H), 7.33 - 7.49 (m, 2H), 7.29 (dd, *J* = 8.93, 4.77 Hz, 1H), 6.83 - 7.01 (m, 2H), 3.57 (m, 4H), 3.36 - 3.45 (m, 4H), 2.50 (s, 3H).

### Example 42

### Synthesis of N-(6-(2-(1H-pyrazol-3-ylamino)imidazo[1,2-b]pyridazin-3-yl)-2-methylpyrimidin4-yl)acetamide

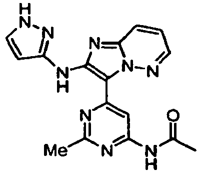

### Step 1

In a 50-mL sealed tube under N₂ were dissolved 3-(6-amino-2-methylpyrimidin-4-yl)-*N*-(1*H*-pyrazol-3-yl)imidazo[1,2-b]pyridazin-2-amine (1.028 g, 3.35 mmol) (see Example 18) and Boc-anhydride (7.77 mL, 33.5 mmol) in 5 mL of DCM and 5 mL of MeCN and the reaction mixture was stirred and heated at 80 °C. After 5 h, the reaction mixture was dissolved in a minimum of MeOH and then pass thru a conditioned Isolute ® SPE column (SCX-2) and then washed with MeOH. The compound was eluted using a 2 M Ammonia in MeOH and then the solution was concentrated under reduced pressure. The crude mixture was purified by MPLC (ISCO) with DCM:MeOH+NH₄OH 100:0 to 90:10 to afforded *tert*-butyl 3-(3-(6-amino-2-methylpyrimidin-4-yl)imidazo[1,2-b]pyridazin-2-ylamino)-1H-pyrazole-1-carboxylate (750 mg, 1.841 mmol, 55.0 % yield). *m*/*z* (ESI, +ve ion) 408.2 (M+H)⁺

### Step 2

In a 10-mL sealed tube under N₂ were dissolved *tert*-butyl 3-(3-(6-amino-2-methylpyrimidin-4-yl)imidazo[1,2-b]pyridazin-2-ylamino)-1*H*-pyrazole-1-carboxylate (200 mg, 0.491 mmol) and acetic anhydride (0.232 mL, 2.454 mmol) in 1 mL of pyridine then stirred and heated at 100 °C. After 1 h, the reaction mixture was concentrated under reduced pressure with silica and purified by MPLC (ISCO) with DCM:MeOH+NH₄OH 100:0 to 90:10 to afford *tert*-butyl 3-(3-(6-acetamido-2-methylpyrimidin-4-yl)imidazo[1,2-b]pyridazin-2-ylamino)-1H-pyrazole-1-carboxylate (20 mg, 0.057 mmol, 11.66 % yield).

### Step 3

In a 25-mL round bottom flask was *tert*-butyl 3-(3-(6-acetamido-2-methylpyrimidin-4-yl)imidazo[1,2-b]pyridazin-2-ylamino)-1*H*-pyrazole-1-carboxylate and TFA (0.945 mL, 12.27 mmol) in 1 mL of DCM then stirred at rt. After 1 h, the reaction mixture was concentrated under reduced pressure. The crude mixture was purified by Gilson reverse phase to afforded *N*-(6-(2-(1*H*-pyrazol-3-ylamino)imidazo[1,2-b]pyridazin-3-yl)-2-methylpyrimidin-4-yl)acetamide (10 mg, 0.029 mmol, 5.8 % yield) as a yellow solid. *m*/*z* (ESI, +ve ion) 350.0 (M+H)⁺.¹H NMR (400 MHz, DMSO-d₆) δ ppm 10.87 (s, 1 H) 10.80 (s, 1 H) 9.30 (s, 1 H) 8.63 (dd, *J*=4.69, 1.56 Hz, 1 H) 8.06 (dd, *J*=9.00, 1.56 Hz, 1 H) 7.65 (d, *J*=2.25 Hz, 1 H) 7.39 (dd, *J*=9.00, 4.69 Hz, 1 H) 6.84 (d, *J*=2.25 Hz, 1 H) 2.64 (s, 3 H) 2.15 (s, 3 H).

### Example 43

### Synthesis of 3-(6-amino-2-methylpyrimidin-4-yl)-6-morpholino-N-(1H-pyrazol-3-yl)imidazo[1,2-b]pyridazin-2-amine

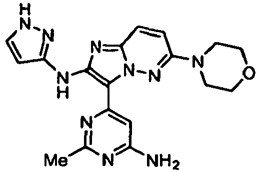

### Step 1

In a 25-mL sealed tube under N₂ was dissolved 2,6-dichloroimidazo[1,2-b]pyridazine (1.00 g, 5.32 mmol) in morpholine (2.317 mL, 26.6 mmol) and heated at 60 °C. After 2 h, the reaction mixture was diluted with water and the solid was filtered to afford 4-(2-chloroimidazo[1,2-b]pyridazin-6-yl)morpholine (1.20 g, 5.03 mmol, 95 % yield). The crude 4-(2-chloroimidazo[1,2-b]pyridazin-6-yl)morpholine (1.20 g, 5.03 mmol, 95 % yield) was used without further purification in the next step. *m*/*z* (ESI, +ve ion) 239.1 (M+H)⁺. 1H NMR (400 MHz, DMSO-*d*₆) δ ppm 10.49 (br. s., 1 H) 7.90 (d, *J*=9.78 Hz, 1 H) 7.62 (s, 2 H) 7.36 (d, *J*=9.49 Hz, 1 H) 6.71 - 6.82 (m, 1 H) 3.78 - 3.87 (m, 4 H) 3.44 - 3.63 (m, 4 H) 2.57 (s, 3 H)

### Step 2 to 4

The title compound was synthesized according the procedures described in Example 18, Step 3 to 5 using 4-(2-chloroimidazo[1,2-b]pyridazin-6-yl)morpholine. *m*/*z* (ESI, +ve ion) 393.2 (M+H)⁺. NMR

### Example 44

### Synthesis of N-(2-methyl-6-(2-(1-methyl-1H-pyrazol-3-ylamino)imidazo[1,2-b]pyridazin-3-yl)pyrimidin-4-yl)acetamide

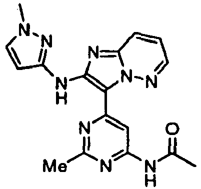

### Step 1

The title compound was synthesized utilizing the procedure described in Example18 Step 5 using 1-methyl-1H-pyrazol-3-amine, followed by procedure described in Example 42, Step 2. *m*/*z* (ESI, +ve ion) 393.2 (M+H)⁺. ¹H NMR (400 MHz, DMSO-d₆) δ ppm 10.81 (s, 1 H) 9.30 (s, 1 H) 8.63 (dd, *J*=4.69, 1.66 Hz, 1 H) 8.05 (dd, *J*=9.00, 1.66 Hz, 1 H) 7.60 (d, *J*=2.05 Hz, I H) 7.38 (dd, *J*=9.00, 4.69 Hz, 1 H) 6.81 (d, *J*=2.25 Hz, 1 H) 5.75 (s, 1 H) 3.78 (s, 3 H) 2.63 (s, 3 H) 2.15 (s, 3 H)

### Example 45

### Synthesis of 3-(6-amino-2-methylpyrimidin-4-yl)-7-morpholino-N-(1H-pyrazol-3-yl)imidazo[1,2-b]pyridazin-2-amine

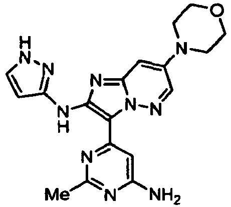

### Step 1

In a 10-mL sealed tube under N₂ were dissolved 3,5-dichloropyridazine (1.00 g, 6.71 mmol) and morpholine (1.754 mL, 20.14 mmol) portion wise in 2 mL of MeCN at 0 °C. After 1 h, the reaction mixture was concentrated under reduced pressure with silica and purified by MPLC (ISCO) with DCM:MeOH+NH₄OH 100:0 to 90:10 to afford 4-(6-chloropyridazin-4-yl)morpholine (1.100 g, 5.51 mmol, 82 % yield). *m*/*z* (ESI, +ve ion) 200.0 (M+H)⁺

### Step 2

In a 10-mL microwave sealed tube under N₂ was dissolved 4-(6-chloropyridazin-4-yl)morpholine (1.100 g, 5.51 mmol, 82 % yield) in ammonium hydroxide saturated with NH₄Cl (5 mL) then stirred and heated at 150 °C with microwave. After 2 h, the reaction mixture was concentrated under reduced pressure with silica and purified by MPLC (ISCO) with DCM:MeOH+NH₄OH 100:0 to 90:10 to afforded 5-morpholinopyridazin-3-amine (0.500g, 2.77 mmol, 41.3 % yield). *m*/*z* (ESI, +ve ion) 181.2 (M+H)⁺

### Step 3 to 7

The title compound was synthesized according to the procedure described in Example 18, Steps 1 to 5 using 5-morpholinopyridazin-3-amine. *m*/*z* (ESI, +ve ion) 393.2 (M+H)⁺. ¹H NMR (400 MHz, DMSO-d₆) δ ppm 10.48 (s, I H) 8.58 (d, *J*=2.84 Hz, 1 H) 7.65 (d, *J*=2.35 Hz, 1 H) 7.54 (s, 1 H) 7.27 (d, *J*=2.84 Hz, 1 H) 6.78 (d, *J*=2.25 Hz, 1 H) 3.74 - 3.85 (m, 4 H) 3.39 - 3.54 (m, 4 H) 2.57 (s, 3 H)

### Example 45

### Synthesis of 3-(6-amino-2-methylpyrimidin-4-yl)-8-fluoro-N-(1H-pyrazol-3-yl)imidazo[1,2-a]pyridin-2-amine

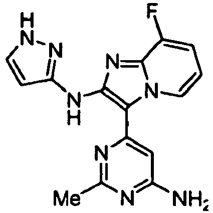

### Step 1 to 5

The title compound was synthesized synthesized according to the procedure described in Example 18, Step 1 to 5 using 3-fluoropyridin-2-amine. *m*/*z* (ESI, +ve ion) 325.0 (M+H)⁺. ¹H NMR (400 MHz, DMSO-d₆) δ ppm 10.04 (br. s., 1 H) 8.61 (d, *J*=7.34 Hz, 1 H) 7.61 (d, *J*=2.25 Hz, 1 H) 7.38 (dd, *J*=10.76, 7.83 Hz, 1 H) 7.03 - 7.11 (m, 1 H) 6.81 (s, 1 H) 6.68 (s, 1 H) 2.52 (br. s., 3 H)

### Example 46

### Synthesis of 3-(2-methyl-6-(6-(4-methylpiperazin-1-yl)pyridazin-3-ylamino)pyrimidin-4-yl)-N-(1H-pyrazol-3-yl)imidazo[1,2-b]pyridazin-2-amine

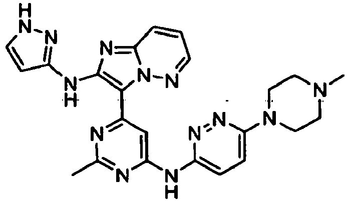

### Step 1

3-Amino-6-chloropyridazine (0.500 g, 3.86 mmol) was combined with 1-methyl-piperazine (2.143 mL, 19.30 mmol) and the reaction was heated at 150 °C for 4 h in the microwave. The reaction mixture was concentrated in vacuo and the crude material purified via MPLC (isocratic 90:10:1 DCM:MeOH:NH₄OH) to provide 6-(4-methylpiperazin-1-yl)pyridazin-3-amine as a orange solid. The material was moved forward without further purification. *m*/*z* (ESI, +ve ion) 194.2 (M+H)⁺

### Step 2

In a sealed tube under nitrogen were dissolved 2-chloro-3-(6-chloro-2-methylpyrimidin-4-yl)imidazo[1,2-b]pyridazine (0.100 g, 0.357 mmol), JosiPhos CyPF-t-Bu (0.040 g, 0.071 mmol), 6-(4-methylpiperazin-1-yl)pyridazin-3-amine (0.083 g, 0.428 mmol), Pd₂(dba)₃ (0.033 g, 0.036 mmol), and cesium carbonate (0.349 g, 1.071 mmol) in *t*-BuOH (1.000 mL) and water (0.064 mL, 3.57 mmol). The vessel was purged with nitrogen, and then stirred at 90 °C for 18 h. The crude material was purified via MPLC, eluting with a gradient of 0% to 100% 90:10:1 DCM:MeOH:NH₄OH to provide N-(6-(2-chloroimidazo[1,2-b]pyridazin-3-yl)-2-methylpyrimidin-4-yl)-6-(4-methylpiperazin-1-yl)pyridazin-3-amine (0.086 g, 0.197 mmol, 55.1 % yield). *m*/*z* (ESI, +ve ion) 437.1 (M+H)⁺

### Step 3

The title compound was synthesized according to the procedure described in Example 27, Step 5 using N-(6-(2-chloroimidazo[1,2-b]pyridazin-3-yl)-2-methylpyrimidin-4-yl)-6-(4-methylpiperazin-1-yl)pyridazin-3-amine. *m*/*z* (ESI, +ve ion) 484.2 (M+H)⁺. ¹H NMR (DMSO-d₆) δ: 12.18 (br. s., 1H), 10.95 (s, 1H), 10.28 (s, 1H), 8.52 - 8.67 (m, 2H), 7.94 - 8.10 (m, 2H), 7.65 (s, 1H), 7.25 - 7.47 (m, 2H), 6.86 (s, 1H), 3.41 - 3.62 (m, 4H), 2.58 (s, 3H), 2.41 - 2.48 (m, 4H), 2.24 (s, 3H)

### Example 47

### Synthesis of 3-(6-(5-(2-methoxyethoxy)pyridazin-3-ylamino)-2-methylpyrimidin-4-yl)-N-(1H-pyrazol-3-yl)imidazo[1,2-b]pyridazin-2-amine

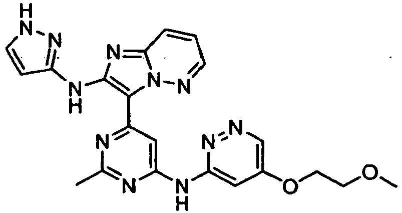

### Step 1

To a solution of 3,5-dichloropyridazine (2.53 g, 16.98 mmol) in THF (16.98 mL) at 0 °C under nitrogen was added sodium methoxyethoxide (8.89 mL, 18.68 mmol) dropwise, after which the flask was warmed to RT and stirred for 1 h. The reaction mixture was concentrated in vacuo, and purified via MPLC (eluting with a gradient 0-100% 90:10:1 DCM:MeOH:NH₄OH in DCM) to yield 3-chloro-5-(2-methoxyethoxy)pyridazine (2.34 g, 12.41 mmol, 73.1 % yield) as an orange oil. *m*/*z* (ESI, +ve ion) 189.3 (M+H)⁺

### Step 2

In a sealed pressure vessel under nitrogen were dissolved 6-(2-chloroimidazo[1,2-b]pyridazin-3-yl)-2-methylpyrimidin-4-amine, JosiPhos CyPF-t-Bu (0.468 g, 0.844 mmol), 3-chloro-5-(2-methoxyethoxy)pyridazine (1.831 g, 9.71 mmol), Pd₂(dba)₃ (0.386 g, 0.422 mmol), and cesium carbonate (6.87 g, 21.10 mmol) in t-BuOH (11.82 mL) and water (0.760 mL, 42.2 mmol), and then heated at 120 °C for 5 h. The crude reaction mixture was concentrated and then purified via MPLC (eluting with 0-100% 90:10:1 DCM:MeOH:NH₄OH in DCM) to yield crude material as a dark brown solid. The solid triturated with IPA, yielding N-(6-(2-chloroimidazo[1,2-b]pyridazin-3-yl)-2-methylpyrimidin-4-yl)-5-(2-methoxyethoxy)pyridazin-3-amine. The material was carried forward without further purification. *m*/z (ESI, +ve ion) 413.0 (M+H)⁺

### Step 3

The title compound was synthesized according to the procedure described in Example 27, Step 5 using N-(6-(2-chloroimidazo[1,2-b]pyridazin-3-yl)-2-methylpyrimidin-4-yl)-5-(2-methoxyethoxy)pyridazin-3-amine. *m*/*z* (ESI, +ve ion) 460.0 (M+H)⁺. ¹H NMR (DMSO-d₆) δ: 12.19 (br. s., 1H), 10.92 (s, 1H), 10.62 (s, 1H), 8.78 (s, 1H), 8.68 (d, J = 2.5 Hz, 1H), 8.61 (d, J = 3.5 Hz, 1H), 8.05 (d, J = 8.7 Hz, 1 H), 7.95 (s, 1H), 7.66 (br. s., 1H), 7.37 (dd, J = 9.1, 4.6 Hz, 1H), 6.86 (br. s., 1H), 4.30 (dd, J = 5.2, 3.5 Hz, 2H), 3.66 - 3.81 (m, 2H), 3.34 (s, 3H), 2.65 (s, 3H).

### Biological Examples

### Example 1

### In vitro assays

The following assays can be used to determine the degree of activity of individual compounds as PI3 kinase and/or mTOR inhibitors.

### Recombinant expression of PI3K enzymes

Full length p110 subunits of PI3K α, β and δ, N-terminally labeled with polyHis tag, can be co-expressed with p85 with Baculo virus expression vectors in sf9 insect cells. P110/p85 heterodimers can be purified by sequential Ni-NTA, Q-HP, Superdex-100 chromatography. Purified α, β and δ isozymes can be stored at -20 °C in 20mM Tris, pH 8, 0.2M NaCl, 50% glycerol, 5mM DTT, 2mM Na cholate. Truncated PI3Kγ, residues 114-1102, N-terminally labeled with polyHis tag, can be expressed with Baculo virus in Hi5 insect cells. The γ isozyme can be purified by sequential Ni-NTA, Superdex-200, Q-HP chromatography. The γ isozyme can be stored frozen at -80 °C in NaH₂PO₄, pH 8, 0.2M NaCl, 1% ethylene glycol, 2mM β-mercaptoethanol.

| | Alpha | Beta | Delta | Gamma |
|---|---|---|---|---|
| 50 mM Tris | pH 8 | pH 7.5 | pH 7.5 | pH 8 |
| MgCl2 | 15 mM | 10 mM | 10 mM | 15 mM |
| Na cholate | 2 mM | 1 mM | 0.5 mM | 2 mM |
| DTT | 2 mM | 1 mM | 1 mM | 2 mM |
| ATP | 1 uM | 0.5 uM | 0.5 uM | 1 uM |
| PIP2 | none | 2.5 uM | 2.5 uM | none |
| time | 1 hr | 2 hr | 2 hr | 1 hr |
| [Enzyme] | 15 nM | 40 nM | 15 nM | 50 nM |

### In vitro PI3 kinase enzyme assays (PI3K ATPLoss)

PI3K enzyme assays (alpha, beta, delta and gamma) can be performed in 25 µL with the above final concentrations of components in white polyproplyene plates. Phosphatidyl inositol phosphoacceptor, PtdIns(4,5)P₂ (e.g,. P4508) can be obtained from Echelon Biosciences, Salt Lake City, UT. The ATPase activity of the alpha and gamma isozymes may not be greatly stimulated by PtdIns(4,5)P₂ under these conditions, it can be omitted from the assay of these isozymes. Test compounds can be dissolved in DMSO and diluted with threefold serial dilutions. The compound in DMSO (1 µL) may be added per test well, and the inhibition relative to reactions containing no compound, with and without enzyme can be determined. After assay incubation at RT, the reaction can be stopped and residual ATP can be determined by addition of an equal volume of a commercial ATP bioluminescence kit (Perkin Elmer EasyLite, Perkin Elmer, Waltham, MA) according to the manufacturer's instructions, and detected using an Analyst GT luminometer.

### Cell-based phospho-AKT Ser473 assay (HCT116 Cell)

This assay determines the ability of a compound to inhibit the phosphorylation of Serine 473 in Akt using a MSD based sandwich immunoassay (Meso Scale Detection, Meso Scale Discovery (MSD), Gaithersburg, MD). HCT 116 human colon carcinoma cell lines can be grown in McCoy's 5A growth medium (GIBCO, Carlsbad, CA) containing 10% FBS (GIBCO, Carlsbad, CA) and X1 Penicillin-streptomycin-glutamine (GIBCO, Carlsbad, CA). Prior to the assay, cells can be detached from the culture flask with trypsin, and re-suspended in complete media to give a final concentration of 1.6 x 10⁵ cells per mL. Aliquots (100 µl) of the HCT116 cell suspension can be seeded into each well of a 96 well tissue culture plate to give a final density of 16,000 cells per well. Cells can then be incubated overnight at 37 °C.

The following day the cells can be treated with serially diluted test compounds and incubated for 2 hours at 37 °C. The culture media on the HCT 116 cells can be replaced with 189 µL McCoys media, supplemented with 0.1% BSA (ICN Biomedicals, Inc., Costa Mesa, CA). Compounds can be prepared as either 10 mM or 0.5 mM stock solutions in DMSO, and serially diluted 3 fold in a 10-point dose-response curve to give final concentrations that are 200-fold greater than the desired final test concentration. Aliquots (1µL) of serially-diluted compounds can be transferred to 96 well tissue culture plates containing the HCT 116 cells. As a minimum response control, each plate can contain wells having a final concentration of 2.5µM of a potent PI3K inhibitor which had previously been shown to completely inhibit Akt phosphorylation at this test concentration. As a maximum response control, wells can contain 0.5% DMSO in place of compound. The plates can be mixed at 700 rpm for 2 min to ensure even distribution of the test compound and incubated for 2 hours at 37 °C. Cells can then be stimulated with insulin-like growth factor 1 (Sigma, St Louis, MO) at final concentration of 100ng/ml for 15 minutes at 37 °C. The media can then be removed and the cells treated with 80 µL cell-lysis buffer (MSD) containing a cocktail of protease and phosphatase inhibitors for one hour at 4 °C.

25 µL Cell lysate can then be transferred to pre-blocked MSD assay plates pre-coated with a capture antibody specific for Akt, and the plates can be incubated for 2 hours at room temperature. The cell lysates can then be removed and plates can then be washed four times with 200 µl per well of Tris wash buffer (500 mM Tris, PH 7.5, 1.5 M NaCl, 0.2% Tween-20). Subsequently cells can be incubated for 1 hour at room temperature with a 25 µL solution containing the detection antibody, anti-phospho Akt (Ser 473) labeled with an electrochemiluminescent compound (Meso Scale Discovery SULPHO-TAG™ label, MSD, Gaithersburg, MD). The detection antibody can be removed and plates can then be washed four times with 200 µL per well of Tris wash buffer. An aliquot of 150 µL of diluted MSD read buffer can then be applied to each well, and the electrochemiluminescent signal can be measured using a MSD SECTOR™ plate reader (Meso Scale Discovery, Gaithersburg, MD). This instrument measures the intensity of emitted light to determine a quantitative measure of phosphorylated Akt in each well. The dose-response data obtained with each compound can be analyzed and the IC₅₀ inhibition of Akt phosphorylation at Ser473 can be calculated.

### pAkt AlphaScreen (U87 Cell)

The pAkt AlphaScreen® assay (PerkinElmer, Waltham, MA) determines whether there is phosphorylation of Akt at Serine 473 by recruitment of a phosphospecific antibody. This assay was performed using U87 MG cells. The U87 growth media consists of MEM (Gibco, Carlsbad, CA) supplemented with 10% FBS (Gibco,), 1x Non-Essential Amino Acids (Gibco,) and 1x Penicillin/Streptomycin/Glutamine (Gibco). The cells were maintained weekly using 0.05% Trypsin (Gibco) and replated in 150 mm TC- Treated Culture Dishes (Corning, Coming, NY).

The first day of the assay, the adherent cells were trypsinized, media was added to the loose cells and cells were mixed to a homogenous mixture. 0.5 ml of the homogenous mixture was counted on the Beckman Coulter^{®} Vi-CELL™ XR (Fullerton, CA). 50 frames of cells were counted and the number of viable cells was determined. The cells were then diluted to 0.25 million cells per ml, and centrifuged at 200 rcf for 5 minutes. The media was removed and the cells were reconstituted in fresh media for plating. The cells were plated at 20 µl per well on the PerkinElmer^{®} FlexDrop PLUS in Low Volume 384 Well White Tissue Culture Plates (Coming) with a final cell density of 5K cells per well. The plates were incubated overnight at 37° Celsius, 5% CO₂.

On the second day, the compound plates were prepared, the cells were treated with compound and the pAkt reaction mix was added to the cell lysate. 384 well compound plates were prepared containing 1 µl of compound per well starting at 5 mM and diluted 1:2 across the row, resulting in a 22 well serial dilution. 39 µl of growth media was added to the compound plate in rows 1-22 using the PerkinElmer^{®} FlexDrop PLUS resulting in a DMSO concentration of 2.5%. The cell plates and diluted compound plates were put onto the VELOCITY 11™ VPREP™ 384 ST where the compound plate was mixed and 5 µl of serially diluted compound or controls was added to the cell plate. The final concentration of the compounds was 25 µM serially diluted to 11.9 pM in 0.5% DMSO.

The cell plates were then incubated with compound for two hours at 37 °C, 5% CO₂. After two hours, the media in the cell plates was aspirated using the BioTek^{®} ELx405HT plate washer (Winooski, VT) removing the majority of media and compound without disturbing the adherent U87 cells. The following assay reagents are components of the SureFire^{®} Akt (Ser 473) Phosphorylation 50K Point Kit (TGR BioSciences, Adelaide, Austalia) and an IgG Detection Kit (PerkinElmer, Waltham, MA). 5 µl of 1x Lysis Buffer was added to each well using the PerkinElmer^{®} FlexDrop PLUS. The plates were then incubated at room temperature on a shaker for ten minutes. The AlphaScreen^{®} reaction was prepared under low light conditions (subdued or green light) including p-Akt (Ser 473) Reaction Buffer, Dilution Buffer, Activation Buffer, Acceptor Beads and Donor Beads at a ratio of 40:20:10:1:1 respectively. The AlphaScreen^{®} reaction was added to the cell lysate at 6 µl per well using the PerkinElmer^{®} FlexDrop PLUS. The plates were placed in a humid environment to reduce edge effects and incubated overnight at room temperature with restricted air flow in the dark.

On the final day of the experiment, the plates were read on the PerkinElmer^{®} EnVision™ 2103 Multilable Reader using the standard AlphaScreen^{®} readout. The POC is calculated and the data is analyzed to report the IC₅₀ IP for pAkt at Serine 473.

Activity data for the compounds tested in the PI3K cell based Akt assay is provided in Table 1 under the column heading U87

### In vitro PI3K AlphaScreen^{®} assay

The PI3K AlphaScreen^{®} assay (PerkinElmer, Waltham, MA) measures the activity of a panel of four phosphoinositide 3-kinases: PI3Kα, PI3Kβ, PI3Kγ, and PI3Kδ. Each of these enzymes phosphorylates the 3'-hydroxyl group on phosphatidylinositiol (4,5)-bisphosphate (PIP₂) to produce phosphatidylinositol (3,4,5)-trisphosphate (PIP₃). This phosphorylation activity is measured using a GST-tagged PIP₃ binding protein (Echelon Biosciences, Salt Lake City, UT), an anti-GST-tagged Acceptor bead, and streptavidin-Donor bead. The interaction of biotinylated-PIP₃ analog (IP₄) and the PIP₃ binding protein brings both Acceptor and Donor beads together producing, upon excitation of the Donor beads at 680 nm, a singlet oxygen species leading to the luminescent AlphaScreen^{®} signal. When PIP₃ is produced *via* phophorylation of PIP2 by a PI3K, PIP3 competes with biotinylated-PIP₃ analog (IP₄) for binding to the PIP₃ binding protein. In the absence of this interaction, proximity of the Donor and Acceptor beads is decreased, producing a loss of luminescent signal which is inversely proportional to PI3K activity. An inhibitor reduces activity of the enzyme, resulting in less PIP₃ production and greater luminescence.

The enzyme reaction buffer is made using sterile water (Baxter, Deerfield, IL) and 50mM Tris HCl pH 7, 14mM MgCl₂, 2mM sodium cholate, and 100mM NaCl. 2mM DTT is added fresh the day of the experiment. The AlphaScreen^{®} reaction buffer is made using sterile water and 10mM Tris HCl pH 7.5, 150mM NaCl, 0.10% Tween 20, and 30mM EDTA. 1mM DTT is added fresh the day of the experiment.

The source plates for this assay are 384-well Greiner clear polypropylene plates containing test compounds at 5mM and diluted 1:2 over 22 points. Columns 23 and 24 contain only DMSO as these are designated for positive and negative controls. Source plates are replicated into 384-well Optiplates (PerkinElmer, Waltham, MA), 0.5µL/well, to make assay-ready plates.

The different PI3K isoforms are each diluted in enzyme reaction buffer to 2X working solutions. PI3Kα is diluted to 1.6nM, PI3Kβ is diluted to 0.8nM, PI3Kγ is diluted to 15nM, and PI3Kδ is diluted to 1.6nM. Two different 2X substrate solutions are made in enzyme reaction buffer. In one solution, PI(4,5)P2 (Echelon Biosciences, Salt Lake City, UT) is diluted to 10µM and ATP is diluted to 20µM. This solution is used in the assays testing PI3Kα and PI3Kβ. In a second solution, PI(4,5)P2 is diluted to 10µM and ATP is diluted to 8µM. This solution is used in the assays testing PI3Kγ and PI3Kδ.

The AlphaScreen^{®} reaction solutions are made using beads from the anti-GST AlphaScreen^{®} kit (PerkinElmer, Waltham, MA). Two solutions are made in Alphascreen reaction buffer to 4X working concentrations. In one solution, biotinylated-IP₄ (Echelon Biosciences, Salt Lake City, UT) is diluted to 40nM and streptavadin-Donor Beads are diluted to 80µg/mL. In the second solution, PIP₃-binding protein (Echelon Biosciences, Salt Lake City, UT) is diluted to 40nM and anti-GST-Acceptor Beads are diluted to 80µg/mL. 10µL/well of enzyme reaction buffer is added to Column 24 of the assay ready plates in place of enzyme. This is done for plates in the PI3Kα, β, and δ assays.

Using a 384-well dispensing Multidrop (Titertek, Huntsville, AL), 10µL/well of 2X enzyme (PI3Kα, β, δ) is added to Columns 1-23 of the appropriate assay ready plates (for PI3Kγ 10µL is added to Columns 1-24). 10µL/well of the appropriate substrate solution (the solution with 20µM ATP for PI3Kα and β assays, and the solution with 8µM ATP for PI3Kγ and δ assays) is then added to Columns 1-24 of the plates. Plates are then incubated at room temperature for 20 minutes.

In the dark, 10µL/well of the Donor Bead solution is added to Columns 1-24 of the plates to quench the enzyme reaction. The plates are incubated at room temperature for 30 minutes. Still in the dark, 10µL/well of the Acceptor Bead solution is also added to Columns 1-24 of the plates. The plates are then incubated in the dark for 1.5 hours. The plates are read on an Envision Multilabel Plate Reader (PerkinElmer, Waltham, MA) with a 680nm excitation filter and a 520-620nm emission filter.

Activity data for the compounds tested in the assay is provided in Table 1 under the column heading PI3Kα AlphaScreen^{®}.

### mTOR .....LanthaScreen

The mTOR LanthaScreen is a TR-FRET assay measuring the phosphorylation of mTOR's substrate 4EBP1. 384 well compound plates were prepared containing 1 µl of compound per well starting at 5 mM and diluted 1:2 across the row, resulting in a 22 well serial dilution. 24 µl of assay buffer (Invitrogen, PV4794) with 2 mM DTT was added to the compound plate in rows 1-24 using the VELOCITY 11™ VPREP™ 384 ST resulting in a DMSO concentration of 4%. The compound plate was mixed and 2.5 µl of serially diluted compound or controls was added to the assay plate (Costar, 3658).

The assay was conducted on the PerkinElmer^{®} FlexDrop PLUS. A 5 µl mix of 800 nM GFP-4E-BP1 (Invitrogen, PV4759) and 20 µM ATP (Amgen) was added to rows 1-24. 2.5 µl of 0.6 µg/ml of mTOR Enzyme (Amgen) was added to rows 1-23. 2.5 µl of assay buffer was added to row 24 for the low control. The final concentration of the compounds was 50 µM serially diluted to 23.84 pM in 1% DMSO. The final high control had 1% DMSO and the low control was a no enzyme control with a concentration of 1% DMSO. The final concentrations in the assay reagents were 400 nM GFP-4E-BP1, 10 µM of ATP and 0.15 µg/ml of mTOR enzyme. The compound, enzyme, and substrate were incubated for 90 minutes. At this point, 10 µl of stop solution was added (20 mM Tris, pH 7.5 (Invitrogen, 15567-027), 0.02 % Sodium Azide (Teknova, S0208), 0.01 % NP-40 (Roche, 11754599001), 20 mM EDTA (Invitrogen, 15575-038) and 4 nM of Tb-anti-p4E-BP1 (Invitrogen, PV4758)) for a final concentration of 2 nM of Tb-anti-p4E-BP1.

Sixty minutes later the plates were read on the PerkinElmer^{®} En Vision™ 2103 Multilable Reader using the Excitation filter 340 nm and the Emission filters 520 nm and 495 nm. The ratio of 520 nm/495 nm was calculated and the POC data was analyzed to report the IC₅₀ IP for the phosphorylation of 4EBP1.

Activity data for the compounds tested in the assay is provided in Table 1 under the column heading mTOR.

### p4EBP1 AlphaScreen

The p4EBP 1 AlphaScreen assay determines whether there is phosphorylation of 4EBP 1 at Thr37/Thr46 by recruitment of a phosphospecific antibody. This assay was performed using U87 MG cells. The U87 growth media consists of MEM (Gibco, 51200-038) supplemented with 10% FBS (Gibco, 16140-071), 1x Non-Essential Amino Acids (Gibco, 11140-050) and 1x Penicillin/Streptomycin/Glutamine (Gibco, 10378-016). The cells were maintained weekly using 0.05% Trypsin (Gibco, 25300-054) and replated in 150 mm TC-Treated Culture Dishes (Corning, 430599).

The first day of the assay, the adherent cells were trypsinized, media was added to the loose cells and cells were mixed to a homogenous mixture. 0.5 ml of the homogenous mixture was counted on the Beckman Coulter^{®} Vi-CELL™ XR. 50 frames of cells were counted and the number of viable cells was determined. The cells were then diluted to 0.25 million cells per ml, and centrifuged at 200 rcf for 5 minutes. The media was removed and the cells were reconstituted in fresh media for plating. The cells were plated at 20 µl per well on the PerkinElmer^{®} FlexDrop PLUS in Low Volume 384 Well White Tissue Culture Plates (Corning, 3826) with a final cell density of 5K cells per well. The plates were incubated overnight at 37° Celsius, 5% CO₂.

On the second day, the compound plates were prepared, the cells were treated with compound and the p4EBP 1 reaction mix was added to the cell lysate. 384 well compound plates were prepared by Amgen's Sample Bank containing 1 µl of compound per well starting at 5 mM and diluted 1:2 across the row, resulting in a 22 well serial dilution. 39 µl of growth media was added to the compound plate in rows 1-22 using the PerkinElmer^{®} FlexDrop PLUS resulting in a DMSO concentration of 2.5%. The control columns were added manually; 40 µl of 2.5% DMSO (Sigma, D4540-100ml) in growth media was added to the plate for the high control and 40 µl of 50 µM of AMG2203766 with 2.5% DMSO was added to the plate as the low control. The cell plates and diluted compound plates were put onto the VELOCITY 11™ VPREP™ 384 ST where the compound plate was mixed and 5 µl of serially diluted compound or controls was added to the cell plate. The final concentration of the compounds was 25 µM serially diluted to 11.9 pM in 0.5% DMSO. The final high control had 0.5% DMSO and the low control concentration was 10 µM AMG2203766 in 0.5% DMSO. The cell plates were then incubated with compound for two hours at 37° Celsius, 5% CO₂. After two hours, the media in the cell plates was aspirated using the BioTek^{®} ELx405HT plate washer removing the majority of media and compound without disturbing the adherent U87 cells. The following assay reagents are components of the SureFire Phospho-4EBP 1 (Thr37/Thr46) 50K Point Kit (TGR BioSciences, TGR4ES50K) and an IgG Detection Kit (PerkinElmer, 6760617R). 5 µl of 1x Lysis Buffer was added to each well using the PerkinElmer^{®} FlexDrop PLUS. The plates were then incubated at room temperature on a shaker for ten minutes. The AlphaScreen reaction was prepared under low light conditions (subdued or green light) including p-4E-BP1 (Thr37/46) Reaction Buffer, Activation Buffer, Acceptor Beads and Donor Beads at a ratio of 60:10:1:1 respectively. The AlphaScreen reaction was added to the cell lysate at 6 µl per well using the PerkinElmer^{®} FlexDrop PLUS. The plates were placed in a humid environment to reduce edge effects and incubated overnight at room temperature with restricted air flow in the dark.

On the final day of the experiment, the plates were read on the PerkinElmer^{®} En Vision™ 2103 Multilable Reader using the standard AlphaScreen readout. The POC is calculated and the data is analyzed to report the IC₅₀IP for p4EBP 1 at Thr37/Thr46.

**Table 1**

| Cpd # | mTOR IC_{50 um} | P13Kalpha IC_{50 um} | pAKT IC_{50 um} | Cpd # | mTOR IC_{50 um} | P13Kalpha IC_{50 um} | pAKT IC_{50 um} |
|---|---|---|---|---|---|---|---|
| 2 | 0.024 | 0.02 | 0.05 | 16 | 0.38 | 2.38 | 0.35 |
| 1 | 0.012 | 0.82 | 0.02 | 11 | 0.82 | 0.66 | 0.60 |
| 3 | 0.053 | 0.82 | 0.05 | 13 | 0.10 | 1.12 | 0.28 |
| 4 | 0.052 | 0.04 | 0.04 | 14 | 0.12 | 2.35 | 0.44 |
| 7 | 0.011 | 0.83 | 0.02 | 18 | 0.86 | 3.23 | 0.71 |
| 26 | - | 0.004 | 0.04 | 57 | 0.01 | 0.01 | 0.07 |
| 29 | 0.28 | 0.007 | 0.026 | 60 | 0.01 | 39.2 | 0.02 |
| 31 | 6.18 | 2.76 | 0.79 | 63 | 0.06 | 2.04 | 0.05 |
| 33 | 2.84 | 0.66 | 0.29 | 65 | 0.34 | 8,69 | 0.38 |
| 34 | 0.44 | 0.75 | 0.06 | 67 | 0.01 | 0.75 | 0.01 |
| 35 | 0.01 | 0.11 | 0.07 | 69 | 0.004 | 0.09 | 0.001 |
| 40 | 0.01 | 0.45 | 0.06 | 72 | 0.04 | 0.11 | 0.003 |
| 41 | 0.40 | 43.2 | 0.4 | 73 | 0.05 | 0.80 | 0.07 |
| 49 | 0.08 | 0.13 | 0.06 | 75 | 1.42 | 42.1 | 0.79 |
| 53 | 0.001 | 0.02 | 0.01 | 77 | 2.72 | 15.3 | 1.64 |
| 88 | - | 4.5 | 1.63 | 109 | 0.05 | 0.47 | 0.22 |
| 90 | 0.03 | 0.59 | 0.02 | 114 | 0.01 | 0.10 | 0.03 |
| 92 | 0.78 | 20.1 | 0.18 | 115 | 0.003 | 0.04 | 0.04 |
| 94 | - | - | 3.92 | 120 | 0.29 | 1.0 | 0.11 |
| 97 | 0.016 | 0.35 | 0.03 | 123 | 0.14 | 6.93 | 0.15 |
| 99 | 0.004 | 0.11 | 0.01 | 126 | 0.69 | 5.34 | 0.22 |
| 100 | 0.001 | 0.42 | 0.01 | 128 | - | 5.03 | 3.16 |
| 105 | 0.014 | 0.04 | 0.05 | 129 | - | 4.94 | 1.47 |
| 107 | 0.04 | 0.25 | 0.13 | 134 | 0.82 | 2.08 | 0.21 |
| 140 | 0.11 | 0.06 | 0.09 | 149 | - | - | 0.28 |
| 151 | 0.43 | 9.52 | 5.38 | 185 | 0.04 | 0.70 | 0.05 |
| 166 | 0.025 | 5.65 | 0.45 | 188 | 0.36 | 7.01 | 0.32 |
| 175 | 0.002 | 0.14 | 0.03 | 198 | 0.11 | 2.21 | - |
| 177 | 0.001 | 0.08 | 0.17 | 201 | 0.25 | 1.39 | - |
| 183 | 0.46 | 0.05 | 0.76 | 204 | 0.09 | 3 | 0.09 |
| 209 | 0.3 | 0.83 | 0.11 | 239 | 0.008 | 3.01 | 0.01 |
| 216 | 0.86 | 1.37 | 0.45 | 242 | 0.08 | 2.32 | 0.08 |
| 217 | 1.74 | - | 1.18 | 246 | - | 15.8 | - |
| 222 | 5.88 | 1.2 | 1.81 | 257 | 1.69 | 46.5 | 1.05 |
| 258 | 0.5 | 0.03 | - | 264 | - | 33.4 | 3.15 |
| 272 | - | 0.32 | 0.11 | 293 | - | 0.30 | 0.71 |
| 277 | - | 0.04 | 0.14 | 295 | - | 2.98 | 0.24 |
| 285 | - | 0.09 | - | 316 | - | - | 0.95 |
| 286 | 0.12 | 0.13 | 0.01 | 318 | - | 12 | - |
| 291 | 1.21 | 0.17 | 0.17 | 334 | - | 4.94 | 0.24 |
| 344 | 0.08 | 0.07 | 0.02 | 354 | 0.32 | 0.77 | 0.10 |
| 351 | 0.23 | 0.48 | 0.03 | | | | |

### Formulation Examples

The following are representative pharmaceutical formulations containing a compound of Formula (I).

### Tablet Formulation

The following ingredients are mixed intimately and pressed into single scored tablets.

| Ingredient | Quantity per tablet Mg |
|---|---|
| compound of this invention | 400 |

| | |
|---|---|
| cornstarch | 50 |
| croscarmellose sodium | 25 |
| lactose | 120 |
| magnesium stearate | 5 |

### Capsule Formulation

The following ingredients are mixed intimately and loaded into a hard-shell gelatin capsule.

| Ingredient | Quantity per capsule |
|---|---|
| | mg |
| compound of this invention | 200 |
| lactose spray dried | 148 |
| magnesium stearate | 2 |

### Capsule Formulation

The following ingredients are mixed intimately and loaded into a hard-shell gelatin capsule.

| Ingredient | Quantity per capsule |
|---|---|
| | mg |
| compound of this invention | 200 |
| lactose spray dried | 148 |
| magnesium stearate | 2 |

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive. The scope of the invention should, therefore, be determined not with reference to the above description, but should instead be determined with reference to the following appended claims, along with the full scope of equivalents to which such claims are entitled.

All patents, patent applications and publications cited in this application are hereby incorporated by reference in their entirety for all purposes to the same extent as if each individual patent, patent application or publication were so individually denoted.

## Claims

1. A compound of Formula (I): 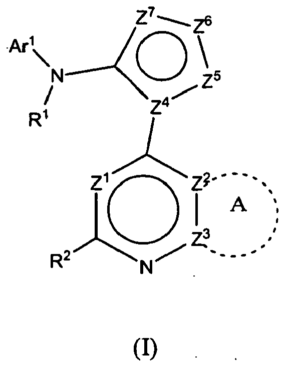 wherein:
Ar¹ is an aryl, heteroaryl, cycloalkyl or heterocyclyl ring, wherein each ring is substituted with R^{d}, R^{e}, or R^{f} where R^{d}, R^{e}, or R^{f} are independently selected from hydrogen, halo, haloalkyl, haloalkoxy, cyano, nitro, alkyl, alkenyl, alkynyl, substituted alkyl, aryl, heteroaryl, heterocycloalkyl, -C(=O)NR^{a}R^{a}, -C(=O)R^{b}, -C(=O)OR^{b}, -C(=NR^{a})NR^{a}R^{a}, -OR^{a}, -OC(=O)R^{b}, -OC(=O)NR^{a}R^{a}, -O-alkylN(R^{a})C(=O)OR^{b}, -OC(=O)N(R^{a})S(=O)₂R^{b}, -O-alkylNR^{a}R^{a}, -O-alkylOR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=S)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}-alkylene-NR^{a}R^{a}, or -NR^{a}-alkylene-OR^{a};
R¹ is hydrogen or alkyl;
R² is methyl or ethyl;
Z¹-N- or -CR³- where R³ is H or alkyl;
Z² is -N- or -CR⁴- where R⁴ is R^{d}, -(alkylene)heterocycloalkyl, -(alkylene)ₙO(alkylene)ₙaryl, -(alkylene)ₙN(R^{a})(alkylene)ₙaryl, -(alkylene)ₙ-N(R^{a})-(alkylene)ₙheteroaryl, or -(alkylene)ₙO(alkylene)ₙheteroaryl;
Z³ is -N- or -CR⁵- where R⁵ is R^{d}, -(alkylene)ₙaryl, -(alkylene)-heteroaryl, -(alkylene)heterocycloalkyl, -(alkylene)ₙO-(alkylene)ₙaryl, -(alkylene)ₙN(R^{a})(alkylene)ₙaryl, -(alkylene)ₙN(R^{a})(alkylene)ₙheteroaryl, or -(alkylene)ₙO(alkylene)ₙheteroaryl provided that only two of Z¹,Z² and Z³ can simultaneously be -N-;
or when Z² is -CR⁴- and Z³ is -CR⁵- then R⁴ and R⁵ together with the atoms to which they are attached can form ring A which is phenyl or a 5 or 6 membered heteroaryl ring and ring A is substituted with R^{g} or R^{h} where R^{g} or R^{h} are independently R^{d}, -(alkylene)ₙ-heterocycloalkyl, -(alkylene)ₙO(alkylene)ₙaryl, -(alkylene)ₙN(R^{a})(alkylene)ₙaryl, -(alkylene)ₙN(R^{a})(alkylene)ₙheteroaryl, or -(alkylene)ₙO(alkylene)ₙheteroaryl; 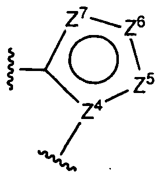 is 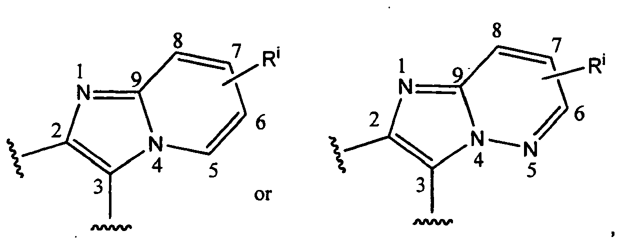
where Rⁱ is R^{d}, -(alkylene)ₙaryl, -(alkylene)heteroaryl, -(alkylene)heterocycloalkyl,-(alkylene)ₙO(alkylene)ₙaryl, -(alkylene)ₙN(R^{a})(alkylene)ₙaryl,-(alkylene₂)ₙN(R^{a})(alkylene)ₙheteroaryl, or -(alkylene)ₙO(alkylene)ₙheteroaryl;
each R^{a} is independently hydrogen or R^{b}; or when two R^{a} are attached to a nitrogen atom, either alone or part of another group, the two R^{a}s together with the nitrogen atom to which they are attached can form a monocyclic heterocyclyl ring with is optionally substituted with one, two or three substitutents independently selected from oxo, halo, alkyl, alkenyl, alkynyl, cyano, nitro, alkylcarbonyl, carboxy, alkoxycarbonyl, hydroxyl, alkoxy, alkonyloxy, alkylthio, alkylsulfonyl, -alkyl-OH, aminosulfonyl, sulfonylamino, amino, alkylamino, or dialkylamino;
each R^{b} is independently alkyl, cycloalkyl, phenyl, heteroaryl, or benzyl wherein the alkyl, cycloalkyl, phenyl, or benzyl is substituted with 0, 1, 2 or 3 substituents independently selected from halo, alkyl, haloalkyl, alkoxy, amino, cyano, hydroxyl, unsubstituted heterocycloalkyl, phenyl, alkylcarbonylamino, alkylamino or dialkylamino; and
each n is independently 0 or 1; or
a pharmaceutically acceptable salt thereof.

2. The compound of Claim 1 wherein each R^{a} is independently hydrogen or R^{b}; or when two R^{a} are attached to a nitrogen atom, either alone or part of another group, the two R^{a}s together with the nitrogen atom to which they are attached can form a monocyclic heterocyclyl ring with is optionally substituted with one, two or three substitutents independently selected from oxo, halo, alkyl, alkenyl, alkynyl, cyano, nitro, alkylcarbonyl, carboxy, alkoxycarbonyl, hydroxyl, alkoxy, alkonyloxy, alkylthio, alkylsulfonyl, aminosulfonyl, sulfonylamino, amino, alkylamino, or dialkylamino; and
each R^{b} is independently alkyl, cycloalkyl, phenyl, heteroaryl, or benzyl wherein the alkyl, cycloalkyl, phenyl, or benzyl is substituted with 0, 1, 2 or 3 substituents independently selected from halo, alkyl, haloalkyl, alkoxy, amino, cyano, alkylamino or dialkylamino.

3. The compound of Claim 1 wherein R¹ is hydrogen and in 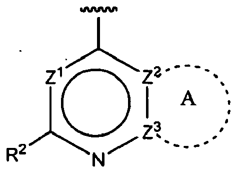 Z¹ is-N-, Z² is -CH- and Z³ is -CR⁵- where R⁵ is R^{d} or -(alkylene)ₙN(R^{a})(alkylene)ₙheteroaryl.

4. The compound of Claim 1 or 3 wherein R² is methyl.

5. The compound of any of the Claims 1, 3 or 4 wherein Ar¹ is aryl substituted as defined above.

6. The compound of any of the Claims 1, 3 or 4 wherein Ar¹ is heteroaryl substituted as defined above.

7. The compound of any of the Claims 1, 3 or 4 wherein Ar¹ is pyrazolyl substituted as defined above.

8. The compound of any of the Claims 1, 3 or 4 wherein Ar¹ is an aryl, heteroaryl, or heterocyclyl ring, wherein each ring is substituted with R^{d}, R^{e}, or R^{f} where R^{d}, R^{e}, or R^{f} are independently selected from hydrogen, halo, haloalkyl, alkyl, alkyl substituted with NR^{y}R^{y} (where R^{y} is alkyl) or alkoxy, -(CR^{y}R^{y})ₙY (where Y is aryl or heteroaryl), -OR^{x} (where R^{x} is hydrogen or alkyl), heteroaryl, heterocycloalkyl, -C(=O)NR^{a}R^{a}, -C(=O)R^{b}, -C(=O)OR^{b}, -OR^{a}, -O-alkylOR^{a}, -S(=O)₂R^{b}, -NR^{a}R^{a}, -N(R^{a})C(=O)R^{b}, -N(R^{a})C(=O)NR^{a}R^{a}, -N(R^{a})S(=O)₂R^{b}, or -NR^{a}-alkylene-OR^{a};
each R^{a} is independently hydrogen or R^{b}; or when two R^{a} are attached to a nitrogen atom, either alone or part of another group, the two R^{a}s together with the nitrogen atom to which they are attached can form a monocyclic heterocyclyl ring with is optionally substituted with one, two or three substitutents independently selected from alkyl, alkylcarbonyl, - alkylOH, or hydroxyl; and
each R^{b} is independently alkyl, cycloalkyl, phenyl, heteroaryl, or benzyl wherein the alkyl, cycloalkyl, phenyl, or benzyl is substituted with 0, 1, 2 or 3 substituents independently selected from halo, alkyl, haloalkyl, alkoxy, amino, cyano, alkylamino dialkylamino, hydroxyl, unsubstituted heterocycloalkyl, or phenyl.

9. The compound of any of the Claims 1, 3 or 4 wherein Ar¹ is 5-fluoro-6-methoxy-3-pyridinyl, 1H-pyrazol-3-yl, 1-methyl-1H-pyrazol-3-yl, 4-(3-ethylureido)phenyl, 1H-pyrrolo[2,3-b]pyridin-6-yl, 6-(trifluoromethyl)-2-pyridinyl, 2-pyridinyl, 1H-pyrrolo[3,2-b]pyridine-5-yl, 4-hydroxypyridin-2-yl, 4-(3-phenylureido)phenyl, 5-(3-ethylureido)pyridine-2-yl, 4-(3-methylureido)phenyl, 3-hydroxyphenyl, 4-acetylaminophenyl, 4-(3-(4-pyridinyl)ureidophenyl, 3-amino-pyrimidin-5-yl, 4-pyridazinyl, 1-acetyl-1H-pyrazol-3-yl, 3-(methylsulfonyl)phenyl, 3-pyridinyl, 3-methyl-5-isothiazolyl, 4-(4-methyl-1-piperazinyl)phenyl, 1H-pyrazolo[3,4-c]pyridin-3-yl, 4-fluoro-1H-indazol-3-yl)imidazo[1,2-b]pyridazin-2-yl, 1-(2-(dimethylamino)ethyl)-1H-pyrazol-3-yl, 1H-pyrazolo[3,4-b]pyridin-3-yl, 3-(4-methyl-1-piperazinyl)-phenyl, 3-acetylaminophenyl, pyridine-2-yl, 1-ethyl-1H-pyrazol-3-yl, quinolin-3-yl, isoquinolin-3-yl, 1-methylaminocarbonyl-1H-pyrazol-3-yl, 1-methylaminocarbonyl-1H-pyrazol-5-yl, 1-(2-hydroxyethylaminocarbonyl)-1H-pyrazol-3-yl, 1-(2-hydroxyethylaminocarbonyl)-1H-pyrazol-5-yl, 1-(morpholin-4-ylcarbonyl)-1H-pyrazol-3-yl, 1-(morpholin-4-ylcarbonyl)-1H-pyrazol-5-yl, 1-(phenoxycarbonyl)-1H-pyrazol-3-yl, 1-(phenylaminocarbonyl)-1H-pyrazol-3-yl, 1-(phenylaminocarbonyl)-1H-pyrazol-5-yl, 1-(methoxycarbonyl)-1H-pyrazol-3-yl, 1-(methoxycarbonyl)-1H-pyrazol-5-yl, 1-(phenoxy-carbonyl)-1H-pyrazol-4-yl, 1-(methylaminocarbonyl)-1H-pyrazol-4-yl, 1-(pyridine-2-yl)-1H-pyrazol-3-yl, 1-(methylsulfonyl)-1H-pyrazol-3-yl, 1-(benzoxazol-2-yl)-1H-pyrazol-3-yl, 1-(benzimidazol-2-yl)-1H-pyrazol-5-yl, 1-(isopropylaminocarbonyl)-1H-pyrazol-3-yl, 1-(benzylaminocarbonyl)-1H-pyrazol-3-yl, 1-(ethylaminocarbonyl)-1H-pyrazol-3-yl, 1-(2-methoxyphenyl-aminocarbonyl)-1H-pyrazol-3-yl, 1-(2-methylphenylaminocarbonyl)-1H-pyrazol-3-yl, 1-(4-methoxyphenylaminocarbonyl)-1H-pyrazol-3-yl, 1-(2-fluorophenyl-aminocarbonyl)-1H-pyrazol-3-yl, 1-(4-cyanophenylaminocarbonyl)-1H-pyrazol-3-yl, 1-(2-trifluoromethyl-phenylaminocarbonyl)-1H-pyrazol-3-yl, 1-(3-methylphenylaminocarbonyl)-1H-pyrazol-3-yl, 1-(2,5-difluorophenylaminocarbonyl)-1H-pyrazol-3-yl, 1-(3,5-difluorophenylaminocarbonyl)-1H-pyrazol-3-yl, 1-(4-methylphenylaminocarbonyl)-1H-pyrazol-3-yl, 1-(3,5-dichlorophenyl-aminocarbonyl)-1H-pyrazol-3-yl, 1-(2-chloro-5-trifluoromethylphenylaminocarbonyl)-1H-pyrazol-3-yl, 1-(2,5-dichlorophenylamino-carbonyl)-1H-pyrazol-3-yl, 1-(2,3-dichloro-phenylaminocarbonyl)-1H-pyrazol-3-yl, 1-(2-chloro-4-trifluoromethyl-phenylaminocarbonyl)-1H-pyrazol-3-yl, 1-(benzimidazol-2-yl)-1H-pyrazol-3-yl, 1-(imidazol-2-yl)-1H-pyrazol-5-yl, 1-methylsulfonyl-1H-pyrazol-3-yl, 1-aminocarbonyl-1H-pyrazol-3-yl, 1-aminocarbonyl-1H-pyrazol-5-yl, 1-azetidin-1-ylcarbonyl-1H-pyrazol-3-yl, 1-azetidin-1-ylcarbonyl-1H-pyrazol-5-yl, 1-(2-methylaminoethylamino)-carbonyl-1H-pyrazol-3-yl, 1-(2-methylaminoethylamino)carbonyl-1H-pyrazol-5-yl, 1-dimethylaminocarbonyl-1H-pyrazol-3-yl, 1-dimethylaminocarbonyl-1H-pyrazol-5-yl, 1-piperazin-1-ylcarbonyl-1H-pyrazol-3-yl, 1-(2-methoxyethylamino)carbonyl-1H-pyrazol-3-yl, 1-(2-methoxyethylamino)carbonyl-1H-pyrazol-5-yl, 1-(2-morpholin-4-ylethylamino)-carbonyl-1H-pyrazol-3-yl, 1-(2-methylpropylaminocarbonyl)-1H-pyrazol-3-yl, 1-(1-phenethylaminocarbonyl)-1H-pyrazol-3-yl, 1-(benzyl)-1H-pyrazol-3-yl, 1-(pyridine-3-ylmethyl)-1H-pyrazol-3-yl, 1-(1,4-benzoxazin-3-(4H)-one-7-yl)-1H-pyrazol-3-yl, 5-(4-hydroxyazetidin-1-yl)pyridine-3-yl, 5-(4-(propan-2-ol)azetidin-1-yl)pyridine-3-yl, quinolin-7-yl, quinolin-6-yl, isoquinolin-7-yl, 6-methoxypyridin-3-yl, 6-ethoxypyridin-3-yl, 1H-indazol-3-yl, 5-fluoro-6-methoxypyridin-3-yl, 1-(6-(2-methoxyethoxy)pyrimidin-3-yl)-1H-pyrazol-3-yl, 3-cyclopropylureidophenyl, 3-benzylcarbonylaminophenyl, 3-benzoylaminophenyl, 3-(4-chlorophenylsulfonylamino)-phenyl, 3-methylsulfonylaminophenyl, 3-(4-fluorophenyl-ureido)phenyl, 3-(benzylureido)-phenyl, 3-ethylaminocarbonylphenyl, 6-ethylamino-carbonylpyridin-3yl, 3-ethylcarbonyl-aminophenyl, 3-methylaminocarbonylphenyl, 3-ethylaminocarbonylphenyl, 3-isopropylaminocarbonylphenyl, 3-phenylaminocarbonylphenyl, 6-methylaminocarbonyl-pyridin-3yl, 6-isopropylaminocarbonylpyridin-3yl, 6-phenylamino-carbonylpyridin-3yl, 3-aminocarbonylphenyl, 4-(2-diethylaminoethylaminocarbonyl)phenyl, 4-phenylaminocarbonylphenyl, 1-(3-methylbutanoyl)-1H-pyrazol-3-yl, 1-isopropyloxy-carbonyl-1H-pyrazol-3-yl, 1-isopropyloxycarbonyl-1H-pyrazol-5-yl, 2-methoxypyridin-4-yl, 1-isopropylcarbonyl-1H-pyrazol-3-yl, 1-isopropylcarbonyl-1H-pyrazol-5-yl, 1-(3,3-dimethylbutanoyl)-1H-pyrazol-3-yl, 1-cyclohexyloxycarbonyl-1H-pyrazol-5-yl, 1-cyclohexyloxycarbonyl-1H-pyrazol-3-yl, 2-methoxypyridin-3-yl, 2-oxopyridin-3-yl, 1-(3-methylbutyl)-1H-pyrazol-3-yl, 1-(2-methoxyethyl)-1H-pyrazol-3-yl, 1H-indazol-4-yl, 6-chloro-5-methylsulfonylaminopyridin-3-yl, 6-methoxy-5-methylsulfonylaminopyridin-3-yl, 1H-indazol-6-yl, 1H-benzimidazol-6-yl, 4-(3-cyclopropylureido)phenyl, 4-piperidin-1-ylcarbonylaminophenyl, 4-pyrrolidin-1-ylcarbonylaminophenyl. 4-azetidin-1-ylcarbonylaminophenyl, 4-(1-methylpiperazin-1-ylcarbonylaminophenyl, 4-(3-(2-methoxyethyl)ureido)phenyl, 4-morpholin-4-yl-ylcarbonylaminophenyl, 4-(1-acetylpiperazin-1-ylcarbonylaminophenyl, thiazol-3-yl, 4-(2-methylmorpholin-4-yl-ylcarbonylamino)phenyl, or 4-(3-methylmorpholin-4-yl-ylcarbonylamino)phenyl.

10. The compound of any of the Claims 1 and 3-9
wherein Rⁱ is R^{d} selected from hydrogen, halo, haloalkyl, substituted alkyl, aryl, heteroaryl, heterocycloalkyl, -C(=O)NR^{a}R^{a}, -C(=O)OR^{b},-COR^{b}, -OR^{a}, -O-alkylNR^{a}R^{a}, -O-alkylOR^{a}, -NR^{a}R^{a}, or -NR^{a}-alkylene-OR^{a} ; each R^{a} is independently hydrogen or R^{b} or when two R^{a} are attached to a nitrogen atom, either alone or part of another group, the two R^{a}s together with the nitrogen atom to which they are attached can form a monocyclic heterocyclyl ring,
each R^{b} is independently alkyl, phenyl, or heteroaryl wherein the alkyl, is substituted with 0, 1, 2 or 3 substituents independently selected from alkyl, or alkoxy, wherein the aryl ring can optionally be substituted with one, two or three substituents independently selected from alkyl, alkoxy, or halo, and/or one or two substituents independently selected from, -C(=O)OR^{y}, -C(=O)NR^{x}R^{x}, -OR^{x}, -SR^{x}, -S(=O)₂R^{y}, -S(=O)₂NR^{x}R^{x}, -NR^{x}R^{x}, -N(R^{x})C(=O)R^{y}, -(CR^{x}R^{x})ₘN(R^{x})C(=O)R^{y} or -(CR^{x}R^{x})ₘOR^{x} where m is 1-3, each R^{x} is hydrogen or R^{y} and R^{y} is independently hydrogen, alkyl, or cycloalkyl;
the heterocycloalkyl ring is optionally be substituted with one, two or three substituents independently selected from alkyl, hydroxy, -C(=O)R^{y}, -C(=O)OR^{y}, -C(=O)NR^{x}R^{x}, -S(=O)₂R^{y}, or -(CR^{x}R^{x})ₘOR^{x} where m is 0-3, each R^{x} is hydrogen or R^{y} and R^{y} is independently hydrogen or alkyl;.
the heteroaryl ring is optionally be substituted with one, two or three substituents independently selected from alkyl, -OR^{x}, -C(=O)NR^{x}R^{x}, or -(CR^{x}R^{x})ₘOR^{x} where m is 1-3, each R^{x} is hydrogen or R^{y} and R^{y} is independently hydrogen or alkyl; and
substituted alkyl is an alkyl radical in which one, two, or three hydrogen atoms are independently replaced by hydroxyl or -NCOR^{y} where R^{y} is alkyl.

11. The compound of any of the Claims 1 and 3-9 wherein
Rⁱ is hydrogen, 1-piperazinyl, 4-methyl-1-piperazinyl, 4-(1-methylethyl)-1-piperazinyl, 4-(2-methoxyethyl)-1-piperazinyl, fluoro, 3-(methylsulfonyl)phenyl, 4-pyridinyl, 2-methoxyethoxy, 4-morpholinyl, 2-(1-pyrrolidinyl)ethoxy, 1-tert-butoxycarbonyl-3,6-dihydro-1(2H)-pyridin-4-yl, 3-methoxyphenyl, 3-fluoro-4-methylaminocarbonylphenyl, 4-acetylaminomethylphenyl, 1-(2-methoxyethyl)-1H-pyrazol-4-yl, 2-fluorophenyl, 2-methylphenyl, 2-methoxy-3-pyridinyl, 3-furanyl, 3,5-dimethyl-4-isoxazolyl, 4-fluoro-2-methylphenyl, 2-(1-methylethoxy)phenyl, 3-(methylsulfanyl)phenyl, 1,2,3,6-tetrahydro-4-pyridinyl, 3-methyl-5-isoxazolyl, 1-(2-methoxyethyl)-1H-pyrazol-4-yl, 3-acetylaminophenyl, 3-cyclopropylaminophenyl, 3-isopropanoylaminophenyl, 3-pyridazinyl, 1-methyl-1,2,3,6-tetrahydro-4-pyridinyl, 4-acetylaminophenyl, 2-methyl-4-pyridinyl, 2-methyl-3-pyridinyl, 5-pyrimidinyl, 3-pyridinyl, phenylamino, 4-methoxyphenylamino, methoxy, 3-fluorophenyl, 4-methylaminocarbonylphenyl, pyridine-3-ylamino, 4-acetylaminophenyl, 3-methoxy-phenylamino, pyridine-4-ylamino, pyrimidin-5-ylamino, 4-acetylaminophenylamino, 2-methyl-4-pyridinylamino, 4-methyl-3-pyridinyl, 2,6-dimethyl-4-pyridinyl, 4-isopropylamino-carbonylphenyl, 3,4-dihydro-1(2H)-isoquinolinone-6-yl, 4-ethylaminocarbonylphenyl, 4-carboxy-2-methylphenyl, 4-methyl-5-pyrimidinyl, 2-methyl-4-methylaminocarbonylphenyl, hydroxyl, 6-methylaminocarbonylpyridin-3-yl, 2-(1-pyrrolidinyl)ethoxy, 4-N,N-dimethylaminocarbonylpiperazin-1-yl, 4-methylsulfonylpiperazin-1-yl, 4-N-methylaminocarbonylpiperazin-1-yl, 4-acetylpiperazin-1-yl, 3-hydroxymethyl-4-methoxyphenyl, pyridine-4-yl, 3,6-dihydro-2H-pyran-4-yl, 2-methyl-4-pyridinyl, 1-methyl-1H-imidazol-5-yl, 3,4-dihydroxybutyl, 1-(1-methylethyl)-1H-pyrazol-4-yl, aminocarbonyl, quinolin-7-yl, 4-methylsulfonylphenyl, 4-methylaminosulfonyl-phenyl, 3-methyl-sulfonylphenyl, 2-methoxyethylamino, 4-hydroxypiperidin-1-yl, 1-methyl-1H-pyrazol-4-yl, 2-hydroxyprop-2-yl, acetyl, or trifluoromethyl.

12. The compound of Claim 1 selected from the group consisting of:
| |
|---|
| 3-(4-amino-6-m ethyl-1,3,5-triazin-2-yl)-N-(1H-pyrazol-3-yl)imidazo[1,2-a]pyridin-2-amine |
| 3-(3-(2-methyl-9H-purin-4-yl)imidazo[1,2-a]pyridin-2-ylamino)phenol |
| 3-(4-methyl-6-(methylamino)-1,3,5-triazin-2-yl)-N-(1H-pyrazol-3-yl)imidazo[1,2-a]pyridin-2-amine |
| 3-(4-amino-6-methyl-1,3,5-triazin-2-yl)-N-(6-methoxypyridin-3-yl)imidazo[1,2-a]pyridin-2-amine |
| N-(3-(2-methyl-9H-purin-4-yl)imidazo[1,2-a]pyridin-2-yl)-1H-indazol-4-amine |
| 7-chloro-3-(4-amino-6-methyl)-1,3,5-triazin-2-yl)-N-(1H-pyrazol-3-yl)imidazo[1,2-a]pyridin-2-amine |
| 3-(6-amino-2-methylpyrimidin-4-yl)-N-(1H-pyrazol-3-yl)imidazo[1,2-b]pyridazin-2-amine |
| 3-(4-amino-6-methyl-1,3,5-triazin-2-yl)-N-(5-fluoro-6-methoxypyridin-3-yl)imidazo[1,2-a]pyridin-2-amine |
| 3-(4-amino-6-methyl-1,3,5-triazin-2-yl)-N-(6-methoxypyridin-3-yl)-6-fluoroimidazo1,2-a]pyridin-2-amine |
| 3-(4-amino-6-methyl-1,3,5-triazin-2-yl)-N-(5-fluoro-6-methoxy-3-pyridinyl)-6-(1-piperazinyl)imidazo[1,2-a]pyridin-2-amine |
| 3-(4-amino-6-methyl-1,3,5-triazin-2-yl)-N-(5-fluoro-6-methoxy-3-pyridinyl)-6-(4-methyl-1-piperazinyl)imidazo[1,2-a]pyridin-2-amine |
| 3-(4-amino-6-methyl-1,3,5-triazin-2-yl)-N-(5-fluoro-6-methoxy-3-pyridinyl)-6-(4-(1-methyl-ethyl)-1-piperazinyl)-imidazo[1,2-a]pyridin-2-amine |
| 3-(4-amino-6-methyl-1,3,5-triazin-2-yl)-N-(5-fluoro-6-methoxy-3-pyridinyl)-6-(4-(2-methoxyethyl)-1-piperazinyl)imidazo[1,2-a]pyridin-2-amine |
| 3-(2-methyl-6-(phenylamino)-4-pyrimidinyl)-N-1H-pyrazol-3-ylimidazo[1,2-b]pyridazin-2-amine |
| 3-(6-((2-fluorophenyl)amino)-2-methyl-4-pyrimidinyl)-N-1H-pyrazol-3-ylimidazo[1,2-b]pyridazin-2-amine |
| 3-(6-((4-fluorophenyl)amino)-2-methyl-4-pyrimidinyl)-N-1H-pyrazol-3-ylimidazo[1,2-b]pyridazin-2-amine |
| 3-(6-((4-methoxyphenyl)amino)-2-methyl-4-pyrimidinyl)-N-1H-pyrazol-3-ylimidazo[1,2-b]pyridazin-2-amine |
| N-(1-methyl-1H-pyrazol-3-yl)-3-(2-methyl-6-(3-pyridazinylamino)-4-pyrimidinyl)imidazo[1,2-b]pyridazin-2-amine |
| 3-(2-methyl-6-((6-(4-methyl-1-piperazinyl)-3-pyridazinyl)amino)-4-pyrimidinyl)-N-1H-pyrazol-3-ylimidazo[1,2-b]pyridazin-2-amine |
| 3-(6-((5-methoxy-2-pyridinyl)amino)-2-methyl-4-pyrimidinyl)-N-1H-pyrazol-3-ylimidazo[1,2-b]pyridazin-2-amine |
| 3-(6-((3-fluorophenyl)amino)-2-methyl-4-pyrimidinyl)-N-1H-pyrazol-3-ylimidazo[1,2-b]pyridazin-2-amine |
| 3-(6-((4-methoxy-2-pyridinyl)amino)-2-methyl-4-pyrimidinyl)-N-1H-pyrazol-3-ylimidazo[1,2-b]pyridazin-2-amine |
| 3-(2-methyl-6-((6-(4-morpholinyl)-3-pyridazinyl)amino)-4-pyrimidinyl)-N-1H-pyrazol-3-ylimidazo[1,2-b]pyridazin-2-amine |
| 1-(4-((3-(6-amino-2-methyl-4-pyrimidinyl)imidazo[1,2-a]pyridin-2-yl)amino)phenyl)-3-ethylurea |
| 3-(6-amino-2-methyl-4-pyrimidinyl)-N-1H-pyrrolo[2,3-b]pyridin-6-ylimidazo[1,2-a]pyridin-2-amine |
| 3-(6-amino-2-methyl-4-pyrimidinyl)-N-(6-(trifluoromethyl)-2-pyridinyl)imidazo[1,2-a]pyridin-2-amine |
| 3-(6-amino-2-methyl-4-pyrimidinyl)-N-2-pyridinylimidazo[1,2-a]pyridin-2-amine |
| 3-(6-((5-(2-methoxyethoxy)-3-pyridazinyl)amino)-2-methyl-4-pyrimidinyl)-N-1H-pyrazol-3-ylimidazo[1,2-b]pyridazin-2-amine |
| N-(3-(6-amino-2-methyl-4-pyrimidinyl)imidazo[1,2-a]pyridin-2-yl)-1H-pyrrolo[3,2-b]pyridin-5-amine |
| 2-((3-(6-amino-2-methyl-4-pyrimidinyl)imidazo[1,2-a]pyridin-2-yl)amino)-4-pyridinol |
| 1-(4-((3-(6-amino-2-methyl-4-pyrimidinyl)imidazo[1,2-a]pyridin-2-yl)amino)phenyl)-3-phenylurea |
| 1-(6-((3-(6-amino-2-methyl-4-pyrimidinyl)imidazo[1,2-a]pyridin-2-yl)amino)-3-pyridinyl)-3-ethylurea |
| 3-(2-methyl-6-((6-(4-morpholinyl)-3-pyridazinyl)amino)-4-pyrimidinyl)-N-1H-pyrazol-3-ylimidazo[1,2-a]pyridin-2-amine |
| 1-(4-((3-(6-amino-2-methyl-4-pyrimidinyl)imidazo[1,2-a]pyridin-2-yl)amino)phenyl)-3-methylurea |
| 3-((3-(6-((5-(2-methoxyethoxy)-3-pyridazinyl)amino)-2-methyl-4-pyrimidinyl)imidazo[1,2-b]pyridazin-2-yl)amino)phenol |
| N-(4-((3-(6-((5-(2-methoxyethoxy)-3-pyridazinyl)amino)-2-methyl-4-pyrimidinyl)imidazo[1,2-b]pyridazin-2-yl)amino)phenyl)acetamide |
| 3-(6-((5-methoxy-3-pyridazinyl)amino)-2-methyl-4-pyrimidinyl)-N-1H-pyrazol-3-ylimidazo-[1,2-b]pyridazin-2-amine |
| 1-ethyl-3-(4-((3-(6-((5-(2-methoxyethoxy)-3-pyridazinyl)amino)-2-methyl-4-pyrimidinyl)-imidazo[1,2-b]pyridazin-2-yl)amino)phenyl)urea |
| 3-(2-methyl-6-((5-methyl-3-pyridazinyl)amino)-4-pyrimidinyl)-N-1H-pyrazol-3-ylimidazo[1,2-b]pyridazin-2-amine |
| 1-(4-((3-(6-amino-2-methyl-4-pyrimidinyl)imidazo[1,2-a]pyridin-2-yl)amino)phenyl)-3-(4-pyridinyl)urea |
| 3-(6-((5-(2-methoxyethoxy)-3-pyridazinyl)amino)-2-methyl-4-pyrimidinyl)-N-1H-pyrazol-3-ylimidazo[1,2-a]pyridin-2-amine |
| N-(6-(2-((4-((ethylcarbamoyl)amino)phenyl)amino)imidazo[1,2-a]pyridin-3-yl)-2-methyl-4-pyrimidinyl)acetamide |
| 3-(2-methyl-6-((5-(2,2,2-trifluoroethoxy)-3-pyridazinyl)amino)-4-pyrimidinyl)-N-1H-pyrazol-3-ylimidazo[1,2-b]pyridazin-2-amine |
| N-'5-'-(3-(6-((5-(2-methoxyethoxy)-3-pyridazinyl)amino)-2-methyl-4-pyrimidinyl)imidazo[1,2-b]pyridazin-2-yl)-2,5-pyrimidinediamine |
| 8-fluoro-3-(6-((5-(2-methoxyethoxy)-3-pyridazinyl)amino)-2-methyl-4-pyrimidinyl)-N-1H-pyrazol-3-ylimidazo[1,2-a]pyridin-2-amine |
| N-(6-(8-fluoro-2-((1-methyl-1H-pyrazol-3-yl)amino)imidazo[1,2-a]pyridin-3-yl)-2-methyl-4-pyrimidinyl)acetamide |
| 3-(6-amino-2-methyl-4-pyrimidinyl)-N-4-pyridazinylimidazo[1,2-b]pyridazin-2-amine |
| 3-(6-amino-2-methyl-4-pyrimidinyl)-N-(1-methyl-1H-pyrazol-3-yl)-6-(3-(methylsulfonyl)phenyl)imidazo[1,2-a]pyridin-2-amine |
| 3-(2-methyl-6-(methylamino)-4-pyrimidinyl)-N-1H-pyrazol-3-ylimidazo[1,2-b]pyridazin-2-amine |
| N-(1-acetyl-1H-pyrazol-3-yl)-3-(6-amino-2-methyl-4-pyrimidinyl)imidazo[1,2-b]pyridazin-2-amine |
| 3-(6-amino-2-methyl-4-pyrimidinyl)-N-1H-pyrazol-3-yl-6-(4-pyridinyl)imidazo[1,2-b]pyridazin-2-amine |
| 3-(6-amino-2-methyl-4-pyrimidinyl)-6-(2-methoxyethoxy)-N-1H-pyrazol-3-ylimidazo[1,2-b]pyridazin-2-amine |
| N-(2-methyl-6-(2-(1H-pyrazol-3-ylamino)imidazo[1,2-b]pyridazin-3-yl)-4-pyrimidinyl)-acetamide |
| 3-(6-amino-2-methyl-4-pyrimidinyl)-6-(4-morpholinyl)-N-1H-pyrazol-3-ylimidazo[1,2-b]pyridazin-2-amine |
| 3-(2-methyl-6-(methylamino)-4-pyrimidinyl)-6-(4-morpholinyl)-N-1H-pyrazol-3-ylimidazo[1,2-b]pyridazin-2-amine |
| 3-(6-amino-2-methyl-4-pyrimidinyl)-N-1H-pyrazol-3-yl-6-(2-(1-pyrrolidinyl)ethoxy)-imidazo[1,2-b]pyridazin-2-amine |
| 3-(2-methyl-6-(2-pyrazinylamino)-4-pyrimidinyl)-N-1H-pyrazol-3-ylimidazo[1,2-b]pyridazin-2-amine |
| 1,1-dimethyl-3-(2-methyl-6-(2-(1H-pyrazol-3-ylamino)imidazo[1,2-b]pyridazin-3-yl)-4-pyrimidinyl)urea |
| 1-ethyl-3-(2-methyl-6-(2-(1H-pyrazol-3-ylamino)imidazo[1,2-b]pyridazin-3-yl)-4-pyrimidinyl)urea |
| 4-methyl-N-(2-methyl-6-(2-(1H-pyrazol-3-ylamino)imidazo[1,2-b]pyridazin-3-yl)-4-pyrimidinyl)-1-piperazinecarboxamide |
| N-(2-methyl-6-(2-(1H-pyrazol-3-ylamino)imidazo[1,2-b]pyridazin-3-yl)-4-pyrimidinyl)-4-morpholinecarboxamide |
| 3-(2-methyl-6-(1H-pyrazol-3-ylamino)-4-pyrimidinyl)-N-1H-pyrazol-3-ylimidazo[1,2-b]pyridazin-2-amine |
| 3-(6-(dimethylamino)-2-methyl-4-pyrimidinyl)-N-(1-methyl-1H-pyrazol-3-yl)imidazo[1,2-b]pyridazin-2-amine |
| 3-(2-methyl-6-(1,3-thiazol-2-ylamino)-4-pyrimidinyl)-N-1H-pyrazol-3-ylimidazo[1,2-b]pyridazin-2-amine |
| 3-(2-methyl-6-(2-pyridinylamino)-4-pyrimidinyl)-N-1H-pyrazol-3-ylimidazo[1,2-b]pyridazin-2-amine |
| 3-(2-methyl-6-(3-pyridazinylamino)-4-pyrimidinyl)-N-1H-pyrazol-3-ylimidazo[1,2-b]pyridazin-2-amine |
| 3-(2-methyl-6-(4-pyrimidinylamino)-4-pyrimidinyl)-N-1H-pyrazol-3-ylimidazo[1,2-b]pyridazin-2-amine |
| 3-(2-methyl-6-(2-pyrimidinylamino)-4-pyrimidinyl)-N-1H-pyrazol-3-ylimidazo[1,2-b]pyridazin-2-amine |
| N-(2-methyl-6-(2-((1-methyl-1H-pyrazol-3-yl)amino)imidazo[1,2-b]pyridazin-3-yl)-4-pyrimidinyl)acetamide |
| 3-(6-amino-2-methyl-4-pyrimidinyl)-7-(4-morpholinyl)-N-1H-pyrazol-3-ylimidazo[1,2-b]pyridazin-2-amine |
| 3-(2-methyl-6-(methylamino)-4-pyrimidinyl)-7-(4-morpholinyl)-N-1H-pyrazol-3-ylimidazo[1,2-b]pyridazin-2-amine |
| N-(2-methyl-6-(2-(1H-pyrazol-3-ylamino)imidazo[1,2-b]pyridazin-3-yl)-4-pyrimidinyl)-methanesulfonamide |
| ((2-methyl-6-(2-(1H-pyrazol-3-ylamino)imidazo[1,2-b]pyridazin-3-yl)-4-pyrimidinyl)amino)-acetonitrile |
| 3-(6-amino-2-methyl-4-pyrimidinyl)-8-fluoro-N-1H-pyrazol-3-ylimidazo[1,2-a]pyridin-2-amine |
| N-2-,N-2--dimethyl-N-(2-methyl-6-(2-((1-methyl-1H-pyrazol-3-yl)amino)imidazo[1,2-b]pyridazin-3-yl)-4-pyrimidinyl)glycinamide |
| N-(2-methyl-6-(2-((1-methyl-1H-pyrazol-3-yl)amino)imidazo[1,2-b]pyridazin-3-yl)-4-pyrimidinyl)glycinamide |
| 3-(6-amino-2-methyl-4-pyrimidinyl)-N-(3-(methylsulfonyl)phenyl)imidazo[1,2-b]pyridazin-2-amine |
| 3-(6-amino-2-methyl-4-pyrimidinyl)-N-3-pyridinylimidazo[1,2-b]pyridazin-2-amine |
| 3-(6-amino-2-methyl-4-pyrimidinyl)-N-(3-methyl-5-isothiazolyl)imidazo[1,2-b]pyridazin-2-amine |
| tert-butyl 4-(3-(4-amino-6-methyl-1,3,5-triazin-2-yl)-2-(1H-pyrazol-3-ylamino)imidazo[1,2-a]pyridin-6-yl)-3,6-dihydro-1(2H)-pyridinecarboxylate |
| 3-(4-amino-6-methyl-1,3,5-triazin-2-yl)-6-(3-methoxyphenyl)-N-1H-pyrazol-3-ylimidazo[1,2-a]pyridin-2-amine |
| 4-(3-(4-amino-6-methyl-1,3,5-triazin-2-yl)-2-(1H-pyrazol-3-ylamino)imidazo[1,2-a]pyridin-6-yl)-2-fluoro-N-methylbenzamide |
| N-(4-(3-(4-amino-6-methyl-1,3,5-triazin-2-yl)-2-(1H-pyrazol-3-ylamino)imidazo[1,2-a]pyridin-6-yl)benzyl)acetamide |
| 3-(4-amino-6-methyl-1,3,5-triazin-2-yl)-6-(1-(2-methoxyethyl)-1H-pyrazol-4-yl)-N-1H-pyrazol-3-ylimidazo[1,2-a]pyridin-2-amine |
| 3-(4-amino-6-methyl-1,3,5-triazin-2-yl)-6-(2-fluorophenyl)-N-1H-pyrazol-3-ylimidazo[1,2-a]pyridin-2-amine |
| 3-(4-amino-6-methyl-1,3,5-triazin-2-yl)-6-(2-methylphenyl)-N-1H-pyrazol-3-ylimidazo[1,2-a]pyridin-2-amine |
| 3-(4-amino-6-methyl-1,3,5-triazin-2-yl)-6-(2-methoxy-3-pyridinyl)-N-1H-pyrazol-3-ylimidazo[1,2-a]pyridin-2-amine |
| 3-(4-amino-6-methyl-1,3,5-triazin-2-yl)-6-(3-furanyl)-N-1H-pyrazol-3-ylimidazo[1,2-a]pyridin-2-amine |
| 3-(4-amino-6-methyl-1,3,5-triazin-2-yl)-6-(3,5-dimethyl-4-isoxazolyl)-N-1H-pyrazol-3-ylimidazo[1,2-a]pyridin-2-amine |
| 3-(4-amino-6-methyl-1,3,5-triazin-2-yl)-6-(4-fluoro-2-methylphenyl)-N-1H-pyrazol-3-ylimidazo[1,2-a]pyridin-2-amine |
| 3-(4-amino-6-methyl-1,3,5-triazin-2-yl)-6-(2-(1-methylethoxy)phenyl)-N-1H-pyrazol-3-ylimidazo[1,2-a]pyridin-2-amine |
| 3-(4-amino-6-methyl-1,3,5-triazin-2-yl)-6-(3-(methylsulfanyl)phenyl)-N-1H-pyrazol-3-ylimidazo[1,2-a]pyridin-2-amine |
| N-(4-(3-(4-methyl-6-(methylamino)-1,3,5-triazin-2-yl)-2-(1H-pyrazol-3-ylamino)imidazo[1,2-a]pyridin-6-yl)benzyl)acetamide |
| 3-(4-amino-6-methyl-1,3,5-triazin-2-yl)-N-1H-pyrazol-3-yl-6-(1,2,3,6-tetrahydro-4-pyridinyl)-imidazo[1,2-a]pyridin-2-amine |
| 3-(4-amino-6-methyl-1,3,5-triazin-2-yl)-6-(3-methyl-5-isoxazolyl)-N-1H-pyrazol-3-ylimidazo-[1,2-a]pyridin-2-amine |
| 6-(1-(2-methoxyethyl)-1H-pyrazol-4-yl)-3-(4-methyl-6-(methylamino)-1,3,5-triazin-2-yl)-N-1H-pyrazol-3-ylimidazo[1,2-a]pyridin-2-amine |
| N-(3-(3-(4-amino-6-methyl-1,3,5-triazin-2-yl)-2-(1H-pyrazol-3-ylamino)imidazo[1,2-a]pyridin-6-yl)phenyl)acetamide |
| N-(3-(3-(4-amino-6-methyl-1,3,5-triazin-2-yl)-2-(1H-pyrazol-3-ylamino)imidazo[1,2-a]pyridin-6-yl)phenyl)cyclopropanecarboxamide |
| N-(4-(3-(4-amino-6-methyl-1,3,5-triazin-2-yl)-2-(1H-pyrazol-3-ylamino)imidazo[1,2-a]pyridin-6-yl)phenyl)-2-methylpropanamide |
| 3-(4-amino-6-methyl-1,3,5-triazin-2-yl)-N-1H-pyrazol-3-yl-6-(3-pyridazinyl)imidazo[1,2-a]pyridin-2-amine |
| 3-(4-amino-6-methyl-1,3,5-triazin-2-yl)-6-(1-methyl-1,2,3,6-tetrahydro-4-pyridinyl)-N-1H-pyrazol-3-ylimidazo[1,2-a]pyridin-2-amine |
| N-(4-(3-(6-amino-2-methyl-4-pyrimidinyl)-2-(1H-pyrazol-3-ylamino)imidazo[1,2-b]pyridazin-6-yl)benzyl)acetamide |
| N-(2-methyl-6-(2-(1H-pyrazol-3-ylamino)imidazo[1,2-b]pyridazin-3-yl)-4-pyrimidinyl)-cyclopropanecarboxamide |
| N-(4-(3-(6-amino-2-methyl-4-pyrimidinyl)-2-(1H-pyrazol-3-ylamino)imidazo[1,2-b]pyridazin-6-yl)phenyl)acetamide |
| 4-(3-(6-amino-2-methyl-4-pyrimidinyl)-2-(1H-pyrazol-3-ylamino)imidazo[1,2-b]pyridazin-6-yl)-N-methylbenzamide |
| 2-methoxy-N-(2-methyl-6-(2-((1-methyl-1H-pyrazol-3-yl)amino)imidazo[1,2-b]pyridazin-3-yl)-4-pyrimidinyl) acetamide |
| 3-(6-amino-2-methyl-4-pyrimidinyl)-N-1H-pyrazol-3-ylimidazo[1,2-a]pyridin-2-amine |
| 3-(6-amino-2-methyl-4-pyrimidinyl)-N-(1-methyl-1H-pyrazol-3-yl)imidazo[1,2-a]pyridin-2-amine |
| 2-cyano-N-(2-methyl-6-(2-((1-methyl-1H-pyrazol-3-yl)amino)imidazo[1,2-a]pyridin-3-yl)-4-pyrimidinyl)acetamide |
| 2-(3-methoxyphenyl)-N-(2-methyl-6-(2-((1-methyl-1H-pyrazol-3-yl)amino)imidazo[1,2-a]-pyridin-3-yl)-4-pyrimidinyl)acetamide |
| 2-(3-fluorophenyl)-N-(2-methyl-6-(2-((1-methyl-1H-pyrazol-3-yl)amino)imidazo[1,2-a]-pyridin-3-yl)-4-pyrimidinyl)acetamide |
| 2-(2,4-difluorophenyl)-N-(2-methyl-6-(2-((1-methyl-1H-pyrazol-3-yl)amino)imidazo[1,2-b]pyridazin-3-yl)-4-pyrimidinyl)acetamide |
| 3-cyano-N-(2-methyl-6-(2-((1-methyl-1H-pyrazol-3-yl)amino)imidazo[1,2-b]pyridazin-3-yl)-4-pyrimidinyl)benzamide |
| 3,3,3-trifluoro-N-(1-methyl-1H-pyrazol-3-yl)-N-(3-(2-methyl-6-((3,3,3-trifluoropropanoyl)-amino)-4-pyrimidinyl)imidazo[1,2-a]pyridin-2-yl)propanamide |
| 3-(6-amino-2-methyl-4-pyrimidinyl)-N-(1-methyl-1H-pyrazol-3-yl)-6-(2-methyl-4-pyridinyl)-imidazo[1,2-b]pyridazin-2-amine |
| 3-(6-amino-2-methyl-4-pyrimidinyl)-N-(1-methyl-1H-pyrazol-3-yl)-6-(2-methyl-3-pyridinyl)-imidazo[1,2-b]pyridazin-2-amine |
| 3,3,3-trifluoro-N-(2-methyl-6-(2-((1-methyl-1H-pyrazol-3-yl)amino)imidazo[1,2-b]pyridazin-3-yl)-4-pyrimidinyl)propanamide |
| 3,3,3-trifluoro-N-(2-methyl-6-(2-((1-methyl-1H-pyrazol-3-yl)amino)imidazo[1,2-a]pyridin-3-yl)-4-pyrimidinyl)propanamide |
| 3-(6-amino-2-methyl-4-pyrimidinyl)-N-(1-methyl-1H-pyrazol-3-yl)-6-(5-pyrimidinyl)imidazo-[1,2-b]pyridazin-2-amine |
| 3-(6-((6-(2-methoxyethoxy)-3-pyridazinyl)amino)-2-methyl-4-pyrimidinyl)-N-1H-pyrazol-3-ylimidazo[1,2-b]pyridazin-2-amine |
| 2-(4-(6-((2-methyl-6-(2-(1H-pyrazol-3-ylamino)imidazo[1,2-b]pyridazin-3-yl)-4-pyrimidinyl)amino)-3-pyridazinyl)-2-morpholinyl)ethanol |
| 3-(6-amino-2-methyl-4-pyrimidinyl)-N-(4-(4-methyl-1-piperazinyl)phenyl)imidazo[1,2-b]pyridazin-2-amine |
| 3-(2-methyl-6-((6-(2-methyl-4-morpholinyl)-3-pyridazinyl)amino)-4-pyrimidinyl)-N-1 H-pyrazol-3-ylimidazo[1,2-b]pyridazin-2-amine |
| 3-(2-methyl-6-((6-(3-methyl-4-morpholinyl)-3-pyridazinyl)amino)-4-pyrimidinyl)-N-1H-pyrazol-3-ylimidazo[1,2-b]pyridazin-2-amine |
| N-(3-(6-amino-2-methyl-4-pyrimidinyl)imidazo[1,2-b]pyridazin-2-yl)-1H-pyrazolo[3,4-c]pyridin-3-amine |
| 3-(6-amino-2-methyl-4-pyrimidinyl)-N-(4-fluoro-1H-indazol-3-yl)imidazo[1,2-b]pyridazin-2-amine |
| N-(3-(6-amino-2-methyl-4-pyrimidinyl)imidazo[1,2-b]pyridazin-2-yl)-1H-pyrazolo[4,3-c]pyridin-3-amine |
| N-(2-methyl-6-(2-(1H-pyrazol-3-ylamino)imidazo[1,2-a]pyridin-3-yl)-4-pyrimidinyl)acetamide |
| 3-(6-amino-2-methyl-4-pyrimidinyl)-N-(1-(2-(dimethylamino)ethyl)-1H-pyrazol-3-yl)imidazo-[1,2-b]pyridazin-2-amine |
| N-(6-(2-((1-(2-(dimethylamino)ethyl)-1H-pyrazol-3-yl)amino)imidazo-[1,2-b]pyridazin-3-yl)-2-methyl-4-pyrimidinyl)acetamide |
| N-(2-methyl-6-(2-((1-methyl-1H-pyrazol-3-yl)amino)imidazo[1,2-a]pyridin-3-yl)-4-pyrimidinyl)acetamide |
| 3-(2-methyl-6-((6-(4-morpholinyl)-3-pyridazinyl)amino)-4-pyrimidinyl)-N-(1-methyl-1H-pyrazol-3-yl)imidazo[1,2-b]pyridazin-2-amine |
| N-(3-(6-amino-2-methyl-4-pyrimidinyl)imidazo[1,2-b]pyridazin-2-yl)-1H-pyrazolo[3,4-b]pyridin-3-amine |
| 3-(6-amino-2-methyl-4-pyrimidinyl)-N-(3-(4-methyl-1-piperazinyl)-phenyl)imidazo[1,2-b]pyridazin-2-amine |
| N-(3-((3-(6-(acetylamino)-2-methyl-4-pyrimidinyl)imidazo[1,2-b]pyridazin-2-yl)amino)-phenyl)acetamide |
| 3-(6-amino-2-methyl-4-pyrimidinyl)-6-(1-(2-methoxyethyl)-1H-pyrazol-4-yl)-N-(1-methyl-1H-pyrazol-3-yl)imidazo[1,2-a]pyridin-2-amine |
| 1-(4-((3-(4-amino-6-methyl-1,3,5-triazin-2-yl)imidazo[1,2-a]pyridin-2-yl)amino)phenyl)-3-methylurea |
| 3-(4-amino-6-methyl-1,3,5-triazin-2-yl)-N-1H-pyrazol-3-yl-6-(3-pyridinyl)imidazo[1,2-a]pyridin-2-amine |
| 3-(4-amino-6-methyl-1,3,5-triazin-2-yl)-N-6--phenyl-N-2--1H-pyrazol-3-ylimidazo[1,2-a]pyridine-2,6-diamine |
| 3-(4-amino-6-methyl-1,3,5-triazin-2-yl)-N-6--(4-methoxyphenyl)-N-2--1H-pyrazol-3-ylimidazo[1,2-a]pyridine-2,6-diamine |
| 3-(4-amino-6-methyl-1,3,5-triazin-2-yl)-7-methoxy-N-1H-pyrazol-3-ylimidazo[1,2-a]pyridin-2-amine |
| 3-(4-amino-6-methyl-1,3,5-triazin-2-yl)-N-1H-pyrazol-3-yl-6-(5-pyrimidinyl)imidazo[1,2-a]pyridin-2-amine |
| 3-(4-methyl-6-(methylamino)-1,3,5-triazin-2-yl)-N-1H-pyrazol-3-yl-6-(5-pyrimidinyl)-imidazo[1,2-a]pyridin-2-amine |
| 3-(4-amino-6-methyl-1,3,5-triazin-2-yl)-6-(3-fluorophenyl)-N-1H-pyrazol-3-ylimidazo[1,2-a]pyridin-2-amine |
| 4-(3-(4-amino-6-methyl-1,3,5-triazin-2-yl)-2-(1H-pyrazol-3-ylamino)imidazo[1,2-a]pyridin-6-yl)-N-methylbenzamide |
| 3-(4-amino-6-methyl-1,3,5-triazin-2-yl)-N-2--1H-pyrazol-3-yl-N-6--3-pyridinylimidazo[1,2-a]pyridine-2,6-diamine |
| N-(4-(3-(4-amino-6-methyl-1,3,5-triazin-2-yl)-2-(1H-pyrazol-3-ylamino)imidazo[1,2-a]pyridin-6-yl)phenyl)acetamide |
| 3-(4-amino-6-methyl-1,3,5-triazin-2-yl)-N-6--(3-methoxyphenyl)-N-2--1H-pyrazol-3-ylimidazo[1,2-a]pyridine-2,6-diamine |
| 3-(4-amino-6-methyl-1,3,5-triazin-2-yl)-N-2--1H-pyrazol-3-yl-N-6--4-pyridinylimidazo[1,2-a]pyridine-2,6-diamine |
| 3-(4-amino-6-methyl-1,3,5-triazin-2-yl)-N-2--1H-pyrazol-3-yl-N-6--5-pyrimidinylimidazo-[1,2-a]pyridine-2,6-diamine |
| N-(4-((3-(4-amino-6-methyl-1,3,5-triazin-2-yl)-2-(1H-pyrazol-3-ylamino)imidazo[1,2-a]pyridin-6-yl)amino)phenyl)acetamide |
| 3-(4-amino-6-methyl-1,3,5-triazin-2-yl)-6-(2-methyl-3-pyridinyl)-N-1H-pyrazol-3-ylimidazo[1,2-a]pyridin-2-amine |
| 3-(4-amino-6-methyl-1,3,5-triazin-2-yl)-N-6--(2-methyl-4-pyridinyl)-N-2--1H-pyrazol-3-ylimidazo[1,2-a]pyridine-2,6-diamine |
| N-methyl-4-(3-(4-methyl-6-(methylamino)-1,3,5-triazin-2-yl)-2-(1H-pyrazol-3-ylamino)-imidazo[1,2-a]pyridin-6-yl)benzamide |
| 3-(4-amino-6-methyl-1,3,5-triazin-2-yl)-6-(4-methyl-3-pyridinyl)-N-1H-pyrazol-3-ylimidazo-[1,2-a]pyridin-2-amine |
| 3-(4-amino-6-methyl-1,3,5-triazin-2-yl)-6-(2,6-dimethyl-4-pyridinyl)-N-1H-pyrazol-3-yl-imidazo[1,2-a]pyridin-2-amine |
| 4-(3-(4-amino-6-methyl-1,3,5-triazin-2-yl)-2-(1H-pyrazol-3-ylamino)imidazo[1,2-a]pyridin-6-yl)-N-(1-methylethyl)benzamide |
| 6-(3-(4-amino-6-methyl-1,3,5-triazin-2-yl)-2-(1H-pyrazol-3-ylamino)imidazo[1,2-a]pyridin-6-yl)-3,4-dihydro-1(2H)-isoquinolinone |
| 4-(3-(4-amino-6-methyl-1,3,5-triazin-2-yl)-2-(1H-pyrazol-3-ylamino)imidazo[1,2-a]pyridin-6-yl)-N-ethylbenzamide |
| 4-(3-(4-amino-6-methyl-1,3,5-triazin-2-yl)-2-(1H-pyrazol-3-ylamino)imidazo[1,2-a]pyridin-6-yl)-3-methylbenzoic acid |
| 3-(2-methyl-6-(methylamino)-4-pyrimidinyl)-N-1H-pyrazol-3-yl-6-(5-pyrimidinyl)imidazo-[1,2-b]pyridazin-2-amine |
| 3-(4-amino-6-methyl-1,3,5-triazin-2-yl)-6-(4-methyl-5-pyrimidinyl)-N-1H-pyrazol-3-ylimidazo[1,2-a]pyridin-2-amine |
| 4-(3-(4-amino-6-methyl-1,3,5-triazin-2-yl)-2-(1H-pyrazol-3-ylamino)imidazo[1,2-a]pyridin-6-yl)-N,3-dimethylbenzamide |
| 5-(3-(4-amino-6-methyl-1,3,5-triazin-2-yl)-2-(1H-pyrazol-3-ylamino)imidazo[1,2-a]pyridin-6-yl)-N-methyl-2-pyridinecarboxamide |
| 3-(4-amino-6-methyl-1,3,5-triazin-2-yl)-2-(1H-pyrazol-3-ylamino)imidazo[1,2-a]pyridin-7-ol |
| tert-butyl 4-(3-(4-amino-6-methyl-1,3,5-triazin-2-yl)-2-(1H-pyrazol-3-ylamino)imidazo[1,2-a]pyridin-7-yl)-3,6-dihydro-1(2H)-pyridinecarboxylate |
| 3-(4-amino-6-methyl-1,3,5-triazin-2-yl)-N-1H-pyrazol-3-yl-7-(1,2,3,6-tetrahydro-4-pyridinyl)-imidazo[1,2-a]pyridin-2-amine |
| 3-(4-amino-6-methyl-1,3,5-triazin-2-yl)-7-(2-methoxyethoxy)-N-1H-pyrazol-3-ylimidazo[1,2-a]pyridin-2-amine |
| 3-(4-amino-6-methyl-1,3,5-triazin-2-yl)-N-1H-pyrazol-3-yl-6-(1H-pyrrolo[2,3-b]pyridin-5-yl)imidazo[1,2-a]pyridin-2-amine |
| 4-(3-(4-amino-6-methyl-1,3,5-triazin-2-yl)-2-(1H-pyrazol-3-ylamino)-imidazo[1,2-a]pyridin-7-yl)-N-methylbenzamide |
| 3-(4-amino-6-methyl-1,3,5-triazin-2-yl)-N-1H-pyrazol-3-yl-7-(2-(1-pyrrolidinyl)ethoxy)-imidazo[1,2-a]pyridin-2-amine |
| 4-(3-(6-amino-2-methyl-4-pyrimidinyl)-2-(1H-pyrazol-3-ylamino)-imidazo[1,2-b]pyridazin-6-yl)-N,N-dimethyl-1-piperazinecarboxamide |
| 3-(6-amino-2-methyl-4-pyrimidinyl)-N-2-pyridinylimidazo[1,2-b]pyridazin-2-amine |
| 3-(6-amino-2-methyl-4-pyrimidinyl)-N-(1-ethyl-1H-pyrazol-3-yl)imidazo[1,2-b]pyridazin-2-amine |
| N-(3-(6-amino-2-methyl-4-pyrimidinyl)imidazo[1,2-b]pyridazin-2-yl)-3-quinolinamine |
| N-(3-(6-amino-2-methyl-4-pyrimidinyl)imidazo[1,2-b]pyridazin-2-yl)-3-isoquinolinamine |
| N-(3-(6-amino-2-methyl-4-pyrimidinyl)imidazo[1,2-b]pyridazin-2-yl)-2-quinolinamine |
| 3-(6-amino-2-methyl-4-pyrimidinyl)-N-(1-methyl-1H-pyrazol-3-yl)imidazo[1,2-b]pyridazin-2-amine |
| 3-((3-(4-amino-6-methyl-1,3,5-triazin-2-yl)imidazo[1,2-a]pyridin-2-yl)amino)-N-methyl-1H-pyrazole-1-carboxamide |
| 5-((3-(4-amino-6-methyl-1,3,5-triazin-2-yl)imidazo[1,2-a]pyridin-2-yl)amino)-N-methyl-1H-pyrazole-1-carboxamide |
| 3-((3-(4-amino-6-methyl-1,3,5-triazin-2-yl)imidazo[1,2-a]pyridin-2-yl)amino)-N-(2-hydroxyethyl)-1H-pyrazole-1-carboxamide |
| 5-((3-(4-amino-6-methyl-1,3,5-triazin-2-yl)imidazo[1,2-a]pyridin-2-yl)amino)-N-(2-hydroxyethyl)-1H-pyrazole-1-carboxamide |
| 3-(4-amino-6-methyl-1,3,5-triazin-2-yl)-N-(1-(4-morpholinylcarbonyl)-1H-pyrazol-3-yl)-imidazo[1,2-a]pyridin-2-amine |
| 3-(4-amino-6-methyl-1,3,5-triazin-2-yl)-N-(1-(4-morpholinylcarbonyl)-1H-pyrazol-5-yl)-imidazo[1,2-a]pyridin-2-amine |
| 5-((3-(4-amino-6-methyl-1,3,5-triazin-2-yl)imidazo[1,2-a]pyridin-2-yl)amino)-N-3-pyridinyl-1H-pyrazole-1-carboxamide |
| 4-((3-(4-amino-6-methyl-1,3,5-triazin-2-yl)imidazo[1,2-a]pyridin-2-yl)amino)-N-methyl-1H-pyrazole-1-carboxamide |
| 3-((3-(4-amino-6-methyl-1,3,5-triazin-2-yl)imidazo[1,2-a]pyridin-2-yl)amino)-N-phenyl-1H-pyrazole-1-carboxamide |
| 5-((3-(4-amino-6-methyl-1,3,5-triazin-2-yl)imidazo[1,2-a]pyridin-2-yl)amino)-N-phenyl-1H-pyrazole-1-carboxamide |
| 3-(4-amino-6-methyl-1,3,5-triazin-2-yl)-N-1H-pyrazol-4-ylimidazo[1,2-a]pyridin-2-amine |
| methyl 3-((3-(4-amino-6-methyl-1,3,5-triazin-2-yl)imidazo[1,2-a]pyridin-2-yl)amino)-1H-pyrazole-1-carboxylate |
| methyl 5-((3-(4-amino-6-methyl-1,3,5-triazin-2-yl)imidazo[1,2-a]pyridin-2-yl)amino)-1H-pyrazole-1-carboxylate |
| phenyl 4-((3-(4-amino-6-methyl-1,3,5-triazin-2-yl)imidazo[1,2-a]pyridin-2-yl)amino)-1H-pyrazole-1-carboxylate |
| 3-((3-(6-amino-2-methyl-4-pyrimidinyl)imidazo[1,2-a]pyridin-2-yl)amino)-N-phenyl-1H-pyrazole-1-carboxamide |
| 5-((3-(6-amino-2-methyl-4-pyrimidinyl)imidazo[1,2-a]pyridin-2-yl)amino)-N-phenyl-1H-pyrazole-1-carboxamide |
| 3-((3-(6-amino-2-methyl-4-pyrimidinyl)imidazo[1,2-a]pyridin-2-yl)amino)-N-methyl-1H-pyrazole-1-carboxamide |
| 5-((3-(6-amino-2-methyl-4-pyrimidinyl)imidazo[1,2-a]pyridin-2-yl)amino)-N-methyl-1H-pyrazole-1-carboxamide |
| 4-((3-(6-amino-2-methyl-4-pyrimidinyl)imidazo[1,2-a]pyridin-2-yl)amino)-N-phenyl-1H-pyrazole-1-carboxamide |
| 4-((3-(6-amino-2-methyl-4-pyrimidinyl)imidazo[1,2-a]pyridin-2-yl)amino)-N-methyl-1H-pyrazole-1-carboxamide |
| 3-(6-amino-2-methyl-4-pyrimidinyl)-N-(1-(2-pyridinyl)-1H-pyrazol-3-yl)imidazo[1,2-a]pyridin-2-amine |
| 3-(6-amino-2-methyl-4-pyrimidinyl)-N-(1-(methylsulfonyl)-1H-pyrazol-3-yl)imidazo[1,2-a]pyridin-2-amine |
| 3-(6-amino-2-methyl-4-pyrimidinyl)-N-(1-(1,3-benzoxazol-2-yl)-1H-pyrazol-5-yl)imidazo[1,2-a]pyridin-2-amine |
| phenyl 3-((3-(6-amino-2-methyl-4-pyrimidinyl)imidazo[1,2-a]pyridin-2-yl)amino)-1H-pyrazole-1-carboxylate |
| 3-(6-amino-2-methyl-4-pyrimidinyl)-N-(1-(1H-benzimidazol-2-yl)-1H-pyrazol-3-yl)imidazo-[1,2-a]pyridin-2-amine |
| 3-((3-(6-amino-2-methyl-4-pyrimidinyl)imidazo[1,2-a]pyridin-2-yl)amino)-N-(1-methylethyl)-1 H-pyrazole-1-carboxamide |
| 3-((3-(6-amino-2-methyl-4-pyrimidinyl)imidazo[1,2-a]pyridin-2-yl)amino)-N-benzyl-1H-pyrazole-1-carboxamide |
| methyl 4-(((3-((3-(6-amino-2-methyl-4-pyrimidinyl)imidazo[1,2-a]pyridin-2-yl)amino)-1H-pyrazol-1-yl)carbonyl)amino)benzoate |
| 3-((3-(6-amino-2-methyl-4-pyrimidinyl)imidazo[1,2-a]pyridin-2-yl)amino)-N-ethyl-1H-pyrazole-1-carboxamide |
| 3-((3-(6-amino-2-methyl-4-pyrimidinyl)imidazo[1,2-a]pyridin-2-yl)amino)-N-(2-methoxyphenyl)-1H-pyrazole-1-carboxamide |
| 3-(6-amino-2-methyl-4-pyrimidinyl)-N-1H-pyrazol-4-ylimidazo[1,2-a]pyridin-2-amine |
| 3-((3-(6-amino-2-methyl-4-pyrimidinyl)imidazo[1,2-a]pyridin-2-yl)amino)-N-(2-methylphenyl)-1H-pyrazole-1-carboxamide |
| 3-((3-(6-amino-2-methyl-4-pyrimidinyl)imidazo[1,2-a]pyridin-2-yl)amino)-N-(4-methoxy-phenyl)-1H-pyrazole-1-carboxamide |
| 3-((3-(6-amino-2-methyl-4-pyrimidinyl)imidazo[1,2-b]pyridazin-2-yl)amino)-N-phenyl-1H-pyrazole-1-carboxamide, trifluoroacetic acid |
| 3-((3-(6-amino-2-methyl-4-pyrimidinyl)imidazo[1,2-b]pyridazin-2-yl)amino)-N-methyl-1H-pyrazole-1-carboxamide |
| 5-((3-(6-amino-2-methyl-4-pyrimidinyl)imidazo[1,2-b]pyridazin-2-yl)amino)-N-methyl-1H-pyrazole-1-carboxamide |
| methyl 4-((3-(6-amino-2-methyl-4-pyrimidinyl)imidazo[1,2-a]pyridin-2-yl)amino)-1H-pyrazole-1-carboxylate |
| 3-(6-amino-2-methyl-4-pyrimidinyl)-N-(1-(1,3-benzoxazol-2-yl)-1H-pyrazol-3-yl)imidazo[1,2-b]pyridazin-2-amine |
| methyl 5-((3-(6-amino-2-methyl-4-pyrimidinyl)imidazo[1,2-b]pyridazin-2-yl)amino)-1H-pyrazole-1-carboxylate |
| 3-((3-(6-amino-2-methyl-4-pyrimidinyl)imidazo[1,2-b]pyridazin-2-yl)amino)-N-(2-methylphenyl)-1H-pyrazole-1-carboxamide |
| 3-((3-(6-amino-2-methyl-4-pyrimidinyl)imidazo[1,2-b]pyridazin-2-yl)amino)-N-(2-fluorophenyl)-1H-pyrazole-1-carboxamide |
| 3-(6-amino-2-methyl-4-pyrimidinyl)-N-(1-(1,3-benzoxazol-2-yl)-1H-pyrazol-5-yl)imidazo[1,2-b]pyridazin-2-amine |
| 3-((3-(6-amino-2-methyl-4-pyrimidinyl)imidazo[1,2-b]pyridazin-2-yl)amino)-N-(4-cyanophenyl)-1H-pyrazole-1-carboxamide |
| 3-((3-(6-amino-2-methyl-4-pyrimidinyl)imidazo[1,2-b]pyridazin-2-yl)amino)-N-(2-(trifluoromethyl)phenyl)-1H-pyrazole-1-carboxam ide |
| 3-((3-(6-amino-2-methyl-4-pyrimidinyl)imidazo[1,2-b]pyridazin-2-yl)amino)-N-(3-methylphenyl)-1H-pyrazole-1-carboxamide |
| 3-((3-(6-amino-2-methyl-4-pyrimidinyl)imidazo[1,2-b]pyridazin-2-yl)amino)-N-(2,5-difluorophenyl)-1H-pyrazole-1-carboxamide |
| 3-((3-(6-amino-2-methyl-4-pyrimidinyl)imidazo[1,2-b]pyridazin-2-yl)amino)-N-(3,5-difluorophenyl)-1H-pyrazole-1-carboxamide |
| 3-((3-(6-amino-2-methyl-4-pyrimidinyl)imidazo[1,2-b]pyridazin-2-yl)amino)-N-(1-methylethyl)-1H-pyrazole-1-carboxamide |
| 3-((3-(6-amino-2-methyl-4-pyrimidinyl)imidazo[1,2-b]pyridazin-2-yl)amino)-N-(4-methylphenyl)-1H-pyrazole-1-carboxamide |
| 3-((3-(6-amino-2-methyl-4-pyrimidinyl)imidazo[1,2-b]pyridazin-2-yl)amino)-N-(3,5-dichlorophenyl)-1H-pyrazole-1-carboxamide |
| 3-((3-(6-amino-2-methyl-4-pyrimidinyl)imidazo[1,2-b]pyridazin-2-yl)amino)-N-(2-chloro-5-(trifluoromethyl)phenyl)-1H-pyrazole-1-carboxam ide |
| 3-((3-(6-amino-2-methyl-4-pyrimidinyl)imidazo[1,2-b]pyridazin-2-yl)amino)-N-(2,5-dichlorophenyl)-1H-pyrazole-1-carboxamide |
| 3-((3-(6-amino-2-methyl-4-pyrimidinyl)imidazo[1,2-b]pyridazin-2-yl)amino)-N-(2,3-dichlorophenyl)-1H-pyrazole-1-carboxamide |
| 3-((3-(6-amino-2-methyl-4-pyrimidinyl)imidazo[1,2-b]pyridazin-2-yl)amino)-N-(2-chloro-4-(trifluoromethyl)phenyl)-1H-pyrazole-1-carboxamide |
| 3-((3-(6-(acetylamino)-2-methyl-4-pyrimidinyl)imidazo[1,2-b]pyridazin-2-yl)amino)-N-(2-methylphenyl)-1H-pyrazole-1-carboxamide |
| 3-(6-amino-2-methyl-4-pyrimidinyl)-N-(1-(1H-benzimidazol-2-yl)-1H-pyrazol-3-yl)imidaz-[1,2-b]pyridazin-2-amine |
| 3-(6-amino-2-methyl-4-pyrimidinyl)-N-(1-(1H-imidazol-2-yl)-1H-pyrazol-3-yl)imidazo[1,2-b]pyridazin-2-amine |
| 3-(6-amino-2-methyl-4-pyrimidinyl)-N-(1-(methylsulfonyl)-1H-pyrazol-3-yl)imidazo[1,2-b]pyridazin-2-amine |
| 3-((3-(6-amino-2-methyl-4-pyrimidinyl)imidazo[1,2-b]pyridazin-2-yl)amino)-1H-pyrazole-1-carboxamide |
| 5-((3-(6-amino-2-methyl-4-pyrimidinyl)imidazo[1,2-b]pyridazin-2-yl)amino)-1H-pyrazole-1-carboxamide |
| 3-(6-amino-2-methyl-4-pyrimidinyl)-N-(1-(1-azetidinylcarbonyl)-1H-pyrazol-3-yl)imidazo[1,2-b]pyridazin-2-amine |
| 3-(6-amino-2-methyl-4-pyrimidinyl)-N-(1-(1-azetidinylcarbonyl)-1H-pyrazol-5-yl)imidazo[1,2-b]pyridazin-2-amine |
| 3-((3-(6-amino-2-methyl-4-pyrimidinyl)imidazo[1,2-b]pyridazin-2-yl)amino)-N-(2-(methyl-amino)-ethyl)-1H-pyrazole-1-carboxamide |
| 5-((3-(6-amino-2-methyl-4-pyrimidinyl)imidazo[1,2-b]pyridazin-2-yl)amino)-N-(2-(methylamino)ethyl)-1H-pyrazole-1-carboxamide |
| 3-((3-(6-amino-2-methyl-4-pyrimidinyl)imidazo[1,2-b]pyridazin-2-yl)amino)-N,N-dimethyl-1 H-pyrazole-1-carboxamide |
| 5-((3-(6-amino-2-methyl-4-pyrimidinyl)imidazo[1,2-b]pyridazin-2-yl)amino)-N,N-dimethyl-1H-pyrazole-1-carboxamide |
| 3-(6-amino-2-methyl-4-pyrimidinyl)-N-(1-(1-piperazinylcarbonyl)-1H-pyrazol-3-yl)imidazo-[1,2-b]pyridazin-2-amine |
| 5-((3-(6-amino-2-methyl-4-pyrimidinyl)imidazo[1,2-b]pyridazin-2-yl)amino)-N-(2-hydroxyethyl)-1H-pyrazole-1-carboxamide |
| 3-((3-(6-amino-2-methyl-4-pyrimidinyl)imidazo[1,2-b]pyridazin-2-yl)amino)-N-(2-methoxy-ethyl)-1H-pyrazole-1-carboxamide |
| 5-((3-(6-amino-2-methyl-4-pyrimidinyl)imidazo[1,2-b]pyridazin-2-yl)amino)-N-(2-methoxy-ethyl)-1H-pyrazole-1-carboxamide |
| 5-((3-(6-amino-2-methyl-4-pyrimidinyl)imidazo[1,2-b]pyridazin-2-yl)amino)-N-(2-methyl-propyl)-1H-pyrazole-1-carboxamide |
| 3-(6-amino-2-methyl-4-pyrimidinyl)-N-1H-pyrazol-3-yl-6-(5-pyrimidinyl)imidazo[1,2-b]-pyridazin-2-amine |
| 3-(6-amino-2-methyl-4-pyrimidinyl)-N-(1-(1-phenylethyl)-1H-pyrazol-3-yl)imidazo[1,2-b]-pyridazin-2-amine |
| 3-(6-amino-2-methyl-4-pyrimidinyl)-N-(1-benzyl-1H-pyrazol-3-yl)imidazo[1,2-b]pyridazin-2-amine |
| 3-(6-amino-2-methyl-4-pyrimidinyl)-N-(1-(3-pyridinylmethyl)-1H-pyrazol-3-yl)imidazo[1,2-b]pyridazin-2-amine |
| 7-((3-(6-amino-2-methyl-4-pyrimidinyl)imidazo[1,2-a]pyridin-2-yl)amino)-2H-1,4-benzoxazin-3(4H)-one |
| 1-(5-((3-(6-amino-2-methyl-4-pyrimidinyl)imidazo[1,2-b]pyridazin-2-yl)amino)-2-pyridinyl)-3-azetidinol |
| 2-(1-(5-((3-(6-amino-2-methyl-4-pyrimidinyl)imidazo[1,2-b]pyridazin-2-yl)amino)-2-pyridinyl)-3-azetidinyl)-2-propanol |
| N-(3-(4-amino-6-methyl-1,3,5-triazin-2-yl)imidazo[1,2-a]pyridin-2-yl)-7-quinolinamine |
| N-(3-(4-amino-6-methyl-1,3,5-triazin-2-yl)imidazo[1,2-a]pyridin-2-yl)-6-quinolinamine |
| N-(3-(4-amino-6-methyl-1,3,5-triazin-2-yl)imidazo[1,2-a]pyridin-2-yl)-7-isoquinolinamine |
| 4-(3-(4-amino-6-methyl-1,3,5-triazin-2-yl)-2-(7-quinolinylamino)-imidazo[1,2-a]pyridin-6-yl)-N,N-dimethyl-1-piperazinecarboxamide |
| N-(3-(4-amino-6-methyl-1,3,5-triazin-2-yl)-6-(4-(methylsulfonyl)-1-piperazinyl)imidazo[1,2-a]pyridin-2-yl)-7-quinolinamine |
| N-(3-(4-amino-6-methyl-1,3,5-triazin-2-yl)-6-(1-piperazinyl)-imidazo[1,2-a]pyridin-2-yl)-7-quinolinamine |
| 4-(3-(4-(acetylamino)-6-methyl-1,3,5-triazin-2-yl)-2-((6-methoxy-3-pyridinyl)amino)-imidazo[1,2-a]pyridin-6-yl)-N,N-dimethyl-1-piperazinecarboxamide |
| 3-(4-amino-6-methyl-1,3,5-triazin-2-yl)-N-(6-ethoxy-3-pyridinyl)-imidazo[1,2-a]pyridin-2-amine |
| N-(3-(4-amino-6-methyl-1,3,5-triazin-2-yl)imidazo[1,2-a]pyridin-2-yl)-1H-indazol-3-amine |
| 4-(3-(4-amino-6-methyl-1,3,5-triazin-2-yl)-2-((5-fluoro-6-methoxy-3-pyridinyl)amino)imidazo[1,2-a]pyridin-6-yl)-N,N-dimethyl-1-piperazinecarboxamide |
| 4-(3-(4-amino-6-methyl-1,3,5-triazin-2-yl)-2-((6-methoxy-3-pyridinyl)amino)imidazo[1,2-a]pyridin-6-yl)-N-methyl-1-piperazinecarboxamide |
| 6-(4-acetyl-1-piperazinyl)-3-(4-amino-6-methyl-1,3,5-triazin-2-yl)-N-(6-methoxy-3-pyridinyl)imidazo[1,2-a]pyridin-2-amine |
| (5-(3-(4-amino-6-methyl-1,3,5-triazin-2-yl)-2-(1H-pyrazol-5-ylamino)imidazo[1,2-a]pyridin-6-yl)-2-methoxyphenyl)methanol |
| 3-(4-amino-6-methyl-1,3,5-triazin-2-yl)-N-1H-pyrazol-5-yl-6-(4-pyridinyl)imidazo[1,2-a]pyridin-2-amine |
| 3-(4-amino-6-methyl-1,3,5-triazin-2-yl)-6-(3,6-dihydro-2H-pyran-4-yl)-N-1H-pyrazol-5-ylimidazo[1,2-a]pyridin-2-amine |
| 3-(4-amino-6-methyl-1,3,5-triazin-2-yl)-6-(2-methyl-4-pyridinyl)-N-1H-pyrazol-5-ylimidazo [1,2-a]pyridin-2-amine |
| 3-(4-amino-6-methyl-1,3,5-triazin-2-yl)-6-(1-methyl-1H-imidazol-5-yl)-N-1H-pyrazol-5-ylimidazo[1,2-a]pyridin-2-amine |
| 4-(3-(4-amino-6-methyl-1,3,5-triazin-2-yl)-2-(1H-pyrazol-5-ylamino)-midazo[1,2-a]pyridin-6-yl)-1,2-butanediol |
| 3-(4-amino-6-methyl-1,3,5-triazin-2-yl)-6-(1-(1-methylethyl)-1H-pyrazol-4-yl)-N-1H-pyrazol-5-ylimidazo[1,2-a]pyridin-2-amine |
| 3-(4-amino-6-methyl-1,3,5-triazin-2-yl)-2-(1H-pyrazol-5-ylamino)-imidazo[1,2-a]pyridine-6-carboxamide |
| N-((3-(4-amino-6-methyl-1,3,5-triazin-2-yl)-2-(1H-pyrazol-5-ylamino)imidazo[1,2-a]pyridin-6-yl)methyl)acetamide |
| 3-(2-methyl-6-((2,2,2-trifluoroethyl)amino)-4-pyrimidinyl)-N-1H-pyrazol-5-ylimidazo[1,2-b]pyridazin-2-amine |
| 3-(2-methyl-6-(2-pyrazinylamino)-4-pyrimidinyl)-N-(1-methyl-1H-pyrazol-3-yl)imidazo[1,2-b]pyridazin-2-amine |
| 3-(2-methyl-6-(3-pyridinylamino)-4-pyrimidinyl)-N-1H-pyrazol-3-ylimidazo[1,2-b]pyridazin-2-amine |
| 2-cyano-N-(2-methyl-6-(2-((1-methyl-1H-pyrazol-3-yl)amino)-imidazo[1,2-b]pyridazin-3-yl)-4-pyrimidinyl)acetamide |
| N-(2-methyl-6-(2-((1-methyl-1H-pyrazol-3-yl)amino)imidazo[1,2-b]pyridazin-3-yl)-4-pyrimidinyl)-1,3-oxazole-5-carboxamide |
| 3-(6-amino-2-methyl-4-pyrimidinyl)-N-(1-methyl-1H-pyrazol-3-yl)-6-(5-pyrimidinyl)imidazo-[1,2-a]pyridin-2-amine |
| N-(4-(3-(6-amino-2-methyl-4-pyrimidinyl)-2-((1-methyl-1H-pyrazol-3-yl)amino)imidazo[1,2-a]pyridin-6-yl)phenyl)acetamide |
| 3-(4-amino-6-methyl-1,3,5-triazin-2-yl)-N-(1-methyl-1H-pyrazol-3-yl)-6-(7-quinolinyl)-imidazo-[1,2-a]pyridin-2-amine |
| N-(4-(3-(6-amino-2-methyl-4-pyrimidinyl)-2-((1-methyl-1H-pyrazol-3-yl)amino)imidazo[1,2-b]pyridazin-6-yl)benzyl)acetamide |
| 3-(4-amino-6-methyl-1,3,5-triazin-2-yl)-N-(1-methyl-1H-pyrazol-3-yl)-6-(5-pyrimidinyl)-imidazo[1,2-a]pyridin-2-amine |
| 4-(3-(6-amino-2-methyl-4-pyrimidinyl)-2-((1-methyl-1H-pyrazol-3-yl)amino)imidazo[1,2-b]pyridazin-6-yl)-N-methylbenzamide |
| 3-(6-amino-2-methyl-4-pyrimidinyl)-N-(1-(6-(2-methoxyethoxy)-4-pyrimidinyl)-1H-pyrazol-3-yl)imidazo[1,2-b]pyridazin-2-amine |
| 3-(6-(3-isoxazolylamino)-2-methyl-4-pyrimidinyl)-N-1H-pyrazol-3-ylimidazo[1,2-b]pyridazin-2-amine |
| 2-cyano-N-(2-methyl-6-(2-((1-methyl-1H-pyrazol-3-yl)amino)-6-(5-pyrimidinyl)imidazo[1,2-a]pyridin-3-yl)-4-pyrimidinyl)acetamide |
| 3-(6-((1-(2-methoxyethyl)-1H-pyrazol-4-yl)amino)-2-methyl-4-pyrimidinyl)-N-1H-pyrazol-3-ylimidazo[1,2-b]pyridazin-2-amine |
| 3-(2-methyl-6-((1-((3R)-tetrahydro-3-furanyl)-1H-pyrazol-4-yl)amino)-4-pyrimidinyl)-N-1H-pyrazol-3-ylimidazo[1,2-b]pyridazin-2-amine, 3-(2-methyl-6-((1-((3S)-tetrahydro-3-furanyl)-1H-pyrazol-4-yl)amino)-4-pyrimidinyl)-N-1H-pyrazol-3-ylimidazo[1,2-b]pyridazin-2-amine |
| 3-(6-(imidazo[1,2-a]pyridin-2-ylamino)-2-methyl-4-pyrimidinyl)-N-1H-pyrazol-3-ylimidazo[1,2-b]pyridazin-2-amine |
| 3-(6-(furo[3,2-b]pyridin-6-ylamino)-2-methyl-4-pyrimidinyl)-N-1H-pyrazol-3-ylimidazo[1,2-b]pyridazin-2-amine |
| 3-(6-amino-2-methyl-4-pyrimidinyl)-N-1H-pyrazol-3-yl-6-(5-pyrimidinyl)imidazo[1,2-a]pyridin-2-amine |
| 3-(2-methyl-6-((1-((3R)-tetrahydro-3-furanyl)-1H-pyrazol-4-yl)amino)-4-pyrimidinyl)-N-1H-pyrazol-3-ylimidazo[1,2-a]pyridin-2-amine, 3-(2-methyl-6-((1-((3S)-tetrahydro-3-furanyl)-1H-pyrazol-4-yl)amino)-4-pyrimidinyl)-N-1H-pyrazol-3-ylimidazo[1,2-a]pyridin-2-amine |
| 3-(6-((6-(2-methoxyethoxy)-4-pyrimidinyl)amino)-2-methyl-4-pyrimidinyl)-N-1H-pyrazol-3-ylimidazo[1,2-b]pyridazin-2-amine |
| 1-(3-((3-(6-amino-2-methyl-4-pyrimidinyl)imidazo[1,2-b]pyridazin-2-yl)amino)phenyl)-3-cyclopropylurea |
| N-(3-((3-(6-amino-2-methyl-4-pyrimidinyl)imidazo[1,2-a]pyridin-2-yl)amino)phenyl)-acetamide |
| N-(3-((3-(6-amino-2-methyl-4-pyrimidinyl)imidazo[1,2-a]pyridin-2-yl)amino)phenyl)-2-phenylacetamide |
| N-(3-((3-(6-amino-2-methyl-4-pyrimidinyl)imidazo[1,2-a]pyridin-2-yl)amino)phenyl)-benzamide |
| N-(3-((3-(6-amino-2-methyl-4-pyrimidinyl)imidazo[1,2-a]pyridin-2-yl)amino)phenyl)-4-chlorobenzenesulfonamide |
| N-(3-((3-(6-amino-2-methyl-4-pyrimidinyl)imidazo[1,2-a]pyridin-2-yl)amino)phenyl)-methanesulfonamide |
| 1-(3-((3-(6-amino-2-methyl-4-pyrimidinyl)imidazo[1,2-a]pyridin-2-yl)amino)phenyl)-3-phenylurea |
| 1-(3-((3-(6-amino-2-methyl-4-pyrimidinyl)imidazo[1,2-a]pyridin-2-yl)amino)phenyl)-3-(4-fluorophenyl)urea |
| 1-(3-((3-(6-amino-2-methyl-4-pyrimidinyl)imidazo[1,2-a]pyridin-2-yl)amino)phenyl)-3-benzylurea |
| 1-(3-((3-(6-amino-2-methyl-4-pyrimidinyl)imidazo[1,2-a]pyridin-2-yl)amino)phenyl)-3-methylurea |
| 1-(3-((3-(6-amino-2-methyl-4-pyrimidinyl)imidazo[1,2-a]pyridin-2-yl)amino)phenyl)-3-ethylurea |
| 4-((3-(6-amino-2-methyl-4-pyrimidinyl)imidazo[1,2-a]pyridin-2-yl)amino)-N-ethylbenzamide |
| 5-((3-(6-amino-2-methyl-4-pyrimidinyl)imidazo[1,2-a]pyridin-2-yl)amino)-N-ethyl-2-pyridinecarboxamide |
| N-(3-((3-(6-amino-2-methyl-4-pyrimidinyl)imidazo[1,2-b]pyridazin-2-yl)amino)phenyl)-acetamide |
| N-(3-((3-(6-amino-2-methyl-4-pyrimidinyl)imidazo[1,2-b]pyridazin-2-yl)amino)phenyl)-propanamide |
| 3-((3-(6-amino-2-methyl-4-pyrimidinyl)imidazo[1,2-a]pyridin-2-yl)amino)-N-methyl-benzamide |
| 3-((3-(6-amino-2-methyl-4-pyrimidinyl)imidazo[1,2-a]pyridin-2-yl)amino)-N-ethylbenzamide |
| 3-((3-(6-amino-2-methyl-4-pyrimidinyl)imidazo[1,2-a]pyridin-2-yl)amino)-N-(1-methylethyl)-benzamide |
| 3-((3-(6-amino-2-methyl-4-pyrimidinyl)imidazo[1,2-b]pyridazin-2-yl)amino)-N-methyl-benzamide |
| 3-((3-(6-amino-2-methyl-4-pyrimidinyl)imidazo[1,2-b]pyridazin-2-yl)amino)-N-ethyl-benzamide |
| 3-((3-(6-amino-2-methyl-4-pyrimidinyl)imidazo[1,2-b]pyridazin-2-yl)amino)-N-phenyl-benzamide |
| 3-((3-(6-amino-2-methyl-4-pyrimidinyl)imidazo[1,2-b]pyridazin-2-yl)amino)-N-(1-methylethyl)benzamide |
| 5-((3-(6-amino-2-methyl-4-pyrimidinyl)imidazo[1,2-a]pyridin-2-yl)amino)-N-methyl-2-pyridinecarboxamide |
| 5-((3-(6-amino-2-methyl-4-pyrimidinyl)imidazo[1,2-a]pyridin-2-yl)amino)-N-(1-methylethyl)-2-pyridinecarboxamide |
| 5-((3-(6-amino-2-methyl-4-pyrimidinyl)imidazo[1,2-a]pyridin-2-yl)amino)-N-phenyl-2-pyridinecarboxamide |
| 4-((3-(6-amino-2-methyl-4-pyrimidinyl)imidazo[1,2-a]pyridin-2-yl)amino)benzamide |
| 4-((3-(6-amino-2-methyl-4-pyrimidinyl)imidazo[1,2-a]pyridin-2-yl)amino)-N-(2-(diethylamino)-ethyl)benzamide |
| 4-((3-(6-amino-2-methyl-4-pyrimidinyl)imidazo[1,2-a]pyridin-2-yl)amino)-N-phenylbenzamide |
| 3-(4-amino-6-methyl-1,3,5-triazin-2-yl)-6-(4-(methylsulfonyl)phenyl)-N-1H-pyrazol-3-ylimidazo [1,2-a]pyridin-2-amine |
| 3-(6-amino-2-methyl-4-pyrimidinyl)-N-(1-(3-methylbutanoyl)-1H-pyrazol-3-yl)imidazo[1,2-b]pyridazin-2-amine |
| 1-methylethyl 5-((3-(6-amino-2-methyl-4-pyrimidinyl)imidazo[1,2-b]pyridazin-2-yl)amino)-1H-pyrazole-1-carboxylate |
| 1-methylethyl 3-((3-(6-amino-2-methyl-4-pyrimidinyl)imidazo[1,2-b]pyridazin-2-yl)amino)-1H-pyrazole-1-carboxylate |
| 3-(6-amino-2-methyl-4-pyrimidinyl)-N-(2-methoxy-4-pyridinyl)imidazo[1,2-b]pyridazin-2-amine |
| 3-(6-amino-2-methyl-4-pyrimidinyl)-N-(1-(2-methylpropanoyl)-1H-pyrazol-3-yl)imidazo[1,2-b]pyridazin-2-amine |
| 3-(6-amino-2-m ethyl-4-pyrimidinyl)-N-(1-(2-methylpropanoyl)-1H-pyrazol-5-yl)imidazo[1,2-b]pyridazin-2-amine |
| 4-(3-(4-amino-6-methyl-1,3,5-triazin-2-yl)-2-(1H-pyrazol-3-ylamino)imidazo[1,2-a]pyridin-6-yl)-N-methylbenzenesulfonamide |
| 3-(4-amino-6-methyl-1,3,5-triazin-2-yl)-6-(3-(methylsulfonyl)phenyl)-N-1H-pyrazol-3-ylimidazo[1,2-a]pyridin-2-amine |
| 3-(6-amino-2-methyl-4-pyrimidinyl)-N-(1-(3,3-dimethylbutanoyl)-1H-pyrazol-3-yl)-imidazo[1,2-b]pyridazin-2-amine |
| 3-(4-amino-6-methyl-1,3,5-triazin-2-yl)-N-(1-methyl-1H-pyrazol-3-yl)-6-(4-(methylsulfonyl)-phenyl)imidazo[1,2-a]pyridin-2-amine |
| cyclohexyl 5-((3-(6-amino-2-methyl-4-pyrimidinyl)imidazo[1,2-b]pyridazin-2-yl)amino)-1H-pyrazole-1-carboxylate |
| cyclohexyl 3-((3-(6-amino-2-methyl-4-pyrimidinyl)imidazo[1,2-b]pyridazin-2-yl)amino)-1H-pyrazole-1-carboxylate |
| 3-(6-amino-2-methyl-4-pyrimidinyl)-N-(2-methoxy-3-pyridinyl)imidazo[1,2-b]pyridazin-2-amine |
| 3-((3-(6-amino-2-methyl-4-pyrimidinyl)imidazo[1,2-b]pyridazin-2-yl)amino)-2(1H)-pyridinone |
| 3-(6-amino-2-methyl-4-pyrimidinyl)-N-(1-(3-methylbutyl)-1H-pyrazol-3-yl)imidazo[1,2-b]pyridazin-2-amine |
| 3-(6-amino-2-methyl-4-pyrimidinyl)-N-(1-(2-methoxyethyl)-1H-pyrazol-3-yl)imidazo[1,2-b]pyridazin-2-amine |
| 3-(6-amino-2-methyl-4-pyrimidinyl)-6-(3-(methylsulfonyl)phenyl)-N-1H-pyrazol-3-ylimidazo[1,2-b]pyridazin-2-amine |
| N-(3-(4-amino-6-methyl-1,3,5-triazin-2-yl)imidazo[1,2-a]pyridin-2-yl)-1H-indazol-4-amine |
| 3-(6-amino-2-methyl-4-pyrimidinyl)-N-(6-methoxy-3-pyridinyl)-6-(4-morpholinyl)-imidazo[1,2-b]pyridazin-2-amine |
| 3-(4-amino-6-methyl-1,3,5-triazin-2-yl)-N-(6-methoxy-3-pyridinyl)-6-(4-morpholinyl)-imidazo[1,2-b]pyridazin-2-amine |
| 3-(6-amino-2-methyl-4-pyrimidinyl)-N-6--(2-methoxyethyl)-N-2--(6-methoxy-3-pyridinyl)imidazo[1,2-b]pyridazine-2,6-diamine |
| 3-(6-amino-2-methyl-4-pyrimidinyl)-N-2--1H-indazol-4-yl-N-6--(2-methoxyethyl)imidazo-[1,2-b]pyridazine-2,6-diamine |
| 1-(3-(6-amino-2-methyl-4-pyrimidinyl)-2-((6-methoxy-3-pyridinyl)amino)imidazo[1,2-b]pyridazin-6-yl)-4-piperidinol |
| 4-(3-(4-amino-6-methyl-1,3,5-triazin-2-yl)-2-((6-methoxy-3-pyridinyl)amino)imidazo[1,2-a]pyridin-7-yl)-N,N-dimethyl-1-piperazinecarboxamide |
| 4-(3-(4-amino-6-methyl-1,3,5-triazin-2-yl)-2-((5-fluoro-6-methoxy-3-pyridinyl)amino)-imidazo[1,2-a]pyridin-7-yl)-N,N-dimethyl-1-piperazinecarboxamide |
| 3-(4-amino-6-methyl-1,3,5-triazin-2-yl)-N-(5-fluoro-6-methoxy-3-pyridinyl)-7-(4-(methylsulfonyl)-1-piperazinyl)imidazo[1,2-a]pyridin-2-amine |
| N-(5-((3-(4-amino-6-methyl-1,3,5-triazin-2-yl)imidazo[1,2-a]pyridin-2-yl)amino)-2-chloro-3-pyridinyl)methanesulfonamide |
| N-(5-((3-(4-amino-6-methyl-1,3,5-triazin-2-yl)imidazo[1,2-a]pyridin-2-yl)amino)-2-methoxy-3-pyridinyl)methanesulfonamide |
| 3-(6-amino-2-methyl-4-pyrimidinyl)-N-1H-indazol-6-ylimidazo[1,2-b]pyridazin-2-amine |
| 3-(6-amino-2-methyl-4-pyrimidinyl)-N-1H-benzimidazol-6-ylimidazo[1,2-b]pyridazin-2-amine |
| N-(3-(6-amino-2-methyl-4-pyrimidinyl)imidazo[1,2-a]pyridin-2-yl)-1H-benzimidazol-6-amine |
| 3-(4-amino-6-methyl-1,3,5-triazin-2-yl)-N-(6-methoxy-3-pyridinyl)-7-(1-methyl-1H-pyrazol-4-yl)imidazo[1,2-a]pyridin-2-amine |
| 3-(4-amino-6-methyl-1,3,5-triazin-2-yl)-N-(6-methoxy-3-pyridinyl)-6-(1-methyl-1H-pyrazol-4-yl)imidazo[1,2-a]pyridin-2-amine |
| 3-(4-amino-6-methyl-1,3,5-triazin-2-yl)-N-(6-methoxy-3-pyridinyl)-6-(4-morpholinyl)-imidazo[1,2-a]pyridin-2-amine |
| 3-(6-amino-2-methyl-4-pyrimidinyl)-N-(6-methoxy-3-pyridinyl)-6-(4-morpholinyl)imidazo-[1,2-a]pyridin-2-amine |
| 3-(4-amino-6-methyl-1,3,5-triazin-2-yl)-N-(6-methoxy-3-pyridinyl)-6-(3-pyridinyl)-imidazo[1,2-a]pyridin-2-amine |
| 4-(3-(4-amino-6-methyl-1,3,5-triazin-2-yl)-2-((6-methoxy-3-pyridinyl)amino)imidazo[1,2-a]pyridin-6-yl)-N,N-dimethyl-1-piperazinecarboxamide |
| 3-(4-amino-6-methyl-1,3,5-triazin-2-yl)-N-(6-methoxy-3-pyridinyl)-6-(4-(methylsulfonyl)-1-piperazinyl)imidazo[1,2-a]pyridin-2-amine |
| 2-(3-(4-amino-6-methyl-1,3,5-triazin-2-yl)-2-((6-methoxy-3-pyridinyl)-amino)imidazo[1,2-a]pyridin-6-yl)-2-propanol |
| 3-(4-amino-6-methyl-1,3,5-triazin-2-yl)-N-(6-methoxy-3-pyridinyl)-6-(1-piperazinyl)imidazo-[1,2-a]pyridin-2-amine |
| 1-(3-(4-amino-6-methyl-1,3,5-triazin-2-yl)-2-(7-quinolinylamino)-imidazo[1,2-a]pyridin-6-yl)ethanone |
| 2-(3-(4-amino-6-methyl-1,3,5-triazin-2-yl)-2-(7-quinolinylamino)-imidazo[1,2-a]pyridin-6-yl)-2-propanol |
| 1-(3-(4-amino-6-methyl-1,3,5-triazin-2-yl)-2-((5-fluoro-6-methoxy-3-pyridinyl)amino)-imidazo[1,2-a]pyridin-6-yl)ethanone |
| 2-(3-(4-amino-6-methyl-1,3,5-triazin-2-yl)-2-((5-fluoro-6-methoxy-3-pyridinyl)amino)-imidazo[1,2-a]pyridin-6-yl)-2-propanol |
| 4-(3-(6-amino-2-methyl-4-pyrimidinyl)-2-((5-fluoro-6-methoxy-3-pyridinyl)amino)-imidazo[1,2-b]pyridazin-6-yl)-N,N-dimethyl-1-piperazinecarboxamide |
| 1-(3-(6-amino-2-methyl-4-pyrimidinyl)-2-((5-fluoro-6-methoxy-3-pyridinyl)amino)imidazo-[1,2-a]pyridin-6-yl)ethanone |
| 2-(3-(6-amino-2-methyl-4-pyrimidinyl)-2-((5-fluoro-6-methoxy-3-pyridinyl)amino)imidazo-[1,2-a]pyridin-6-yl)-2-propanol |
| 4-(3-(6-amino-2-methyl-4-pyrimidinyl)-2-((5-fluoro-6-methoxy-3-pyridinyl)amino)-imidazo[1,2-a]pyridin-6-yl)-N,N-dimethyl-1-piperazinecarboxamide |
| 3-(4-amino-6-methyl-1,3,5-triazin-2-yl)-N-1H-pyrazol-3-yl-6-(trifluoromethyl)imidazo[1,2-a]pyridin-2-amine |
| 3-(4-amino-6-methyl-1,3,5-triazin-2-yl)-N-1H-pyrazol-3-yl-7-(trifluoromethyl)imidazo[1,2-a]-pyridin-2-amine |
| 3-(6-amino-2-methyl-4-pyrimidinyl)-N-1H-pyrazol-3-yl-7-(trifluoromethyl)imidazo[1,2-a]-pyridin-2-amine |
| 3-(6-amino-2-methyl-4-pyrimidinyl)-N-1H-pyrazol-3-yl-6-(trifluoromethyl)imidazo[1,2-a]-pyridin-2-amine |
| 1-(4-((3-(6-amino-2-methyl-4-pyrimidinyl)imidazo[1,2-b]pyridazin-2-yl)amino)phenyl)-3-cyclopropylurea |
| 1-(4-((3-(6-amino-2-methyl-4-pyrimidinyl)imidazo[1,2-b]pyridazin-2-yl)amino)phenyl)-3-ethylurea |
| N-(4-((3-(6-amino-2-methyl-4-pyrimidinyl)imidazo[1,2-b]pyridazin-2-yl)amino)phenyl)-1-piperidinecarboxamide |
| N-(4-((3-(6-amino-2-methyl-4-pyrimidinyl)imidazo[1,2-b]pyridazin-2-yl)amino)phenyl)-1-pyrrolidinecarboxamide |
| N-(4-((3-(6-amino-2-methyl-4-pyrimidinyl)imidazo[1,2-b]pyridazin-2-yl)amino)phenyl)-1-azetidinecarboxamide |
| N-(4-((3-(6-amino-2-methyl-4-pyrimidinyl)imidazo[1,2-b]pyridazin-2-yl)amino)phenyl)-4-methyl-1-piperazinecarboxamide |
| 1-(4-((3-(6-amino-2-methyl-4-pyrimidinyl)imidazo[1,2-b]pyridazin-2-yl)amino)phenyl)-3-(2-methoxyethyl)urea |
| N-(4-((3-(6-amino-2-methyl-4-pyrimidinyl)imidazo[1,2-b]pyridazin-2-yl)amino)phenyl)-4-morpholinecarboxamide |
| 4-acetyl-N-(4-((3-(6-amino-2-methyl-4-pyrimidinyl)imidazo[1,2-b]pyridazin-2-yl)amino)-phenyl)-1-piperazinecarboxamide |
| 3-(6-amino-2-methyl-4-pyrimidinyl)-N-1,3-thiazol-4-ylimidazo[1,2-b]pyridazin-2-amine |
| N-(4-((3-(6-amino-2-methyl-4-pyrimidinyl)imidazo[1,2-a]pyridin-2-yl)amino)phenyl)-4-morpholinecarboxamide |
| 4-acetyl-N-(4-((3-(6-amino-2-methyl-4-pyrimidinyl)imidazo[1,2-a]pyridin-2-yl)amino)phenyl)-1-piperazinecarboxamide |
| N-(4-((3-(6-amino-2-methyl-4-pyrimidinyl)imidazo[1,2-a]pyridin-2-yl)amino)phenyl)-2-methyl-4-morpholinecarboxamide |
| 3-(4-((3-(6-amino-2-methyl-4-pyrimidinyl)imidazo[1,2-a]pyridin-2-yl)amino)phenyl)-1-methyl-1-(-methylethyl)urea |
| N-(4-((3-(6-amino-2-methyl-4-pyrimidinyl)imidazo[1,2-a]pyridin-2-yl)amino)phenyl)-3-methyl-4-morpholinecarboxamide |
| 1-(4-((3-(6-amino-2-methyl-4-pyrimidinyl)imidazo[1,2-a]pyridin-2-yl)amino)phenyl)-3-cyclopropylurea |
| ethyl 2-((3-(6-amino-2-methyl-4-pyrimidinyl)imidazo[1,2-b]-pyridazin-2-yl)amino)-1H-imidazole-4-carboxylate |
| 3-(2-methyl-4-pyrimidinyl)-6-(3-(methylsulfonyl)phenyl)-N-1H-pyrazol-3-ylimidazo[1,2-b]pyridazin-2-amine |
| N-(3-(6-amino-2-methyl-4-pyrimidinyl)imidazo[1,2-b]-pyridazin-2-yl)-3H-imidazo[4,5-b]pyridin-5-amine |
| N-(2-methyl-6-(2-((1-methyl-1H-pyrazol-3-yl)amino)-6-(3-(methylsulfonyl)phenyl)imidazo[1,2-a]pyridin-3-yl)-4-pyrimidinyl)acetamide |
| 3-(4-amino-6-methyl-1,3,5-triazin-2-yl)-6-(3-(ethylsulfonyl)-phenyl)-N-1H-pyrazol-3-ylimidazo[1,2-a]pyridin-2-amine |
| 3-(6-amino-2-methyl-4-pyrimidinyl)-6-(3,6-dihydro-2H-pyran-4-yl)-N-1H-pyrazol-3-ylimidazo[1,2-b]pyridazin-2-amine |
| N-(5-((3-(6-ammo-2-methyl-4-pyrimidinyl)imidazo[1,2-b]-pyridazin-2-yl)amino)-3-pyridinyl)acetamide |
| 3-(6-amino-2-methyl-4-pyrimidinyl)-N-(2-methyl-1,3-thiazol-4-yl)imidazo[1,2-b]pyridazin-2-amine |
| 3-(6-amino-2-methyl-4-pyrimidinyl)-N-1,3-thiazol-5-ylimidazo-[1,2-b]pyridazin-2-amine |
| 1-(4-((3-(6-amino-2-methyl-4-pyrimidinyl)imidazo[1,2-a]-pyridin-2-yl)amino)phenyl)-3-(2-methylpropyl)urea |
| N-(4-((3-(6-amino-2-methyl-4-pyrimidinyl)imidazo[1,2-a]-pyridin-2-yl)amino)phenyl)-4,4-difluoro-1-piperidinecarboxamide |
| (2S)-N-(4-((3-(6-amino-2-methyl-4-pyrimidinyl)imidazo[1,2-a]pyridin-2-yl)amino)phenyl)-2-methyl-1-pyrrolidine-carboxamide |
| N-(2-methyl-6-(2-((1-methyl-1H-pyrazol-3-yl)amino)-6-(2-methyl-3-pyridinyl)imidazo[1,2-a]pyridin-3-yl)-4-pyrimidinyl)acetamide |
| N-(6-(6-(1-(2-methoxyethyl)-1H-pyrazol-4-yl)-2-((1-methyl-1H-pyrazol-3-yl)amino)imidazo[1,2-a]pyridin-3-yl)-2-methyl-4-pyrimidinyl)acetamide |
| N-(5-((3-(6-(acetylamino)-2-methyl-4-pyrimidinyl)imidazo-[1,2-b]pyridazin-2-yl)amino)-3-pyridinyl)acetamide |
| N-(2-methyl-6-(2-(1H-pyrazol-3-ylamino)-6-(5-pyrimidinyl)-imidazo[1,2-a]pyridin-3-yl)-4-pyrimidinyl)acetamide |
| 8-fluoro-3-(2-methyl-4-pyrimidinyl)-N-1H-pyrazol-3-ylimidazo-[1,2-a]pyridin-2-amine |
| 3-(4-amino-6-methyl-1,3,5-triazin-2-yl)-6-(4-(ethylsulfonyl)-phenyl)-N-1H-pyrazol-3-ylimidazo[1,2-a]pyridin-2-amine |
| 3-(2,6-dimethyl-4-pyrimidinyl)-6-(3-(methylsulfonyl)phenyl)-N-1H-pyrazol-3-ylimidazo[1,2-b]pyridazin-2-amine |
| N-(6-(8-fluoro-2-(1H-pyrazol-3-ylamino)imidazo[1,2-a]-pyridin-3-yl)-2-methyl-4-pyrimidinyl)acetamide |
| N-(3-(6-amino-2-methyl-4-pyrimidinyl)-6-(3-(methylsulfonyl)-phenyl)imidazo[1,2-b]pyridazin-2-yl)-1,3-benzenediamine |
| 3-((8-fluoro-3-(2-methyl-4-pyrimidinyl)imidazo[1,2-a]pyridin-2-yl)amino)-N-(1-methylethyl)-1H-pyrazole-1-carboxamide |
| 5-((3-(6-(acetylamino)-2-methyl-4-pyrimidinyl)-8-fluoro-imidazo[1,2-a]pyridin-2-yl)amino)-1 H-pyrazole-1-carboxamide |
| 3-((3-(6-(acetylamino)-2-methyl-4-pyrimidinyl)-8-fluoroimidazo[1,2-a]pyridin-2-yl)amino)-1 H-pyrazole-1-carboxamide |
| N-(2-methyl-6-(2-(1H-pyrazol-3-ylamino)-6-(5-pyrimidinyl)-imidazo[1,2-b]pyridazin-3-yl)-4-pyrimidinyl)acetamide |
| 5-((8-fluoro-3-(2-methyl-4-pyrimidinyl)imidazo[1,2-a]pyridin-2-yl)amino)-N-(1-methylethyl)-1H-pyrazole-1-carboxamide |
| (2R)-N-(4-((3-(6-amino-2-methyl-4-pyrimidinyl)imidazo[1,2-b]pyridazin-2-yl)amino)phenyl)-2-methyl-4-morpholine-carboxamide |
| 3-(4-((3-(6-amino-2-methyl-4-pyrimidinyl)imidazo[1,2-b]pyridazin-2-yl)amino)phenyl)-1-methyl-1-(1-methylethyl)urea |
| (3R)-N-(4-((3-(6-amino-2-methyl-4-pyrimidinyl)imidazo[1,2-b]pyridazin-2-yl)amino)phenyl)-3-methyl-4-morpholine-carboxamide |
| (2S)-N-(4-((3-(6-amino-2-methyl-4-pyrimidinyl)imidazo[1,2-b]pyridazin-2-yl)amino)phenyl)-2-methyl-1-pyrrolidine-carboxamide |
| (3S)-N-(4-((3-(6-amino-2-methyl-4-pyrimidinyl)imidazo[1,2-b]pyridazin-2-yl)amino)phenyl)-3-methyl-1-pyrrolidine-carboxamide |
| N-(3-((3-(6-amino-2-methyl-4-pyrimidinyl)-6-(3-(methyl-sulfonyl)-phenyl)imidazo[1,2-b]pyridazin-2-yl)amino)phenyl)acetamide |
| N-(6-(2-((4-((cyclopropylcarbamoyl)amino)phenyl)amino)-imidazo[1,2-b]pyridazin-3-yl)-2-methyl-4-pyrimidinyl)-acetamide |
| 3-(6-amino-2-methyl-4-pyrimidinyl)-6-(5,6-dihydro-2H-pyran-3-yl)-N-1H-pyrazol-3-ylimidazo[1,2-b]pyridazin-2-amine |
| 2-(4-((3-(6-amino-2-methyl-4-pyrimidinyl)imidazo[1,2-b]-pyridazin-2-yl)amino)phenyl)-N-cyclopropylacetamide |
| 3-((3-(6-amino-2-methyl-4-pyrimidinyl)imidazo[1,2-b]-pyridazin-2-yl)amino)-N-(5-fluoro-2-(trifluoromethyl)phenyl)-1H-pyrazole-1-carboxamide |
| 6-fluoro-8-methoxy-3-(2-methyl-4-pyrimidinyl)-N-1H-pyrazol-3-ylimidazo[1,2-a]pyridin-2-amine |
| N-(3-(6-amino-2-methyl-4-pyrimidinyl)-6-(3-(methylsulfonyl)-phenyl)imidazo[1,2-b]pyridazin-2-yl)-1,4-benzenediamine |
| N-(4-((3-(6-amino-2-methyl-4-pyrimidinyl)-6-(3-(methyl-sulfonyl)-phenyl)imidazo[1,2-b]pyridazin-2-yl)amino)phenyl)acetamide |
| 3-(6-amino-2-methyl-4-pyrimidinyl)-N-(1-methyl-1H-pyrazol-3-yl)-6-(3-(methylsulfonyl)phenyl)imidazo[1,2-b]pyridazin-2-amine |
| 3-(6-amino-2-methyl-4-pyrimidinyl)-N-(1-(4,5-dihydro-1,3-oxazol-2-yl)-1H-pyrazol-3-yl)imidazo[1,2-b]pyridazin-2-amine |
| 6-fluoro-3-(2-methyl-4-pyrimidinyl)-N-1H-pyrazol-3-yl-8-(3-pyridinyloxy)imidazo[1,2-a]pyridin-2-amine |
or a pharmaceutically acceptable salt thereof.

13. A pharmaceutical composition comprising a compound of any of the Claims 1-12 or a pharmaceutically acceptable salt thereof; and a pharmaceutically acceptable excipient.

14. A compound of any of the claims 1-12, or a pharmaceutically acceptable salt thereof, for use in a method of treating melanoma, ovarian cancer, cervical cancer, breast cancer, colon cancer, rectal cancer, endometrial cancer, pancreatic cancer, lung cancer, stomach cancer, glioblastoma, liver cancer, prostate cancer, acute myelogenous leukemia, chronic myelogenous leukemia, or thyroid cancer.

15. Compound of any of the Claims 1-12 for use in therapy.

16. The compound of Claim 15 wherein the therapy is for cancer.

## Patentansprüche

1. Verbindung der Formel (I): 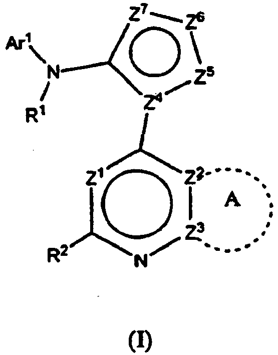 wobei gilt:
Ar¹ ist ein Aryl-, Heteroaryl-, Cycloalkyl- oder Heterocyclylring, wobei jeder Ring mit R^{d}, R^{e} oder R^{f} substituiert ist, wobei R^{d}, R^{e} oder R^{f} unabhängig aus Wasserstoff, Halo, Haloalkyl, Haloalkoxy, Cyano, Nitro, Alkyl, Alkenyl, Alkinyl, substituiertes Alkyl, Aryl, Heteroaryl, Heterocycloalkyl, -C(=O)NR^{a}R^{a}, -C(=O)R^{b}, -C(=O)OR^{b}, -C(ONR^{a})NR^{a}R^{a}, -OR^{a}, -OC(=O)R^{b}, -OC(=O)NR^{a}R^{a}, -O-Alkyl-N(R^{a})C(=O)OR^{b}, -OC(=O)N(R^{a})S(=O)₂R^{b}, -O-Alkyl-NR^{a}R^{a}, -O-Alkyl-OR^{a}, -SR^{a}, -S(=O)R^{b}, -S(=O)₂R^{b}, -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=S)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}-Alkylen-NR^{a}R^{a} oder -NR^{a}-Alkylen-OR^{a} ausgewählt sind;
R¹ ist Wasserstoff oder Alkyl;
R² ist Methyl oder Ethyl;
Z¹ ist -N- oder -CR³-, wobei R³ H oder Alkyl ist;
Z² ist -N- oder -CR⁴-, wobei R⁴ R^{d}, -(Alkylen)heterocycloalkyl, -(Alkylen)ₙO(alkylen)ₙaryl, -(Alkylen)ₙN(R^{a})(alkylen)ₙaryl, -(Alkylen)ₙ-N(R^{a})-(alk*ylen)ₙheteroaryl oder -(Alkylen)ₙO(alkylen)ₙheteroaryl ist;
Z³ ist -N- oder -CR⁵-, wobei R⁵ ist R^{d} -(Alkylen)ₙaryl, -(Alkylen)-heteroaryl, -(Alkylen)heterocycloalkyl, -(Alkylen)ₙO-(alkylen)ₙaryl, -(Alkylen)ₙN(R^{a})(alkylen)ₙaryl, -(Alkylen)ₙN(R^{a})(alkylen)ₙheteroaryl oder -(Alkylen)ₙO(alkylen)ₙheteroaryl ist, mit der Maßgabe, dass nur zwei Vertreter von Z¹, Z² und Z³ gleichzeitig N sein können;
Oder wenn Z² -CR⁴ ist und Z³ -CR⁵ ist, dann können R⁴ und R⁵ zusammen mit den Atomen, an die sie gebunden sind, Ring A bilden, der Phenyl oder ein 5- oder 6-gliedriger Heteroarylring ist und Ring A mit R^{g} oder R^{h} substituiert ist, wobei R^{g} oder R^{h} unabhängig R^{d}, -(Alkylen)ₙ-heterocycloalkyl, -(Alkylen)ₙO(-alkylen)ₙaryl, -(Alkylen)ₙN(R^{a})(alkylen)ₙaryl, -(Alkylen)ₙN(R^{a})(alkylen)ₙheteroaryl oder -(Alkylen)ₙO(alkylen)ₙheteroaryl sind; 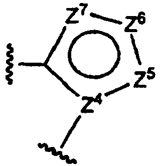 ist 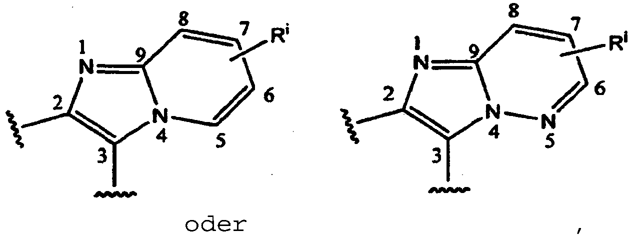 wobei R¹ R^{d}, -(Alkylen)ₙaryl, -(Alkylen)heteroaryl, -(Alkylen)heterocycloalkyl, -(Alkylen)ₙO(alkylen)ₙaryl, -(Alkylen)ₙN(R^{a})(alkylen)ₙaryl, -(Alkylen₂)ₙN(R^{a})(alkylen)ₙheteroaryl oder -(Alkylen)ₙO(alkylen)ₙheteroaryl ist;
R^{a} ist jeweils unabhängig Wasserstoff oder R^{b}; oder wenn zwei R^{a} an ein Stickstoffatom gebunden sind, entweder einzeln oder als Teil einer anderen Gruppe, können die beiden R^{a} zusammen mit dem Stickstoffatom, an das sie gebunden sind, einen monozyklischen Heterocyclylring bilden, der gegebenenfalls mit einem, zwei oder drei Substituenten, unabhängig ausgewählt aus Oxo, Halo, Alkyl, Alkenyl, Alkinyl, Cyano, Nitro, Alkylcarbonyl, Carboxy, Alkoxycarbonyl, Hydroxyl, Alkoxy, Alkonyloxy, Alkylthio, Alkylsulfonyl, -Alkyl-OH, Aminosulfonyl, Sulfonylamino, Amino, Alkylamino oder Dialkylamino, substituiert ist;
R^{b} ist jeweils unabhängig Alkyl, Cycloalkyl, Phenyl, Heteroaryl oder Benzyl, wobei Alkyl, Cycloalkyl, Phenyl oder Benzyl mit 0, 1, 2, oder 3 Substituenten, unabhängig ausgewählt aus Halo, Alkyl, Haloalkyl, Alkoxy, Amino, Cyano, Hydroxyl, unsubstituiertem Heterocycloalkyl, Phenyl, Alkylcarbonylamino, Alkylamino oder Dialkylamino, substituiert ist; und
n ist jeweils unabhängig 0 oder 1; oder
ein pharmazeutisch annehmbares Salz hiervon.

2. Verbindung gemäß Anspruch 1, wobei R^{a} jeweils unabhängig Wasserstoff oder R^{b} ist; oder wenn zwei R^{a} an ein Stickstoffatom gebunden sind, entweder einzeln oder als Teil einer anderen Gruppe, können die beiden R^{a} zusammen mit dem Stickstoffatom, an das sie gebunden sind, einen monozyklische Heterocyclylring bilden, der gegebenenfalls mit einem, zwei oder drei Substituenten, unabhängig ausgewählt aus Oxo, Halo, Alkyl, Alkenyl, Alkinyl, Cyano, Nitro, Alkylcarbonyl, Carboxy, Alkoxycarbonyl, Hydroxyl, Alkoxy, Alkonyloxy, Alkylthio, Alkylsulfonyl, Aminosulfonyl, Sulfonylamino, Amino, Alkylamino oder Dialkylamino, substituiert ist; und
R^{b} jeweils unabhängig Alkyl, Cycloalkyl, Phenyl, Heteroaryl oder Benzyl ist, wobei Alkyl, Cycloalkyl, Phenyl oder Benzyl mit 0, 1, 2 oder 3 Substituenten substituiert ist, unabhängig ausgewählt aus Halo, Alkyl, Haloalkyl, Alkoxy, Amino, Cyano, Alkylamino oder Dialkylamino.

3. Verbindung gemäß Anspruch 1, wobei R¹ Wasserstoff ist und in 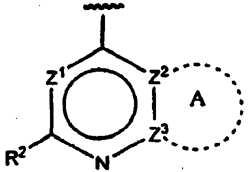 Z¹ -N- ist; Z² -CH- ist und Z³ -CR⁵- ist,
wobei R⁵ R^{d} oder -(Alkylen)ₙN(R^{a})(alkylen)ₙheteroaryl ist.

4. Verbindung gemäß Anspruch 1 oder 3, wobei R² Methyl ist.

5. Verbindung gemäß einem der Ansprüche 1, 3 oder 4, wobei Ar¹ Aryl ist, das, wie oben definiert, substituiert ist.

6. Verbindung gemäß einem der Ansprüche 1, 3 oder 4, wobei Ar¹ Heteroaryl ist, das, wie oben definiert, substituiert ist.

7. Verbindung gemäß einem der Ansprüche 1, 3 oder 4, wobei Ar¹ Pyrazolyl ist, das, wie oben definiert, substituiert ist.

8. Verbindung gemäß einem der Ansprüche 1, 3 oder 4, wobei Ar¹ ein Aryl-, Heteroaryl- oder Heterocyclylring ist, wobei jeder Ring mit R^{d}, R^{e} oder R^{f} substituiert ist, wobei R^{d}, R^{e} oder R^{f} unabhängig aus Wasserstoff, Halo, Haloalkyl, Alkyl, Alkyl, das mit -NR^{y}R^{y} (wobei R^{y} Alkyl ist) oder Alkoxy substituiert ist, -(CR^{y}R^{y})ₙY (wobei Y Aryl oder Heteroaryl ist), -OR^{X} (wobei R^{X} Wasserstoff oder Alkyl ist), Heteroaryl, Heterocycloalkyl, -C(=O)NR^{a}R^{a}, -C(=O)R^{b}, -C(=O)OR^{b}, -OR^{a}, -O-Alkyl-OR^{a}, -S(=O)₂R^{b}, -NR^{a}R^{a}, -N(R^{a})C(=O)R^{b}, -N(R^{a})C(=O)NR^{a}Ra, -N(R^{a})S(=O)₂R^{b} oder -NR^{a}-Alkylen-OR^{a} ausgewählt sind; R^{a} jeweils unabhängig Wasserstoff oder R^{b} ist; oder wenn zwei R^{a} an ein Stickstoffatom gebunden sind, entweder einzeln oder als Teil einer anderen Gruppe, können die beiden R^{a} zusammen mit dem Stickstoffatom, an das sie gebunden ist, einen monozyklischen Heterocyclylring bilden, der gegebenenfalls mit einem, zwei oder drei Substituenten, ausgewählt aus Alkyl, Alkylcarbonyl, -Alkyl-OH oder Hydroxyl, substituiert ist; und
R^{b} jeweils unabhängig Alkyl, Cycloalkyl, Phenyl, Heteroaryl oder Benzyl ist, wobei Alkyl, Cycloalkyl, Phenyl oder Benzyl mit 0, 1, 2 oder 3 Substituenten, unabhängig ausgewählt aus Halo, Alkyl, Haloalkyl, Alkoxy, Amino, Cyano, Alkylamino, Dialkylamino, Hydroxyl, unsubstutiertes Heterocycloalkyl oder Phenyl substituiert ist.

9. Verbindung gemäß einem der Ansprüche 1, 3 oder 4, wobei Ar¹ 5-Fluor-6-methoxy-3-pyridinyl, 1H-Pyrazol-3-yl, 1-Methyl-1H-pyrazol-3-yl, 4-(3-Ethylureido)phenyl, 1H-pyrrolo[2,3-b]pyridin-6-yl, 6-(Trifluormethyl)-2-pyridinyl, 2-Pyridinyl, 1H-Pyrrolo[3,2-b]pyridin-5-yl, 4-Hydroxypyridin-2-yl, 4-(3-Phenylureido)phenyl, 5-(3-Ethylureido)pyridin-2-yl, 4-(3-Methylureido)phenyl, 3-Hydroxyphenyl, 4-Acetylaminophenyl, 4-(3-(4-Pyridinyl)ureidophenyl, 3-Aminopyrimidin-5-yl, 4-Pyridazinyl, 1-Acetyl-1H-pyrazol-3-yl, 3-(Methylsulfonyl)phenyl, 3-Pyridinyl, 3-Methyl-5-isothiazolyl, 4-(4-Methyl-1-piperazinyl)phenyl, 1H-Pyrazolo-[3,4-c]pyridin-3-yl, 4-Fluor-1H-indazol-3-yl)imidazo[1,2-b]pyridazin-2-yl, 1-(2-(Dimethylamino)ethyl)-1H-pyrazol-3-yl, 1H-Pyrazolo[3,4-b]pyridin-3-yl, 3-(4-Methyl-1-piperazinyl)phenyl, 3-Acetylaminophenyl, Pydridin-2-yl, 1-Ethyl-1H-pyrazol-3-yl, Chinolin-3-yl, Isochinolin-3-yl, 1-Methylaminocarbonyl-1H-pyrazol-3-yl, 1-Methylaminocarbonyl-1H-pyrazol-5-yl, 1-(2-Hydroxyethylaminocarbonyl)-1H-pyrazol-3-yl, 1-(2-Hydroxyethylaminocarbonyl)-1H-pyrazol-5-yl, 1-(Morpholin-4-ylcarbonyl)-1H-pyrazol-3-yl, 1-(Morpholin-4-ylcarbonyl)-1H-pyrazol-5-yl, 1-(Phenoxycarbonyl)-1H-pyrazol-3-yl, 1-(Phenylaminocarbonyl)-1H-pyrazol-3-yl, 1-(Phenylaminocarbonyl)-1H-pyrazol-5-yl, 1-(Methoxycarbonyl)-1H-pyrazol-3-yl, 1-(Methoxycarbonyl)-1H-pyrazol-5-yl, 1-(Phenoxycarbonyl)-1H-pyrazol-4-yl, 1-(Methylaminocarbonyl)-1H-pyrazol-4-yl, 1-(Pyridin-2-yl)-1H-pyrazol-3-yl, 1-(Methylsulfonyl)-1H-pyrazol-3-yl, (1-(benzoxazol-2-yl)-1H-pyrazol-3-yl, 1-(Benzimidazol-2-yl)-1H-pyrazol-5-yl, 1-(Isopropylaminocarbonyl)-1H-pyrazol-3-yl, 1-(Benzylaminocarbonyl)-1H-pyrazol-3-yl, 1-(Ethylaminocarbonyl)-1H-pyrazol-3-yl, 1-(2-Methoxyphenylaminocarbonyl)-1H-pyrazol-3-yl, 1-(2-Methylphenylaminocarbonyl)-1H-pyrazol-3-yl, 1-(4-Methoxyphenylaminocarbonyl)-1H-pyrazol-3-yl, 1-(2-Fluorphenylaminocarbonyl)-1H-pyrazol-3-yl, 1-(4-Cyanophenylaminocarbonyl)-1H-pyrazol-3-yl, 1-(2-Trifluormethylphenylaminocarbonyl)-1H-pyrazol-3-yl, 1-(3-Methylphenylaminocarbonyl)-1H-pyrazol-3-yl, 1-(2,5-Difluorphenylaminocarbonyl)-1H-pyrazol-3-yl, 1-(3,5-Difluorphenylaminocarbonyl)-1H-pyrazol-3-yl, 1-(4-Methylphenylaminocarbonyl)-1H-pyrazol-3-yl, 1-(3,5-Dichlorphenylaminocarbonyl)-1H-pyrazol-3-yl, 1-(2-Chlor-5-trifluormethylphenylaminocarbonyl)-1H-pyrazol-3-yl, 1-(2,5-Dichlorphenylaminocarbonyl)-1H-pyrazol-3-yl, 1-(2,3-Dichlorphenylaminocarbonyl)-1H-pyrazol-3-yl, 1-(2-Chlor-4-trifluormethylphenylaminocarbonyl)-1H-pyrazol-3-yl, 1-(Benzimidazol-2-yl)-1H-pyrazol-3-yl, 1-(Imidazol-2-yl, 1-(Imidazol-2-yl)-1H-pyrazol-5-yl, 1-Methylsulfonyl-1H-pyrazol-3-yl, 1-aminocarbonyl-1H-pyrazol-3-yl, 1-Aminocarbonyl-1H-pyrazol-5-yl, 1-Azetidin-1-ylcarbonyl-1H-pyrazol-3-yl, 1-Azetidin-1-ylcarbonyl-1H-pyrazol-5-yl, 1-(2-Methylaminoethylamino)-carbonyl-1H-pyrazol-3-yl, 1-(2-Methylaminoethylamino)carbonyl-1H-pyrazol-5-yl, 1-Dimethylaminocarbonyl-1H-pyrazol-3-yl, 2-Dimethylaminocarbonyl-1H-pyrazol-5-yl, 1-Piperazin-1-ylcarbonyl-1H-pyrazol-3-yl, 1-(2-Methoxyethylamino)carbonyl-1H-pyrazol-3-yl, 1-(2-Methoxyethylamino)carbonyl-1H-pyrazol-5-yl, 1-(2-Morpholin-4-ylethylamino)carbonyl-1H-pyrazol-3-yl, 1-(2-Methylpropylaminocarbonyl)-1H-pyrazol-3-yl, 1-(1-Phenethylaminocarbonyl)-1H-pyrazol-3-yl, 1-(Benzyl)-1H-pyrazol-3-yl, 1-(Pyridin-3-ylmethyl)-1H-pyrazol-3-yl, 1-(1,4-Benzoxazin-3-(4H)-on-7-yl)-1H-pyrazol-3-yl, 5-(4-Hydroxyazetidin-1-yl)pyridin-3-yl, 5-(4-)Propan-2-ol)azetidin-1-yl)pyridin-3-yl, Chinolin-7-yl, Chinolin-6-yl, Isochinolin-7-yl, 6-Methoxypyridin-3-yl, 6-Ethoxypyridin-3-yl, 1H-Indazol-3-yl, 5-Fluor-6-methoxypyridin-3-yl, 1-(6-(2-Methoxyethoxy)pyrimidin-3-yl)-1H-pyrazol-3-yl, 3-Cyclopropylureidophenyl, 3-Benzylcarbonylaminophenyl, 3-Benzoylaminophenyl, 3-(4-Chlorphenylsulfonylamino)phenyl, 3-Methylsulfonylaminophenyl, 3-(4-Fluorphenylureido)phenyl, 3-(Benzylureido)phenyl, 3-Ethylaminocarbonylphenyl, 6-Ethylaminocarbonylpyridin-3-yl, 3-Ethylcarbonylaminophenyl, 3-Methylaminocarbonylphenyl, 3-Ethylaminocarbonylphenyl, 3-Isopropylaminocarbonylphenyl, 3-Phenylaminocarbonylphenyl, 6-Methylaminocarbonylpyridin-3-yl, 6-Isopropylaminocarbonylpyridin-3-yl, 6-Phenylaminocarbonylpyridin-3-yl, 3-Aminocarbonylphenyl, 4-(2-Diethylaminoethylaminocarbonyl)phenyl, 4-Phenylaminocarbonylphenyl, 1-(3-Methylbutanoyl)-1H-pyrazol-3-yl, 1-Isopropyloxycarbonyl-1H-pyrazol-3-yl, 1-Isopropyloxycarbonyl-1H-pyrazol-5-yl, 2-Methoxypyridin-4-yl, 1-Isopropylcarbonyl-1H-pyrazol-3-yl, 1-Isopropylcarbonyl-1H-pyrazol-5-yl, 1-(3,3-Dimethylbutanoyl)-1H-pyrazol-3-yl, 1-Cyclohexyloxycarbonyl-1H-pyrazol-5-yl, 1-Cyclohexyloxycarbonyl-1H-pyrazol-3-yl, 2-Methoxypyridin-3-yl, 2-Oxopyridin-3-yl, 1-(3-Methylbutyl)-1H-pyrazol-3-yl, 1-(2-Methoxyethyl)-1H-pyrazol-3-yl, 1H-Indazol-4-yl, 6-Chlor-5-methylsulfonylaminopyridin-3-yl, 6-Methoxy-5-methylsulfonylaminopyridin-3-yl, 1H-Indazol-6-yl, 1H-Benzimidazol-6-yl, 4-(3-Cyclopropylureido)phenyl, 4-Piperidin-1-ylcarbonylaminophenyl, 4-Pyrrolidin-1-ylcarbonylaminophenyl, 4-Azetidin-1-ylcarbonylaminophenyl, 4-(1-Methylpiperazin-1-ylcarbonylaminophenyl, 4-(3-(2-Methoxyethyl)ureido)phenyl, 4-Morpholin-4-yl-ylcarbonylaminophenyl, 4-(1-Acetylpiperazin-1-ylcarbonylaminophenyl, Thiazol-3-yl, 4-(2-Methylmorpholin-4-yl-ylcarbonylamino)phenyl oder 4-(3-Methylmorpholin-4-yl-ylcarbonylamino)phenyl ist.

10. Verbindung gemäß einem der Ansprüche 1 und 3 bis 9, wobei R¹ R^{d} ist, ausgewählt aus Wasserstoff, Halo, Haloalkyl, substituiertes Alkyl, Aryl, Heteroaryl, Heterocycloalkyl, -C(=O)NR^{a}R^{a}, -C(=O)OR^{b}, -COR^{b}, -OR^{a}, -O-Alkyl-NR^{a}R^{a}, -O-Alkyl-OR^{a}, -NR^{a}R^{a} oder -NR^{a}-Alkylen-OR^{a}; R^{a} jeweils unabhängig Wasserstoff oder R^{b} ist oder wenn zwei R^{a} an ein Stickstoffatom gebunden sind, entweder einzeln oder als Teil einer anderen Gruppe, die beiden R^{a} zusammen mit dem Stickstoffatom, an das sie gebunden sind, einen monozyklischen Heterocyclylring bilden können,
R^{b} jeweils unabhängig Alkyl, Phenyl oder Heteroaryl ist, wobei Alkyl mit 0, 1, 2 oder 3 Substituenten, ausgewählt aus Alkyl oder Alkoxy substituiert ist,
wobei der Arylring gegebenenfalls mit einem, zwei oder drei Substituenten, unabhängig ausgewählt aus Alkyl, Alkoxy oder Halo und/oder einem oder zwei Substituenten, unabhängig ausgewählt aus -C(=O)OR^{y}, -C(=O)NR^{x}R^{x}, -OR^{x}, -SR^{x}, -S(=O)₂R^{y}, -S(=O)₂NR^{x}R^{x}, -NR^{x}R^{x}, -N(R^{x})C(=O)R^{y}, -(CR^{x}R^{x})ₘN(R^{x})C(=O)R^{y} oder -(CR^{x}R^{x})ₘOR^{x} substituiert ist, wobei m 1 bis 3 ist, R^{x} jeweils Wasserstoff oder R^{y} ist und RY unabhängig Wasserstoff, Alkyl oder Cycloalkyl ist;
der Heterocycloalkylring gegebenenfalls mit einem, zwei oder drei Substituenten, ausgewählt aus Alkyl, Hydroxy, -C(=O)R^{y}, -C(=O)OR^{y}, -C(=O)NR^{x}R^{x}, -S(=O)₂R^{y} oder -(CR^{x}R^{x})ₘOR, substituiert ist, wobei m 0 bis 3 ist, R^{x} jeweils Wasserstoff oder R^{y} ist und R^{y} unabhängig Wasserstoff oder Alkyl ist;
der Heteroarylring gegebenenfalls mit einem, zwei oder drei Substituenten, unabhängig ausgewählt aus Alkyl, -OR^{x}, -C(=O)NR^{x}R^{x} oder -(CR^{x}R^{x})ₘOR^{x}, substituiert ist, wobei m 1 bis 3 ist, R^{x} jeweils Wasserstoff oder R^{y} ist und R^{y} unabhängig Wasserstoff oder Alkyl ist; und
substituiertes Alkyl ein Alkylrest ist, in dem ein, zwei oder drei Wasserstoffatome unabhängig durch Hydroxyl oder -NCOR^{y} ersetzt sind, wobei R^{y} Alkyl ist.

11. Verbindung gemäß einem der Ansprüche 1 und 3 bis 9, wobei
R¹ Wasserstoff, 1-Piperazinyl, 4-Methyl-1-piperazinyl, 4-(1-Methylethyl)-1-piperazinyl, 4-(2-Methoxyethyl)-1-piperazinyl, Fluor, 3-(Methylsulfonyl)phenyl, 4-Pyridinyl, 2-Methoxyethoxy, 4-Morpholinyl, 2-(1-Pyrrolidinyl)ethoxy, 1-tert-Butoxycarbonyl-3,6-dihydro-1(2H)-pyridin-4-yl, 3-Methoxyphenyl, 3-Fluor-4-methylaminocarbonylphenyl, 4-Acetylaminomethylphenyl, 1-(2-Methoxyethyl)-1H-pyrazol-4-yl, 2-Fluorphenyl, 2-Methylphenyl, 2-Methoxy-3-pyridinyl, 3-Furanyl, 3,5-Dimethyl-4-isoxazolyl, 4-Fluor-2-methylphenyl, 2-(1-Methylethoxy)phenyl, 3-(Methylsulfanyl)phenyl, 1,2,3,6-Tetrahydro-4-pyridinyl, 3-Methyl-5-isoxazolyl, 1-(2-Methoxyethyl)-1H-pyrazol-4-yl, 3-Acetylaminophenyl, 3-Cyclopropylaminophenyl, 3-Isopropanoylaminophenyl, 3-Pyridazinyl, 1-Methyl-1,2,3,6-tetrahydro-4-pyridinyl, 4-Acetylaminophenyl, 2-Methyl-4-pyridinyl, 2-Methyl-3-pyridinyl, 5-Pyrimidinyl, 3-Pyridinyl, Phenylamino, 4-Methoxyphenylamino, Methoxy, 3-Fluorphenyl, 4-Methylaminocarbonylphenyl, Pyridin-3-ylamino, 4-Acetylaminophenyl, 3-Methoxyphenylamino, Pyridin-4-ylamino, Pyrimidin-5-ylamino, 4-Acetylaminophenylamino, 2-Methyl-4-pyridinylamino, 4-Methyl-3-pyridinyl, 2,6-Dimethyl-4-pyridinyl, 4-Isopropylaminocarbonylphenyl, 3,4-Dihydro-1(2H)-isochinolinon-6-yl, 4-Ethylaminocarbonylphenyl, 4-Carboxy-2-methylphenyl, 4-Methyl-5-pyrimidinyl, 2-Methyl-4-methylaminocarbonylphenyl, Hydroxyl, 6-Methylaminocarbonylpyridin-3-yl, 2-(1-Pyrrolidinyl)ethoxy, 4-N,N-Dimethylaminocarbonylpiperazin-1-yl, 4-Methylsulfonylpiperazin-1-yl, 4-N-Methylaminocarbonylpiperazin-1-yl, 4-Acetylpiperazin-1-yl, 3-Hydroxymethyl-4-methoxyphenyl, Pyridin-4-yl, 3,6-Dihydro-2H-pyran-4-yl, 2-Methyl-4-pyridinyl, 1-Methyl-1H-imidazol-5-yl, 3,4-Dihydroxybutyl, 1-(1-Methylethyl)-1H-pyrazol-4-yl, Aminocarbonyl, Chinolin-7-yl, 4-Methylsulfonylphenyl, 4-Methylaminosulfonylphenyl, 3-Methylsulfonylphenyl, 2-Methoxyethylamino, 4-Hydroxypiperidin-1-yl, 1-Methyl-1H-pyrazol-4-yl, 2-Hydroxyprop-2-yl, Acetyl oder Trifluormethyl ist.

12. Verbindung gemäß Anspruch 1, ausgewählt aus der Gruppe, bestehend aus:
| |
|---|
| 3-(4-Amino-6-methyl-1,3,5-triazin-2-yl)-N-(1H-pyrazol-3-yl)imidazo[1,2-a]pyridin-2-amin |
| 3-(3-(2-Methyl-9H-purin-4-yl)imidazo[1,2-a]pyridin-2-ylamino)phenol |
| 3-(4-Methyl-6-(methylamino)-1,3,5-triazin-2-yl)-N-(1H-pyrazol-3-yl)imidazo[1,2-a]pyridin-2-amin |
| 3-(4-Amino-6-methyl-1,3,5-triazin-2-yl)-N-(6-methoxypyridin-3-yl)imidazo[1,2-a]pyridin-2-amin |
| N-(3-(2-Methyl-9H-purin-4-yl)imidazo[1,2-a]pyridin-2-yl)-1H-indazol-4-amin |
| 7-Chlor-3-(4-amino-6-methyl)-1,3,5-triazin-2-yl)-N-(1H-pyrazol-3-yl)imidazo[1,2-a]pyridin-2-amin |
| 3-(6-Amino-2-methylpyrimidin-4-yl)-N-(1H-pyrazol-3-yl)imidazo[1,2-b]pyridazin-2-amin |
| 3-(4-Amino-6-methyl-1,3,5-triazin-2-yl)-N-(5-fluor-6-methoxypyridin-3-yl)imidazo[1,2-a]pyridin-2-amin |
| 3-(4-Amino-6-methyl-1,3,5-triazin-2-yl)-N-(6-methoxypyridin-3-yl)-6-fluorimidazo[1,2-a]pyridin-2-amin |
| 3-(4-Amino-6-methyl-1,3,5-triazin-2-yl)-N-(5-fluor-6-methoxy-3-pyridinyl)-6-(1-piperazinyl)imidazo[1,2-a]pyridin-2-amin |
| 3-(4-Amino-6-methyl-1,3,5-triazin-2-yl)-N-(5-fluor-6-methoxy-3-pyridinyl)-6-(4-methyl-1-piperazinyl)imidazo[1,2-a]pyridin-2-amin |
| 3-(4-Amino-6-methyl-1,3,5-triazin-2-yl)-N-(5-fluor-6-methoxy-3-pyridinyl)-6-(4-(1-methyl-ethyl)-1-piperazinyl)-imidazo[1,2-a]pyridin-2-amin |
| 3-(4-Amino-6-methyl-1,3,5-triazin-2-yl)-N-(5-fluor-6-methoxy-3-pyridinyl)-6-(4-(2-methoxyethyl)-1-piperazinyl)imidazo[1,2-a]pyridin-2-amin |
| 3-(2-Methyl-6-(phenylamino)-4-pyrimidinyl)-N-1H-pyrazol-3-ylimidazo[1,2-b]pyridazin-2-amin |
| 3-(6-((2-Fluorphenyl)amino)-2-methyl-4-pyrimidinyl)-N-1H-pyrazol-3-ylimidazo[1,2-b]pyridazin-2-amin |
| 3-(6-((4-Fluorphenyl)amino)-2-methyl-4-pyrimidinyl)-N-1H-pyrazol-3-ylimidazo[1,2-b]pyridazin-2-amin |
| 3-(6-((4-Methoxyphenyl)amino)-2-methyl-4-pyrimidinyl)-N-1H-pyrazol-3-ylimidazo[1,2-b]pyridazin-2-amin |
| N-(1-Methyl-1H-pyrazol-3-yl)-3-(2-methyl-6-(3-pyridazinylamino)-4-pyrimidinyl)imidazo[1,2-b]pyridazin-2-amin |
| 3-(2-Methyl-6-((6-(4-methyl-1-piperazinyl)-3-pyridazinyl)amino)-4-pyrimidinyl)-N-1H-pyrazol-3-ylimidazo[1,2-b]pyridazin-2-amin |
| 3-(6-((5-Methoxy-2-pyridinyl)amino)-2-methyl-4-pyrimidinyl)-N-1H-pyrazol-3-ylimidazo[1,2-b]pyridazin-2-amin |
| 3-(6-((3-Fluorphenyl)amino)-2-methyl-4-pyrimidinyl)-N-1H-pyrazol-3-ylimidazo[1,2-b]pyridazin-2-amin |
| 3-(6-((4-Methoxy-2-pyridinyl)amino)-2-methyl-4-pyrimidinyl)-N-1H-pyrazol-3-ylimidazo[1,2-b]pyridazin-2-amin |
| 3-(2-Methyl-6-((6-(4-morpholinyl)-3-pyridazinyl)amino)-4-pyrimidinyl)-N-1H-pyrazol-3-ylimidazo[1,2-b]pyridazin-2-amin |
| 1-(4-((3-(6-Amino-2-methyl-4-pyrimidinyl)imidazo[1,2-a]pyridin-2-yl)amino)phenyl)-3-ethylharnstoff |
| 3-(6-Amino-2-methyl-4-pyrimidinyl)-N-1H-pyrrolo[2,3-b]pyridin-6-ylimidazo[1,2-a]pyridin-2-amin |
| 3-(6-Amino-2-methyl-4-pyrimidinyl)-N-(6-(trifluormethyl)-2-pyridinyl)imidazo[1,2-a]pyridin-2-amin |
| 3-(6-Amino-2-methyl-4-pyrimidinyl)-N-2-pyridinylimidazo[1,2-a]pyridin-2-amin |
| 3-(6-((5-(2-Methoxyethoxy)-3-pyridazinyl)amino)-2-methyl-4-pyrimidinyl)-N-1H-pyrazol-3-ylimidazo[1,2-b]pyridazin-2-amin |
| N-(3-(6-Amino-2-methyl-4-pyrimidinyl)imidazo[1,2-a]pyridin-2-yl)-1H-pyrrolo[3,2-b]pyridin-5-amin |
| 2-((3-(6-Amino-2-methyl-4-pyrimidinyl)imidazo[1,2-a]pyridin-2-yl)amino)-4-pyridinol |
| 1-(4-((3-(6-Amino-2-methyl-4-pyrimidinyl)imidazo[1,2-a]pyridin-2-yl)amino)phenyl)-3-phenylharnstoff |
| 1-(6-((3-(6-Amino-2-methyl-4-pyrimidinyl)imidazo[1,2-a]pyridin-2-yl)amino)-3-pyridinyl)-3-ethylharnstoff |
| 3-(2-Methyl-6-((6-(4-morpholinyl)-3-pyridazinyl)amino)-4-pyrimidinyl)-N-1H-pyrazol-3-ylimidazo[1,2-a]pyridin-2-amin |
| 1-(4-((3-(6-Amino-2-methyl-4-pyrimidinyl)imidazo[1,2-a]pyridin-2-yl)amino)phenyl)-3-methylharnstoff |
| 3-((3-(6-((5-(2-Methoxyethoxy)-3-pyridazinyl)amino)-2-methyl-4-pyrimidinyl)imidazo[1,2-b]pyridazin-2-yl)amino)phenol |
| N-(4-((3-(6-((5-(2-Methoxyethoxy)-3-pyridazinyl)amino)-2-methyl-4-pyrimidinyl)imidazo[1,2-b]pyridazin-2-yl)amino)phenyl)acetamid |
| 3-(6-((5-Methoxy-3-pyridazinyl)amino)-2-methyl-4-pyrimidinyl)-N-1H-pyrazol-3-ylimidazo-[1,2-b]pyridazin-2-amin |
| 1-Ethyl-3-(4-((3-(6-((5-(2-methoxyethoxy)-3-pyridazinyl)amino)-2-methyl-4-pyrimidinyl)-imidazo[1,2-b]pyridazin-2-yl)amino)phenyl)harnstoff |
| 3-(2-Methyl-6-((5-methyl-3-pyridazinyl)amino)-4-pyrimidinyl)-N-1H-pyrazol-3-ylimidazo[1,2-b]pyridazin-2-amin |
| 1-(4-((3-(6-Amino-2-methyl-4-pyrimidinyl)imidazo[1,2-a]pyridin-2-yl)amino)phenyl)-3-(4-pyridinyl)harnstoff |
| 3-(6-((5-(2-Methoxyethoxy)-3-pyridazinyl)amino)-2-methyl-4-pyrimidinyl)-N-1H-pyrazol-3-ylimidazo[1,2-a]pyridin-2-amin |
| N-(6-(2-((4-((Ethylcarbamoyl)amino)phenyl)amino)imidazo-[1,2-a]pyridin-3-yl)-2-methyl-4-pyrimidinyl)acetamid |
| 3-(2-Methyl-6-((5-(2,2,2-trifluorethoxy)-3-pyridazinyl)amino)-4-pyrimidinyl)-N-1H-pyrazol-3-ylimidazo[1,2-b]pyridazin-2-amin |
| N-5--(3-(6-((5-(2-Methoxyethoxy)-3-pyridazinyl)amino)-2-methyl-4-pyrimidinyl)imidazo[1,2-b]pyridazin-2-yl)-2,5-pyrimidin-diamin |
| 8-Fluor-3-(6-((5-(2-methoxyethoxy)-3-pyridazinyl)amino)-2-methyl-4-pyrimidiny1)-N-1H-pyrazol-3-ylimidazo[1,2-a]pyridin-2-amin |
| N-(6-(8-Fluor-2-((1-methyl-1H-pyrazol-3-yl)amino)imidazo[1,2-a]pyridin-3-yl)-2-methyl-4-pyrimidinyl)acetamid |
| 3-(6-Amino-2-methyl-4-pyrimidinyl)-N-4-pyridazinylimidazo[1,2-b]pyridazin-2-amin |
| 3-(6-Amino-2-methyl-4-pyrimidinyl)-N-(1-methyl-1H-pyrazol-3-yl)-6-(3-(methylsulfonyl)phenyl)imidazo[1,2-a]pyridin-2-amin |
| 3-(2-Methyl-6-(methylamino)-4-pyrimidinyl)-N-1H-pyrazol-3-ylimidazo[1,2-b]pyridazin-2-amin |
| N-(1-Acetyl-1H-pyrazol-3-yl)-3-(6-amino-2-methyl-4-pyrimidinyl)imidazo[1,2-b]pyridazin-2-amin |
| 3-(6-Amino-2-methyl-4-pyrimidinyl)-N-1H-pyrazol-3-yl-6-(4-pyridinyl)imidazo[1,2-b]pyridazin-2-amin |
| 3-(6-Amino-2-methyl-4-pyrimidinyl)-6-(2-methoxyethoxy)-N-1H-pyrazol-3-ylimidazo[1,2-b]pyridazin-2-amin |
| N-(2-Methyl-6-(2-(1H-pyrazol-3-ylamino)imidazo[1,2-b]pyridazin-3-yl)-4-pyrimidinyl)-acetamid |
| 3-(6-Amino-2-methyl-4-pyrimidinyl)-6-(4-morpholinyl)-N-1H-pyrazol-3-ylimidazo[1,2-b]pyridazin-2-amin |
| 3-(2-Methyl-6-(methylamino)-4-pyrimidinyl)-6-(4-morpho-linyl)-N-1H-pyrazol-3-ylimidazo[1,2-b]pyridazin-2-amin |
| 3-(6-Amino-2-methyl-4-pyrimidinyl)-N-1H-pyrazol-3-yl-6-(2-(1-pyrrolidinyl)ethoxy)-imidazo[1,2-b]pyridazin-2-amin |
| 3-(2-Methyl-6-(2-pyrazinylamino)-4-pyrimidinyl)-N-1H-pyrazol-3-ylimidazo[1,2-b]pyridazin-2-amin |
| 1,1-Dimethyl-3-(2-methyl-6-(2-(1H-pyrazol-3-ylamino)-imidazo[1,2-b]pyridazin-3-yl)-4-pyrimidinyl)harnstoff |
| 1-Ethyl-3-(2-methyl-6-(2-(1H-pyrazol-3-ylamino)-imidazo[1,2-b]pyridazin-3-yl)-4-pyrimidinyl)harnstoff |
| 4-Methyl-N-(2-methyl-6-(2-(1H-pyrazol-3-ylamino)imidazo-[1,2-b]pyridazin-3-yl)-4-pyrimidinyl)-1-piperazin-carboxamid |
| N-(2-Methyl-6-(2-(1H-pyrazol-3-ylamino)imidazo[1,2-b]pyridazin-3-yl)-4-pyrimidinyl)-4-morpholin-carboxamid |
| 3-(2-Methyl-6-(1H-pyrazol-3-ylamino)-4-pyrimidinyl)-N-1H-pyrazol-3-ylimidazo[1,2-b]pyridazin-2-amin |
| 3-(6-(Dimethylamino)-2-methyl-4-pyrimidinyl)-N-(1-methyl-1H-pyrazol-3-yl)imidazo[1,2-b]pyridazin-2-amin |
| 3-(2-Methyl-6-(1,3-thiazol-2-ylamino)-4-pyrimidinyl)-N-1H-pyrazol-3-ylimidazo[1,2-b]pyridazin-2-amin |
| 3-(2-Methyl-6-(2-pyridinylamino)-4-pyrimidinyl)-N-1H-pyrazol-3-ylimidazo[1,2-b]pyridazin-2-amin |
| 3-(2-Methyl-6-(3-pyridazinylamino)-4-pyrimidinyl)-N-1H-pyrazol-3-ylimidazo[1,2-b]pyridazin-2-amin |
| 3-(2-Methyl-6-(4-pyrimidinylamino)-4-pyrimidinyl)-N-1H-pyrazol-3-ylimidazo[1,2-b]pyridazin-2-amin |
| 3-(2-Methyl-6-(2-pyrimidinylamino)-4-pyrimidinyl)-N-1H-pyrazol-3-ylimidazo[1,2-b]pyridazin-2-amin |
| N-(2-Methyl-6-(2-((1-methyl-1H-pyrazol-3-yl)amino)-imidazo[1,2-b]pyridazin-3-yl)-4-pyrimidinyl)acetamid |
| 3-(6-Amino-2-methyl-4-pyrimidinyl)-7-(4-morpholinyl)-N-1H-pyrazol-3-ylimidazo[1,2-b]pyridazin-2-amin |
| 3-(2-Methyl-6-(methylamino)-4-pyrimidinyl)-7-(4-morpholi-nyl)-N-1H-pyrazol-3-ylimidazo[1,2-b]pyridazin-2-amin |
| N-(2-Methyl-6-(2-(1H-pyrazol-3-ylamino)imidazo[1,2-b]pyridazin-3-yl)-4-pyrimidinyl)-methan-sulfonamid |
| ((2-Methyl-6-(2-(1H-pyrazol-3-ylamino)imidazo[1,2-b]pyridazin-3-yl)-4-pyrimidinyl)amino)-acetonitril |
| 3-(6-Amino-2-methyl-4-pyrimidinyl)-8-fluor-N-1H-pyrazol-3-ylimidazo[1,2-a]pyridin-2-amin |
| N-2-,N-2--Dimethyl-N-(2-methyl-6-(2-((1-methyl-1H-pyrazol-3-yl)amino)imidazo[1,2-b]pyridazin-3-yl)-4-pyrimidinyl)-glycinamid |
| N-(2-Methyl-6-(2-((1-methyl-1H-pyrazol-3-yl)amino)imidazo-[1,2-b]pyridazin-3-yl)-4-pyrimidinyl)glycinamid |
| 3-(6-Amino-2-methyl-4-pyrimidinyl)-N-(3-(methylsulfonyl)-phenyl)imidazo[1,2-b]pyridazin-2-amin |
| 3-(6-Amino-2-methyl-4-pyrimidinyl)-N-3-pyridinylimidazo-[1,2-b]pyridazin-2-amin |
| 3-(6-Amino-2-methyl-4-pyrimidinyl)-N-(3-methyl-5-isothiazolyl)imidazo[1,2-b]pyridazin-2-amin |
| tert-butyl-4-(3-(4-Amino-6-methyl-1,3,5-triazin-2-yl)-2-(1H-pyrazol-3-ylamino)imidazo[1,2-a]pyridin-6-yl)-3,6-dihydro-1(2H)-pyridin-carboxylat |
| 3-(4-Amino-6-methyl-1,3,5-triazin-2-yl)-6-(3-methoxy-phenyl)-N-1H-pyrazol-3-ylimidazo[1,2-a]pyridin-2-amin |
| 4-(3-(4-Amino-6-methyl-1,3,5-triazin-2-yl)-2-(1H-pyrazol-3-ylamino)imidazo[1,2-a]pyridin-6-yl)-2-fluor-N-methylbenzamid |
| N-(4-(3-(4-Amino-6-methyl-1,3,5-triazin-2-yl)-2-(1H-pyrazol-3-ylamino)imidazo[1,2-a]pyridin-6-yl)benzyl)-acetamid |
| 3-(4-Amino-6-methyl-1,3,5-triazin-2-yl)-6-(1-(2-methoxy-ethyl)-1H-pyrazol-4-yl)-N-1H-pyrazol-3-ylimidazo[1,2-a]pyridin-2-amin |
| 3-(4-Amino-6-methyl-1,3,5-triazin-2-yl)-6-(2-fluorphenyl)-N-1H-pyrazol-3-ylimidazo[1,2-a]pyridin-2-amin |
| 3-(4-Amino-6-methyl-1,3,5-triazin-2-yl)-6-(2-methyl-phenyl)-N-1H-pyrazol-3-ylimidazo[1,2-a]pyridin-2-amin |
| 3-(4-Amino-6-methyl-1,3,5-triazin-2-yl)-6-(2-methoxy-3-pyridinyl)-N-1H-pyrazol-3-ylimidazo[1,2-a]pyridin-2-amin |
| 3-(4-Amino-6-methyl-1,3,5-triazin-2-yl)-6-(3-furanyl)-N-1H-pyrazol-3-ylimidazo[1,2-a]pyridin-2-amin |
| 3-(4-Amino-6-methyl-1,3,5-triazin-2-yl)-6-(3,5-dimethyl-4-isoxazolyl)-N-1H-pyrazol-3-ylimidazo[1,2-a]pyridin-2-amin |
| 3-(4-Amino-6-methyl-1,3,5-triazin-2-yl)-6-(4-fluor-2-methylphenyl)-N-1H-pyrazol-3-ylimidazo[1,2-a]pyridin-2-amin |
| 3-(4-Amino-6-methyl-1,3,5-triazin-2-yl)-6-(2-(1-methylethoxy)phenyl)-N-1H-pyrazol-3-ylimidazo[1,2-a]pyridin-2-amin |
| 3-(4-Amino-6-methyl-1,3,5-triazin-2-yl)-6-(3-(methylsulfanyl)phenyl)-N-1H-pyrazol-3-ylimidazo[1,2-a]pyridin-2-amin |
| N-(4-(3-(4-Methyl-6-(methylamino)-1,3,5-triazin-2-yl)-2-(1H-pyrazol-3-ylamino)imidazo[1,2-a]pyridin-6-yl)benzyl)acetamid |
| 3-(4-Amino-6-methyl-1,3,5-triazin-2-yl)-N-1H-pyrazol-3-yl-6-(1,2,3,6-tetrahydro-4-pyridinyl)-imidazo[1,2-a]pyridin-2-amin |
| 3-(4-Amino-6-methyl-1,3,5-triazin-2-yl)-6-(3-methyl-5-isoxazolyl)-N-1H-pyrazol-3-ylimidazo-[1,2-a]pyridin-2-amin |
| 6-(1-(2-Methoxyethyl)-1H-pyrazol-4-yl)-3-(4-methyl-6-(methylamino)-1,3,5-triazin-2-yl)-N-1H-pyrazol-3-ylimidazo[1,2-a]pyridin-2-amin |
| N-(3-(3-(4-Amino-6-methyl-1,3,5-triazin-2-yl)-2-(1H-pyrazol-3-ylamino)imidazo[1,2-a]pyridin-6-yl)phenyl)-acetamid |
| N-(3-(3-(4-Amino-6-methyl-1,3,5-triazin-2-yl)-2-(1H-pyrazol-3-ylamino)imidazo[1,2-a]pyridin-6-yl)phenyl)-cyclopropan-carboxamid |
| N-(4-(3-(4-Amino-6-methyl-1,3,5-triazin-2-yl)-2-(1H-pyrazol-3-ylamino)imidazo[1,2-a]pyridin-6-yl)phenyl)-2-methylpropanamid |
| 3-(4-Amino-6-methyl-1,3,5-triazin-2-yl)-N-1H-pyrazol-3-yl-6-(3-pyridazinyl)imidazo[1,2-a]pyridin-2-amin |
| 3-(4-Amino-6-methyl-1,3,5-triazin-2-yl)-6-(1-methyl-1,2,3,6-tetrahydro-4-pyridinyl)-N-1H-pyrazol-3-ylimidazo[1,2-a]pyridin-2-amin |
| N-(4-(3-(6-Amino-2-methyl-4-pyrimidinyl)-2-(1H-pyrazol-3-ylamino)imidazo[1,2-b]pyridazin-6-yl)benzyl)acetamid |
| N-(2-Methyl-6-(2-(1H-pyrazol-3-ylamino)imidazo[1,2-b]pyridazin-3-yl)-4-pyrimidinyl)-cyclopropan-carboxamid |
| N-(4-(3-(6-Amino-2-methyl-4-pyrimidinyl)-2-(1H-pyrazol-3-ylamino)imidazo[1,2-b]pyridazin-6-yl)phenyl)acetamid |
| 4-(3-(6-Amino-2-methyl-4-pyrimidinyl)-2-(1H-pyrazol-3-ylamino)imidazo[1,2-b]pyridazin-6-yl)-N-methylbenzamid |
| 2-Methoxy-N-(2-methyl-6-(2-((1-methyl-1H-pyrazol-3-yl)amino)imidazo[1,2-b]pyridazin-3-yl)-4-pyrimidinyl)-acetamid |
| 3-(6-Amino-2-methyl-4-pyrimidinyl)-N-1H-pyrazol-3-ylimidazo[1,2-a]pyridin-2-amin |
| 3-(6-Amino-2-methyl-4-pyrimidinyl)-N-(1-methyl-1H-pyrazol-3-yl)imidazo[1,2-a]pyridin-2-amin |
| 2-Cyano-N-(2-methyl-6-(2-((1-methyl-1H-pyrazol-3-yl)amino)imidazo[1,2-a]pyridin-3-yl)-4-pyrimidinyl)-acetamid |
| 2-(3-Methoxyphenyl)-N-(2-methyl-6-(2-((1-methyl-1H-pyrazol-3-yl)amino)imidazo[1,2-a]-pyridin-3-yl)-4-pyrimidinyl)acetamid |
| 2-(3-Fluorphenyl)-N-(2-methyl-6-(2-((1-methyl-1H-pyrazol-3-yl)amino)imidazo[1,2-a]-pyridin-3-yl)-4-pyrimidinyl)-acetamid |
| 2-(2,4-Difluorphenyl)-N-(2-methyl-6-(2-((1-methyl-1H-pyrazol-3-yl)amino)imidazo[1,2-b]pyridazin-3-yl)-4-pyrimidinyl)acetamid |
| 3-Cyano-N-(2-methyl-6-(2-((1-methyl-1H-pyrazol-3-yl)amino)imidazo[1,2-b]pyridazin-3-yl)-4-pyrimidinyl)-benzamid |
| 3,3,3-Trifluor-N-(1-methyl-1H-pyrazol-3-yl)-N-(3-(2-methyl-6-((3,3,3-trifluorpropanoyl)-amino)-4-pyrimidinyl)imidazo[1,2-a]pyridin-2-yl)propanamid |
| 3-(6-Amino-2-methyl-4-pyrimidinyl)-N-(1-methyl-1H-pyrazol-3-yl)-6-(2-methyl-4-pyridinyl)-imidazo[1,2-b]pyridazin-2-amin |
| 3-(6-Amino-2-methyl-4-pyrimidinyl)-N-(1-methyl-1H-pyrazol-3-yl)-6-(2-methyl-3-pyridinyl)-imidazo[1,2-b]pyridazin-2-amin |
| 3,3,3-Trifluor-N-(2-methyl-6-(2-((1-methyl-1H-pyrazol-3-yl)amino)imidazo[1,2-b]pyridazin-3-yl)-4-pyrimidinyl)-propanamid |
| 3,3,3-Trifluor-N-(2-methyl-6-(2-((1-methyl-1H-pyrazol-3-yl)amino)imidazo[1,2-a]pyridin-3-yl)-4-pyrimidinyl)-propanamid |
| 3-(6-Amino-2-methyl-4-pyrimidinyl)-N-(1-methyl-1H-pyrazol-3-yl)-6-(5-pyrimidinyl)imidazo-[1,2-b]pyridazin-2-amin |
| 3-(6-((6-(2-Methoxyethoxy)-3-pyridazinyl)amino)-2-methyl-4-pyrimidinyl)-N-1H-pyrazol-3-ylimidazo[1,2-b]pyridazin-2-amin |
| 2-(4-(6-((2-Methyl-6-(2-(1H-pyrazol-3-ylamino)imidazo[1,2-b]pyridazin-3-yl)-4-pyrimidinyl)amino)-3-pyridazinyl)-2-morpholinyl)ethanol |
| 3-(6-Amino-2-methyl-4-pyrimidinyl)-N-(4-(4-methyl-1-piperazinyl)phenyl)imidazo[1,2-b]pyridazin-2-amin |
| 3-(2-Methyl-6-((6-(2-methyl-4-morpholinyl)-3-pyridazinyl)-amino)-4-pyrimidiny1)-N-1H-pyrazol-3-ylimidazo[1,2-b]-pyridazin-2-amin |
| 3-(2-Methyl-6-((6-(3-methyl-4-morpholinyl)-3-pyridazinyl)-amino)-4-pyrimidiny1)-N-1H-pyrazol-3-ylimidazo[1,2-b]-pyridazin-2-amin |
| N-(3-(6-Amino-2-methyl-4-pyrimidinyl)imidazo[1,2-b]pyridazin-2-yl)-1H-pyrazolo[3,4-c]pyridin-3-amin |
| 3-(6-Amino-2-methyl-4-pyrimidinyl)-N-(4-fluor-1H-indazol-3-yl)imidazo[1,2-b]pyridazin-2-amin |
| N-(3-(6-Amino-2-methyl-4-pyrimidinyl)imidazo[1,2-b]pyridazin-2-yl)-1H-pyrazolo[4,3-c]pyridin-3-amin |
| N-(2-Methyl-6-(2-(1H-pyrazol-3-ylamino)imidazo[1,2-a]pyridin-3-yl)-4-pyrimidinyl)acetamid |
| 3-(6-Amino-2-methyl-4-pyrimidinyl)-N-(1-(2-(dimethyl-amino)ethyl)-1H-pyrazol-3-yl)imidazo-[1,2-b]pyridazin-2-amin |
| N-(6-(2-((1-(2-(Dimethylamino)ethyl)-1H-pyrazol-3-yl)amino)imidazo-[1,2-b]pyridazin-3-yl)-2-methyl-4-pyrimidinyl)acetamid |
| N-(2-Methyl-6-(2-((1-methyl-1H-pyrazol-3-yl)amino)-imidazo[1,2-a]pyridin-3-yl)-4-pyrimidinyl)acetamid |
| 3-(2-Methyl-6-((6-(4-morpholinyl)-3-pyridazinyl)amino)-4-pyrimidinyl)-N-(1-methyl-1H-pyrazol-3-yl)imidazo[1,2-b]pyridazin-2-amin |
| N-(3-(6-Amino-2-methyl-4-pyrimidinyl)imidazo[1,2-b]-pyridazin-2-yl)-1H-pyrazolo[3,4-b]pyridin-3-amin |
| 3-(6-Amino-2-methyl-4-pyrimidinyl)-N-(3-(4-methyl-1-piperazinyl)-phenyl)imidazo[1,2-b]pyridazin-2-amin |
| N-(3-((3-(6-(Acetylamino)-2-methyl-4-pyrimidinyl)-imidazo[1,2-b]pyridazin-2-yl)amino)-phenyl)acetamid |
| 3-(6-Amino-2-methyl-4-pyrimidinyl)-6-(1-(2-methoxyethyl)-1H-pyrazol-4-yl)-N-(1-methyl-1H-pyrazol-3-yl)imidazo[1,2-a]pyridin-2-amin |
| 1-(4-((3-(4-Amino-6-methyl-1,3,5-triazin-2-yl)imidazo[1,2-a]pyridin-2-yl)amino)phenyl)-3-methylharnstoff |
| 3-(4-Amino-6-methyl-1,3,5-triazin-2-yl)-N-1H-pyrazol-3-yl-6-(3-pyridinyl)imidazo[1,2-a]pyridin-2-amin |
| 3-(4-Amino-6-methyl-1,3,5-triazin-2-yl)-N-6--phenyl-N-2-1H-pyrazol-3-ylimidazo[1,2-a]pyridin-2,6-diamin |
| 3-(4-Amino-6-methyl-1,3,5-triazin-2-yl)-N-6--(4-methoxyphenyl)-N-2--1H-pyrazol-3-ylimidazo[1,2-a]pyridin-2,6-diamin |
| 3-(4-Amino-6-methyl-1,3,5-triazin-2-yl)-7-methoxy-N-1H-pyrazol-3-ylimidazo[1,2-a]pyridin-2-amin |
| 3-(4-Amino-6-methyl-1,3,5-triazin-2-yl)-N-1H-pyrazol-3-yl-6-(5-pyrimidinyl)imidazo[1,2-a]pyridin-2-amin |
| 3-(4-Methyl-6-(methylamino)-1,3,5-triazin-2-yl)-N-1H-pyrazol-3-yl-6-(5-pyrimidinyl)-imidazo[1,2-a]pyridin-2-amin |
| 3-(4-Amino-6-methyl-1,3,5-triazin-2-yl)-6-(3-fluorphenyl)-N-1H-pyrazol-3-ylimidazo[1,2-a]pyridin-2-amin |
| 4-(3-(4-Amino-6-methyl-1,3,5-triazin-2-yl)-2-(1H-pyrazol-3-ylamino)imidazo[1,2-a]pyridin-6-yl)-N-methylbenzamid |
| 3-(4-Amino-6-methyl-1,3,5-triazin-2-yl)-N-2--1H-pyrazol-3-yl-N-6--3-pyridinylimidazo[1,2-a]pyridin-2,6-diamin |
| N-(4-(3-(4-Amino-6-methyl-1,3,5-triazin-2-yl)-2-(1H-pyra-zol-3-ylamino)imidazo[1,2-a]pyridin-6-yl)phenyl)acetamid |
| 3-(4-Amino-6-methyl-1,3,5-triazin-2-yl)-N-6--(3-methoxy-phenyl)-N-2--1H-pyrazol-3-ylimidazo[1,2-a]pyridin-2,6-diamin |
| 3-(4-Amino-6-methyl-1,3,5-triazin-2-yl)-N-2--1H-pyrazol-3-yl-N-6--4-pyridinylimidazo[1,2-a]pyridin-2,6-diamin |
| 3-(4-Amino-6-methyl-1,3,5-triazin-2-yl)-N-2--1H-pyrazol-3-yl-N-6--5-pyrimidinylimidazo-[1,2-a]pyridin-2,6-diamin |
| N-(4-((3-(4-Amino-6-methyl-1,3,5-triazin-2-yl)-2-(1H-pyrazol-3-ylamino)imidazo[1,2-a]pyridin-6-yl)amino)-phenyl)acetamid |
| 3-(4-Amino-6-methyl-1,3,5-triazin-2-yl)-6-(2-methyl-3-pyridinyl)-N-1H-pyrazol-3-ylimidazo[1,2-a]pyridin-2-amin |
| 3-(4-Amino-6-methyl-1,3,5-triazin-2-yl)-N-6--(2-methyl-4-pyridinyl)-N-2--1H-pyrazol-3-ylimidazo[1,2-a]pyridin-2,6-diamin |
| N-Methyl-4-(3-(4-methyl-6-(methylamino)-1,3,5-triazin-2-yl)-2-(1H-pyrazol-3-ylamino)-imidazo[1,2-a]pyridin-6-yl)benzamid |
| 3-(4-Amino-6-methyl-1,3,5-triazin-2-yl)-6-(4-methyl-3-pyridinyl)-N-1H-pyrazol-3-ylimidazo-[1,2-a]pyridin-2-amin |
| 3-(4-Amino-6-methyl-1,3,5-triazin-2-yl)-6-(2,6-dimethyl-4-pyridinyl)-N-1H-pyrazol-3-yl-imidazo[1,2-a]pyridin-2-amin |
| 4-(3-(4-Amino-6-methyl-1,3,5-triazin-2-yl)-2-(1H-pyrazol-3-ylamino)imidazo[1,2-a]pyridin-6-yl)-N-(1-methylethyl)-benzamid |
| 6-(3-(4-Amino-6-methyl-1,3,5-triazin-2-yl)-2-(1H-pyrazol-3-ylamino)imidazo[1,2-a]pyridin-6-yl)-3,4-dihydro-1(2H)-isochinolinon |
| 4-(3-(4-Amino-6-methyl-1,3,5-triazin-2-yl)-2-(1H-pyrazol-3-ylamino)imidazo[1,2-a]pyridin-6-yl)-N-ethylbenzamid |
| 4-(3-(4-Amino-6-methyl-1,3,5-triazin-2-yl)-2-(1H-pyrazol-3-ylamino)imidazo[1,2-a]pyridin-6-yl)-3-methylbenzoesäure |
| 3-(2-Methyl-6-(methylamino)-4-pyrimidinyl)-N-1H-pyrazol-3-yl-6-(5-pyrimidinyl)imidazo-[1,2-b]pyridazin-2-amin |
| 3-(4-Amino-6-methyl-1,3,5-triazin-2-yl)-6-(4-methyl-5-pyrimidinyl)-N-1H-pyrazol-3-ylimidazo[1,2-a]pyridin-2-amin |
| 4-(3-(4-Amino-6-methyl-1,3,5-triazin-2-yl)-2-(1H-pyrazol-3-ylamino)imidazo[1,2-a]pyridin-6-yl)-N,3-dimethylbenzamid |
| 5-(3-(4-Amino-6-methyl-1,3,5-triazin-2-yl)-2-(1H-pyrazol-3-ylamino)imidazo[1,2-a]pyridin-6-yl)-N-methyl-2-pyridincarboxamid |
| 3-(4-Amino-6-methyl-1,3,5-triazin-2-yl)-2-(1H-pyrazol-3-ylamino)imidazo[1,2-a]pyridin-7-ol |
| tert-butyl-4-(3-(4-Amino-6-methyl-1,3,5-triazin-2-yl)-2-(1H-pyrazol-3-ylamino)imidazo[1,2-a]pyridin-7-yl)-3,6-dihydro-1(2H)-pyridincarboxylat |
| 3-(4-Amino-6-methyl-1,3,5-triazin-2-yl)-N-1H-pyrazol-3-yl-7-(1,2,3,6-tetrahydro-4-pyridinyl)-imidazo[1,2-a]pyridin-2-amin |
| 3-(4-Amino-6-methyl-1,3,5-triazin-2-yl)-7-(2-methoxy-ethoxy)-N-1H-pyrazol-3-ylimidazo[1,2-a]pyridin-2-amin |
| 3-(4-Amino-6-methyl-1,3,5-triazin-2-yl)-N-1H-pyrazol-3-yl-6-(1H-pyrrolo[2,3-b]pyridin-5-yl)imidazo[1,2-a]pyridin-2-amin |
| 4-(3-(4-Amino-6-methyl-1,3,5-triazin-2-yl)-2-(1H-pyrazol-3-ylamino)-imidazo[1,2-a]pyridin-7-yl)-N-methylbenzamid |
| 3-(4-Amino-6-methyl-1,3,5-triazin-2-yl)-N-1H-pyrazol-3-yl-7-(2-(1-pyrrolidinyl)ethoxy)-imidazo[1,2-a]pyridin-2-amin |
| 4-(3-(6-Amino-2-methyl-4-pyrimidinyl)-2-(1H-pyrazol-3-ylamino)-imidazo[1,2-b]pyridazin-6-yl)-N,N-dimethyl-1-piperazin-carboxamid |
| 3-(6-Amino-2-methyl-4-pyrimidinyl)-N-2-pyridinylimidazo-[1,2-b]pyridazin-2-amin |
| 3-(6-Amino-2-methyl-4-pyrimidinyl)-N-(1-ethyl-1H-pyrazol-3-yl)imidazo[1,2-b]pyridazin-2-amin |
| N-(3-(6-Amino-2-methyl-4-pyrimidinyl)imidazo[1,2-b]-pyridazin-2-yl)-3-chinolinamin |
| N-(3-(6-Amino-2-methyl-4-pyrimidinyl)imidazo[1,2-b]-pyridazin-2-yl)-3-isochinolinamin |
| N-(3-(6-Amino-2-methyl-4-pyrimidinyl)imidazo[1,2-b]-pyridazin-2-yl)-2-chinolinamin |
| 3-(6-Amino-2-methyl-4-pyrimidinyl)-N-(1-methyl-1H-pyrazol-3-yl)imidazo[1,2-b]pyridazin-2-amin |
| 3-((3-(4-Amino-6-methyl-1,3,5-triazin-2-yl)imidazo[1,2-a]-pyridin-2-yl)amino)-N-methyl-1H-pyrazol-1-carboxamid |
| 5-((3-(4-Amino-6-methyl-1,3,5-triazin-2-yl)imidazo[1,2-a]-pyridin-2-yl)amino)-N-methyl-1H-pyrazol-1-carboxamid |
| 3-((3-(4-Amino-6-methyl-1,3,5-triazin-2-yl)imidazo[1,2-a]-pyridin-2-yl)amino)-N-(2-hydroxyethyl)-1H-pyrazol-1-carboxamid |
| 5-((3-(4-Amino-6-methyl-1,3,5-triazin-2-yl)imidazo[1,2-a]-pyridin-2-yl)amino)-N-(2-hydroxyethyl)-1H-pyrazol-1-carboxamid |
| 3-(4-Amino-6-methyl-1,3,5-triazin-2-yl)-N-(1-(4-morpholinylcarbonyl)-1H-pyrazo1-3-yl)-imidazo[1,2-a]-pyridin-2-amin |
| 3-(4-Amino-6-methyl-1,3,5-triazin-2-yl)-N-(1-(4-morpholinylcarbonyl)-1H-pyrazol-5-yl)-imidazo[1,2-a]-pyridin-2-amin |
| 5-((3-(4-Amino-6-methyl-1,3,5-triazin-2-yl)imidazo[1,2-a]-pyridin-2-yl)amino)-N-3-pyridinyl-1H-pyrazol-1-carboxamid |
| 4-((3-(4-Amino-6-methyl-1,3,5-triazin-2-yl)imidazo[1,2-a]-pyridin-2-yl)amino)-N-methyl-1H-pyrazol-1-carboxamid |
| 3-((3-(4-Amino-6-methyl-1,3,5-triazin-2-yl)imidazo[1,2-a]-pyridin-2-yl)amino)-N-phenyl-1H-pyrazol-1-carboxamid |
| 5-((3-(4-Amino-6-methyl-1,3,5-triazin-2-yl)imidazo[1,2-a]-pyridin-2-yl)amino)-N-phenyl-1H-pyrazol-1-carboxamid |
| 3-(4-Amino-6-methyl-1,3,5-triazin-2-yl)-N-1H-pyrazol-4-ylimidazo[1,2-a]pyridin-2-amin |
| Methyl-3-((3-(4-amino-6-methyl-1,3,5-triazin-2-yl)-imidazo[1,2-a]pyridin-2-yl)amino)-1H-pyrazol-1-carboxylat |
| Methyl-5-((3-(4-amino-6-methyl-1,3,5-triazin-2-yl)-imidazo[1,2-a]pyridin-2-yl)amino)-1H-pyrazol-1-carboxylat |
| Phenyl-4-((3-(4-amino-6-methyl-1,3,5-triazin-2-yl)-imidazo[1,2-a]pyridin-2-yl)amino)-1H-pyrazol-1-carboxylat |
| 3-((3-(6-Amino-2-methyl-4-pyrimidinyl)imidazo[1,2-a]-pyridin-2-yl)amino)-N-phenyl-1H-pyrazol-1-carboxamid |
| 5-((3-(6-Amino-2-methyl-4-pyrimidinyl)imidazo[1,2-a]-pyridin-2-yl)amino)-N-phenyl-1H-pyrazol-1-carboxamid |
| 3-((3-(6-Amino-2-methyl-4-pyrimidinyl)imidazo[1,2-a]-pyridin-2-yl)amino)-N-methyl-1H-pyrazol-1-carboxamid |
| 5-((3-(6-Amino-2-methyl-4-pyrimidinyl)imidazo[1,2-a]-pyridin-2-yl)amino)-N-methyl-1H-pyrazol-1-carboxamid |
| 4-((3-(6-Amino-2-methyl-4-pyrimidinyl)imidazo[1,2-a]-pyridin-2-yl)amino)-N-phenyl-1H-pyrazol-1-carboxamid |
| 4-((3-(6-Amino-2-methyl-4-pyrimidinyl)imidazo[1,2-a]-pyridin-2-yl)amino)-N-methyl-1H-pyrazol-1-carboxamid |
| 3-(6-Amino-2-methyl-4-pyrimidinyl)-N-(1-(2-pyridinyl)-1H-pyrazol-3-yl)imidazo[1,2-a]pyridin-2-amin |
| 3-(6-Amino-2-methyl-4-pyrimidinyl)-N-(1-(methylsulfonyl)-1H-pyrazol-3-yl)imidazo[1,2-a]pyridin-2-amin |
| 3-(6-Amino-2-methyl-4-pyrimidinyl)-N-(1-(1,3-benzoxazol-2-yl)-1H-pyrazol-5-yl)imidazo[1,2-a]pyridin-2-amin |
| Phenyl-3-((3-(6-amino-2-methyl-4-pyrimidinyl)imidazo-[1,2-a]pyridin-2-yl)amino)-1H-pyrazol-1-carboxylat |
| 3-(6-Amino-2-methyl-4-pyrimidinyl)-N-(1-(1H-benzimidazol-2-yl)-1H-pyrazol-3-yl)imidazo-[1,2-a]pyridin-2-amin |
| 3-((3-(6-Amino-2-methyl-4-pyrimidinyl)imidazo[1,2-a]-pyridin-2-yl)amino)-N-(1-methylethyl)-1H-pyrazol-1-carboxamid |
| 3-((3-(6-Amino-2-methyl-4-pyrimidinyl)imidazo[1,2-a]-pyridin-2-yl)amino)-N-benzyl-1H-pyrazol-1-carboxamid |
| Methyl-4-(((3-((3-(6-amino-2-methyl-4-pyrimidinyl)imidazo-[1,2-a]pyridin-2-yl)amino)-1H-pyrazol-1-yl)carbonyl)-amino)benzoat |
| 3-((3-(6-Amino-2-methyl-4-pyrimidinyl)imidazo[1,2-a]-pyridin-2-yl)amino)-N-ethyl-1H-pyrazol-1-carboxamid |
| 3-((3-(6-Amino-2-methyl-4-pyrimidinyl)imidazo[1,2-a]-pyridin-2-yl)amino)-N-(2-methoxyphenyl)-1H-pyrazol-1-carboxamid |
| 3-(6-Amino-2-methyl-4-pyrimidinyl)-N-1H-pyrazol-4-ylimidazo[1,2-a]pyridin-2-amin |
| 3-((3-(6-Amino-2-methyl-4-pyrimidinyl)imidazo[1,2-a]-pyridin-2-yl)amino)-N-(2-methyl-phenyl)-1H-pyrazol-1-carboxamid |
| 3-((3-(6-Amino-2-methyl-4-pyrimidinyl)imidazo[1,2-a]-pyridin-2-yl)amino)-N-(4-methoxy-phenyl)-1H-pyrazol-1-carboxamid |
| 3-((3-(6-Amino-2-methyl-4-pyrimidinyl)imidazo[1,2-b]-pyridazin-2-yl)amino)-N-phenyl-1H-pyrazol-1-carboxamid trifluoressigsäure |
| 3-((3-(6-Amino-2-methyl-4-pyrimidinyl)imidazo[1,2-b]-pyridazin-2-yl)amino)-N-methyl-1H-pyrazol-1-carboxamid |
| 5-((3-(6-Amino-2-methyl-4-pyrimidinyl)imidazo[1,2-b]-pyridazin-2-yl)amino)-N-methyl-1H-pyrazol-1-carboxamid |
| Methyl-4-((3-(6-amino-2-methyl-4-pyrimidinyl)imidazo[1,2-a]pyridin-2-yl)amino)-1H-pyrazol-1-carboxylat |
| 3-(6-Amino-2-methyl-4-pyrimidinyl)-N-(1-(1,3-benzoxazol-2-yl)-1H-pyrazol-3-yl)imidazo[1,2-b]pyridazin-2-amin |
| Methyl-5-((3-(6-amino-2-methyl-4-pyrimidinyl)imidazo-[1,2-b]pyridazin-2-yl)amino)-1H-pyrazol-1-carboxylat |
| 3-((3-(6-Amino-2-methyl-4-pyrimidinyl)imidazo[1,2-b]-pyridazin-2-yl)amino)-N-(2-methylphenyl)-1H-pyrazol-1-carboxamid |
| 3-((3-(6-Amino-2-methyl-4-pyrimidinyl)imidazo[1,2-b]-pyridazin-2-yl)amino)-N-(2-fluorphenyl)-1H-pyrazol-1-carboxamid |
| 3-(6-Amino-2-methyl-4-pyrimidinyl)-N-(1-(1,3-benzoxazol-2-yl)-1H-pyrazol-5-yl)imidazo[1,2-b]pyridazin-2-amin |
| 3-((3-(6-Amino-2-methyl-4-pyrimidinyl)imidazo[1,2-b]-pyridazin-2-yl)amino)-N-(4-cyanophenyl)-1H-pyrazol-1-carboxamid |
| 3-((3-(6-Amino-2-methyl-4-pyrimidinyl)imidazo[1,2-b]-pyridazin-2-yl)amino)-N-(2-(trifluormethyl)phenyl)-1H-pyrazol-1-carboxamid |
| 3-((3-(6-Amino-2-methyl-4-pyrimidinyl)imidazo[1,2-b]-pyridazin-2-yl)amino)-N-(3-methylphenyl)-1H-pyrazol-1-carboxamid |
| 3-((3-(6-Amino-2-methyl-4-pyrimidinyl)imidazo[1,2-b]-pyridazin-2-yl)amino)-N-(2,5-difluorphenyl)-1H-pyrazol-1-carboxamid |
| 3-((3-(6-Amino-2-methyl-4-pyrimidinyl)imidazo[1,2-b]-pyridazin-2-yl)amino)-N-(3,5-difluorphenyl)-1H-pyrazol-1-carboxamid |
| 3-((3-(6-Amino-2-methyl-4-pyrimidinyl)imidazo[1,2-b]-pyridazin-2-yl)amino)-N-(1-methylethyl)-1H-pyrazol-1-carboxamid |
| 3-((3-(6-Amino-2-methyl-4-pyrimidinyl)imidazo[1,2-b]-pyridazin-2-yl)amino)-N-(4-methylphenyl)-1H-pyrazol-1-carboxamid |
| 3-((3-(6-Amino-2-methyl-4-pyrimidinyl)imidazo[1,2-b]-pyridazin-2-yl)amino)-N-(3,5-dichlorphenyl)-1H-pyrazol-1-carboxamid |
| 3-((3-(6-Amino-2-methyl-4-pyrimidinyl)imidazo[1,2-b]-pyridazin-2-yl)amino)-N-(2-chlor-5-(trifluormethyl)phenyl)-1H-pyrazol-1-carboxamid |
| 3-((3-(6-Amino-2-methyl-4-pyrimidinyl)imidazo[1,2-b]-pyridazin-2-yl)amino)-N-(2,5-dichlorphenyl)-1H-pyrazol-1-carboxamid |
| 3-((3-(6-Amino-2-methyl-4-pyrimidinyl)imidazo[1,2-b]-pyridazin-2-yl)amino)-N-(2,3-dichlorphenyl)-1H-pyrazol-1-carboxamid |
| 3-((3-(6-Amino-2-methyl-4-pyrimidinyl)imidazo[1,2-b]-pyridazin-2-yl)amino)-N-(2-chlor-4-(trifluormethyl)-phenyl)-1H-pyrazol-1-carboxamid |
| 3-((3-(6-(Acetylamino)-2-methyl-4-pyrimidinyl)imidazo-[1,2-b]pyridazin-2-yl)amino)-N-(2-methylphenyl)-1H-pyrazol-1-carboxamid |
| 3-(6-Amino-2-methyl-4-pyrimidinyl)-N-(1-(1H-benzimidazol-2-yl)-1H-pyrazol-3-yl)imidaz-[1,2-b]pyridazin-2-amin |
| 3-(6-Amino-2-methyl-4-pyrimidinyl)-N-(1-(1H-imidazol-2-yl)-1H-pyrazol-3-yl)imidazo[1,2-b]pyridazin-2-amin |
| 3-(6-Amino-2-methyl-4-pyrimidinyl)-N-(2-(methylsulfonyl)-1H-pyrazol-3-yl)imidazo[1,2-b]pyridazin-2-amin |
| 3-((3-(6-Amino-2-methyl-4-pyrimidinyl)imidazo[1,2-b]-pyridazin-2-yl)amino)-1H-pyrazol-1-carboxamid |
| 5-((3-(6-Amino-2-methyl-4-pyrimidinyl)imidazo[1,2-b]-pyridazin-2-yl)amino)-1H-pyrazol-1-carboxamid |
| 3-(6-Amino-2-methyl-4-pyrimidinyl)-N-(1-(1-azetidinyl-carbonyl)-1H-pyrazol-3-yl)imidazo[1,2-b]pyridazin-2-amin |
| 3-(6-Amino-2-methyl-4-pyrimidinyl)-N-(1-(1-azetidinyl-carbonyl)-1H-pyrazol-5-yl)imidazo[1,2-b]pyridazin-2-amin |
| 3-((3-(6-Amino-2-methyl-4-pyrimidinyl)imidazo[1,2-b]-pyridazin-2-yl)amino)-N-(2-(methyl-amino)-ethyl)-1H-pyrazol-1-carboxamid |
| 5-((3-(6-Amino-2-methyl-4-pyrimidinyl)imidazo[1,2-b]-pyridazin-2-yl)amino)-N-(2-(methylamino)ethyl)-1H-pyrazol-1-carboxamid |
| 3-((3-(6-Amino-2-methyl-4-pyrimidinyl)imidazo[1,2-b]-pyridazin-2-yl)amino)-N,N-dimethyl-1H-pyrazol-1-carboxamid |
| 5-((3-(6-Amino-2-methyl-4-pyrimidinyl)imidazo[1,2-b]-pyridazin-2-yl)amino)-N,N-dimethyl-1H-pyrazol-1-carboxamid |
| 3-(6-Ainino-2-methyl-4-pyrimidinyl)-N-(1-(1-piperazinyl-carbonyl)-1H-pyrazol-3-yl)imidazo-[1,2-b]pyridazin-2-amin |
| 5-((3-(6-Amino-2-methyl-4-pyrimidinyl)imidazo[1,2-b]-pyridazin-2-yl)amino)-N-(2-hydroxyethyl)-1H-pyrazol-1-carboxamid |
| 3-((3-(6-Amino-2-methyl-4-pyrimidinyl)imidazo[1,2-b]-pyridazin-2-yl)amino)-N-(2-methoxy-ethyl)-1H-pyrazol-1-carboxamid |
| 5-((3-(6-Amino-2-methyl-4-pyrimidinyl)imidazo[1,2-b]-pyridazin-2-yl)amino)-N-(2-methoxy-ethyl)-1H-pyrazol-1-carboxamid |
| 5-((3-(6-Amino-2-methyl-4-pyrimidinyl)imidazo[1,2-b]-pyridazin-2-yl)amino)-N-(2-methyl-propyl)-1H-pyrazol-1-carboxamid |
| 3-(6-Amino-2-methyl-4-pyrimidinyl)-N-1H-pyrazol-3-yl-6-(5-pyrimidinyl)imidazo[1,2-b]-pyridazin-2-amin |
| 3-(6-Amino-2-methyl-4-pyrimidinyl)-N-(1-(1-phenylethyl)-1H-pyrazol-3-yl)imidazo[1,2-b]-pyridazin-2-amin |
| 3-(6-Amino-2-methyl-4-pyrimidinyl)-N-(1-benzyl-1H-pyrazol-3-yl)imidazo[1,2-b]pyridazin-2-amin |
| 3-(6-Amino-2-methyl-4-pyrimidinyl)-N-(1-(3-pyridinyl-methyl)-1H-pyrazol-3-yl)imidazo[1,2-b]pyridazin-2-amin |
| 7-((3-(6-Amino-2-methyl-4-pyrimidinyl)imidazo[1,2-a]-pyridin-2-yl)amino)-2H-1,4-benzoxazin-3(4H)-one |
| 1-(5-((3-(6-Amino-2-methyl-4-pyrimidinyl)imidazo[1,2-b]-pyridazin-2-yl)amino)-2-pyridinyl)-3-azetidinol |
| 2-(1-(5-((3-(6-Amino-2-methyl-4-pyrimidinyl)imidazo-[1,2-b]pyridazin-2-yl)amino)-2-pyridinyl)-3-azetidinyl)-2-propanol |
| N-(3-(4-Amino-6-methyl-1,3,5-triazin-2-yl)imidazo[1,2-a]-pyridin-2-yl)-7-chinolinamin |
| N-(3-(4-Amino-6-methyl-1,3,5-triazin-2-yl)imidazo[1,2-a]-pyridin-2-yl)-6-chinolinamin |
| N-(3-(4-Amino-6-methyl-1,3,5-triazin-2-yl)imidazo[1,2-a]pyridin-2-yl)-7-isochinolinamin |
| 4-(3-(4-Amino-6-methyl-1,3,5-triazin-2-yl)-2-(7-chinolinylamino)-imidazo[1,2-a]pyridin-6-yl)-N,N-dimethyl-1-piperazin-carboxamid |
| N-(3-(4-Amino-6-methyl-1,3,5-triazin-2-yl)-6-(4-(methyl-sulfonyl)-1-piperazinyl)imidazo[1,2-a]pyridin-2-yl)-7-chinolinamin |
| N-(3-(4-Amino-6-methyl-1,3,5-triazin-2-yl)-6-(1-piperazinyl)-imidazo[1,2-a]pyridin-2-yl)-7-chinolinamin |
| 4-(3-(4-(Acetylamino)-6-methyl-1,3,5-triazin-2-yl)-2-((6-methoxy-3-pyridinyl)amino)-imidazo[1,2-a]pyridin-6-yl)-N,N-dimethyl-1-piperazin-carboxamid |
| 3-(4-Amino-6-methyl-1,3,5-triazin-2-yl)-N-(6-ethoxy-3-pyridinyl)-imidazo[1,2-a]pyridin-2-amin |
| N-(3-(4-Amino-6-methyl-1,3,5-triazin-2-yl)imidazo[1,2-a]-pyridin-2-yl)-1H-indazol-3-amin |
| 4-(3-(4-Amino-6-methyl-1,3,5-triazin-2-yl)-2-((5-fluor-6-methoxy-3-pyridinyl)amino)imidazo[1,2-a]pyridin-6-yl)-N,N-dimethyl-1-piperazincarboxamid |
| 4-(3-(4-Amino-6-methyl-1,3,5-triazin-2-yl)-2-((6-methoxy-3-pyridinyl)amino)imidazo[1,2-a]pyridin-6-yl)-N-methyl-1-piperazincarboxamid |
| 6-(4-Acetyl-1-piperazinyl)-3-(4-amino-6-methyl-1,3,5-triazin-2-yl)-N-(6-methoxy-3-pyridinyl)imidazo[1,2-a]-pyridin-2-amin |
| (5-(3-(4-Amino-6-methyl-1,3,5-triazin-2-yl)-2-(1H-pyrazol-5-ylamino)imidazo[1,2-a]pyridin-6-yl)-2-methoxyphenyl)-methanol |
| 3-(4-Amino-6-methyl-1,3,5-triazin-2-yl)-N-1H-pyrazol-5-yl-6-(4-pyridinyl)imidazo[1,2-a]pyridin-2-amin |
| 3-(4-Amino-6-methyl-1,3,5-triazin-2-yl)-6-(3,6-dihydro-2H-pyran-4-yl)-N-1H-pyrazol-5-ylimidazo[1,2-a]pyridin-2-amin |
| 3-(4-Amino-6-methyl-1,3,5-triazin-2-yl)-6-(2-methyl-4-pyridinyl)-N-1H-pyrazol-5-ylimidazo[1,2-a]pyridin-2-amin |
| 3-(4-Amino-6-methyl-1,3,5-triazin-2-yl)-6-(1-methyl-1H-imidazol-5-yl)-N-1H-pyrazol-5-ylimidazo[1,2-a]pyridin-2-amin |
| 4-(3-(4-Amino-6-methyl-1,3,5-triazin-2-yl)-2-(1H-pyrazol-5-ylamino)-midazo[1,2-a]pyridin-6-yl)-1,2-butanediol |
| 3-(4-Amino-6-methyl-1,3,5-triazin-2-yl)-6-(1-(1-methyl-ethyl)-1H-pyrazol-4-yl)-N-1H-pyrazol-5-ylimidazo[1,2-a]-pyridin-2-amin |
| 3-(4-Amino-6-methyl-1,3,5-triazin-2-yl)-2-(1H-pyrazol-5-ylamino)-imidazo[1,2-a]pyridin-6-carboxamid |
| N-((3-(4-Amino-6-methyl-1,3,5-triazin-2-yl)-2-(1H-pyrazol-5-ylamino)imidazo[1,2-a]pyridin-6-yl)methyl)acetamid |
| 3-(2-methyl-6-((2,2,2-trifluorethyl)amino)-4-pyrimidinyl)-N-1H-pyrazol-5-ylimidazo[1,2-b]pyridazin-2-amin |
| 3-(2-Methyl-6-(2-pyrazinylamino)-4-pyrimidinyl)-N-(1-methyl-1H-pyrazol-3-yl)imidazo[1,2-b]pyridazin-2-amin |
| 3-(2-Methyl-6-(3-pyridinylamino)-4-pyrimidinyl)-N-1H-pyrazol-3-ylimidazo[1,2-b]pyridazin-2-amin |
| 2-Cyano-N-(2-methyl-6-(2-((1-methyl-1H-pyrazol-3-yl)-amino)-imidazo[1,2-b]pyridazin-3-yl)-4-pyrimidinyl)-acetamid |
| N-(2-Methyl-6-(2-((1-methyl-1H-pyrazol-3-yl)amino)-imidazo[1,2-b]pyridazin-3-yl)-4-pyrimidinyl)-1,3-oxazol-5-carboxamid |
| 3-(6-Amino-2-methyl-4-pyrimidinyl)-N-(1-methyl-1H-pyrazol-3-yl)-6-(5-pyrimidinyl)imidazo-[1,2-a]pyridin-2-amin |
| N-(4-(3-(6-Amino-2-methyl-4-pyrimidinyl)-2-((1-methyl-1H-pyrazol-3-yl)amino)imidazo[1,2-a]pyridin-6-yl)phenyl)-acetamid |
| 3-(4-Amino-6-methyl-1,3,5-triazin-2-yl)-N-(1-methyl-1H-pyrazol-3-yl)-6-(7-chinolinyl)-imidazo-[1,2-a]pyridin-2-amin |
| N-(4-(3-(6-Amino-2-methyl-4-pyrimidinyl)-2-((1-methyl-1H-pyrazol-3-yl)amino)imidazo[1,2-b]pyridazin-6-yl)benzyl)-acetamid |
| 3-(4-Amino-6-methyl-1,3,5-triazin-2-yl)-N-(1-methyl-1H-pyrazol-3-yl)-6-(5-pyrimidinyl)-imidazo[1,2-a]pyridin-2-amin |
| 4-(3-(6-Amino-2-methyl-4-pyrimidinyl)-2-((1-methyl-2H-pyrazol-3-yl)amino)imidazo[1,2-b]pyridazin-6-yl)-N-methylbenzamid |
| 3-(6-Amino-2-methyl-4-pyrimidinyl)-N-(1-(6-(2-methoxy-ethoxy)-4-pyrimidinyl)-1H-pyrazol-3-yl)imidazo[1,2-b]-pyridazin-2-amin |
| 3-(6-(3-Isoxazolylamino)-2-methyl-4-pyrimidinyl)-N-1H-pyrazol-3-ylimidazo[1,2-b]pyridazin-2-amin |
| 2-Cyano-N-(2-methyl-6-(2-((1-methyl-1H-pyrazol-3-yl)-amino)-6-(5-pyrimidinyl)imidazo[1,2-a]pyridin-3-yl)-4-pyrimidinyl)acetamid |
| 3-(6-((1-(2-Methoxyethyl)-1H-pyrazol-4-yl)amino)-2-methyl-4-pyrimidinyl)-N-1H-pyrazol-3-ylimidazo[1,2-b]pyridazin-2-amin |
| 3-(2-Methyl-6-((1-((3R)-tetrahydro-3-furanyl)-1H-pyrazol-4-yl)amino)-4-pyrimidinyl)-N-1H-pyrazol-3-ylimidazo-[1,2-b]pyridazin-2-amin, 3-(2-Methyl-6-((1-((3S)-tetrahydro-3-furanyl)-1H-pyrazol-4-yl)amino)-4-pyrimidinyl)-N-1H-pyrazol-3-ylimidazo[1,2-b]pyridazin-2-amin |
| 3-(6-(Imidazo[1,2-a]pyridin-2-ylamino)-2-methyl-4-pyrimidinyl)-N-1H-pyrazol-3-ylimidazo[1,2-b]pyridazin-2-amin |
| 3-(6-(Furo[3,2-b]pyridin-6-ylamino)-2-methyl-4-pyrimidinyl)-N-1H-pyrazol-3-ylimidazo[1,2-b]pyridazin-2-amin |
| 3-(6-Amino-2-methyl-4-pyrimidinyl)-N-1H-pyrazol-3-yl-6-(5-pyrimidinyl)imidazo[1,2-a]pyridin-2-amin |
| 3-(2-Methyl-6-((1-((3R)-tetrahydro-3-furanyl)-1H-pyrazol-4-yl)amino)-4-pyrimidinyl)-N-1H-pyrazol-3-ylimidazo-[1,2-a]pyridin-2-amin, 3-(2-Methyl-6-((1-((3S)-tetrahydro-3-furanyl)-1H-pyrazol-4-yl)amino)-4-pyrimidinyl)-N-1H-pyrazol-3-ylimidazo[1,2-a]pyridin-2-amin |
| 3-(6-((6-(2-Methoxyethoxy)-4-pyrimidinyl)amino)-2-methyl-4-pyrimidinyl)-N-1H-pyrazol-3-ylimidazo[1,2-b]pyridazin-2-amin |
| 1-(3-((3-(6-Amino-2-methyl-4-pyrimidinyl)imidazo[1,2-b]-pyridazin-2-yl)amino)phenyl)-3-cyclopropylharnstoff |
| N-(3-((3-(6-Amino-2-methyl-4-pyrimidinyl)imidazo[1,2-a]-pyridin-2-yl)amino)phenyl)-acetamid |
| N-(3-((3-(6-Amino-2-methyl-4-pyrimidinyl)imidazo[1,2-a]-pyridin-2-yl)amino)phenyl)-2-phenylacetamid |
| N-(3-((3-(6-Amino-2-methyl-4-pyrimidinyl)imidazo[1,2-a]-pyridin-2-yl)amino)phenyl)-benzamid |
| N-(3-((3-(6-Amino-2-methyl-4-pyrimidinyl)imidazo[1,2-a]-pyridin-2-yl)amino)phenyl)-4-chlorbenzolsulfonamid |
| N-(3-((3-(6-Amino-2-methyl-4-pyrimidinyl)imidazo[1,2-a]-pyridin-2-yl)amino)phenyl)-methansulfonamid |
| 1-(3-((3-(6-Amino-2-methyl-4-pyrimidinyl)imidazo[1,2-a]-pyridin-2-yl)amino)phenyl)-3-phenylharnstoff |
| 1-(3-((3-(6-Amino-2-methyl-4-pyrimidinyl)imidazo[1,2-a]-pyridin-2-yl)amino)phenyl)-3-(4-fluorphenyl)harnstoff |
| 1-(3-((3-(6-Amino-2-methyl-4-pyrimidinyl)imidazo[1,2-a]-pyridin-2-yl)amino)phenyl)-3-benzylharnstoff |
| 1-(3-((3-(6-Amino-2-methyl-4-pyrimidinyl)imidazo[1,2-a]-pyridin-2-yl)amino)phenyl)-3-methylharnstoff |
| 1-(3-((3-(6-Amino-2-methyl-4-pyrimidinyl)imidazo[1,2-a]-pyridin-2-yl)amino)phenyl)-3-ethylharnstoff |
| 4-((3-(6-Amino-2-methyl-4-pyrimidinyl)imidazo[1,2-a]-pyridin-2-yl)amino)-N-ethylbenzamid |
| 5-((3-(6-Amino-2-methyl-4-pyrimidinyl)imidazo[1,2-a]-pyridin-2-yl)amino)-N-ethyl-2-pyridincarboxamid |
| N-(3-((3-(6-Amino-2-methyl-4-pyrimidinyl)imidazo[1,2-b]-pyridazin-2-yl)amino)phenyl)-acetamid |
| N-(3-((3-(6-Amino-2-methyl-4-pyrimidinyl)imidazo[1,2-b]-pyridazin-2-yl)amino)phenyl)-propanamid |
| 3-((3-(6-Amino-2-methyl-4-pyrimidinyl)imidazo[1,2-a]-pyridin-2-yl)amino)-N-methyl-benzamid |
| 3-((3-(6-Amino-2-methyl-4-pyrimidinyl)imidazo[1,2-a]-pyridin-2-yl)amino)-N-ethylbenzamid |
| 3-((3-(6-Amino-2-methyl-4-pyrimidinyl)imidazo[1,2-a]-pyridin-2-yl)amino)-N-(1-methylethyl)-benzamid |
| 3-((3-(6-Amino-2-methyl-4-pyrimidinyl)imidazo[1,2-b]pyridazin-2-yl)amino)-N-methyl-benzamid |
| 3-((3-(6-Amino-2-methyl-4-pyrimidinyl)imidazo[1,2-b]-pyridazin-2-yl)amino)-N-ethyl-benzamid |
| 3-((3-(6-Amino-2-methyl-4-pyrimidinyl)imidazo[1,2-b]-pyridazin-2-yl)amino)-N-phenyl-benzamid |
| 3-((3-(6-Amino-2-methyl-4-pyrimidinyl)imidazo[1,2-b]-pyridazin-2-yl)amino)-N-(1-methyl-ethyl)benzamid |
| 5-((3-(6-Amino-2-methyl-4-pyrimidinyl)imidazo[1,2-a]-pyridin-2-yl)amino)-N-methyl-2-pyridincarboxamid |
| 5-((3-(6-Amino-2-methyl-4-pyrimidinyl)imidazo[1,2-a]-pyridin-2-yl)amino)-N-(1-methylethyl)-2-pyridincarboxamid |
| 5-((3-(6-Amino-2-methyl-4-pyrimidinyl)imidazo[1,2-a]-pyridin-2-yl)amino)-N-phenyl-2-pyridincarboxamid |
| 4-((3-(6-Amino-2-methyl-4-pyrimidinyl)imidazo[1,2-a]-pyridin-2-yl)amino)benzamid |
| 4-((3-(6-Amino-2-methyl-4-pyrimidinyl)imidazo[1,2-a]-pyridin-2-yl)amino)-N-(2-(diethylamino)-ethyl)benzamid |
| 4-((3-(6-Amino-2-methyl-4-pyrimidinyl)imidazo[1,2-a]-pyridin-2-yl)amino)-N-phenylbenzamid |
| 3-(4-Amino-6-methyl-1,3,5-triazin-2-yl)-6-(4-(methylsulfonyl)phenyl)-N-1H-pyrazol-3-ylimidazo[1,2-a]-pyridin-2-amin |
| 3-(6-Amino-2-methyl-4-pyrimidinyl)-N-(1-(3-methyl-butanoyl)-1H-pyrazol-3-yl)imidazo[1,2-b]pyridazin-2-amin |
| 1-Methylethyl 5-((3-(6-amino-2-methyl-4-pyrimidinyl)-imidazo[1,2-b]pyridazin-2-yl)amino)-1H-pyrazol-1-carboxylat |
| 1-Methylethyl 3-((3-(6-amino-2-methyl-4-pyrimidinyl)-imidazo[1,2-b]pyridazin-2-yl)amino)-1H-pyrazol-1-carboxylat |
| 3-(6-Amino-2-methyl-4-pyrimidinyl)-N-(2-methoxy-4-pyridinyl)imidazo[1,2-b]pyridazin-2-amin |
| 3-(6-Amino-2-methyl-4-pyrimidinyl)-N-(1-(2-methylpropanoyl)-1H-pyrazol-3-yl)imidazo[1,2-b]pyridazin-2-amin |
| 3-(6-Amino-2-methyl-4-pyrimidinyl)-N-(1-(2-methylpropanoyl)-1H-pyrazol-5-yl)imidazo[1,2-b]pyridazin-2-amin |
| 4-(3-(4-Amino-6-methyl-1,3,5-triazin-2-yl)-2-(1H-pyrazol-3-ylamino)imidazo[1,2-a]pyridin-6-yl)-N-methylbenzol-sulfonamid |
| 3-(4-Amino-6-methyl-1,3,5-triazin-2-yl)-6-(3-(methyl-sulfonyl)phenyl)-N-1H-pyrazol-3-ylimidazo[1,2-a]pyridin-2-amin |
| 3-(6-Amino-2-methyl-4-pyrimidinyl)-N-(1-(3,3-dimethyl-butanoyl)-1H-pyrazol-3-yl)-imidazo[1,2-b]pyridazin-2-amin |
| 3-(4-Amino-6-methyl-1,3,5-triazin-2-yl)-N-(1-methyl-1H-pyrazol-3-yl)-6-(4-(methylsulfonyl)-phenyl)imidazo[1,2-a]-pyridin-2-amin |
| Cyclohexyl-5-((3-(6-amino-2-methyl-4-pyrimidinyl)imidazo-[1,2-b]pyridazin-2-yl)amino)-1H-pyrazol-1-carboxylat |
| Cyclohexyl-3-((3-(6-amino-2-methyl-4-pyrimidinyl)imidazo-[1,2-b]pyridazin-2-yl)amino)-1H-pyrazol-1-carboxylat |
| 3-(6-Amino-2-methyl-4-pyrimidinyl)-N-(2-methoxy-3-pyridinyl)imidazo[1,2-b]pyridazin-2-amin |
| 3-((3-(6-Amino-2-methyl-4-pyrimidinyl)imidazo[1,2-b]-pyridazin-2-yl)amino)-2(1H)-pyridinon |
| 3-(6-Amino-2-methyl-4-pyrimidinyl)-N-(1-(3-methylbutyl)-1H-pyrazol-3-yl)imidazo[1,2-b]pyridazin-2-amin |
| 3-(6-Amino-2-methyl-4-pyrimidinyl)-N-(1-(2-methoxyethyl)-1H-pyrazol-3-yl)imidazo[1,2-b]pyridazin-2-amin |
| 3-(6-Amino-2-methyl-4-pyrimidinyl)-6-(3-(methylsulfonyl)-phenyl)-N-1H-pyrazol-3-ylimidazo[1,2-b]pyridazin-2-amin |
| N-(3-(4-Amino-6-methyl-1,3,5-triazin-2-yl)imidazo[1,2-a]-pyridin-2-yl)-1H-indazol-4-amin |
| 3-(6-Amino-2-methyl-4-pyrimidinyl)-N-(6-methoxy-3-pyridinyl)-6-(4-morpholinyl)-imidazo[1,2-b]pyridazin-2-amin |
| 3-(4-Amino-6-methyl-1,3,5-triazin-2-yl)-N-(6-methoxy-3-pyridinyl)-6-(4-morpholinyl)-imidazo[1,2-b]pyridazin-2-amin |
| 3-(6-Amino-2-methyl-4-pyrimidinyl)-N-6--(2-methoxyethyl)-N-2--(6-methoxy-3-pyridinyl)imidazo[1,2-b]pyridazin-2,6-diamin |
| 3-(6-Amino-2-methyl-4-pyrimidinyl)-N-2--1H-indazol-4-yl-N-6--(2-methoxyethyl)imidazo-[1,2-b]pyridazin-2,6-diamin |
| 1-(3-(6-Amino-2-methyl-4-pyrimidinyl)-2-((6-methoxy-3-pyridinyl)amino)imidazo[1,2-b]pyridazin-6-yl)-4-piperidinol |
| 4-(3-(4-Amino-6-methyl-1,3,5-triazin-2-yl)-2-((6-methoxy-3-pyridinyl)amino)imidazo[1,2-a]pyridin-7-yl)-N,N-dimethyl-1-piperazincarboxamid |
| 4-(3-(4-Amino-6-methyl-1,3,5-triazin-2-yl)-2-((5-fluor-6-methoxy-3-pyridinyl)amino)-imidazo[1,2-a]pyridin-7-yl)-N,N-dimethyl-1-piperazincarboxamid |
| 3-(4-Amino-6-methyl-1,3,5-triazin-2-yl)-N-(5-fluor-6-methoxy-3-pyridinyl)-7-(4-(methylsulfonyl)-1-piperazinyl)-imidazo[1,2-a]pyridin-2-amin |
| N-(5-((3-(4-Amino-6-methyl-1,3,5-triazin-2-yl)imidazo[1,2-a]pyridin-2-yl)amino)-2-chlor-3-pyridinyl)methansulfonamid |
| N-(5-((3-(4-Amino-6-methyl-1,3,5-triazin-2-yl)imidazo[1,2-a]pyridin-2-yl)amino)-2-methoxy-3-pyridinyl)methansulfon-amid |
| 3-(6-Amino-2-methyl-4-pyrimidinyl)-N-1H-indazol-6-ylimidazo[1,2-b]pyridazin-2-amin |
| 3-(6-Amino-2-methyl-4-pyrimidinyl)-N-1H-benzimidazol-6-ylimidazo[1,2-b]pyridazin-2-amin |
| N-(3-(6-Amino-2-methyl-4-pyrimidinyl)imidazo[1,2-a]-pyridin-2-yl)-1H-benzimidazol-6-amin |
| 3-(4-Amino-6-methyl-1,3,5-triazin-2-yl)-N-(6-methoxy-3-pyridinyl)-7-(1-methyl-1H-pyrazol-4-yl)imidazo[1,2-a]-pyridin-2-amin |
| 3-(4-Amino-6-methyl-1,3,5-triazin-2-yl)-N-(6-methoxy-3-pyridinyl)-6-(1-methyl-1H-pyrazol-4-yl)imidazo[1,2-a]-pyridin-2-amin |
| 3-(4-Amino-6-methyl-1,3,5-triazin-2-yl)-N-(6-methoxy-3-pyridinyl)-6-(4-morpholinyl)-imidazo[1,2-a]pyridin-2-amin |
| 3-(6-Amino-2-methyl-4-pyrimidinyl)-N-(6-methoxy-3-pyridinyl)-6-(4-morpholinyl)imidazo-[1,2-a]pyridin-2-amin |
| 3-(4-Amino-6-methyl-1,3,5-triazin-2-yl)-N-(6-methoxy-3-pyridinyl)-6-(3-pyridinyl)-imidazo[1,2-a]pyridin-2-amin |
| 4-(3-(4-Amino-6-methyl-1,3,5-triazin-2-yl)-2-((6-methoxy-3-pyridinyl)amino)imidazo[1,2-a]pyridin-6-yl)-N,N-dimethyl-1-piperazincarboxamid |
| 3-(4-Amino-6-methyl-1,3,5-triazin-2-yl)-N-(6-methoxy-3-pyridinyl)-6-(4-(methylsulfonyl)-1-piperazinyl)imidazo-[1,2-a]pyridin-2-amin |
| 2-(3-(4-Amino-6-methyl-1,3,5-triazin-2-yl)-2-((6-methoxy-3-pyridinyl)-amino)imidazo[1,2-a]pyridin-6-yl)-2-propanol |
| 3-(4-Amino-6-methyl-1,3,5-triazin-2-yl)-N-(6-methoxy-3-pyridinyl)-6-(1-piperazinyl)imidazo-[1,2-a]pyridin-2-amin |
| 1-(3-(4-Amino-6-methyl-1,3,5-triazin-2-yl)-2-(7-chinolinylamino)-imidazo[1,2-a]pyridin-6-yl)ethanon |
| 2-(3-(4-Amino-6-methyl-1,3,5-triazin-2-yl)-2-(7-chinolinylamino)-imidazo[1,2-a]pyridin-6-yl)-2-propanol |
| 1-(3-(4-Amino-6-methyl-1,3,5-triazin-2-yl)-2-((5-fluor-6-methoxy-3-pyridinyl)amino)-imidazo[1,2-a]pyridin-6-yl)-ethanon |
| 2-(3-(4-Amino-6-methyl-1,3,5-triazin-2-yl)-2-((5-fluor-6-methoxy-3-pyridinyl)amino)-imidazo[1,2-a]pyridin-6-yl)-2-propanol |
| 4-(3-(6-Amino-2-methyl-4-pyrimidinyl)-2-((5-fluor-6-methoxy-3-pyridinyl)amino)-imidazo[1,2-b]pyridazin-6-yl)-N,N-dimethyl-1-piperazincarboxamid |
| 1-(3-(6-Amino-2-methyl-4-pyrimidinyl)-2-((5-fluor-6-methoxy-3-pyridinyl)amino)imidazo-[1,2-a]pyridin-6-yl)-ethanon |
| 2-(3-(6-Amino-2-methyl-4-pyrimidinyl)-2-((5-fluor-6-methoxy-3-pyridinyl)amino)imidazo-[1,2-a]pyridin-6-yl)-2-propanol |
| 4-(3-(6-Amino-2-methyl-4-pyrimidinyl)-2-((5-fluor-6-methoxy-3-pyridinyl)amino)-imidazo[1,2-a]pyridin-6-yl)-N,N-dimethyl-1-piperazincarboxamid |
| 3-(4-Amino-6-methyl-1,3,5-triazin-2-yl)-N-1H-pyrazol-3-yl-6-(trifluormethyl)imidazo[1,2-a]pyridin-2-amin |
| 3-(4-Amino-6-methyl-1,3,5-triazin-2-yl)-N-1H-pyrazol-3-yl-7-(trifluormethyl)imidazo[1,2-a]-pyridin-2-amin |
| 3-(6-Amino-2-methyl-4-pyrimidinyl)-N-1H-pyrazol-3-yl-7-(trifluormethyl)imidazo[1,2-a]-pyridin-2-amin |
| 3-(6-Amino-2-methyl-4-pyrimidinyl)-N-1H-pyrazol-3-yl-6-(trifluormethyl)imidazo[1,2-a]-pyridin-2-amin |
| 1-(4-((3-(6-Amino-2-methyl-4-pyrimidinyl)imidazo[1,2-b]-pyridazin-2-yl)amino)phenyl)-3-cyclopropylharnstoff |
| 1-(4-((3-(6-Amino-2-methyl-4-pyrimidinyl)imidazo[1,2-b]-pyridazin-2-yl)amino)phenyl)-3-ethylharnstoff |
| N-(4-((3-(6-Amino-2-methyl-4-pyrimidinyl)imidazo[1,2-b]-pyridazin-2-yl)amino)phenyl)-1-piperidin-carboxamid |
| .N-(4-((3-(6-Amino-2-methyl-4-pyrimidinyl)imidazo[1,2-b]-pyridazin-2-yl)amino)phenyl)-1-pyrrolidin-carboxamid |
| N-(4-((3-(6-Amino-2-methyl-4-pyrimidinyl)imidazo[1,2-b]-pyridazin-2-yl)amino)phenyl)-1-azetidin-carboxamid |
| N-(4-((3-(6-Amino-2-methyl-4-pyrimidinyl)imidazo[1,2-b]-pyridazin-2-yl)amino)phenyl)-4-methyl-1-piperazincarbox-amid |
| 1-(4-((3-(6-Amino-2-methyl-4-pyrimidinyl)imidazo[1,2-b]-pyridazin-2-yl)amino)phenyl)-3-(2-methoxyethyl)harnstoff |
| N-(4-((3-(6-Amino-2-methyl-4-pyrimidinyl)imidazo[1,2-b]-pyridazin-2-yl)amino)phenyl)-4-morpholin-carboxamid |
| 4-Acetyl-N-(4-((3-(6-amino-2-methyl-4-pyrimidinyl)imidazo-[1,2-b]pyridazin-2-yl)amino)-phenyl)-1-piperazincarboxamid |
| 3-(6-Amino-2-methyl-4-pyrimidinyl)-N-1,3-thiazol-4-ylimidazo[1,2-b]pyridazin-2-amin |
| N-(4-((3-(6-Amino-2-methyl-4-pyrimidinyl)imidazo[1,2-a]-pyridin-2-yl)amino)phenyl)-4-morpholinecarboxamid |
| 4-Acetyl-N-(4-((3-(6-amino-2-methyl-4-pyrimidinyl)imidazo-[1,2-a]pyridin-2-yl)amino)phenyl)-1-piperazincarboxamid |
| N-(4-((3-(6-Amino-2-methyl-4-pyrimidinyl)imidazo[1,2-a]-pyridin-2-yl)amino)phenyl)-2-methyl-4-morpholin-carboxamid |
| 3-(4-((3-(6-Amino-2-methyl-4-pyrimidinyl)imidazo[1,2-a]-pyridin-2-yl)amino)phenyl)-1-methyl-1-(1-methylethyl)-harnstoff |
| N-(4-((3-(6-Amino-2-methyl-4-pyrimidinyl)imidazo[1,2-a]-pyridin-2-yl)amino)phenyl)-3-methyl-4-morpholin-carboxamid |
| 1-(4-((3-(6-Amino-2-methyl-4-pyrimidinyl)imidazo[1,2-a]-pyridin-2-yl)amino)phenyl)-3-cyclopropylharnstoff |
| Ethyl-2-((3-(6-amino-2-methyl-4-pyrimidinyl)imidazo-[1,2-b]pyridazin-2-yl)amino)-1H-imidazol-4-carboxylat |
| 3-(2-Methyl-4-pyrimidinyl)-6-(3-(methylsulfonyl)phenyl)-N-1H-pyrazol-3-ylimidazo[1,2-b]pyridazin-2-amin |
| N-(3-(6-Amino-2-methyl-4-pyrimidinyl)imidazo[1,2-b]-pyridazin-2-yl)-3H-imidazo[4,5-b]pyridin-5-amin |
| N-(2-Methyl-6-(2-((1-methyl-1H-pyrazol-3-yl)amino)-6-(3-(methylsulfonyl)phenyl)imidazo[1,2-a]pyridin-3-yl)-4-pyrimidinyl)acetamid |
| 3-(4-Amino-6-methyl-1,3,5-triazin-2-yl)-6-(3-(ethyl-sulfonyl)-phenyl)-N-1H-pyrazol-3-ylimidazo[1,2-a]pyridin-2-amin |
| 3-(6-Amino-2-methyl-4-pyrimidinyl)-6-(3,6-dihydro-2H-pyran-4-yl)-N-1H-pyrazol-3-ylimidazo[1,2-b]pyridazin-2-amin |
| N-(5-((3-(6-Amino-2-methyl-4-pyrimidinyl)imidazo[1,2-b]-pyridazin-2-yl)amino)-3-pyridinyl)acetamid |
| 3-(6-Amino-2-methyl-4-pyrimidinyl)-N-(2-methyl-1,3-thiazol-4-yl)imidazo[1,2-b]pyridazin-2-amin |
| 3-(6-Amino-2-methyl-4-pyrimidinyl)-N-1,3-thiazol-5-ylimidazo-[1,2-b]pyridazin-2-amin |
| 1-(4-((3-(6-Amino-2-methyl-4-pyrimidinyl)imidazo[1,2-a]-pyridin-2-yl)amino)phenyl)-3-(2-methylpropyl)harnstoff |
| N-(4-((3-(6-Amino-2-methyl-4-pyrimidinyl)imidazo[1,2-a]-pyridin-2-yl)amino)phenyl)-4,4-difluor-1-piperidin-carboxamid |
| (2S)-N-(4-((3-(6-Amino-2-methyl-4-pyrimidinyl)imidazo-[1,2-a]pyridin-2-yl)amino)phenyl)-2-methyl-1-pyrrolidin-carboxamid |
| N-(2-Methyl-6-(2-((1-methyl-1H-pyrazol-3-yl)amino)-6-(2-methyl-3-pyridinyl)imidazo[1,2-a]pyridin-3-yl)-4-pyrimidinyl)acetamid |
| N-(6-(6-(1-(2-Methoxyethyl)-1H-pyrazol-4-yl)-2-((1-methyl-1H-pyrazol-3-yl)amino)imidazo[1,2-a]pyridin-3-yl)-2-methyl-4-pyrimidinyl)acetamid |
| N-(5-((3-(6-(Acetylamino)-2-methyl-4-pyrimidinyl)imidazo-[1,2-b]pyridazin-2-yl)amino)-3-pyridinyl)acetamid |
| N-(2-Methyl-6-(2-(1H-pyrazol-3-ylamino)-6-(5-pyrimidinyl)-imidazo[1,2-a]pyridin-3-yl)-4-pyrimidinyl)acetamid |
| 8-Fluor-3-(2-methyl-4-pyrimidinyl)-N-1H-pyrazol-3-ylimidazo-[1,2-a]pyridin-2-amin |
| 3-(4-Amino-6-methyl-1,3,5-triazin-2-yl)-6-(4-(ethyl-sulfonyl)phenyl)-N-1H-pyrazol-3-ylimidazo[1,2-a]pyridin-2-amin |
| 3-(2,6-Dimethyl-4-pyrimidinyl)-6-(3-(methylsulfonyl)-phenyl)-N-1H-pyrazol-3-ylimidazo[1,2-b]pyridazin-2-amin |
| N-(6-(8-Fluor-2-(1H-pyrazol-3-ylamino)imidazo[1,2-a]-pyridin-3-yl)-2-methyl-4-pyrimidinyl)acetamid |
| N-(3-(6-Amino-2-methyl-4-pyrimidinyl)-6-(3-(methyl-sulfonyl)phenyl)imidazo[1,2-b]pyridazin-2-yl)-1,3-benzoldiamin |
| 3-((8-Fluor-3-(2-methyl-4-pyrimidinyl)imidazo[1,2-a]-pyridin-2-yl)amino)-N-(1-methylethyl)-1H-pyrazol-1-carboxamid |
| 5-((3-(6-(Acetylamino)-2-methyl-4-pyrimidinyl)-8-fluor-imidazo[1,2-a]pyridin-2-yl)amino)-1H-pyrazol-1-carboxamid |
| 3-((3-(6-(Acetylamino)-2-methyl-4-pyrimidinyl)-8-fluorimidazo[1,2-a]pyridin-2-yl)amino)-1H-pyrazol-1-carboxamid |
| N-(2-Methyl-6-(2-(1H-pyrazol-3-ylamino)-6-(5-pyrimidinyl)-imidazo[1,2-b]pyridazin-3-yl)-4-pyrimidinyl)acetamid |
| 5-((8-Fluor-3-(2-methyl-4-pyrimidinyl)imidazo[1,2-a]-pyridin-2-yl)amino)-N-(1-methylethyl)-1H-pyrazol-1-carboxamid |
| (2R)-N-(4-((3-(6-Amino-2-methyl-4-pyrimidinyl)imidazo-[1,2-b]pyridazin-2-yl)amino)phenyl)-2-methyl-4-morpholine-carboxamid |
| 3-(4-((3-(6-Amino-2-methyl-4-pyrimidinyl)imidazo[1,2-b]-pyridazin-2-yl)amino)phenyl)-1-methyl-1-(1-methylethyl)-harnstoff |
| (3R)-N-(4-((3-(6-Amino-2-methyl-4-pyrimidinyl)imidazo-[1,2-b]pyridazin-2-yl)amino)phenyl)-3-methyl-4-morpholin-carboxamid |
| (2S)-N-(4-((3-(6-Amino-2-methyl-4-pyrimidinyl)imidazo-[1,2-b]pyridazin-2-yl)amino)phenyl)-2-methyl-1-pyrrolidin-carboxamid |
| (3S)-N-(4-((3-(6-Amino-2-methyl-4-pyrimidinyl)imidazo-[1,2-b]pyridazin-2-yl)amino)phenyl)-3-methyl-1-pyrrolidin-carboxamid |
| N-(3-((3-(6-Amino-2-methyl-4-pyrimidinyl)-6-(3-(methyl-sulfonyl)-phenyl)imidazo[1,2-b]pyridazin-2-yl)amino)-phenyl)acetamid |
| N-(6-(2-((4-((Cyclopropylcarbamoyl)amino)phenyl)amino)-imidazo[1,2-b]pyridazin-3-yl)-2-methyl-4-pyrimidinyl)-acetamid |
| 3-(6-Amino-2-methyl-4-pyrimidinyl)-6-(5,6-dihydro-2H-pyran-3-yl)-N-1H-pyrazol-3-ylimidazo[1,2-b]pyridazin-2-amin |
| 2-(4-((3-(6-Amino-2-methyl-4-pyrimidinyl)imidazo[1,2-b]-pyridazin-2-yl)amino)phenyl)-N-cyclopropylacetamid |
| 3-((3-(6-Amino-2-methyl-4-pyrimidinyl)imidazo[1,2-b]-pyridazin-2-yl)amino)-N-(5-fluor-2-(trifluormethyl)-phenyl)-1H-pyrazol-1-carboxamid |
| 6-Fluor-8-methoxy-3-(2-methyl-4-pyrimidinyl)-N-1H-pyrazol-3-ylimidazo[1,2-a]pyridin-2-amin |
| N-(3-(6-Amino-2-methyl-4-pyrimidinyl)-6-(3-(methyl-sulfonyl)phenyl)imidazo[1,2-b]pyridazin-2-yl)-1,4-benzoldiamin |
| N-(4-((3-(6-Amino-2-methyl-4-pyrimidinyl)-6-(3-(methyl-sulfonyl)-phenyl)imidazo[1,2-b]pyridazin-2-yl)amino)-phenyl)acetamid |
| 3-(6-Amino-2-methyl-4-pyrimidinyl)-N-(1-methyl-1H-pyrazol-3-yl)-6-(3-(methylsulfonyl)phenyl)imidazo[1,2-b]pyridazin-2-amin |
| 3-(6-Amino-2-methyl-4-pyrimidinyl)-N-(1-(4,5-dihydro-1,3-oxazol-2-yl)-1H-pyrazol-3-yl)imidazo[1,2-b]pyridazin-2-amin |
| 6-Fluor-3-(2-methyl-4-pyrimidinyl)-N-1H-pyrazol-3-yl-8-(3-pyridinyloxy)imidazo[1,2-a]pyridin-2-amin |
oder ein pharmazeutisch annehmbares Salz hiervon.

13. Pharmazeutische Zusammensetzung, umfassend eine Verbindung gemäß einem der Ansprüche 1 bis 12 oder ein pharmazeutisch annehmbares Salz hiervon; und einen pharmazeutisch annehmbaren Hilfsstoff.

14. Verbindung gemäß einem der Ansprüche 1 bis 12 oder ein pharmazeutisch annehmbares Salz hiervon zur Verwendung bei einem Verfahren zur Behandlung eines Melanoms, von Eierstockkrebs, von Gebärmutterhalskrebs, von Brustkrebs, von Darmkrebs, von Mastdarmkrebs, eines Endometriumkarzinoms, von Pankreaskrebs, von Lungenkrebs, von Magenkrebs, eines Glioblastoms, von Leberkrebs, von Prostatakrebs, von akuter myeloischer Leukämie, von chronischer myeloischer Leukämie oder von Schilddrüsenkrebs.

15. Verbindung gemäß einem der Ansprüche 1 bis 12 zur therapeutischen Verwendung.

16. Verbindung gemäß Anspruch 15, wobei die Therapie für Krebs ist.

## Revendications

1. Composé de formule (I) : 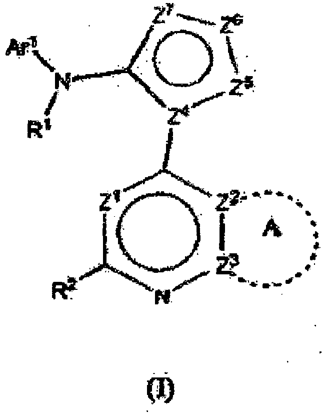 dans laquelle :
Ar¹ représente un cycle aryle, hétéroaryle, cycloalkyle ou hétérocyclyle, dans laquelle chaque cycle est substitué par R^{d}, R^{e}, ou R^{f} où R^{d}, R^{e}, ou R^{f} sont choisis indépendamment parmi un atome d'hydrogène, un groupe halogéno, halogénoalkyle, halogénoalcoxy, cyano, nitro, alkyle, alcényle, alcynyle, alkyle substitué, aryle, hétéroaryle, hétérocycloalkyle, -C(=O)NR^{a}R^{a}, -C(=O)R^{b}, -C(=O)OR^{b},-C(=NR^{a})NR^{a}R^{a}, -OR^{a}, -OC(=O)R^{b}, -OC(=O)NR^{a}R^{a}, -O-alkyl-N(R^{a})C(=O)OR^{b}, -OC(=O)N(R^{a})S(=O)₂R^{b}, -O-alkyl-NR^{a}R^{a}, -O-alkyl-OR^{a}, -SR^{a}, -S(=O)R^{b}, -S(=O)₂R^{b} -S(=O)₂NR^{a}R^{a}, -S(=O)₂N(R^{a})C(=O)R^{b}, -S(=O)₂N(R^{a})C(=O)OR^{b}, -S(=O)₂N(R^{a})C(=O)NR^{a}R^{a}, -NR^{a}R^{a}, -N(R^{a})C(=O)R^{b}, -N(R^{a})C(=O)OR^{b}, -N(R^{a})C(=O)NR^{a}R^{a}, -N(R^{a})C(=S)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}-alkylène-NR^{a}R^{a}, ou -NR^{a}-alkylène-OR^{a} ;
R¹ représente un atome d'hydrogène ou un groupe alkyle ;
R² représente un groupe méthyle ou éthyle ;
Z¹ représente -N- ou -CR³- où R³ représente H ou un groupe alkyle ;
Z² représente N- ou -CR⁴- où R⁴ représente R^{d}, un groupe -(alkylène)-hétérocycloalkyle, -(alkylène)ₙO(alkylène)ₙaryle, -(alkylène)ₙN(R^{a})(alkylène)ₙaryle, -(alkylène)ₙ-N(R^{a})-(alkylène)ₙhétéroaryle, ou -(alkylène)ₙO(alkylène)ₙhétéroaryle ;
Z³ représente -N- ou -CR⁵- où R⁵ représente R^{d}, un groupe -(alkylène)ₙaryle, -(alkylène)-hétéroaryle, -(alkylène)hétérocycloalkyle, -(alkylène)ₙO-(alkylène)ₙaryle, -(alkylène)ₙN(R^{a})(alkylène)ₙaryle, -(alkylène)ₙN(R^{a})(alkylène)ₙhétéroaryle, ou -(alkylène)ₙO(alkylène)ₙhétéroaryle à condition que seulement deux de Z¹, Z² et Z³ puissent représenter simultanément -N- ;
ou lorsque Z² représente -CR⁴- et Z³ représente -CR⁵-, alors R⁴ et R⁵ conjointement avec les atomes auxquels ils sont fixés peuvent former le cycle A qui est un cycle phényle ou hétéroaryle de 5 ou 6 chaînons et le cycle A est substitué par R^{g} ou R^{h} où R^{g} ou R^{h} représentent indépendamment R^{d}, un groupe -(alkylène)ₙ-hétérocycloalkyle, -(alkylène)ₙO(alkylène)ₙaryle, -(alkylène)ₙN(R^{a})(alkylène)ₙaryle, -(alkylène)ₙN(R^{a})(alkylène)ₙhétéroaryle, ou -(alkylène)ₙO(alkylène)ₙhétéroaryle ; 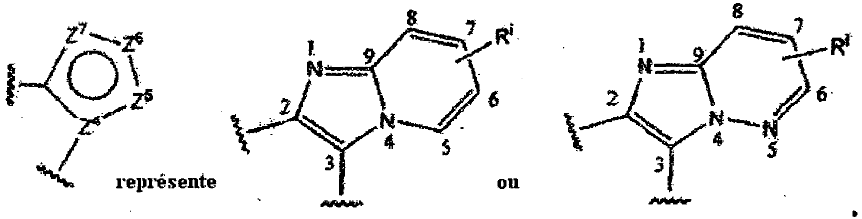
où R¹ représente R^{d}, un groupe -(alkylène)ₙaryle, -(alkylène)hétéroaryle, -(alkylène)-hétérocycloalkyle, -(alkylène)ₙO(alkylène)ₙaryle, -(alkylène)ₙN(R^{a})(alkylène)ₙaryle, -(alkylène₂)ₙN(R^{a})(alkylène)ₙhétéroaryle, ou -(alkylène)ₙO(alkylène)ₙhétéroaryle ; chaque R^{a} représente indépendamment un atome d'hydrogène ou R^{b} ; ou lorsque deux R^{a} sont fixés à un atome d'azote, soit seuls soit faisant partie d'un autre groupe, les deux R^{a} conjointement avec l'atome d'azote auquel ils sont fixés peuvent former un cycle hétérocyclyle monocyclique qui est éventuellement substitué par un, deux ou trois substituants choisis indépendamment parmi les groupes oxo, halogéno, alkyle, alcényle, alcynyle, cyano, nitro, alkylcarbonyle, carboxy, alcoxycarbonyle, hydroxyle, alcoxy, alconyloxy, alkylthio, alkylsulfonyle, -alkyl-OH, aminosulfonyle, sulfonylamino, amino, alkylamino, ou dialkylamino ;
chaque R^{b} représente indépendamment un groupe alkyle, cycloalkyle, phényle, hétéroaryle ou benzyle dans laquelle le groupe alkyle, cycloalkyle, phényle, ou benzyle est substitué par 0, 1, 2 ou 3 substituants choisis indépendamment parmi les groupes halogéno, alkyle, halogénoalkyle, alcoxy, amino, cyano, hydroxyle, hétérocycloalkyle non substitué, phényle, alkylcarbonylamino, alkylamino ou dialkylamino ; et
chaque n vaut indépendamment 0 ou 1 ; ou
un sel pharmaceutiquement acceptable de celui-ci.

2. Composé selon la revendication 1 dans lequel chaque R^{a} représente indépendamment un atome d'hydrogène ou R^{b} ; ou lorsque deux R^{a} sont fixés à un atome d'azote, soit seuls soit faisant partie d'un autre groupe, les deux R^{a} conjointement avec l'atome d'azote auquel ils sont fixés peuvent former un cycle hétérocyclyle monocyclique qui est éventuellement substitué par un, deux ou trois substituants choisis indépendamment parmi les groupes oxo, halogéno, alkyle, alcényle, alcynyle, cyano, nitro, alkylcarbonyle, carboxy, alcoxycarbonyle, hydroxyle, alcoxy, alconyloxy, alkylthio, alkylsulfonyle, aminosulfonyle, sulfonylamino, amino, alkylamino, ou dialkylamino ; et
chaque R^{b} représente indépendamment un groupe alkyle, cycloalkyle, phényle, hétéroaryle, ou benzyle dans lequel le groupe alkyle, cycloalkyle, phényle, ou benzyle est substitué par 0, 1, 2 ou 3 substituants choisis indépendamment parmi les groupes halogéno, alkyle, halogénoalkyle, alcoxy, amino, cyano, alkylamino ou dialkylamino.

3. Composé selon la revendication 1 dans lequel R¹ représente un atome d'hydrogène
et dans 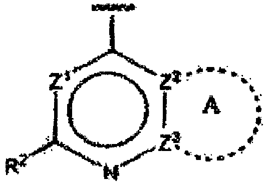 Z¹ représente -N-, Z² représente -CH- et Z³ représente -CR⁵- où R⁵ représente R^{d} ou un groupe -(alkylène)ₙN(R⁸)(alkylène)ₙ-hétéroaryle.

4. Composé selon la revendication 1 ou 3 dans lequel R² représente un groupe méthyle.

5. Composé selon l'une quelconque des revendications 1, 3 ou 4, dans lequel Ar¹ représente un cycle aryle substitué tel que défini ci-dessus.

6. Composé selon l'une quelconque des revendications 1, 3 ou 4, dans lequel Ar¹ représente un cycle hétéroaryle substitué tel que défini ci-dessus.

7. Composé selon l'une quelconque des revendications 1, 3 ou 4, dans lequel Ar¹ représente un cycle pyrazolyle substitué tel que défini ci-dessus.

8. Composé selon l'une quelconque des revendications 1, 3 ou 4, dans lequel Ar¹ représente un cycle aryle, hétéroaryle, ou hétérocyclyle, dans lequel chaque cycle est substitué par R^{d}, R^{e}, ou R^{f} où R^{d}, R^{e}, ou R^{f} sont choisis indépendamment parmi un atome d'hydrogène, un groupe halogéno, halogénoalkyle, alkyle, alkyle substitué par -NR^{y}R^{y} (où R^{y} représente un groupe alkyle) ou alcoxy, -(CR^{y}R^{y})ₙY (où Y représente un groupe aryle ou hétéroaryle), -OR^{x} (où R^{x} représente un atome d'hydrogène ou un groupe alkyle), hétéroaryle, hétérocycloalkyle, -C(=O)NR^{a}R^{a}, -C(=O)R^{b}, -C(=O)OR^{b}, -OR^{a}, -O-alkyl-OR^{a}, -S(=O)₂R^{b}, -NR^{a}R^{a}, -N(R^{a})C(=O)R^{b}, -N(R^{a})C(=O)NR^{a}R^{a}, -N(R^{a})S(=O)₂R^{b}, ou -NR^{a}-alkylène-OR^{a} ;
chaque R^{a} représente indépendamment un atome d'hydrogène ou R^{b} ; ou lorsque deux R^{a} sont fixés à un atome d'azote, soit seuls soit faisant partie d'un autre groupe, les deux R^{a} conjointement avec l'atome d'azote auquel ils sont fixés peuvent former un cycle hétérocyclyle monocyclique qui est éventuellement substitué par un, deux ou trois substituants choisis parmi les groupes alkyle, alkylcarbonyle, -alkyl-OH, ou hydroxyle ; et
chaque R^{b} représente indépendamment un groupe alkyle, cycloalkyle, phényle, hétéroaryle, ou benzyle dans lequel le groupe alkyle, cycloalkyle, phényle, ou benzyle est substitué par 0, 1, 2 ou 3 substituants choisis indépendamment parmi les groupes halogéno, alkyle, halogénoalkyle, alcoxy, amino, cyano, alkylamino, dialkylamino, hydroxyle, hétérocycloalkyle non substitué, ou phényle.

9. Composé selon l'une quelconque des revendications 1, 3 ou 4, dans lequel Ar¹ représente un groupe 5-fluoro-6-méthoxy-3-pyridinyle, 1H-pyrazol-3-yle, 1-méthyl-1H-pyrazol-3-yle, 4-(3-éthyluréido)phényle, 1H-pyrrolo[2,3-b]pyridin-6-yle, 6-(trifluorométhyl)-2-pyridinyle, 2-pyridinyle, 1H-pyrrolo[3,2-b]pyridin-5-yle, 4-hydroxypyridin-2-yle, 4-(3-phényluréido)phényle, 5-(3-éthyluréido)pyridin-2-yle, 4-(3-méthyluréido)phényle, 3-hydroxyphényle, 4-acétylaminophényle, 4-(3-(4-pyridinyl)uréidophényle, 3-amino-pyrimidin-5-yle, 4-pyridazinyle, 1-acétyl-1H-pyrazol-3-yle, 3-(méthylsulfonyl)phényle, 3-pyridinyle, 3-méthyl-5-isothiazolyle, 4-(4-méthyl-1-pipérazinyl)phényle, 1H-pyrazolo[3,4-c]pyridin-3-yle, 4-fluoro-1H-indazol-3-yl)imidazo[1,2-b]pyridazin-2-yle, 1-(2-(diméthylamino)éthyl)-1H-pyrazol-3-yle, 1H-pyrazolo[3,4-b)pyridin-3-yle, 3-(4-méthyl-1-pipérazinyl)phényle, 3-acétylaminophényle, pyridin-2-yle, 1-éthyl-1H-pyrazol-3-yle, quinoléin-3-yle, isoquinoléin-3-yle, 1-méthylaminocarbonyl-1H-pyrazol-3-yle, 1-méthylamino-carbonyl-1H-pyrazol-5-yle, 1-(2-hydroxyéthylaminocarbonyl)-1H-pyrazol-3-yle, 1-(2-hydroxyéthylaminocarbonyl)-1 H-pyrazol-5-yle, 1-(morpholin-4-ylcarbonyl)-1H-pyrazol-3-yle, 1-(morpholin-4-ylcarbonyl)-1H-pyrazol-5-yle, 1-(phénoxy-carbonyl)-1H-pyrazol-3-yle, 1-(phénylaminocarbonyl)-1H-pyrazol-3-yle, 1-(phényl-aminocarbonyl)-1H-pyrazol-5-yle, 1-(méthoxycarbonyl)-1H-pyrazol-3-yle, 1-(méthoxycarbonyl)-1H-pyrazol-5-yle, 1-(phénoxycarbonyl)-1H-pyrazol-4-yle, 1-(méthylaminocarbonyl)-1H-pyrazol-4-yle, 1-(pyridin-2-yl)-1H-pyrazol-3-yle, 1-(méthylsulfonyl)-1H-pyrazol-3-yle, 1-(benzoxazol-2-yl)-1H-pyrazol-3-yle, 1-(benzimidazol-2-yl)-1H-pyrazol-5-yle, 1-(isopropylaminocarbonyl)-1H-pyrazol-3-yle, 1-(benzylaminocarbonyl)-1H-pyrazol-3-yle, 1-(éthylaminocarbonyl)-1H-pyrazol-3-yle, 1-(2-méthoxyphénylaminocarbonyl)-1H-pyrazol-3-yle, 1-(2-méthyl-phénylaminocarbonyl)-1H-pyrazol-3-yle, 1-(4-méthoxyphénylaminocarbonyl)-1H-pyrazol-3-yle, 1-(2-fluorophénylaminocarbonyl)-1H-pyrazol-3-yle, 1-(4-cyano-phénylaminocarbonyl)-1H-pyrazol-3-yle, 1-(2-trifluorométhylphénylamino-carbonyl)-1H-pyrazol-3-yle, 1-(3-méthylphénylaminocarbonyl)-1H-pyrazol-3-yle, 1-(2,5-difluorophénylaminocarbonyl)-1H-pyrazol-3-yle, 1-(3,5-difluorophényl-aminocarbonyl)-1H-pyrazol-3-yle, 1-(4-méthylphénylaminocarbonyl)-1H-pyrazol-3-yle, 1-(3,5-dichlorophényl-aminocarbonyl)-1H-pyrazol-3-yle, 1-(2-chloro-5-trifluorométhylphénylaminocarbonyl)-1H-pyrazol-3-yle, 1-(2,5-dichlorophényl-aminocarbonyl)-1H-pyrazol-3-yle, 1-(2,3-dichloro-phénylaminocarbonyl)-1H-pyrazol-3-yle, 1-(2-chloro-4-trifluorométhyl-phénylaminocarbonyl)-1H-pyrazol-3-yle, 1-(benzimidazol-2-yl)-1H-pyrazol-3-yle, 1-(imidazol-2-yl)-1H-pyrazol-5-yle, 1-méthylsulfonyl-1H-pyrazol-3-yle, 1-aminocarbonyl-1H-pyrazol-3-yle, 1-amino-carbonyl-1H-pyrazol-5-yle, 1-azétidin-1-ylcarbonyl-1H-pyrazol-3-yle, 1-azétidin-1-ylcarbonyl-1H-pyrazol-5-yle, 1-(2-méthylaminoéthylamino)-carbonyl-1H-pyrazol-3-yle, 1-(2-méthylaminoéthylamino)carbonyl-1H-pyrazol-5-yle, 1-diméthylamino-carbonyl-1H-pyrazol-3-yle, 1-diméthylaminocarbonyl-1H-pyrazol-5-yle, 1-pipérazin-1-ylcarbonyl-1H-pyrazol-3-yle, 1-(2-méthoxyéthylamino)carbonyl-1H-pyrazol-3-yle, 1-(2-méthoxyéthylamino)carbonyl-1H-pyrazol-5-yle, 1-(2-morpholin-4-yléthylamino)-carbonyl-1H-pyrazol-3-yle, 1-(2-méthylpropylaminocarbonyl)-1H-pyrazol-3-yle, 1-(1-phenéthylaminocarbonyl)-1H-pyrazol-3-yle, 1-(benzyl)-1H-pyrazol-3-yle, 1-(pyridin-3-ylméthyl)-1H-pyrazol-3-yle, 1-(1,4-benzoxazin-3-(4H)-on-7-yl)-1H-pyrazol-3-yle, 5-(4-hydroxyazétidin-1-yl)pyridin-3-yle, 5-(4-(propan-2-ol)azétidin-1-yl)pyridin-3-yle, quinoléin-7-yle, quinoléin-6-yle, isoquinoléin-7-yle, 6-méthoxypyridin-3-yle, 6-éthoxypyridin-3-yle, 1H-indazol-3-yle, 5-fluoro-6-méthoxypyridin-3-yle, 1-(6-(2-méthoxyéthoxy)pyrimidin-3-yl)-1H-pyrazol-3-yle, 3-cyclopropyluréidophényle, 3-benzylcarbonylaminophényle, 3-benzoylamino-phényle, 3-(4-chlorophénylsulfonylamino)phényle, 3-méthylsulfonylaminophényle, 3-(4-fluorophényl-uréido)phényle, 3-(benzyluréido)-phényle, 3-éthylaminocarbonylphényle, 6-éthylaminocarbonylpyridin-3yle, 3-éthylcarbonylaminophényle, 3-méthylaminocarbonylphényle, 3-éthylaminocarbonylphényle, 3-isopropylamino-carbonylphényle, 3-phénylaminocarbonylphényle, 6-méthylaminocarbonyl-pyridin-3-yle, 6-isopropylaminocarbonylpyridin-3-yle, 6-phénylamino-carbonylpyridin-3-yle, 3-aminocarbonylphényle, 4-(2-diéthylaminoéthylaminocarhonyl)phényle, 4-phénylaminocarbonylphényle, 1-(3-méthylbutanoyl)-1H-pyrazol-3-yle, 1-isopropyloxycarbonyl-1H-pyrazol-3-yle, 1-isopropyloxycarbonyl-1H-pyrazol-5-yle, 2-méthoxy-pyridin-4-yle, 1-isopropylcarbonyl-1H-pyrazol-3-yle, 1-isopropylcarbonyl-1H-pyrazol-5-yle, 1-(3,3-diméthylbutanoyl)-1H-pyrazol-3-yle, 1-cyclohexyloxy-carbornyl-1H-pyrazol-5-yle, 1-cyclohexyloxycarbonyl-1H-pyrazol-3-yle, 2-méthoxypyridin-3-yle, 2-oxopyridin-3-yle, 1-(3-méthylbutyl)-1H-pyrazol-3-yle, 1-(2-méthoxyéthyl)-1H-pyrazol-3-yle, 1H-indazol-4-yle, 6-chloro-5-méthylsulfonyl-aminopyridin-3-yle, 6-méthoxy-5-méthylsulfonylaminopyridin-3-yle, 1H-indazol-6-yle, 1H-benzimidazol-6-yle, 4-(3-cyclopropyluréido)phényle, 4-pipéridin-1-yl-carbonylaminophényle, 4-pyrrolidin-1-ylcarbonylaminophényle, 4-azétidin-1-yl-carbonylaminophényle, 4-(1-méthylpipérazin-1-ylcarbonylamino-phényle, 4-(3-(2-méthoxyéthyl)uréido)phényle, 4-morpholin4-yl-ylcarbonylamino-phényle, 4-(1-acétylpipérazin-1-ylcarbonylaminophényle, thiazol-3-yle, 4-(2-méthyl-morpholin-4-yl-ylcarbonylamino)phényle, ou 4-(3-méthylinorpholin-4-yl-yl-carbonylamino)-phényle.

10. Composé selon l'une quelconque des revendications 1 et 3 à 9 dans lequel Rⁱ représente R^{d} choisi parmi un atome d'hydrogène, un groupe halogéno, halogénoalkyle, alkyl substitué, aryle, hétéroaryle, hétérocycloalkyle, -C(=O)NR^{a}R^{a}, -C(=O)OR^{b}, -COR^{b}, -OR^{a}, -O-alkyl-NR^{a}R^{a}, -O-alkyl-OR^{a}, -NR^{a}R^{a}, ou NR^{a}-alkylène-OR^{a} ; chaque R^{a} représente indépendamment un atome d'hydrogène ou R^{b} ou lorsque deux R^{a} sont fixés à un atome d'azote, soit seuls soit faisant partie d'un autre groupe, les deux R^{a} conjointement avec l'atome d'azote auquel ils sont fixés peuvent former un cycle hétérocyclyle monocyclique,
chaque R^{b} représente indépendamment un groupe alkyle, phényle, ou hétéroaryle dans lequel le groupe alkyle est substitué par 0, 1, 2 ou 3 substituants choisis indépendamment parmi un groupe alkyle ou alcoxy et dans lequel :
le cycle aryle peut être éventuellement substitué par un, deux ou trois substituants choisis indépendamment parmi les groupes alkyle, alcoxy, ou halogéno, et/ou un ou deux substituants choisis indépendamment parmi -C(=O)OR^{y}, -C(=O)NR^{x}R^{x}, -OR^{x}, -SR^{x}, -S(O)₂R^{y}, -S(=O)₂NR^{x}R^{x}, -NR^{x}R^{x}, -N(R^{x})C(=O)R^{y}, -(CR^{x}R^{x})ₘN(R^{x})C(=O)R^{y} ou -(CR^{x}R^{x})ₘOR^{x} où m vaut 1 à 3, chaque R^{x} représente un atome d'hydrogène ou R^{y} et R^{y} représente indépendamment un atome d'hydrogène, un groupe alkyle, ou cycloalkyle ;
le cycle hétérocycloalkyle est éventuellement substitué par un, deux ou trois substituants choisis indépendamment parmi les groupes alkyle, hydroxy, -C(=O)R^{y}, -C(=O)OR^{y}, -C(=O)NR^{x}R^{x}, -S(=O)₂R^{y}, ou -(CR^{x}R^{x})ₘOR^{x} où m vaut 0 à 3, chaque R^{x} représente un atome d'hydrogène ou R^{y} et R^{y} représente indépendamment un atome d'hydrogène ou un groupe alkyle ;
le cycle hétéroaryle est éventuellement substitué par un, deux ou trois substituants choisis indépendamment parmi les groupes alkyle, -OR^{x}, -C(=O)NR^{x}R^{x}, ou -(CR^{x}R^{x})ₘOR^{x} où m vaut 1 à 3, chaque R^{x} représente un atome d'hydrogène ou R^{y} et R^{y} représente indépendamment un atome d'hydrogène ou un groupe alkyle ; et
un groupe alkyle substitué est un radical alkyle dans lequel un, deux ou trois atomes d'hydrogène sont remplacés indépendamment par un groupe hydroxyle ou -NCOR^{y} où R^{y} représente un groupe alkyle.

11. Composé selon l'une quelconque des revendications 1 et 3 à 9 dans lequel R¹ représente un atome d'hydrogène, un groupe 1-pipérazinyle, 4-méthyl-1-pipérazinyle, 4-(1-méthyléthyl)-1-pipérazinyle, 4-(2-méthoxyéthyl)-1-pipérazinyle, fluoro, 3-(méthylsulfonyl)phényle, 4-pyridinyle, 2-méthoxyéthoxy, 4-morpholinyle, 2-(1-pyrrolidinyl)éthoxy, 1-tert-butoxycarbonyl-3,6-dihydro-1(2H)-pyridin-4-yle, 3-méthoxyphényle, 3-fluoro-4-méthylaminocarbonylphényle, 4-acétylaminométhyl-phényle, 1-(2-méthoxyéthyl)-1H-pyrazol-4-yle, 2-fluorophényle, 2-méthylphényle, 2-méthoxy-3-pyridinyle, 3-furanyle, 3,5-diméthyl-4-isoxazolyle, 4-fluoro-2-méthylphényle, 2-(1-méthyléthoxy)phényle, 3-(méthylsulfanyl)phényle, 1,2,3,6-tétrahydro-4-pyridinyle, 3-méthyl-5-isoxazolyle, 1-(2-méthoxyéthyl)-1H-pyrazol-4-yle, 3-acétylaminophényle, 3-cyclopropylaminophényle, 3-isopropanoylamino-phényle, 3-pyridazinyle, 1-méthyl-1,2,3,6-tétrahydro-4-pyridinyle, 4-acétylaminophényle, 2-méthyl-4-pyridinyle, 2-méthyl-3-pyridinyle, 5-pyrimidinyle, 3-pyridinyle, phénylamino, 4-méthoxyphénylamino, méthoxy, 3-fluorophényle, 4-méthylamino-carbonylphényle, pyridin-3-ylamino, 4-acétylaminophényle, 3-méthoxyphénylamino, pyridin-4-ylamino, pyrimidin-5-ylamino, 4-acétylaminophénylamino, 2-méthyl-4-pyridinylamino, 4-méthyl-3-pyridinyle, 2,6-diméthyl-4-pyridinyle, 4-isopropylamino-carbonylphényle, 3,4-dihydro-1(2H)-isoquinoléinon-6-yle, 4-éthylaminocarbonylphényle, 4-carboxy-2-méthylphényle, 4-méthyl-5-pyrimidinyle, 2-méthyl-4-méthylaminocarbonylphényle, hydroxyle, 6-méthylamino-carbonylpyridin-3-yle, 2-(1-pyrrolidinyl)éthoxy, 4-N,N-diméthylaminocarbonyl-pipérazin-1-yle, 4-méthylsulfonylpipérazin-1-yle, 4-N-méthylaminocarbonyl-pipérazin-1-yle, 4-acétylpipérazin-1-yle, 3-hydroxyméthyl-4-méthoxyphényle, pyridin-4-yle, 3,6-dihydro-2H-pyran-4-yle, 2-méthyl-4-pyridinyle, 1-méthyl-1H-imidazol-5-yle, 3,4-dihydroxybutyle, 1-(1-méthyléthyl)-1H-pyrazol-4-yle, aminocarbonyle, quinoléin-7-yle, 4-méthylsulfonylphényle, 4-méthylaminosulfonyl-phényle, 3-méthylsulfonylphényle, 2-méthoxyéthylamino, 4-hydroxypipéridin-1-yle, 1-méthyl-1H-pyrazol-4-yle, 2-hydroxyprop-2-yle, acétyle, ou trifluorométhyle.

12. Composé selon la revendication 1 choisi dans le groupe constitué de :
| |
|---|
| 3-(4-ammo-6-méthyl-1,3,5-triazin-2-yl)-N-(1H-pyrazol-3-yl)imidazo[1,2-a]-pyridin-2-amine |
| 3-(3-(2-méthyl-9H-purin-4-yl)imidazo[1,2-a]pyridin-2-ylamino)phénol |
| 3-(4-méthyl-6-(méthylamino)-1,3,5-triazin-2-yl)-N-(1H-pyrazol-3-yl)imidazo-[1,2-a]pyridin-2-amine |
| 3-(4-amino-6-méthyl-1,3,5-triazin-2-yl)-N-(6-méthoxypyridin-3-yl)imidazo[1,2-a]-pyridin-2-amine |
| N-(3-(2-méthyl-9H-purin-4-yl)imidazo[1,2-a]pyridin-2-yl)-1H-indazol-4-amine |
| 7-chloro-3-(4-amino-6-méthyl)-1,3,5-triazin-2-yl)-N-(1H-pyrazol-3-yl)imidazo-[1,2-a]pyridin-2-amine |
| 3-(6-amino-2-méthylpyrimidin-4-yl)-N-(1H-pyrazol-3-yl)imidazo[1,2-b]pyridazin-2-amine |
| 3-(4-amino-6-méthyl-1,3,5-triazin-2-yl)-N-(5-fluoro-6-méthoxypyridin-3-yl)-imidazo[1,2-a]pyridin-2-amine |
| 3-(4-amino-6-méthyl-1,3,5-triazin-2-yl)-N-(6-méthoxypyridin-3-yl)-6-fluoro-imidazo[1,2-a]pyridin-2-amine |
| 3-(4-amino-6-méthyl-1,3,5-triazin-2-yl)-N-(5-fluoro-6-méthoxy-3-pyridinyl)-6-(1-pipérazinyl)imidazo[1,2-a]pyridin-2-amine |
| 3-(4-amino-6-méthyl-1,3,5-triazin-2-yl)-N-(5-fluoro-6-méthoxy-3-pyridinyl)-6-(4-méthyl-1-pipérazinyl)imidazo[1,2-a]pyridin-2-amine |
| 3-(4-amino-6-méthyl-1,3,5-triazin-2-yl)-N-(5-fluoro-6-méthoxy-3-pyridinyl)-6-(4-(1-méthyléthyl)-1-pipérazinyl)imidazo[1,2-a]pyridin-2-amine |
| 3-(4-amino-6-méthyl-1,3,5-triazin-2-yl)-N-(5-fluoro-6-méthoxy-3-pyridinyl)-6-(4-(2-éthoxyéthyl)-1-pipérazinyl)imidazo[1,2-a]pyridin-2-amine |
| 3-(2-méthyl-6-(phénylamino)-4-pyrimidinyl)-N-1H-pyrazol-3-ylimidazo[1,2-b]-pyridazin-2-amine |
| 3-(6-((2-fluorophényl)amino)-2-méthyl-4-pyrimidinyl)-N-1H-pyrazol-3-yl-imidazo[1,2-b]pyridazin-2-amine |
| 3-(6-((4-fluorophényl)amino)-2-méthyl-4-pyrimidinyl)-N-1H-pyrazol-3-yl-imidazo[1,2-b]pyridazin-2-amine |
| 3-(6-((4-méthoxyphényl)amino)-2-méthyl-4-pyrimidinyl)-N-1H-pyrazol-3-yl-imidazo[1,2-b]pyridazin-2-amine |
| N-(1-méthyl-1H-pyrazol-3-yl)-3-(2-méthyl-6-(3-pyridazinylamino)-4-pyrimidinyl)-imidazo[1,2-b]pyridazin-2-amine |
| 3-(2-méthyl-6-((6-(4-méthyl-1-pipérazinyl)-3-pyridazinyl)amino)-4-pyrimidinyl)-N-1H-pyrazol-3-ylimidazo[1,2-b]pyridazin-2-amine |
| 3-(6-((5-méthoxy-2-pyridinyl)amino)-2-méthyl-4-pyrimidinyl)-N-1H-pyrazol-3-yl-imidazo[1,2-b]pyridazin-2-amine |
| 3-(6-((3-fluorophényl)amino)-2-méthyl-4-pyrimidinyl)-N-1H-pyrazol-3-yl-imidazo[1,2-b]pyridazin-2-amine |
| 3-(6-((4-méthoxy-2-pyridmyl)ammo)-2-méthyl-4-pyrimidinyl)-N-1H-pyrazol-3-yl-imidazo[1,2-b]pyridazin-2-amine |
| 3-(2-méthyl-6-((6-(4-morpholinyl)-3-pyridazinyl)amino)-4-pyrimidinyl)-N-1H-pyrazol-3-ylimidazo[1,2-b]pyridazin-2-amine |
| 1-(4-((3-(6-amino-2-méthyl-4-pyrimidinyl)imidazo[1,2-a]pyridin-2-yl)amino)-phényl)-3-éthylurée |
| 3-(6-amino-2-méthyl-4-pyrimidinyl)-N-1H-pyrrolo[2,3-b]pyridin-6-ylimidazo-[1,2-a]pyridin-2-amine |
| 3-(6-amino-2-méthyl-4-pyrimidinyl)-N-(6-(trifluorométhyl)-2-pyridinyl)-imidazo[1,2-a]pyridin-2-amine |
| 3-(6-amino-2-méthyl-4-pyrimidinyl)-N-2-pyridinylimidazo[1,2-a]pyridin-2-amine |
| 3-(6-((5-(2-méthoxyéthoxy)-3-pyridazinyl)amino)-2-méthyl-4-pyrimidinyl)-N-1H-pyrazol-3-ylimidazo[1,2-b]pyridazin-2-amine |
| N-(3-(6-amino-2-méthyl-4-pyrimidinyl)imidazo[1,2-a]pyridin-2-yl)-1H-pyrrolo-[3,2-b]pyridin-5-amine |
| 2-((3-(6-amino-2-méthyl-4-pyrimidinyl)imidazo[1,2-a]pyridin-2-yl)amino)-4-pyridinol |
| 1-(4-((3-(6-amino-2-méthyl-4-pyrimidinyl)imidazo[1,2-a]pyridin-2-yl)amino)-phényl)-3-phénylurée |
| 1-(6-((3-(6-amino-2-méthyl-4-pyrimidinyl)imidazo[1,2-a]pyridin-2-yl)amino)-3-pyridinyl)-3-éthylurée |
| 3-(2-méthyl-6-((6-(4-morpholinyl)-3-pyridazinyl)amino)-4-pyrimidinyl)-N-1H-pyrazol-3-ylimidazo[1,2-a]pyridin-2-amine |
| 1-(4-((3-(6-amino-2-méthyl-4-pyrimidinyl)imidazo[1,2-a]pyridin-2-yl)amino)-phényl)-3-méthylurée |
| 3-((3-(6-((5-(2-méthoxyéthoxy)-3-pyridazinyl)amino)-2-méthyl-4-pyrimidinyl)-imidazo[1,2-b]pyridazin-2-yl)amino)phénol |
| N-(4-((3-(6-((5-(2-méthoxyéthoxy)-3-pyridazinyl)amino)-2-méthyl-4-pyrimidinyl)-imidazo[1,2-b]pyridazin-2-yl)amino)phényl)acétamide |
| 3-(6-((5-méthoxy-3-pyridazinyl)amino)-2-méthyl-4-pyrimidinyl)-N-1H-pyrazol-3-ylimidazo[1,2-b]pyridazin-2-amine |
| 1-éthyl-3-(4-((3-(6-((5-(2-méthoxyéthoxy)-3-pyridazinyl)amino)-2-méthyl-4-pyrimidinyl)imidazo[1,2-b]pyridazin-2-yl)amino)phényl)urée |
| 3-(2-méthyl-6-((5-méthyl-3-pyridazinyl)amino)-4-pyrimidinyl)-N-1H-pyrazol-3-ylimidazo[1,2-b]pyridazin-2-amine |
| 1-(4-((3-(6-amino-2-méthyl-4-pyrimidinyl)imidazo[1,2-a]pyridin-2-yl)amino)-phényl)-3-(4-pyridinyl)urée |
| 3-(6-((5-(2-méthoxyéthoxy)-3-pyridazinyl)amino)-2-méthyl-4-pyrimidinyl)-N-1H-pyrazol-3-ylimidazo[1,2-a]pyridin-2-amine |
| N-(6-(2-((4-((éthylcarbamoyl)amino)phényl)amino)imidazo[1,2-a]pyridin-3-yl)-2-méthyl-4-pyrimidinyl)acétamide |
| 3-(2-méthyl-6-((5-(2,2,2-trifluoroéthoxy)-3-pyridazinyl)amino)-4-pyrimidinyl)-N-1H-pyrazol-3-ylimidazo[1,2-b]pyridazin-2-amine |
| N-5∼-(3-(6-((5-(2-méthoxyéthoxy)-3-pyridazinyl)amino)-2-méthyl-4-pyrimidinyl)-imidazo[1,2-b]pyridazin-2-yl)-2,5-pyrimidinediamine |
| 8-fluoro-3-(6-((5-(2-méthoxyéthoxy)-3-pyridazinyl)amino)-2-méthyl-4-pyrimidinyl)-N-1H-pyrazol-3-ylimidazo[1,2-a]pyridin-2-amine |
| N-(6-(8-fluoro-2-((1-méthyl-1H-pyrazol-3-yl)amino)imidazo[1,2-a]pyridin-3-yl)-2-méthyl-4-pyrimidinyl)acétamide |
| 3-(6-amino-2-méthyl-4-pyrimidinyl)-N-4-pyridazinylimidazo[1,2-b]pyridazin-2-amine |
| 3 -(6-amino-2-méthyl-4-pyrimidinyl)-N-(1-méthyl-1H-pyrazol-3-yl)-6-(3-(méthyl-sulfonyl)phényl)imidazo[1,2-a]pyridin-2-amine |
| 3-(2-méthyl-6-(méthylamino)-4-pyrimidinyl)-N-1H-pyrazol-3-ylimidazo[1,2-b]-pyridazin-2-amine |
| N-(1-acétyl-1H-pyrazol-3-yl)-3-(6-amino-2-méthyl-4-pyrimidinyl)imidazo[1,2-b]-pyridazin-2-amine |
| 3-(6-amino-2-méthyl-4-pyrimidinyl)-N-1H-pyrazol-3-yl-6-(4-pyridinyl)imidazo-[1,2-b]pyridazin-2-amine |
| 3-(6-amino-2-méthyl-4-pyrimidinyl)-6-(2-méthoxyéthoxy)-N-1H-pyrazol-3-yl-imidazo[1,2-b]pyridazin-2-amine |
| N-(2-méthyl-6-(2-(1H-pyrazol-3-ylamino)imidazo[1,2-b]pyridazin-3-yl)-4-pyrimidinyl)acétamide |
| 3-(6-amino-2-méthyl-4-pyrimidinyl)-6-(4-morpholinyl)-N-1H-pyrazol-3-yl-imidazo[1,2-b]pyridazin-2-amine |
| 3-(2-méthyl-6-(méthylamino)-4-pyrimidinyl)-6-(4-morpholinyl)-N-1H-pyrazol-3-ylimidazo[1,2-b]pyridazin-2-amine |
| 3-(6-amino-2-méthyl-4-pyrimidinyl)-N-1H-pyrazol-3-yl-6-(2-(1-pyrrolidinyl)-éthoxy)imidazo[1,2-b]pyridazin-2-amine |
| 3-(2-méthyl-6-(2-pyrazinylamino)-4-pyrimidinyl)-N-1H-pyrazol-3-ylimidazo-[1,2-b]pyridazin-2-amine |
| 1,1-diméthyl-3-(2-méthyl-6-(2-(1H-pyrazol-3-ylamino)imidazo[1,2-b]pyridazin-3-yl)-4-pyrimidinyl)urée |
| 1-éthyl-3-(2-méthyl-6-(2-(1H-pyrazol-3-ylamino)imidazo[1,2-b]pyridazin-3-yl)-4-pyrimidinyl)urée |
| 4-méthyl-N-(2-méthyl-6-(2-(1H-pyrazol-3-ylamino)imidazo[1,2-b]pyridazin-3-yl)-4-pyrimidinyl)-1-pipérazinecarboxamide |
| N-(2-méthyl-6-(2-(1H-pyrazol-3-ylamino)imidazo[1,2-b]pyridazin-3-yl)-4-pyrimidinyl)-4-morpholinecarboxamide |
| 3-(2-méthyl-6-(1H-pyrazol-3-ylamino)-4-pyrimidinyl)-N-1H-pyrazol-3-yl-imidazo[1,2-b]pyridazin-2-amine |
| 3-(6-(diméthylamino)-2-méthyl-4-pyrimidinyl)-N-(1-méthyl-1H-pyrazol-3-yl)-imidazo[1,2-b]pyridazin-2-amine |
| 3-(2-méthyl-6-(1,3-thiazol-2-ylamino)-4-pyrimidinyl)-N-1H-pyrazol-3-ylimidazo-[1,2-b]pyridazin-2-amine |
| 3-(2-méthyl-6-(2-pyridinylamino)-4-pyrimidinyl)-N-1H-pyrazol-3-ylimidazo-[1,2-b]pyridazin-2-amine |
| 3-(2-méthyl-6-(3-pyridazinylamino)-4-pyrimidinyl)-N-1H-pyrazol-3-ylimidazo-[1,2-b]pyridazin-2-amine |
| 3-(2-méthyl-6-(4-pyrimidinylamino)-4-pyrimidinyl)-N-1H-pyrazol-3-ylimidazo-[1,2-b]pyridazin-2-amine |
| 3-(2-méthyl-6-(2-pyrimidinylamino)-4-pyrimidinyl)-N-1H-pyrazol-3-ylimidazo-[1,2-b]pyridazin-2-amine |
| N-(2-méthyl-6-(2-((1-méthyl-1H-pyrazol-3-yl)amino)imidazo[1,2-b]pyridazin-3-yl)-4-pyrimidinyl)acétamide |
| 3-(6-amino-2-méthyl-4-pyrimidinyl)-7-(4-morpholinyl)-N-1H-pyrazol-3-yl-imidazo[1,2-b]pyridazin-2-amine |
| 3-(2-méthyl-6-(méthylamino)-4-pyrimidinyl)-7-(4-morpholinyl)-N-1H-pyrazol-3-ylimidazo[1,2-b]pyridazin-2-amine |
| N-(2-méthyl-6-(2-(1H-pyrazol-3-ylamino)imidazo[1,2-b]pyridazin-3-yl)-4-pyrimidinyl)méthanesulfonamide |
| ((2-méthyl-6-(2-(1H-pyrazol-3-ylamino)imidazo[1,2-b]pyridazin-3-yl)-4-pyrimidinyl)amino)acétonitrile |
| 3-(6-amino-2-méthyl-4-pyrimidinyl)-8-fluoro-N-1H-pyrazol-3-ylimidazo[1,2-a]-pyridin-2-amine |
| N~2~,N~2~-diméthyl-N-(2-méthyl-6-(2-((1-méthyl-1H-pyrazol-3-yl)amino)-imidazo[1,2-b]pyridazin-3-yl)-4-pyrimidinyl)glycinamide |
| N-(2-méthyl-6-(2-((1-méthyl-1H-pyrazol-3-yl)amino)imidazo[1,2-b]pyridazin-3-yl)-4-pyrimidinyl)glycinamide |
| 3-(6-amino-2-méthyl-4-pyrimidinyl)-N-(3-(méthylsulfonyl)phényl)imidazo-[1,2-b]pyridazin-2-amine |
| 3-(6-amino-2-méthyl-4-pyrimidinyl)-N-3-pyridinylimidazo[1,2-b]pyridazin-2-amine |
| 3-(6-amino-2-méthyl-4-pyrimidinyl)-N-(3-méthyl-5-isothiazolyl)imidazo[1,2-b]-pyridazin-2-amine |
| 4-(3-(4-amino-6-méthyl-1,3,5-triazin-2-yl)-2-(1H-pyrazol-3-ylamino)imidazo-[1,2-a]pyridin-6-yl)-3,6-dihydro-1(2H)-pyridinecarboxylate de tert-butyle |
| 3-(4-amino-6-méthyl-1,3,5-triazin-2-yl)-6-(3-méthoxyphényl)-N-1H-pyrazol-3-ylimidazo[1,2-a]pyridin-2-amine |
| 4-(3-(4-amino-6-méthyl-1,3,5-triazin-2-yl)-2-(1H-pyrazol-3-ylamino)imidazo-[1,2-a]pyridin-6-yl)-2-fluoro-N-méthylbenzamide |
| N-(4-(3-(4-amino-6-méthyl-1,3,5-triazin-2-yl)-2-(1H-pyrazol-3-ylamino)imidazo-[1,2-a]pyridin-6-yl)benzyl)acétamide |
| 3-(4-amino-6-méthyl-1,3,5-triazin-2-yl)-6-(1-(2-méthoxyéthyl)-1H-pyrazol-4-yl)-N-1H-pyrazol-3-ylimidazo[1,2-a]pyridin-2-amine |
| 3-(4-amino-6-méthyl-1,3,5-triazin-2-yl)-6-(2-fluorophényl)-N-1H-pyrazol-3-yl-imidazo[1,2-a]pyridin-2-amine |
| 3-(4-amino-6-méthyl-1,3,5-triazin-2-yl)-6-(2-méthylphényl)-N-1H-pyrazol-3-yl-imidazo[1,2-a]pyridin-2-amine |
| 3-(4-amino-6-méthyl-1,3,5-triazin-2-yl)-6-(2-méthoxy-3-pyridinyl)-N-1H-pyrazol-3-ylimidazo[1,2-a]pyridin-2-amine |
| 3-(4-amino-6-méthyl-1,3,5-triazin-2-yl)-6-(3-furanyl)-N-1H-pyrazol-3-ylimidazo-[1,2-a]pyridin-2-amine |
| 3-(4-amino-6-méthyl-1,3,5-triazin-2-yl)-6-(3,5-diméthyl-4-isoxazolyl)-N-1H-pyrazol-3-ylimidazo[1,2-a]pyridin-2-amine |
| 3-(4-amino-6-méthyl-1,3,5-triazin-2-yl)-6-(4-fluoro-2-méthylphényl)-N-1H-pyrazol-3-ylimidazo[1,2-a]pyridin-2-amine |
| 3-(4-amino-6-méthyl-1,3,5-triazin-2-yl)-6-(2-(1-méthyléthoxy)phényl)-N-1H-pyrazol-3-ylimidazo[1,2-a]pyridin-2-amine |
| 3-(4-amino-6-méthyl-1,3,5-triazin-2-yl)-6-(3-(méthylsulfanyl)phényl)-N-1H-pyrazol-3-ylimidazo[1,2-a]pyridin-2-amine |
| N-(4-(3-(4-méthyl-6-(méthylamino)-1,3,5-triazin-2-yl)-2-(1H-pyrazol-3-ylamino)-imidazo[1,2-a]pyridin-6-yl)benzyl)acétamide |
| 3-(4-amino-6-méthyl-1,3,5-triazin-2-yl)-N-1H-pyrazol-3-yl-6-(1,2,3,6-tétrahydro-4-pyridinyl)imidazo[1,2-a]pyridin-2-amine |
| 3-(4-amino-6-méthyl-1,3,5-triazin-2-yl)-6-(3-méthyl-5-isoxazolyl)-N-1H-pyrazol-3-ylimidazo[1,2-a]pyridin-2-amine |
| 6-(1-(2-méthoxyéthyl)-1H-pyrazol-4-yl)-3-(4-méthyl-6-(méthylamino)-1,3,5-triazin-2-yl)-N-1H-pyrazol-3 -ylimidazo [1,2-a]pyridin-2-amine |
| N-(3-(3-(4-amino-6-méthyl-1,3,5-triazin-2-yl)-2-(1H-pyrazol-3-ylamino)imidazo-[1,2-a]pyridin-6-yl)phényl)acétamide |
| N-(3-(3-(4-amino-6-méthyl-1,3,5-triazin-2-yl)-2-(1H-pyrazol-3-ylamino)imidazo-[1,2-a]pyridin-6-yl)phényl)cyclopropanecarboxamide |
| N-(4-(3-(4-amino-6-méthyl-1,3,5-triazin-2-yl)-2-(1H-pyrazol-3-ylamino)imidazo-[1,2-a]pyridin-6-yl)phényl)-2-méthylpropanamide |
| 3-(4-amino-6-méthyl-1,3,5-triazin-2-yl)-N-1H-pyrazol-3-yl-6-(3-pyridazinyl)-imidazo[1,2-a]pyridin-2-amine |
| 3-(4-amino-6-méthyl-1,3,5-triazin-2-yl)-6-(1-méthyl-1,2,3,6-tétrahydro-4-pyridinyl)-N-1H-pyrazol-3-ylimidazo[1,2-a]pyridin-2-amine |
| N-(4-(3-(6-amino-2-méthyl-4-pyrimidinyl)-2-(1H-pyrazol-3-ylamino)imidazo-[1,2-b]pyridazin-6-yl)benzyl)acétamide |
| N-(2-méthyl-6-(2-(1H-pyrazol-3-ylamino)imidazo[1,2-b]pyridazin-3-yl)-4-pyrimidinyl)cyclopropanecarboxamide |
| N-(4-(3-(6-amino-2-méthyl-4-pyrimidinyl)-2-(1H-pyrazol-3-ylamino)imidazo-[1,2-b]pyridazin-6-yl)phényl)acétamide |
| 4-(3-(6-amino-2-méthyl-4-pyrimidinyl)-2-(1H-pyrazol-3-ylamino)imidazo[1,2-b]-pyridazin-6-yl)-N-méthylbenzamide |
| 2-méthoxy-N-(2-méthyl-6-(2-((1-méthyl-1H-pyrazol-3-yl)amino)imidazo[1,2-b]-pyridazin-3-yl)-4-pyrimidinyl)acétamide |
| 3-(6-amino-2-méthyl-4-pyrimidinyl)-N-1H-pyrazol-3-ylimidazo[1,2-a]pyridin-2-amine |
| 3-(6-amino-2-méthyl-4-pyrimidinyl)-N-(1-méthyl-1H-pyrazol-3-yl)imidazo[1,2-a]-pyridin-2-amine |
| 2-cyano-N-(2-méthyl-6-(2-((1-méthyl-1H-pyrazol-3-yl)amino)imidazo[1,2-a]-pyridin-3-yl)-4-pyrimidinyl)acétamide |
| 2-(3-méthoxyphényl)-N-(2-méthyl-6-(2-((1-méthyl-1H-pyrazol-3-yl)amino)-imidazo[1,2-a]pyridin-3-yl)-4-pyrimidinyl)acétamide |
| 2-(3-fluorophényl)-N-(2-méthyl-6-(2-((1-méthyl-1H-pyrazol-3-yl)amino)imidazo-[1,2-a]pyridin-3-yl)-4-pyrimidinyl)acétamide |
| 2-(2,4-difluorophényl)-N-(2-méthyl-6-(2-((1-méthyl-1H-pyrazol-3-yl)amino)-imidazo[1,2-b]pyridazin-3-yl)-4-pyrimidinyl)acétamide |
| 3-cyano-N-(2-méthyl-6-(2-((1-méthyl-1H-pyrazol-3-yl)amino)imidazo[1,2-b]-pyridazin-3-yl)-4-pyrimidinyl)benzamide |
| 3,3,3-trifluoro-N-(1-méthyl-1H-pyrazol-3-yl)-N-(3-(2-méthyl-6-((3,3,3-trifluoro-propanoyl)amino)-4-pyrimidinyl)imidazo[1,2-a]pyridin-2-yl)propanamide |
| 3-(6-amino-2-méthyl-4-pyrimidinyl)-N-(1-méthyl-1H-pyrazol-3-yl)-6-(2-méthyl-4-pyridinyl)imidazo[1,2-b]pyridazin-2-amine |
| 3 -(6-amino-2-méthyl-4-pyrimidinyl)-N-(1-méthyl-1H-pyrazol-3-yl)-6-(2-méthyl-3 - pyridinyl)imidazo[1,2-b]pyridazin-2-amine |
| 3,3,3-trifluoro-N-(2-méthyl-6-(2-((1-méthyl-1H-pyrazol-3-yl)amino)imidazo[1,2-b]-pyridazin-3-yl)-4-pyrimidinyl)propanamide |
| 3,3,3-trifluoro-N-(2-méthyl-6-(2-((1-méthyl-1H-pyrazol-3-yl)amino)imidazo[1,2-a]-pyridin-3-yl)-4-pyrimidinyl)propanamide |
| 3-(6-amino-2-méthyl-4-pyrimidinyl)-N-(1-méthyl-1H-pyrazol-3-yl)-6-(5-pyrimidinyl)imidazo[1,2-b]pyridazin-2-amine |
| 3-(6-((6-(2-méthoxyéthoxy)-3-pyridazinyl)amino-2-méthyl-4-pyrimidinyl)-N-1 H-pyrazol-3-ylimidazo[1,2-b]pyridazin-2-amine |
| 2-(4-(6-((2-méthyl-6-(2-(1H-pyrazol-3-ylamino)imidazo[1,2-b]pyridazin-3-yl)-4-pyrimidinyl)amino)-3-pyridazinyl)-2-morpholinyl)éthanol |
| 3-(6-amino-2-méthyl-4-pyrimidinyl)-N-(4-(4-méthyl-1-pipérazinyl)phényl)imidazo-[1,2-b]pyridazin-2-amine |
| 3-(2-méthyl-6-((6-(2-méthyl-4-morpholinyl)-3-pyridazinyl)amino)-4-pyrimidinyl)-N-1 H-pyrazol-3-ylimidazo[1,2-b]pyridazin-2-amine |
| 3-(2-méthyl-6-((6-(3-méthyl-4-morpholinyl)-3-pyridazinyl)amino)-4-pyrimidinyl)-N-1 H-pyrazol-3-ylimidazo[1,2-b]pyridazin-2-amine |
| N-(3-(6-amino-2-méthyl-4-pyrimidinyl)imidazo[1,2-b]pyridazin-2-yl)-1H-pyrazolo-[3,4-c]pyridin-3-amine |
| 3-(6-amino-2-méthyl-4-pyrimidinyl)-N-(4-fluoro-1H-indazol-3-yl)imidazo[1,2-b]-pyridazin-2-amine |
| N-(3-(6-amino-2-méthyl-4-pyrimidinyl)imidazo[1,2-b]pyridazin-2-yl)-1H-pyrazolo-[4,3-c]pyridin-3-amine |
| N-(2-méthyl-6-(2-(1 H-pyrazol-3-ylamino)imidazo[1,2-a]pyridin-3-yl)-4-pyrimidinyl)acétamide |
| 3-(6-amino-2-méthyl-4-pyrimidinyl)-N-(1-(2-(diméthylamino)éthyl)-1H-pyrazol-3-yl)imidazo[1,2-b]pyridazin-2-amine |
| N-(6-(2-((1-(2-(diméthylamino)éthyl)-1H-pyrazol-3-yl)amino)imidazo[1,2-b]-pyridazin-3-yl)-2-méthyl-4-pyrimidinyl)acétamide |
| N-(2-méthyl-6-(2-((1-méthyl-1H-pyrazol-3-yl)amino)imidazo[1,2-a]pyridin-3-yl)-4-pyrimidinyl)acétamide |
| 3-(2-méthyl-6-((6-(4-morpholinyl)-3-pyridazinyl)amino)-4-pyrimidinyl)-N-(1-méthyl-1 H-pyrazol-3-yl)imidazo[1,2-b]pyridazin-2-amine |
| N-(3-(6-amino-2-méthyl-4-pyrimidinyl)imidazo[1,2-b]pyridazin-2-yl)-1H-pyrazolo-[3,4-b]pyridin-3-amine |
| 3-(6-amino-2-méthyl-4-pyrimidinyl)-N-(3-(4-méthyl-1-pipérazinyl)phényl)imidazo-[1,2-b]pyridazin-2-amine |
| N-(3-((3-(6-(acétylamino)-2-méthyl-4-pyrimidinyl)imidazo[1,2-b]pyridazin-2-yl)-amino)phényl)-acétamide |
| 3-(6-amino-2-méthyl-4-pyrimidinyl)-6-(1-(2-méthoxyéthyl)-1H-pyrazol-4-yl)-N-(1-méthyl-1 H-pyrazol-3-yl)imidazo[1,2-a]pyridin-2-amine |
| 1-(4-((3-(4-amino-6-méthyl-1,3,5-triazin-2-yl)imidazo[1,2-a]pyridin-2-yl)amino)-phényl)-3-méthylurée |
| 3-(4-amino-6-méthyl-1,3,5-triazin-2-yl)-N-1H-pyrazol-3-yl-6-(3-pyridinyl)imidazo-[1,2-a]pyridin-2-amine |
| 3-(4-amino-6-méthyl-1,3,5-triazin-2-yl)-N-6∼-phényl-N-2∼-1H-pyrazol-3-yl-imidazo[1,2-a]pyridine-2,6-diamine |
| 3-(4-amino-6-méthyl-1,3,5-triazin-2-yl)-N-6∼-(4-méthoxyphényl)-N-2∼-1H-pyrazol-3-ylimidazo[1,2-a]pyridine-2,6-diamine |
| 3-(4-amino-6-méthyl-1,3,5-triazin-2-yl)-7-méthoxy-N-1H-pyrazol-3-ylimidazo-[1,2-a]pyridin-2-amine |
| 3-(4-amino-6-méthyl-1,3,5-triazin-2-yl)-N-1H-pyrazol-3-yl-6-(5-pyrimidinyl)-imidazo[1,2-a]pyridin-2-amine |
| 3-(4-méthyl-6-(méthylamino)-1,3,5-triazin-2-yl)-N-1H-pyrazol-3-yl-6-(5-pyrimidinyl)imidazo[1,2-a]pyridin-2-amine |
| 3-(4-amino-6-méthyl-1,3,5-triazin-2-yl)-6-(3-fluorophényl)-N-1H-pyrazol-3-ylimidazo [1,2-a]pyridin-2-amine |
| 4-(3-(4-amino-6-méthyl-1,3,5-triazin-2-yl)-2-(1H-pyrazol-3-ylamino)imidazo-[1,2-a]pyridin-6-yl)-N-méthylbenzamide |
| 3-(4-amino-6-méthyl-1,3,5-triazin-2-yl)-N-2∼-1H-pyrazol-3-yl-N∼6∼-3-pyridinyl-imidazo[1,2-a]pyridine-2,6-diamine |
| N-(4-(3-(4-amino-6-méthyl-1,3,5-triazin-2-yl)-2-(1H-pyrazol-3-ylamino)imidazo-[1,2-a]pyridin-6-yl)phényl)acétamide |
| 3-(4-amino-6-méthyl-1,3,5-triazin-2-yl)-N∼6∼-(3-méthoxyphényl)-N∼2∼-1H-pyrazol-3-ylimidazo[1,2-a]pyridine-2,6-diamine |
| 3-(4-amino-6-méthyl-1,3,5-triazin-2-yl)-N∼2∼-1H-pyrazol-3-yl-N∼6∼-4-pyridinyl-imidazo[1,2-a]pyridine-2,6-diamine |
| 3-(4-amino-6-méthyl-1,3,5-triazin-2-yl)-N∼2∼-1H-pyrazol-3-yl-N∼6∼-5-pyrimidinylimidazo[1,2-a]pyridine-2,6-diamine |
| N-(4-((3-(4-amino-6-méthyl-1,3,5-triazin-2-yl)-2-(1H-pyrazol-3-ylamino)imidazo-[1,2-a]pyridin-6-yl)amino)phényl)acétamide |
| 3-(4-amino-6-méthyl-1,3,5-triazin-2-yl)-6-(2-méthyl-3-pyridinyl)-N-1H-pyrazol-3-ylimidazo[1,2-a]pyridin-2-amine |
| 3-(4-amino-6-méthyl-1,3,5-triazin-2-yl)-N∼6∼-(2-méthyl-4-pyridinyl)-N∼2∼-1H-pyrazol-3-ylimidazo[1,2-a]pyridine-2,6-diamine |
| N-méthyl-4-(3-(4-méthyl-6-(méthylamino)-1,3,5-triazin-2-yl)-2-(1H-pyrazol-3-yl-amino)imidazo[1,2-a]pyridin-6-yl)benzamide |
| 3-(4-amino-6-méthyl-1,3,5-triazin-2-yl)-6-(4-méthyl-3-pyridinyl)-N-1H-pyrazol-3-ylimidazo[1,2-a]pyridin-2-amine |
| 3-(4-amino-6-méthyl-1,3,5-triazin-2-yl)-6-(2,6-diméthyl-4-pyridinyl)-N-1H-pyrazol-3-yl-imidazo[1,2-a]pyridin-2-amine |
| 4-(3-(4-amino-6-méthyl-1,3,5-triazin-2-yl)-2-(1H-pyrazol-3-ylamino)imidazo-[1,2-a]pyridin-6-yl)-N-(1-méthyléthyl)benzamide |
| 6-(3-(4-amino-6-méthyl-1,3,5-triazin-2-yl)-2-(1H-pyrazol-3-ylamino)imidazo-[1-,2-a]pyridin-6-yl)-3,4-dihydro-1(2H)-isoquinoléinone |
| 4-(3-(4-amino-6-méthyl-1,3,5-triazin-2-yl)-2-(1H-pyrazol-3-ylamino)imidazo-[1,2-a]pyridin-6-yl)-N-éthylbenzamide |
| acide 4-(3-(4-amino-6-méthyl-1,3,5-triazin-2-yl)-2-(1H-pyrazol-3-ylamino)imidazo-[1,2-a]pyridin-6-yl)-3-méthylbenzoïque |
| 3-(2-méthyl-6-(méthylamino)-4-pyrimidinyl)-N-1H-pyrazol-3-yl-6-(5-pyrimidinyl)-imidazo[1,2-b]pyridazin-2-amine |
| 3-(4-amino-6-méthyl-1,3,5-triazin-2-yl)-6-(4-méthyl-5-pyrimidinyl)-N-1H-pyrazol-3-ylimidazo[1,2-a]pyridin-2-amine |
| 4-(3-(4-amino-6-méthyl-1,3,5-triazin-2-yl)-2-(1H-pyrazol-3-ylamino)imidazo-[1,2-a]pyridin-6-yl)-N,3-diméthylbenzamide |
| 5-(3-(4-amino-6-méthyl-1,3,5-triazin-2-yl)-2-(1H-pyrazol-3-ylamino)imidazo-[1,2-a]pyridin-6-yl)-N-méthyl-2-pyridinecarboxamide |
| 3-(4-amino-6-méthyl-1,3,5-triazin-2-yl)-2-(1H-pyrazol-3-ylamino)imidazo-[1,2-a]pyridin-7-ol |
| 4-(3-(4-amino-6-méthyl-1,3,5-triazin-2-yl)-2-(1H-pyrazol-3-ylamino)imidazo-[1,2-a]pyridin-7-yl)-3,6-dihydro-1(2H)-pyridinecarboxylate de tert-butyle |
| 3-(4-amino-6-méthyl-1,3,5-triazin-2-yl)-N-1H-pyrazol-3-yl-7-(1,2,3,6-tétrahydro-4-pyridinyl)imidazo[1,2-a]pyridin-2-amine |
| 3-(4-amino-6-méthyl-1,3,5-triazin-2-yl)-7-(2-méthoxyéthoxy)-N-1H-pyrazol-3-yl-imidazo[1,2-a]pyridin-2-amine |
| 3-(4-amino-6-méthyl-1,3,5-triazin-2-yl)-N-1H-pyrazol-3-yl-6-(1H-pyrrolo[2,3-b]-pyridin-5-yl)imidazo[1,2-a]pyridin-2-amine |
| 4-(3-(4-amino-6-methyl-1,3,5-triazin-2-yl)-2-(1H-pyrazol-3-ylamino)imidazo-[1,2-a]pyridin-7-yl)-N-méthylbenzamide |
| 3-(4-amino-6-méthyl-1,3,5-triazin-2-yl)-N-1H-pyrazol-3-yl-7-(2-(1-pyrrolidinyl)-éthoxy)imidazo[1,2-a]pyridin-2-amine |
| 4-(3-(6-amino-2-méthyl-4-pyrimidinyl)-2-(1H-pyrazol-3-ylamino)imidazo[1,2-b]-pyridazin-6-yl) -N,N-diméthyl-1-pipérazinecarboxamide |
| 3-(6-amino-2-méthyl-4-pyrimidinyl)-N-2-pyridinylimidazo[1,2-b]pyridazin-2-amine |
| 3-(6-amino-2-méthyl-4-pyrimidinyl)-N-(1-éthyl-1H-pyrazol-3-yl)imidazo[1,2-b]-pyridazin-2-amine |
| N-(3-(6-amino-2-méthyl-4-pyrimidinyl)imidazo[1,2-b]pyridazin-2-yl)-3-quinoléinamine |
| N-(3-(6-amino-2-méthyl-4-pyrimidinyl)imidazo[1,2-b]pyridazin-2-yl)-3-isoquinoléinamine |
| N-(3-(6-amino-2-méthyl-4-pyrimidinyl)imidazo[1,2-b]pyridazin-2-yl)-2-quinoléinamine |
| 3-(6-amino-2-méthyl-4-pyrimidinyl)-N-(1-méthyl-1H-pyrazol-3-yl)imidazo[1,2-b]-pyridazin-2-amine |
| 3-((3-(4-amino-6-méthyl-1,3,5-triazin-2-yl)imidazo[1,2-a]pyridin-2-yl)amino)-N-méthyl-1 H-pyrazole-1-carboxamide |
| 5-((3-(4-amino-6-méthyl-1,3,5-triazin-2-yl)imidazo[1,2-a]pyridin-2-yl)amino)-N-méthyl-1 H-pyrazole-1-carboxamide |
| 3-((3-(4-amino-6-méthyl-1,3,5-triazin-2-yl)imidazo[1,2-a]pyridin-2-yl)amino)-N-(2-hydroxyéthyl)-1H-pyrazole-1-carboxamide |
| 5-((3-(4-amino-6-méthyl-1,3,5-triazin-2-yl)imidazo[1,2-a]pyridin-2-yl)amino)-N-(2-hydroxyéthyl)-1H-pyrazole-1-carboxamide |
| 3-(4-amino-6-méthyl-1,3,5-triazin-2-yl)-N-(1-(4-morpholinylcarbonyl)-1H-pyrazol-3-yl)imidazo[1,2-a]pyridin-2-amine |
| 3-(4-amino-6-méthyl-1,3,5-triazin-2-yl)-N-(1-(4-morpholinylcarbonyl)-1H-pyrazol-5-yl)-imidazo[1,2-a]pyridin-2-amine |
| 5-((3-(4-amino-6-méthyl-1,3,5-triazin-2-yl)imidazo[1,2-a]pyridin-2-yl)amino)-N-3-pyridinyl-1 H-pyrazole-1-carboxamide |
| 4-((3-(4-amino-6-méthyl-1,3,5-triazin-2-yl)imidazo[1,2-a]pyridin-2-yl)amino)-N-méthyl-1 H-pyrazole-1-carboxamide |
| 3-((3-(4-amino-6-méthyl-1,3,5-triazin-2-yl)imidazo[1,2-a]pyridin-2-yl)amino)-N-phényl-1H-pyrazole-1-carboxamide |
| 5-((3-(4-amino-6-méthyl-1,3,5-triazin-2-yl)imidazo[1,2-a]pyridin-2-yl)amino)-N-phényl-1H-pyrazole-1-carboxamide |
| 3-(4-amino-6-méthyl-1,3,5-triazin-2-yl)-N-1H-pyrazol-4-ylimidazo[1,2-a]pyridin-2-amine |
| 3-((3-(4-amino-6-méthyl-1,3,5-triazin-2-yl)imidazo[1,2-a]pyridin-2-yl)amino)-1H-pyrazole-1-carboxylate de méthyle |
| 5-((3-(4-amino-6-méthyl-1,3,5-triazin-2-yl)imidazo[1,2-a]pyridin-2-yl)amino)-1H-pyrazole-1-carboxylate de méthyle |
| 4-((3-(4-amino-6-méthyl-1,3,5-triazin-2-yl)imidazo[1,2-a]pyridin-2-yl)amino)-1 H-pyrazole-1-carboxylate de phényle |
| 3-((3-(6-amino-2-méthyl-4-pyrimidinyl)imidazo[1,2-a]pyridin-2-yl)amino)-N-phényl-1H-pyrazole-1-carboxamide |
| 5-((3-(6-amino-2-méthyl-4-pyrimidinyl)imidazo[1,2-a]pyridin-2-yl)amino)-N-phényl-1H-pyrazole-1-carboxamide |
| 3-((3-(6-amino-2-méthyl-4-pyrimidinyl)imidazo[1,2-a]pyridin-2-yl)amino)-N-méthyl-1H-pyrazole-1-carboxamide |
| 5-((3-(6-amino-2-méthyl-4-pyrimidinyl)imidazo[1,2-a]pyridin-2-yl)amino)-N-méthyl-1H-pyrazole-1-carboxamide |
| 4-((3-(6-amino-2-méthyl-4-pyrimidinyl)imidazo[1,2-a]pyridin-2-yl)amino)-N-phényl-1H-pyrazole-1-carboxamide |
| 4-((3-(6-amino-2-méthyl-4-pyrimidinyl)imidazo[1,2-a]pyridin-2-yl)amino)-N-méthyl-1 H-pyrazole-1-carboxamide |
| 3-(6-amino-2-méthyl-4-pyrimidinyl)-N-(-(2-pyridinyl)-1H-pyrazol-3-yl)imidazo-[1,2-a]pyridin-2-amine |
| 3-(6-amino-2-méthyl-4-pyrimidinyl)-N-(1-(méthylsulfonyl)-1H-pyrazol-3-yl)-imidazo[1,2-a]pyridin-2-amine |
| 3-(6-amino-2-méthyl-4-pyrimidinyl)-N-(1-(1,3-benzoxazol-2-yl)-1H-pyrazol-5-yl)-imidazo[[1,2-a]pyridin-2-amine |
| 3-((3-(6-amino-2-méthyl-4-pyrimidinyl)imidazo[1,2-a]pyridin-2-yl)amino)-1H-pyrazole-1-carboxylate de phényle |
| 3-(6-amino-2-méthyl-4-pyrimidinyl)-N-(1-(1H-benzimidazol-2-yl)-1H-pyrazol-3-yl)imidazo[1,2-a]pyridin-2-amine |
| 3-((3-(6-amino-2-méthyl-4-pyrimidinyl)imidazo[1,2-a]pyridin-2-yl)amino)-N-(1-méthyléthyl)-1H-pyrazole-1-carboxamide |
| 3-((3-(6-amino-2-méthyl-4-pyrimidinyl)imidazo[1,2-a]pyridin-2-yl)amino)-N-benzyl-1 H-pyrazole-1-carboxamide |
| 4-(((3-((3-(6-amino-2-méthyl-4-pyrimidinyl)imidazo[1,2-a]pyridin-2-yl)amino)-1H-pyrazol-1-yl)carbonyl)amino)benzoate de méthyle |
| 3-((3-(6-amino-2-méthyl-4-pyrimidinyl)imidazo[1,2-a]pyridin-2-yl)amino)-N-éthyl-1 H-pyrazole-1-carboxamide |
| 3-((3-(6-amino-2-méthyl-4-pyrimidinyl)imidazo[1,2-a]pyridin-2-yl)amino)-N-(2-méthoxyphényl)-1H-pyrazole-1-carboxamide |
| 3-(6-amino-2-méthyl-4-pyrimidinyl)-N-1H-pyrazol-4-ylimidazo[1,2-a]pyridin-2-amine |
| 3-((3-(6-amino-2-méthyl-4-pyrimidinyl)imidazo[1,2-a]pyridin-2-yl)amino)-N-(2-méthylphényl)-1H-pyrazole-1-carboxamide |
| 3-((3-(6-amino-2-méthyl-4-pyrimidinyl)imidazo[1,2-a]pyridin-2-yl)amino)-N-(4-méthoxyphényl)-1H-pyrazole-1-carboxamide |
| 3-((3-(6-amino-2-méthyl-4-pyrimidinyl)imidazo[1,2-b]pyridazin-2-yl)amino)-N-phényl-1H-pyrazole-1-carboxamide, acide trifluoroacétique |
| 3-((3-(6-amino-2-méthyl-4-pyrimidinyl)imidazo[1,2-b]pyridazin-2-yl)amino)-N-méthyl-1 H-pyrazole-1-carboxamide |
| 5-((3-(6-amino-2-méthyl-4-pyrimidinyl)imidazo[1,2-b]pyridazin-2-yl)amino)-N-méthyl-1 H-pyrazole-1-carboxamide |
| 4-((3-(6-amino-2-méthyl-4-pyrimidinyl)imidazo[1,2-a]pyridin-2-yl)amino)-1H-pyrazole-1-carboxylate de méthyle |
| 3-(6-amino-2-méthyl-4-pyrimidinyl)-N-(1-(1,3-benzoxazol-2-yl)-1H-pyrazol-3-yl)-imidazo[1,2-b]pyridazin-2-amine |
| 5-((3-(6-amino-2-méthyl-4-pyrimidinyl)imidazo[1,2-b]pyridazin-2-yl)amino)-1H-pyrazole-1-carboxylate de méthyle |
| 3-((3-(6-amino-2-méthyl-4-pyrimidinyl)imidazo[1,2-b]pyridazin-2-yl)amino)-N-(2-méthylphényl)-1H-pyrazole-1-carboxamide |
| 3-((3-(6-amino-2-méthyl-4-pyrimidinyl)imidazo[1,2-b]pyridazin-2-yl)amino)-N-(2-fluorophényl)-1H-pyrazole-1-carboxamide |
| 3-(6-amino-2-méthyl-4-pyrimidinyl)-N-(1-(1,3-benzoxazol-2-yl)-1H-pyrazol-5-yl)-imidazo[1,2-b]pyridazin-2-amine |
| 3-((3-(6-amino-2-méthyl-4-pyrimidinyl)imidazo[1,2-b]pyridazin-2-yl)amino)-N-(4-cyanophényl)-1H-pyrazole-1-carboxamide |
| 3-((3-(6-amino-2-méthyl-4-pyrimidinyl)imidazo[1,2-b]pyridazin-2-yl)amino)-N-(2-(trifluorométhyl)phényl)-1H-pyrazole-1-carboxamide |
| 3-((3-(6-amino-2-méthyl-4-pyrimidinyl)imidazo[1,2-b]pyridazin-2-yl)amino)-N-(3-méthylphényl)-1H-pyrazole-1-carboxamide |
| 3-((3-(6-amino-2-méthyl-4-pyrimidinyl)imidazo[1,2-b]pyridazin-2-yl)amino)-N-(2,5-difluorophényl)-1H-pyrazole-1-carboxamide |
| 3-((3-(6-amino-2-méthyl-4-pyrimidinyl)imidazo[1,2-b]pyridazin-2-yl)amino)-N-(3,5-difluorophényl)-1H-pyrazole-1-carboxamide |
| 3-((3-(6-amino-2-méthyl-4-pyrimidinyl)imidazo[1,2-b]pyridazin-2-yl)amino)-N-(1-méthyléthyl)-1 H-pyrazole-1-carboxamide |
| 3-((3-(6-amino-2-méthyl-4-pyrimidinyl)imidazo[1,2-b]pyridazin-2-yl)amino)-N-(4-méthylphényl)-1H-pyrazole-1-carboxamide |
| 3-((3-(6-amino-2-méthyl-4-pyrimidinyl)imidazo[1,2-b]pyridazin-2-yl)amino)-N-(3,5-dichlorophényl)-1H-pyrazole-1-carboxamide |
| 3-((3-(6-amino-2-méthyl-4-pyrimidinyl)imidazo[1,2-b]pyridazin-2-yl)amino)-N-(2-chloro-5-(trifluorométhyl)phényl)-1H-pyrazole-1-carboxamide |
| 3-((3-(6-amino-2-méthyl-4-pyrimidinyl)imidazo[1,2-b]pyridazin-2-yl)amino)-N-(2,5-dichlorophényl)-1H-pyrazole-1-carboxamide |
| 3-((3-(6-amino-2-méthyl-4-pyrimidinyl)imidazo[1,2-b]pyridazin-2-yl)amino)-N-(2,3-dichlorophényl)-1H-pyrazole-1-carboxamide |
| 3-((3-(6-amino-2-méthyl-4-pyrimidinyl)imidazo[1,2-b]pyridazin-2-yl)amino)-N-(2-chloro-4-(trifluorométhyl)phényl)-1H-pyrazole-1-carboxamide |
| 3-((3-(6-(acétylamino)-2-méthyl-4-pyrimidinyl)imidazo[1,2-b]pyridazin-2-yl)-amino)-N-(2-méthylphényl)-1H-pyrazole-1-carboxamide |
| 3-(6-amino-2-méthyl-4-pyrimidinyl)-N-(1-(1H-benzimidazol-2-yl)-1H-pyrazol-3-yl)imidazo[1,2-b]pyridazin-2-amine |
| 3-(6-amino-2-méthyl-4-pyrimidinyl)-N-(1-(1H-imidazol-2-yl)-1H-pyrazol-3-yl)-imidazo[1,2-b]pyridazin-2-amine |
| 3-(6-amino-2-méthyl-4-pyrimidinyl)-N-(1-(méthylsulfonyl)-1H-pyrazol-3-yl)-imidazo[1,2-b]pyridazin-2-amine |
| 3-((3-(6-amino-2-méthyl-4-pyrimidinyl)imidazo[1,2-b]pyridazin-2-yl)amino)-1H-pyrazole-1-carboxamide |
| 5-((3-(6-amino-2-méthyl-4-pyrimidinyl)imidazo[1,2-b]pyridazin-2-yl)amino)-1H-pyrazole-1-carboxamide |
| 3-(6-amino-2-méthyl-4-pyrimidinyl)-N-(1-(1-azétidinylcarbonyl)-1H-pyrazol-3-yl)-imidazo[1,2-b]pyridazin-2-amine |
| 3-(6-amino-2-méthyl-4-pyrimidinyl)-N-(1-(1-azétidinylcarbonyl)-1H-pyrazol-5-yl)-imidazo[1,2-b]pyridazin-2-amine |
| 3-((3-(6-amino-2-méthyl-4-pyrimidinyl)imidazo[1,2-b]pyridazin-2-yl)amino)-N-(2-(méthylamino)éthyl)-1H-pyrazole-1-carboxamide |
| 5-((3-(6-amino-2-méthyl-4-pyrimidinyl)imidazo[1,2-b]pyridazin-2-yl)amino)-N-(2-(méthylamino)éthyl)-1H-pyrazole-1-carboxamide |
| 3-((3-(6-amino-2-méthyl-4-pyrimidinyl)imidazo[1,2-b]pyridazin-2-yl)amino)-N,N-diméthyl-1 H-pyrazole-1-carboxamide |
| 5-((3-(6-amino-2-méthyl-4-pyrimidinyl)imidazo[1,2-b]pyridazin-2-yl)amino)-N,N-diméthyl-1H-pyrazole-1-carboxamide |
| 3 -(6-amino-2-méthyl-4-pyrimidinyl)-N-(1-(1-pipérazinylcarbonyl)-1H-pyrazol-3-yl)imidazo-[1,2-b]pyridazin-2-amine |
| 5-((3-(6-amino-2-méthyl-4-pyrimidinyl)imidazo[1,2-b]pyridazin-2-yl)amino)-N-(2-hydroxyéthyl)-1H-pyrazole-1-carboxamide |
| 3-((3-(6-amino-2-méthyl-4-pyrimidinyl)imidazo[1,2-b]pyridazin-2-yl)amino)-N-(2-méthoxyéthyl)-1H-pyrazole-1-carboxamide |
| 5-((3-(6-amino-2-méthyl-4-pyrimidinyl)imidazo[1,2-b]pyridazin-2-yl)amino)-N-(2-méthoxyéthyl)-1H-pyrazole-1-carboxamide |
| 5-((3-(6-amino-2-méthyl-4-pyrimidinyl)imidazo[1,2-b]pyridazin-2-yl)amino)-N-(2-méthylpropyl)-1H-pyrazole-1-carboxamide |
| 3-(6-amino-2-méthyl-4-pyrimidinyl)-N-1H-pyrazol-3-yl-6-(5-pyrimidinyl)imidazo-[1,2-b]pyridazin-2-amine |
| 3-(6-amino-2-méthyl-4-pyrimidinyl)-N-(1-(1-phényléthyl)-1H-pyrazol-3-yl)-imidazo[1,2-b]pyridazin-2-amine |
| 3-(6-amino-2-méthyl-4-pyrimidinyl)-N-(1-benzyl-1H-pyrazol-3-yl)imidazo[1,2-b]-pyridazin-2-amine |
| 3-(6-amino-2-méthyl-4-pyrimidinyl)-N-(1-(3-pyridinylméthyl)-1H-pyrazol-3-yl)-imidazo[1,2-b]pyridazin-2-amine |
| 7-((3-(6-amino-2-méthyl-4-pyrimidinyl)imidazo[1,2-a]pyridin-2-yl)amino)-2H-1,4-benzoxazin-3(4H)-one |
| 1-(5-((3-(6-amino-2-méthyl-4-pyrimidinyl)imidazo[1,2-b]pyridazin-2-yl)amino)-2-pyridinyl)-3-azétidinol |
| 2-(1-(5-((3-(6-amino-2-méthyl-4-pyrimidinyl)imidazo[1,2-b]pyridazin-2-yl)amino)-2-pyridinyl)-3-azétidinyl)-2-propanol |
| N-(3-(4-amino-6-méthyl-1,3,5-triazin-2-yl)imidazo[1,2-a]pyridin-2-yl)-7-quinoléinamine |
| N-(3-(4-amino-6-méthyl-1,3,5-triazin-2-yl)imidazo[1,2-a]pyridin-2-yl)-6-quinoléinamine |
| N-(3-(4-amino-6-méthyl-1,3,5-triazin-2-yl)imidazo[1,2-a]pyridin-2-yl)-7-isoquinoléinamine |
| 4-(3-(4-amino-6-méthyl-1,3,5-triazin-2-yl)-2-(7-quinoléinylamino)imidazo[1,2-a]-pyridin-6-yl)-N,N-diméthyl-1-pipérazinecarboxamide |
| N-(3-(4-amino-6-méthyl-1,3,5-triazin-2-yl)-6-(4-(méthylsulfonyl)-1-pipérazinyl)-imidazo[1,2-a]pyridin-2-yl)-7-quinoléinamine |
| N-(3-(4-amino-6-méthyl-1,3,5-triazin-2-yl)-6-(1-pipérazinyl)-imidazo[1,2-a]-pyridin-2-yl)-7-quinoléinamine |
| 4-(3-(4-(acétylamino)-6-méthyl-1,3,5-triazin-2-yl)-2-((6-méthoxy-3-pyridinyl)-amino)imidazo[1,2-a]pyridin-6-yl)-N,N-diméthyl-1-pipérazinecarboxamide |
| 3-(4-amino-6-méthyl-1,3,5-triazin-2-yl)-N-(6-éthoxy-3-pyridinyl)-imidazo[1,2-a]-pyridin-2-amine |
| N-(3-(4-amino-6-méthyl-1,3,5-triazin-2-yl)imidazo[1,2-a]pyridin-2-yl)-1H-indazol-3-amine |
| 4-(3-(4-amino-6-méthyl-1,3,5-triazin-2-yl)-2-((5-fluoro-6-méthoxy-3-pyridinyl)-amino)imidazo[1,2-a]pyridin-6-yl)-N,N-diméthyl-1-pipérazinecarboxamide |
| 4-(3-(4-amino-6-méthyl-1,3,5-triazin-2-yl)-2-((6-méthoxy-3-pyridinyl)amino)-imidazo[1,2-a]pyridin-6-yl)-N-méthyl-1-pipérazinecarboxamide |
| 6-(4-acétyl-1-pipérazinyl)-3-(4-amino-6-méthyl-1,3,5-triazin-2-yl)-N-(6-méthoxy-3-pyridinyl)imidazo[1,2-a]pyridin-2-amine |
| (5-(3-(4-amino-6-méthyl-1,3,5-triazin-2-yl)-2-(1H-pyrazol-5-ylamino)imidazo-[1,2-a]pyridin-6-yl)-2-méthoxyphényl)méthanol |
| 3-(4-amino-6-méthyl-1,3,5-triazin-2-yl)-N-1H-pyrazol-5-yl-6-(4-pyridinyl)imidazo-[1,2-a]pyridin-2-amine |
| 3-(4-amino-6-méthyl-1,3,5-triazin-2-yl)-6-(3,6-dihydro-2H-pyran-4-yl)-N-1H-pyrazol-5-ylimidazo[1,2-a]pyridin-2-amine |
| 3-(4-amino-6-méthyl-1,3,5-triazin-2-yl)-6-(2-méthyl-4-pyridinyl)-N-1H-pyrazol-5-ylimidazo[1,2-a]pyridin-2-amine |
| 3-(4-amino-6-méthyl-1,3,5-triazin-2-yl)-6-(1-méthyl-1H-imidazol-5-yl)-N-1H-pyrazol-5-ylimidazo[1,2-a]pyridin-2-amine |
| 4-(3-(4-amino-6-méthyl-1,3,5-triazin-2-yl)-2-(1H-pyrazol-5-ylamino)imidazo-[1,2-a]pyridin-6-yl)-1,2-butanediol |
| 3-(4-amino-6-méthyl-1,3,5-triazin-2-yl)-6-(1-(1-méthyléthyl)-1H-pyrazol-4-yl)-N-1H-pyrazol-5-ylimidazo[1,2-a]pyridin-2-amine |
| 3-(4-amino-6-méthyl-1,3,5-triazin-2-yl)-2-(1H-pyrazol-5-ylamino)imidazo[1,2-a]-pyridine-6-carboxamide |
| N-((3-(4-amino-6-méthyl-1,3,5-triazin-2-yl)-2-(1H-pyrazol-5-ylamino)imidazo-[1,2-a]pyridin-6-yl)méthyl)acétamide |
| 3-(2-méthyl-6-((2,2,2-trifluoroéthyl)amino)-4-pyrimidinyl)-N-1H-pyrazol-5-yl-imidazo[1,2-b]pyridazin-2-amine |
| 3-(2-méthyl-6-(2-pyrazinylamino)-4-pyrimidinyl)-N-(1-méthyl-1H-pyrazol-3-yl)-imidazo[1,2-b]pyridazin-2-amine |
| 3-(2-méthyl-6-(3-pyridinylamino)-4-pyrimidinyl)-N-1H-pyrazol-3-ylimidazo-[1,2-b]pyridazin-2-amine |
| 2-cyano-N-(2-méthyl-6-(2-((1-méthyl-1H-pyrazol-3-yl)amino)imidazo[1,2-b]-pyridazin-3-yl)-4-pyrimidinyl)acétamide |
| N-(2-méthyl-6-(2-((1-méthyl-1H-pyrazol-3-yl)amino)imidazo[1,2-b]pyridazin-3-yl)-4-pyrimidinyl)-1,3-oxazole-5-carboxamide |
| 3-(6-amino-2-méthyl-4-pyrimidinyl)-N-(1-méthyl-1H-pyrazol-3-yl)-6-(5-pyrimidinyl)imidazo[1,2-a]pyridin-2-amine |
| N-(4-(3-(6-amino-2-méthyl-4-pyrimidinyl)-2-((1-méthyl-1H-pyrazol-3-yl)amino)-imidazo[1,2-a]pyridin-6-yl)phényl)acétamide |
| 3-(4-amino-6-méthyl-1,3,5-triazin-2-yl)-N-(1-méthyl-1H-pyrazol-3-yl)-6-(7-quinoléinyl)imidazo[1,2-a]pyridin-2-amine |
| N-(4-(3-(6-amino-2-méthyl-4-pyrimidinyl)-2-((1-méthyl-1H-pyrazol-3-yl)amino)-imidazo[1,2-b]pyridazin-6-yl)benzyl)acétamide |
| 3-(4-amino-6-méthyl-1,3,5-triazin-2-yl)-N-(1-méthyl-1H-pyrazol-3-yl)-6-(5-pyrimidinyl)imidazo[1,2-a]pyridin-2-amine |
| 4-(3-(6-amino-2-méthyl-4-pyrimidinyl)-2-((1-méthyl-1H-pyrazol-3-yl)amino)-imidazo[1,2-b]pyridazin-6-yl)-N-méthylbenzamide |
| 3-(6-amino-2-méthyl-4-pyrimidinyl)-N-(1-(6-(2-méthoxyéthoxy)-4-pyrimidinyl)-1 H-pyrazol-3-yl)imidazo[1,2-b]pyridazin-2-amine |
| 3-(6-(3-isoxazolylamino)-2-méthyl-4-pyrimidinyl)-N-1H-pyrazol-3-ylimidazo-[1,2-b]pyridazin-2-amine |
| 2-cyano-N-(2-méthyl-6-(2-((1-méthyl-1H-pyrazol-3-yl)amino)-6-(5-pyrimidinyl)-imidazo[1,2-a]pyridin-3-yl)-4-pyrimidinyl)acétamide |
| 3-(6-((1-(2-méthoxyéthyl)-1H-pyrazol-4-yl)amino)-2-méthyl-4-pyrimidinyl)-N-1H-pyrazol-3-ylimidazo[1,2-b]pyridazin-2-amine |
| 3-(2-méthyl-6-((1-((3R)-tétrahydro-3-furanyl)-1H-pyrazol-4-yl)amino)-4-pyrimidinyl)-N-1H-pyrazol-3-ylimidazo[1,2-b]pyridazin-2-amine, 3-(2-méthyl-6-((1-(3S)-tétrahydro-3-furanyl)-1H-pyrazol-4-yl)amino)-4-pyrimidinyl)-N-1H-pyrazol-3-ylimidazo[1,2-b]pyridazin-2-amine |
| 3-(6-(imidazo[1,2-a]pyridin-2-ylamino)-2-méthyl-4-pyrimidinyl)-N-1H-pyrazol-3-ylimidazo[1,2-b]pyridazin-2-amine |
| 3-(6-(furo[3,2-b]pyridin-6-ylamino)-2-méthyl-4-pyrimidinyl)-N-1H-pyrazol-3-yl-imidazo[1,2-b]pyridazin-2-amine |
| 3-(6-amino-2-méthyl-4-pyrimidinyl)-N-1H-pyrazol-3-yl-6-(5-pyrimidinyl)imidazo-[1,2-a]pyridin-2-amine |
| 3-(2-méthyl-6-((1-((3R)-tétrahydro-3-furanyl)-1H-pyrazol-4-yl)amino)-4-pyrimidinyl)-N-1H-pyrazol-3-ylimidazo[1,2-a]pyridin-2-amine, 3-(2-méthyl-6-((1-((3S)-tétrahydro-3-furanyl)-1H-pyrazol-4-yl)amino)-4-pyrimidinyl)-N-1 H-pyrazol-3-ylimidazo[1,2-a]pyridin-2-amine |
| 3-(6-((6-(2-méthoxyéthoxy)-4-pyrimidinyl)amino)-2-méthyl-4-pyrimidinyl)-N-1H-pyrazol-3-ylimidazo[1,2-b]pyridazin-2-amine |
| 1-(3-((3-(6-amino-2-méthyl-4-pyrimidinyl)imidazo[1,2-b]pyridazin-2-yl)amino)-phényl)-3-cyclopropylurée |
| N-(3-((3-(6-amino-2-méthyl-4-pyrimidinyl)imidazo[1,2-a]pyridin-2-yl)amino)-phényl)acétamide |
| N-(3-((3-(6-amino-2-méthyl-4-pyrimidinyl)imidazo[1,2-a]pyridin-2-yl)amino)-phényl)-2-phénylacétamide |
| N-(3-((3-(6-amino-2-méthyl-4-pyrimidinyl)imidazo[1,2-a]pyridin-2-yl)amino)-phényl)benzamide |
| N-(3-((3-(6-amino-2-méthyl-4-pyrimidinyl)imidazo[1,2-a]pyridin-2-yl)amino)-phényl)-4-chlorobenzènesulfonamide |
| N-(3-((3-(6-amino-2-méthyl-4-pyrimidinyl)imidazo[1,2-a]pyridin-2-yl)amino)-phényl)méthanesulfonamide |
| 1-(3-((3-(6-amino-2-méthyl-4-pyrimidinyl)imidazo[1,2-a]pyridin-2-yl)amino)-phényl)-3-phénylurée |
| 1-(3-((3-(6-amino-2-méthyl-4-pyrimidinyl)imidazo[1,2-a]pyridin-2-yl)amino)-phényl)-3-(4-fluorophényl)urée |
| 1-(3-((3-(6-amino-2-méthyl-4-pyrimidinyl)imidazo[1,2-a]pyridin-2-yl)amino)-phényl)-3-benzylurée |
| 1-(3-((3-(6-amino-2-méthyl-4-pyrimidinyl)imidazo[1,2-a]pyridin-2-yl)amino)-phényl)-3-méthylurée |
| 1-(3-((3-(6-amino-2-méthyl-4-pyrimidinyl)imidazo[1,2-a]pyridin-2-yl)amino)-phényl)-3-éthylurée |
| 4-((3-(6-amino-2-méthyl-4-pyrimidinyl)imidazo[1,2-a]pyridin-2-yl)amino)-N-éthylbenzamide |
| 5-((3-(6-amino-2-méthyl-4-pyrimidinyl)imidazo[1,2-a]pyridin-2-yl)amino)-N-éthyl-2-pyridinecarboxamide |
| N-(3-((3-(6-amino-2-méthyl-4-pyrimidinyl)imidazo[1,2-b]pyridazin-2-yl)amino)-phényl)acétamide |
| N-(3-((3-(6-amino-2-méthyl-4-pyrimidinyl)imidazo[1,2-b]pyridazin-2-yl)amino)-phényl)propanamide |
| 3-((3-(6-amino-2-méthyl-4-pyrimidinyl)imidazo[1,2-a]pyridin-2-yl)amino)-N-méthylbenzamide |
| 3-((3-(6-amino-2-méthyl-4-pyrimidinyl)imidazo[1,2-a]pyridin-2-yl)amino)-N-éthylbenzamide |
| 3-((3-(6-amino-2-méthyl-4-pyrimidinyl)imidazo[1,2-a]pyridin-2-yl)amino)-N-(1-méthyléthyl)benzamide |
| 3-((3-(6-amino-2-méthyl-4-pyrimidinyl)imidazo[1,2-b]pyridazin-2-yl)amino)-N-méthylbenzamide |
| 3-((3-(6-amino-2-méthyl-4-pyrimidinyl)imidazo[1,2-b]pyridazin-2-yl)amino)-N-éthylbenzamide |
| 3-((3-(6-amino-2-méthyl-4-pyrimidinyl)imidazo[1,2-b]pyridazin-2-yl)amino)-N-phénylbenzamide |
| 3-((3-(6-amino-2-méthyl-4-pyrimidinyl)imidazo[1,2-b]pyridazin-2-yl)amino)-N-(1-méthyléthyl)benzamide |
| 5-((3-(6-amino-2-méthyl-4-pyrimidinyl)imidazo[1,2-a]pyridin-2-yl)amino)-N-méthyl-2-pyridinecarboxamide |
| 5-((3-(6-amino-2-méthyl-4-pyrimidinyl)imidazo[1,2-a]pyridin-2-yl)amino)-N-(1-méthyléthyl)-2-pyridinecarboxamide |
| 5-((3-(6-amino-2-méthyl-4-pyrimidinyl)imidazo[1,2-a]pyridin-2-yl)amino)-N-phényl-2-pyridinecarboxamide |
| 4-((3-(6-amino-2-méthyl-4-pyrimidinyl)imidazo[1,2-a]pyridin-2-yl)amino)-benzamide |
| 4-((3-(6-amino-2-méthyl-4-pyrimidinyl)imidazo[1,2-a]pyridin-2-yl)amino)-N-(2-(diéthylamino)éthyl)benzamide |
| 4-((3-(6-amino-2-méthyl-4-pyrimidinyl)imidazo[1,2-a]pyridin-2-yl)amino)-N-phénylbenzamide |
| 3-(4-amino-6-méthyl-1,3,5-triazin-2-yl)-6-(4-(méthylsulfonyl)phényl)-N-1H-pyrazol-3-ylimidazo[1,2-a]pyridin-2-amine |
| 3-(6-amino-2-méthyl-4-pyrimidinyl)-N-(1-(3-méthylbutanoyl)-1H-pyrazol-3-yl)-imidazo[1,2-b]pyridazin-2-amine |
| 5-((3-(6-amino-2-méthyl-4-pyrimidinyl)imidazo[1,2-b]pyridazin-2-yl)amino)-1H-pyrazole-1-carboxylate de 1-méthyléthyle |
| 3-((3-(6-amino-2-méthyl-4-pyrimidinyl)imidazo[1,2-b]pyridazin-2-yl)amino)-1 H-pyrazole-1-carboxylate de 1-méthyléthyle |
| 3-(6-amino-2-méthyl-4-pyrimidinyl)-N-(2-méthoxy-4-pyridinyl)imidazo[1,2-b]-pyridazin-2-amine |
| 3-(6-amino-2-méthyl-4-pyrimidinyl)-N-(1-(2-méthylpropanoyl)-1H-pyrazol-3-yl)-imidazo[1,2-b]pyridazin-2-amine |
| 3-(6-amino-2-méthyl-4-pyrimidinyl)-N-(1-(2-méthylpropanoyl)-1H-pyrazol-5-yl)-imidazo[1,2-b]pyridazin-2-amine |
| 4-(3-(4-amino-6-méthyl-1,3,5-triazin-2-yl)-2-(1H-pyrazol-3-ylamino)imidazo-[1,2-a]pyridin-6-yl)-N-méthylbenzènesulfonamide |
| 3-(4-amino-6-méthyl-1,3,5-triazin-2-yl)-6-(3-(méthylsulfonyl)phényl)-N-1H-pyrazol-3-ylimidazo[1,2-a]pyridin-2-amine |
| 3-(6-amino-2-méthyl-4-pyrimidinyl)-N-(1-(3,3 -diméthylbutanoyl)-1H-pyrazol-3-yl)imidazo[1,2-b]pyridazin-2-amine |
| 3-(4-amino-6-méthyl-1,3,5-triazin-2-yl)-N-(1-méthyl-1H-pyrazol-3-yl)-6-(4-(méthylsulfonyl)phényl)imidazo[1,2-a]pyridin-2-amine |
| 5-((3-(6-amino-2-méthyl-4-pyrimidinyl)imidazo[1,2-b]pyridazin-2-yl)amino)-1H-pyrazole-1-carboxylate de cyclohexyle |
| 3-((3-(6-amino-2-méthyl-4-pyrimidinyl)imidazo[1,2-b]pyridazin-2-yl)amino)-1H-pyrazole-1-carboxylate de cyclohexyle |
| 3-(6-amino-2-méthyl-4-pyrimidinyl)-N-(2-méthoxy-3-pyridinyl)imidazo[1,2-b]-pyridazin-2-amine |
| 3-((3-(6-amino-2-méthyl-4-pyrimidinyl)imidazo[1,2-b]pyridazin-2-yl)amino)-2(1H)-pyridinone |
| 3-(6-amino-2-méthyl-4-pyrimidinyl)-N-(1-(3-méthylbutyl)-1H-pyrazol-3-yl)-imidazo[[1,2-b]pyridazin-2-amine |
| 3-(6-amino-2-méthyl-4-pyrimidinyl)-N-(1-(2-méthoxyéthyl)-1H-pyrazol-3-yl)-imidazo[1,2-b]pyridazin-2-amine |
| 3-(6-amino-2-méthyl-4-pyrimidinyl)-6-(3-(méthylsulfonyl)phényl)-N-1H-pyrazol-3-ylimidazo[1,2-b]pyridazin-2-amine |
| N-(3-(4-amino-6-méthyl-1,3,5-triazin-2-yl)imidazo[1,2-a]pyridin-2-yl)-1H-indazol-4-amine |
| 3-(6-amino-2-méthyl-4-pyrimidinyl)-N-(6-méthoxy-3-pyridinyl)-6-(4-morpholinyl)-imidazo[1,2-b]pyridazin-2-amine |
| 3-(4-amino-6-méthyl-1,3,5-triazin-2-yl)-N-(6-méthoxy-3-pyridinyl)-6-(4-morpholinyl)imidazo[1,2-b]pyridazin-2-amine |
| 3-(6-amino-2-méthyl-4-pyrimidinyl)-N∼6∼-(2-méthoxyéthyl)-N-2∼-(6-méthoxy-3-pyridinyl)imidazo[1,2-b]pyridazine-2,6-diamine |
| 3-(6-amino-2-méthyl-4-pyrimidinyl)-N∼2∼-1H-indazol-4-yl-N∼6∼-(2-méthoxyéthyl)-imidazo[1,2-b]pyridazine-2,6-diamine |
| 1-(3-(6-amino-2-méthyl-4-pyrimidinyl)-2-((6-méthoxy-3-pyridinyl)amino)imidazo-[1,2-b]pyridazin-6-yl)-4-pipéridinol |
| 4-(3-(4-amino-6-méthyl-1,3,5-triazin-2-yl)-2-((6-méthoxy-3-pyridinyl)amino)-imidazo[1,2-a]pyridin-7-yl)-N,N-diméthyl-1-pipérazinecarboxamide |
| 4-(3-(4-amino-6-méthyl-1,3,5-triazin-2-yl)-2-((5-fluoro-6-méthoxy-3-pyridinyl)-amino)imidazo[1,2-a]pyridin-7-yl)-N,N-diméthyl-1-pipérazinecarboxamide |
| 3-(4-amino-6-méthyl-1,3,5-triazin-2-yl)-N-(5-fluoro-6-méthoxy-3-pyridinyl)-7-(4-(méthylsulfonyl)-1-pipérazinyl)imidazo[1,2-a]pyridin-2-amine |
| N-(5-((3-(4-amino-6-méthyl-1,3,5-triazin-2-yl)imidazo[1,2-a]pyridin-2-yl)amino-2-chloro-3-pyridinyl)méthanesulfonamide |
| N-(5-((3-(4-amino-6-méthyl-1,3,5-triazin-2-yl)imidazo[1,2-a]pyridin-2-yl)amino)-2-méthoxy-3-pyridinyl)méthanesulfonamide |
| 3-(6-amino-2-méthyl-4-pyrimidinyl)-N-1H-indazol-6-ylimidazo[1,2-b]pyridazin-2-amine |
| 3-(6-amino-2-méthyl-4-pyrimidinyl)-N-1H-benzimidazol-6-ylimidazo[1,2-b]-pyridazin-2-amine |
| N-(3-(6-amino-2-méthyl-4-pyrimidinyl)imidazo[1,2-a]pyridin-2-yl)-1H-benzimidazol-6-amine |
| 3-(4-amino-6-méthyl-1,3,5-triazin-2-yl)-N-(6-méthoxy-3-pyridinyl)-7-(1-méthyl-1H-pyrazol-4-yl)imidazo[1,2-a]pyridin-2-amine |
| 3-(4-amino-6-méthyl-1,3,5-triazin-2-yl)-N-(6-méthoxy-3-pyridinyl)-6-(1-méthyl-1 H-pyrazol-4-yl)imidazo[1,2-a]pyridin-2-amine |
| 3-(4-amino-6-méthyl-1,3,5-triazin-2-yl)-N-(6-méthoxy-3-pyridinyl)-6-(4-morpholinyl)imidazo[1,2-a]pyridin-2-amine |
| 3-(6-amino-2-méthyl-4-pyrimidinyl)-N-(6-méthoxy-3-pyridinyl)-6-(4-morpholinyl)-imidazo[1,2-a]pyridin-2-amine |
| 3-(4-amino-6-méthyl-1,3,5-triazin-2-yl)-N-(6-méthoxy-3-pyridinyl)-6-(3-pyridinyl)-imidazo[1,2-a]pyridin-2-amine |
| 4-(3-(4-amino-6-méthyl-1,3,5-triazin-2-yl)-2-((6-méthoxy-3-pyridinyl)amino)-imidazo[1,2-a]pyridin-6-yl)-N,N-diméthyl-1-pipérazinecarboxamide |
| 3-(4-amino-6-méthyl-1,3,5-triazin-2-yl)-N-(6-méthoxy-3-pyridinyl)-6-(4-(méthyl-sulfonyl)-1-pipérazinyl)imidazo[1,2-a]pyridin-2-amine |
| 2-(3-(4-amino-6-méthyl-1,3,5-triazin-2-yl)-2((6-méthoxy-3-pyridinyl)amino-imidazo[1,2-a]pyridin-6-yl)-2-propanol |
| 3-(4-amino-6-méthyl-1,3,5-triazin-2-yl)-N-(6-méthoxy-3-pyridinyl)-6-(1-pipérazinyl)imidazo[1,2-a]pyridin-2-amine, |
| 1-(3-(4-amino-6-méthyl-1,3,5-triazin-2-yl)-2-(7-quinoléinylamino)imidazo[1,2-a]-pyridin-6-yl)éthanone |
| 2-(3-(4-amino-6-méthyl-1,3,5-triazin-2-yl)-2-(7-quinoléinylamino)imidazo[1,2-a]-pyridin-6-yl)-2-propanol |
| 1-(3-(4-amino-6-méthyl-1,3,5-triazin-2-yl)-2-((5-fluoro-6-méthoxy-3-pyridinyl)-amino)imidazo[1,2-a]pyridin-6-yl)éthanone |
| 2-(3-(4-amino-6-méthyl-1,3,5-triazin-2-yl)-2-((5-fluoro-6-méthoxy-3-pyridinyl)-amino)imidazo[1,2-a]pyridin-6-yl)-2-propanol |
| 4-(3-(6-amino-2-méthyl-4-pyrimidinyl)-2-((5-fluoro-6-méthoxy-3-pyridinyl)-amino)imidazo[1,2-b]pyridazin-6-yl)-N,N-diméthyl-1-pipérazinecarboxamide |
| 1-(3-(6-amino-2-méthyl-4-pyrimidinyl)-2-((5-fluoro-6-méthoxy-3-pyridinyl)-amino)imidazo[1,2-a]pyridin-6-yl)éthanone |
| 2-(3-(6-amino-2-méthyl-4-pyrimidinyl)-2-((5-fluoro-6-méthoxy-3-pyridinyl)-amino)imidazo[1,2-a]pyridin-6-yl)-2-propanol |
| 4-(3-(6-amino-2-méthyl-4-pyrimidinyl)-2-((5-fluoro-6-méthoxy-3-pyridinyl)-amino)imidazo[1,2-a]pyridin-6-yl)-N,N-diméthyl-1-pipérazinecarboxamide |
| 3-(4-amino-6-méthyl-1,3,5-triazin-2-yl)-N-1H-pyrazol-3-yl-6-(trifluorométhyl)-imidazo[1,2-a]pyridin-2-amine |
| 3-(4-amino-6-méthyl-1,3,5-triazin-2-yl)-N-1H-pyrazol-3-yl-7-(trifluorométhyl)-imidazo[1,2-a]pyridin-2-amine |
| 3-(6-amino-2-méthyl-4-pyrimidinyl)-N-1H-pyrazol-3-yl-7-(trifluorométhyl)-imidazo[1,2-a]pyridin-2-amine |
| 3-(6-amino-2-méthyl-4-pyrimidinyl)-N-1H-pyrazol-3-yl-6-(trifluorométhyl)-imidazo[1,2-a]pyridin-2-amine |
| 1-(4-((3-(6-amino-2-méthyl-4-pyrimidinyl)imidazo[1,2-b]pyridazin-2-yl)amino)-phényl)-3-cyclopropylurée |
| 1-(4-((3-(6-amino-2-méthyl-4-pyrimidinyl)imidazo[1,2-b]pyridazin-2-yl)amino)-phényl)-3-éthylurée |
| N-(4-((3-(6-amino-2-méthyl-4-pyrimidinyl)imidazo[1,2-b]pyridazin-2-yl)amino)-phényl)-1-pipéridinecarboxamide |
| N-(4-((3-(6-amino-2-méthyl-4-pyrimidinyl)imidazo[1,2-b]pyridazin-2-yl)amino)-phényl-1-pyrrolidinecarboxamide |
| N-(4-((3-(6-amino-2-méthyl-4-pyrimidinyl)imidazo[1,2-b]pyridazin-2-yl)amino)-phényl)-1-azétidinecarboxamide |
| N-(4-((3-(6-amino-2-méthyl-4-pyrimidinyl)imidazo[1,2-b]pyridazin-2-yl)amino)-phényl)-4-méthyl-1-pipérazinecarboxamide |
| 1-(4-((3-(6-amino-2-méthyl-4-pyrimidinyl)imidazo[1,2-b]pyridazin-2-yl)amino)-phényl)-3-(2-méthoxyéthyl)urée |
| N-(4-((3-(6-amino-2-méthyl-4-pyrimidinyl)imidazo[1,2-b]pyridazin-2-yl)amino)-phényl)-4-morpholinecarboxamide |
| 4-acétyl-N-(4-((3-(6-amino-2-méthyl-4-pyrimidinyl)imidazo[1,2-b]pyridazin-2-yl)-amino)phényl)-1-pipérazinecarboxamide |
| 3-(6-amino-2-méthyl-4-pyrimidinyl)-N-1,3-thiazol-4-ylimidazo[1,2-b]pyridazin-2-amine |
| N-(4-((3-(6-amino-2-méthyl-4-pyrimidinyl)imidazo[1,2-a]pyridin-2-yl)amino)-phényl)-4-morpholinecarboxamide |
| 4-acétyl-N-(4-((3-(6-amino-2-méthyl-4-pyrimidinyl)imidazo[1,2-a]pyridin-2-yl)-amino)phényl)-1-pipérazinecarboxamide |
| N-(4-((3-(6-amino-2-méthyl-4-pyrimidinyl)imidazo[1,2-a]pyridin-2-yl)amino)-phényl)-2-méthyl-4-morpholinecarboxamide |
| 3-(4-((3-(6-amino-2-méthyl-4-pyrimidinyl)imidazo[1,2-a]pyridin-2-yl)amino)-phényl)-1-méthyl-1-(1-méthyléthyl)urée |
| N-(4-((3-(6-amino-2-méthyl-4-pyrimidinyl)imidazo[1,2-a]pyridin-2-yl)amino)-phényl)-3-méthyl-4-morpholinecarboxamide |
| 1-(4-((3-(6-amino-2-méthyl-4-pyrimidinyl)imidazo[1,2-a]pyridin-2-yl)amino)-phényl)-3-cyclopropylurée |
| 2-((3-(6-amino-2-méthyl-4-pyrimidinyl)imidazo[1,2-b]-pyridazin-2-yl)amino)-1H-imidazole-4-carboxylate d'éthyle |
| 3-(2-méthyl-4-pyrimidinyl)-6-(3-(méthylsulfonyl)phényl)-N-1H-pyrazol-3-yl-imidazo[1,2-b]pyridazin-2-amine |
| N-(3-(6-amino-2-méthyl-4-pyrimidinyl)imidazo[1,2-b]pyridazin-2-yl)-3H-imidazo[4,5-b]pyridin-5-amine |
| N-(2-méthyl-6-(2-((1-méthyl-1H-pyrazol-3-yl)amino)-6-(3-méthylsulfonyl)phényl-imidazo[1,2-a]pyridin-3-yl)-4-pyrimidinyl)acétamide |
| 3-(4-amino-6-méthyl-1,3,5-triazin-2-yl)-6-(3-(éthylsulfonyl)phényl)-N-1H-pyrazol-3-ylimidazo[1,2-a]pyridin-2-amine |
| 3-(6-amino-2-méthyl-4-pyrimidinyl)-6-(3,6-dihydro-2H-pyran-4-yl)-N-1H-pyrazol-3-ylimidazo[1,2-b]pyridazin-2-amine |
| N-(5-((3-(6-amino-2-méthyl-4-pyrimidinyl)imidazo[1,2-b]pyridazin-2-yl)amino)-3-pyridinyl)acétamide |
| 3-(6-amino-2-méthyl-4-pyrimidinyl)-N-(2-méthyl-1,3-thiazol-4-yl)imidazo[1,2-b]-pyridazin-2-amine |
| 3-(6-amino-2-méthyl-4-pyrimidinyl)-N-1,3-thiazol-5-ylimidazo[1,2-b]pyridazin-2-amine |
| 1-(4-((3-(6-amino-2-méthyl-4-pyrimidinyl)imidazo[1,2-a]pyridin-2-yl)amino)-phényl)-3-(2-méthylpropyl)urée |
| N-(4-((3-(6-amino-2-méthyl-4-pyrimidinyl)imidazo[1,2-a]pyridin-2-yl)amino)-phényl)-4,4-difluoro-1-pipéridinecarboxamide |
| (2S)-N-(4-((3-(6-amino-2-méthyl-4-pyrimidinyl)imidazo[1,2-a]pyridin-2-yl)amino)-phényl)-2-méthyl-1-pyrrolidine-carboxamide |
| N-(2-méthyl-6-(2-((1-méthyl-1H-pyrazol-3-yl)amino)-6-(2-méthyl-3-pyridinyl)-imidazo[1,2-a]pyridin-3-yl)-4-pyrimidinyl)acétamide |
| N-(6-(6-(1-(2-méthoxyéthyl)-1H-pyrazol-4-yl)-2-((1-méthyl-1H-pyrazol-3-yl)-amino)imidazo[1,2-a]pyridin-3-yl)-2-méthyl-4-pyrimidinyl)acétamide |
| N-(5-((3-(6-(acétylamino)-2-méthyl-4-pyrimidinyl)imidazo[1,2-b]pyridazin-2-yl)-amino)-3-pyridinyl)acétamide |
| N-(2-méthyl-6-(2-(1H-pyrazol-3-ylamino)-6-(5-pyrimidinyl)imidazo[1,2-a]pyridin-3-yl)-4-pyrimidinyl)acétamide |
| 8-fluoro-3-(2-méthyl-4-pyrimidinyl)-N-1H-pyrazol-3-ylimidazo[1,2-a]pyridin-2-amine |
| 3-(4-amino-6-méthyl-1,3,5-triazin-2-yl)-6-(4-(éthylsulfonyl)-phényl)-N-1H-pyrazol-3-ylimidazo[1,2-a]pyridin-2-amine |
| 3-(2,6-diméthyl-4-pyrimidinyl)-6-(3-(méthylsulfonyl)phényl)-N-1H-pyrazol-3-yl-imidazo[1,2-b]pyridazin-2-amine |
| N-(6-(8-fluoro-2-(1H-pyrazol-3-ylamino)imidazo[1,2-a]pyridin-3-yl)-2-méthyl-4-pyrimidinyl)acétamide |
| N-(3-(6-amino-2-méthyl-4-pyrimidinyl)-6-(3-(méthylsulfonyl)-phényl)imidazo-[1,2-b]pyridazin-2-yl)-1,3-benzènediamine |
| 3-((8-fluoro-3-(2-méthyl-4-pyrimidinyl)imidazo[1,2-a]pyridin-2-yl)amino)-N-(1-méthyléthyl)-1H-pyrazole-1-carboxamide |
| 5-((3-(6-(acétylamino)-2-méthyl-4-pyrimidinyl)-8-fluoro-imidazo[1,2-a]pyridin-2-yl)amino)-1H-pyrazole-1-carboxamide |
| 3-((3-(6-(acétylamino)-2-méthyl-4-pyrimidinyl)-8-fluoroimidazo[1,2-a]pyridin-2-yl)amino)-1H-pyrazole-1-carboxamide |
| N-(2-méthyl-6-(2-(1H-pyrazol-3-ylamino)-6-(5-pyrimidinyl)-imidazo[1,2-b]-pyridazin-3-yl)-4-pyrimidinyl)acétamide |
| 5-((8-fluoro-3-(2-méthyl-4-pyrimidinyl)imidazo[1,2-a]pyridin-2-yl)amino)-N-(1-méthyléthyl)-1H-pyrazole-1-carboxamide |
| (2R)-N-(4-((3-(6-amino-2-méthyl-4-pyrimidinyl)imidazo[1,2-b]pyridazin-2-yl)-amino)phényl)-2-méthyl-4-morpholine-carboxamide |
| 3-(4-((3-(6-amino-2-méthyl-4-pyrimidinyl)imidazo[1,2-b]pyridazin-2-yl)amino)-phényl)-1-méthyl-1-(1-méthyléthyl)urée |
| (3R)-N-(4-((3-(6-amino-2-méthyl-4-pyrimidinyl)imidazo[1,2-b]pyridazin-2-yl)-amino)phényl)-3-méthyl-4-morpholine-carboxamide |
| (2S)-N-(4-((3-(6-amino-2-méthyl-4-pyrimidinyl)imidazo[1,2-b]pyridazin-2-yl)-amino)phényl)-2-méthyl-1-pyrrolidine-carboxamide |
| (3S)-N-(4-((3-(6-amino-2-méthyl-4-pyrimidinyl)imidazo[1,2-b]pyridazin-2-yl)-amino)phényl)-3-méthyl-1-pyrrolidine-carboxamide |
| N-(3-((3-(6-amino-2-méthyl-4-pyrimidinyl)-6-(3-(méthylsulfonyl)phényl)imidazo-[1,2-b]pyridazin-2-yl)amino)phényl)acétamide |
| N-(6-(2-((4-((cyclopropylcarbamoyl)amino)phényl)amino)imidazo[1,2-b]pyridazin-3-yl)-2-méthyl-4-pyrimidinyl)acétamide |
| 3-(6-amino-2-méthyl-4-pyrimidinyl)-6-(5,6-dihydro-2H-pyran-3-yl)-N-1H-pyrazol-3-ylimidazo[1,2-b]pyridazin-2-amine |
| 2-(4-((3-(6-amino-2-méthyl-4-pyrimidinyl)imidazo[1,2-b]pyridazin-2-yl)amino)-phényl)-N-cyclopropylacétamide |
| 3-((3-(6-amino-2-méthyl-4-pyrimidinyl)imidazo[1,2-b]pyridazin-2-yl)amino)-N-(5-fluoro-2-(trifluorométhyl)phényl)-1H-pyrazole-1-carboxamide |
| 6-fluoro-8-méthoxy-3-(2-méthyl-4-pyrimidinyl)-N-1H-pyrazol-3-ylimidazo[1,2-a]-pyridin-2-amine |
| N-(3-(6-amino-2-méthyl-4-pyrimidinyl)-6-(3-(méthylsulfonyl)phényl)imidazo-[1,2-b]pyridazin-2-yl)-1,4-benzènediamine |
| N-(4-((3-(6-amino-2-méthyl-4-pyrimidinyl)-6-(3-(méthylsulfonyl)-phényl)imidazo-[1,2-b]pyridazin-2-yl)amino)phényl)acétamide |
| 3-(6-amino-2-méthyl-4-pyrimidinyl)-N-(1-méthyl-1H-pyrazol-3-yl)-6-(3-(méthyl-sulfonyl)phényl)imidazo[1,2-b]pyridazin-2-amine |
| 3-(6-amino-2-méthyl-4-pyrimidinyl)-N-(1-(4,5-dihydro-1,3-oxazol-2-yl)-1H-pyrazol-3-yl)imidazo[1,2-b]pyridazin-2-amine |
| 6-fluoro-3-(2-méthyl-4-pyrimidinyl)-N-1H-pyrazol-3-yl-8-(3-pyridinyloxy)imidazo-[1,2-a]pyridin-2-amine |
ou sel pharmaceutiquement acceptable de celui-ci.

13. Composition pharmaceutique comprenant un composé selon l'une quelconque des revendications 1 à 12 ou sel pharmaceutiquement acceptable de celui-ci ; et excipient pharmaceutiquement acceptable.

14. Composé selon l'une quelconque des revendications 1 à 12, ou sel pharmaceutiquement acceptable de celui-ci, pour une utilisation dans un procédé de traitement d'un mélanome, d'un cancer ovarien, d'un cancer du col de l'utérus, d'un cancer du sein, d'un cancer du côlon, d'un cancer rectal, d'un cancer endométrial, d'un cancer pancréatique, d'un cancer du poumon, d'un cancer de l'estomac, d'un glioblastome, d'un cancer du foie, d'un cancer de la prostate, d'une leucémie myélogène aiguë, d'une leucémie myélogène chronique, ou d'un cancer de la thyroïde.

15. Composé selon l'une quelconque des revendications 1 à 12 pour une utilisation en thérapie.

16. Composé selon la revendication 15 dans lequel la thérapie est celle du cancer.
